(19) Europäisches Patentamt / European Patent Office / Office européen des brevets

(11) **EP 3 436 048 B1**

(12) **EUROPEAN PATENT SPECIFICATION**

(45) Date of publication and mention of the grant of the patent:
**18.06.2025 Bulletin 2025/25**

(21) Application number: **17776811.6**

(22) Date of filing: **31.03.2017**

(51) International Patent Classification (IPC):
*A61P 35/00* (2006.01)    *A61K 39/00* (2006.01)
*A61K 38/00* (2006.01)    *C07K 14/47* (2006.01)

(52) Cooperative Patent Classification (CPC):
**C07K 14/47; A61K 39/0011; A61K 39/001102;
A61K 39/001103; A61K 39/001104;
A61K 39/001106; A61K 39/001107;
A61K 39/001108; A61K 39/001134;
A61K 39/001151; A61K 39/001152;
A61K 39/001154; A61K 39/001162;
A61K 39/001163; A61K 39/001164;**    (Cont.)

(86) International application number:
**PCT/US2017/025462**

(87) International publication number:
**WO 2017/173321 (05.10.2017 Gazette 2017/40)**

(54) **NEOANTIGENS AND METHODS OF THEIR USE**

NEOANTIGENE UND VERFAHREN ZU DEREN VERWENDUNG

NÉOANTIGÈNES ET LEURS PROCÉDÉS D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **31.03.2016   US 201662316530 P
31.03.2016   US 201662316533 P
31.03.2016   US 201662316547 P
31.03.2016   US 201662316552 P
01.04.2016   US 201662316567 P
01.04.2016   US 201662316571 P**

(43) Date of publication of application:
**06.02.2019   Bulletin 2019/06**

(73) Proprietor: **BioNTech US Inc.**
**Cambridge, MA 02139 (US)**

(72) Inventor: **ROONEY, Michael Steven**
**Boston, Massachusetts 02124 (US)**

(74) Representative: **Avidity IP**
**Broers Building**
**Hauser Forum**
**21 JJ Thomson Avenue**
**Cambridge CB3 0FA (GB)**

(56) References cited:
WO-A1-2016/164833    WO-A1-2016/172722
WO-A2-2013/151672    WO-A2-2016/187508
CA-A1- 2 343 602      US-A1- 2011 097 743
US-B2- 8 501 167

• DU FENG ET AL: "The Significance and Therapeutic Potential of GATA3 Expression and Mutation in Breast Cancer: A Systematic Review", MEDICINAL RESEARCH REVIEWS,, vol. 35, no. 6, 1 November 2015 (2015-11-01), pages 1300 - 1315, XP002791786, ISSN: 1098-1128
• E. F. FRITSCH ET AL: "HLA-Binding Properties of Tumor Neoepitopes in Humans", CANCER IMMUNOLOGY RESEARCH, vol. 2, no. 6, 3 March 2014 (2014-03-03), US, pages 522 - 529, XP055420002, ISSN: 2326-6066, DOI: 10.1158/2326-6066.CIR-13-0227

EP 3 436 048 B1

(52) Cooperative Patent Classification (CPC): (Cont.)
**A61K 40/11; A61K 40/19; A61K 40/24;**
**A61K 40/4201; A61P 35/00; A61P 35/02;**
**A61P 35/04; A61P 37/04; C07K 4/12;**
**C07K 14/705; C12N 9/00;** A61K 38/00;

A61K 2039/505; A61K 2039/804; A61K 2039/812;
A61K 2039/82; A61K 2039/828; A61K 2039/836;
A61K 2039/844; A61K 2039/852; A61K 2039/86;
A61K 2039/868; A61K 2039/876; A61K 2039/884;
A61K 2039/892

**Description**

This application claims priority to U.S. Provisional Application No. 62/316,530, filed March 31, 2016, U.S. Provisional Application No. 62/316,533, filed March 31, 2016, U.S. Provisional Application No. 62/316,547, filed March 31, 2016, U.S. Provisional Application No. 62/316,552, filed March 31, 2016, U.S. Provisional Application No. 62/316,567, filed April 1, 2016, and U.S. Provisional Application No. 62/316,571, filed April 1, 2016.

**FIELD OF THE INVENTION**

The field of the present invention relates to immunotherapeutic peptides, nucleic acids encoding the peptides, peptide binding agents, and their use in the immunotherapy of cancer. In one aspect, the invention provides neoantigenic peptides, useful alone or in combination with other tumor-associated peptides, anticancer, or immunomodulatory agents to treat cancer.

**BACKGROUND OF THE INVENTION**

Tumor vaccines are typically composed of tumor antigens and immunostimulatory molecules (e.g., adjuvants, cytokines or TLR ligands) that work together to induce antigen-specific cytotoxic T cells (CTLs) that recognize and lyse tumor cells. Such vaccines contain either shared tissue restricted tumor antigens or a mixture of shared and patient-specific antigens in the form of whole tumor cell preparations. The shared tissue restricted tumor antigens are ideally immunogenic proteins with selective expression in tumors across many individuals and are commonly delivered to patients as synthetic peptides or recombinant proteins. In contrast, whole tumor cell preparations are delivered to patients as autologous irradiated cells, cell lysates, cell fusions, heat-shock protein preparations or total mRNA. Since whole tumor cells are isolated from the autologous patient, the cells may include patient-specific tumor antigens as well as shared tumor antigens. Finally, there is a third class of tumor antigens, neoantigens, that has rarely been used in vaccines, which consists of proteins with tumor-specific mutations (which can be patient-specific or shared) that result in altered amino acid sequences. Such mutated proteins are: (a) unique to the tumor cell as the mutation and it's corresponding protein are present only in the tumor; (b) avoid central tolerance and are therefore more likely to be immunogenic; (c) provide an excellent target for immune recognition including by both humoral and cellular immunity. WO 2016/187508, WO 2016/164833 and WO 2016/172722 disclose tumor-specific neoepitopes. Feng Du et al., Medicinal Research Reviews, volume 35, No. 6, pages 1300-1315 (2015) discloses GATA3 mutations in breast cancer patients. Fritsch et al., Cancer Immunology Research, volume 2, No. 6, pages 522-529 (2014) discloses HLA-binding properties of tumor neoepitopes. However, the use of personalized neoantigens requires sequencing of each patient's genome and then production of a patient-specific neoantigen composition. Accordingly, there is still a need for developing additional cancer therapeutics.

**BRIEF SUMMARY OF THE INVENTION**

The present invention is as set out in the claims. Other disclosures herein not falling within the claims are for the assistance of the skilled person in understanding and practicing the disclosed invention and are not part of the invention. For the avoidance of doubt, it is noted that the present invention does not extend to methods of treatment of the human or animal body. Any references in the description to methods of treatment refer to compounds, pharmaceutical compositions, and medicaments of the present disclosure for use in a method of treatment of the human or animal body by therapy.

BRIEF SUMMARY OF THE DISCLOSURE

Provided herein is an isolated neoantigenic peptide comprising a tumor-specific neoepitope, wherein the isolated neoantigenic peptide is not a native polypeptide, wherein the neoepitope comprises at least 8 contiguous amino acids of an amino acid sequence represented by: AxByCz, wherein each A and C represents an amino acid corresponding to the native polypeptide, y is at least 1 and B represents an amino acid substitution or insert of the native polypeptide, x + y + z is at least 8, the at least 8 contiguous amino acids comprises By, and the native polypeptide is encoded by a gene selected from the group consisting of: (a) ABL1, wherein AxByCz is (i) VADGLITTLHYPAPKRNKPTVYGVSPNYDKWEMERT DITMKHKLGGGQYGKVYEGVWKKYSLTV AVKTLKEDTMEVEEFLKEAAVMKEIKHPNLVQLLGVC, (ii) VADGLITTLH YPAPKRNKPTVYGVSPNYDKWEMERTDITMKHKLGGGQYGVVYEGVWKKYSLTV AVKTLKEDTMEVEEFLK-EAAVMKEIKHPNLVQLLGVC, (iii) LLGVCTREPPFYIITEFMTYGNLLDYLRECNRQEVNAVVLLYMATQISSATEYLEKK NFIHRDLAARN CLVGENHLVKVADFGLSRLMTGDTYTAHAGAKF, (iv) SLTVAVKTLKEDTMEVEEFLKEAAVMKEIKH PNLVQLLGVCTREPPFYIIIEFMTYGNLLDYLRECNR QEVNAVVLLYMATQISSAMEYLEKKNFIHRDLA, or (v) STVAD GLITTLHYPAPKRNKPTVYGVSPNYDKWEMERTDITMKHKLGGGQHGEVYEGVWKKYSLT VAVKTLKEDTME-VEEFLKEAAVMKEIKHPNLVQLLG; (b) ALK, wherein AxByCz is (i) SSLAMLDLLHVARDIACGCQYLEENHFIHRDIAA

RNCLLTCPGPGRVAKIADFGMARDIYRASYYRK GGCAMLPVKWMPPEAFMEGIFTSKTDTWSFGVLL, or (ii) QVAVK TLPEVCSEQDELDFLMEALIISKFNHQNIVRCIGVSLQSLPRFILMELMAGGDLKSFLRETRPR PSQPSSLAMLDLLH-VARDIACGCQYLEENHFI; (c) BRAF, wherein AxByCz is MIKLIDIARQTAQGMDYLHAKSIIHRDLKSNNIFLHEDLTVKI GDFGLATEKSRWSGSHQFEQLSGSIL WMAPEVIRMQDKNPYSFQSDVYAFGIVLYELM; (d) BTK, wherein AxByCz is MIKEGSMSEDEFIEEAKVMMNLSHEKLVQLYGVCTKQRPIFIITEYMANGSLLNYLREMRHRFQTQQ LLEMCKDVCEAMEYLESKQFLHRDLAARNCLVND; (e) EEF1B2, wherein AxByCz is MGFGDLKSPAGLQVLNDYLADKSYIEGYV PSQADVAVFEAVSGPPPADLCHALRWYNHIKSYEKEK ASLPGVKKALGKYGPADVEDTTGSGAT; (f) EGFR, wherein AxByCz is (i) SLNITSLGLRSLKEISDGDVIISGNKNLCYANTINWKKLFGTSGQKTKIIRNRGENSCKATGQVCHALC SPEGCWGPEPRDCVSCRNVSRGRECVDKCNLL, or (ii) IPVAIKELREATSPKANKEILDEAYVMASVDNPHVCRLLGI CLTSTVQLIMQLMPFGCLLDYVREHKD NIGSQYLLNWCVQIAKGMNYLEDRRLVHRDLAA; (g) ERBB3, wherein Ax-ByCz is ERCEVVMGNLEIVLTGHNADLSFLQWIREVTGYVLVAMNEFSTLPLPNLRMVRGTQVYDGKFAIFV MLNYNTNSSHALRQLRLTQLTEILSGGVYIEKNDK; (h) ESR1, wherein AxByCz is (i) HLMAKAGLTLQQQHQRLAQLL LILSHIRHMSNKGMEHLYSMKCKNVVPLYGLLLEMLDAHRLHAP TSRGGASVEETDQSHLATAGSTSSHSLQKYYIT-GEA, (ii) NQGKCVEGMVEIFDMLLATSSRFRMMNLQGEEFVCLKSIILLNSGVYTFLPSTLKSLEEKDHIHRVLD KITD-TLIHLMAKAGLTLQQQHQRLAQLLLILSH, (iii) IHLMAKAGLTLQQQHQRLAQLLLILSHIRHMSNKGMEHLYSMKCKNV VPLCDLLLEMLDAHRLHAP TSRGGASVEETDQSHLATAGSTSSHSLQKYYITGE, (iv) IHLMAKAGLTLQQQHQRLAQ LLLILSHIRHMSNKGMEHLYSMKCKNVVPLNDLLLEMLDAHRLHAP TSRGGASVEETDQSHLATAGSTSSHSLQ-KYYITGE, or (v) IHLMAKAGLTLQQQHQRLAQLLLILSHIRHMSNKGMEHLYSMKCKNVVPLSDLLLEMLDAHRLHAP TSRGGASVEETDQSHLATAGSTSSHSLQKYYITGE; (i) FGFR3, wherein AxByCz is HRIGGIKLRHQQWSLVMESVV PSDRGNYTCVVENKFGSIRQTYTLDVLERCPHRPILQAGLPANQTA VLGSDVEFHCKVYSDAQPHIQWLKH-VEVNGSKVG; (j) FRG1B, wherein AxByCz is AVKLSDSRIALKSGYGKYLGINSDELVGHSDAIGPREQWEPVFQNGK MALSASNSCFIRCNEAGDIEA KSKTAGEEEMIKIRSCAEKETKKKDDIPEEDKG; (k) HER2, wherein AxByCz is GSG A F G T V Y K G I W I P D G E N V K I P V A I K V L R E N T S P K A N K E I L D E A Y V M A G L G S P Y V S R L L G I C L T S T V QLVTQLMPYGCLLDHVRENRGRLGSQDLLNWCM; (1) IDH1, wherein AxByCz is (i) RVEEFKLKQMWKSPNGTIRNI LGGTVFREAIICKNIPRLVSGWVKPIIIGHHAYGDQYRATDFVVPGP GKVEITYTPSDGTQKVTYLVHNFEEGGG-VAMGM, (ii) RVEEFKLKQMWKSPNGTIRNILGGTVFREAIICKNIPRLVSGWVKPIIIGCHAYGDQYRATDFVVPGPG KVEITYTPSDGTQKVTYLVHNFEEGGGVAMGM, (iii) RVEEFKLKQMWKSPNGTIRNILGGTVFREAIICKNIPRLVSGW VKPIIIGGHAYGDQYRATDFVVPGP GKVEITYTPSDGTQKVTYLVHNFEEGGGVAMGM, or (iv) RVEEFKLKQMWKSP NGTIRNILGGTVFREAIICKNIPRLVSGWVKPIIIGSHAYGDQYRATDFVVPGPG KVEITYTPSDGTQKVTYLVHN-FEEGGGVAMGM; (m) KIT, wherein AxByCz is (i) VEATAYGLIKSDAAMTVAVKMLKPSAHLTEREALMSELKVLSYLG NHMNIANLLGACTIGGPTLVIT EYCCYGDLLNFLRRKRDSFICSKQEDHAEAALYK, or (ii) VEATAYGLIKSDAAMTVAV KMLKPSAHLTEREALMSELKVLSYLGNHMNIANLLGACTIGGPTLVIT EYCCYGDLLNFLRRKRDSFICSKQEDHAEAA-LYK; (n) MEK, wherein AxByCz is (i) ISELGAGNGGVVFKVSHKPSGLVMARKLIHLEIKPAIRNQIIRELQVLHESNSPYI VGFYGAFYSDGEIS ICMEHMDGGSLDQVLKKAGRIPEQILGKVSI, or (ii) LGAGNGGVVFKVSHKPSGLVMARKLIHL EIKPAIRNQIIRELQVLHECNSLYIVGFYGAFYSDGEISIC MEHMDGGSLDQVLKKAGRIPEQILGKVSIAVI; (o) MYC, wherein AxByCz is (i) MPLNVSFTNRNYDLDYDSVQPYFYCDEEENFYQQQQQSDLQPPAPSEDIWKKFELLPTPPLS PSRRSG LCSPSYVAVTPFSLRGDNDGG, (ii) FTNRNYDLDYDSVQPYFYCDEEENFYQQQQQSELQPPAPSEDIWKK FELLSTPPLSPSRRSGLCSPSY VAVTPFSLRGDNDGGGGSFSTADQLEMVTELLG, or (iii) TNRNYDLDYDSVQPYFY CDEEENFYQQQQQSELQPPAPSEDIWKKFELLPIPPLSPSRRSGLCSPSYVA VTPFSLRGDNDGGGGSFSTADQ-LEMVTELLGG; (p) PDGFRa, wherein AxByCz is VAVKMLKPTARSSEKQALMSELKIMTHLGPHLNIVNLLGACTKSG PIYIIIEYCFYGDLVNYLHKNRD SFLSHHPEKPKKELDIFGLNPADESTRSYVILS; (q) PIK3CA, wherein AxByCz is (i) I EEHANWSVSREAGFSYSHAGLSNRLARDNELRENDKEQLKAISTRDPLSKITEQEKDFLWSHRHYC VTI-PEILPKLLLSVKWNSRDEVAQMYCLVKDWPP, (ii) HANWSVSREAGFSYSHAGLSNRLARDNELRENDKEQLKAISTR DPLSEITKQEKDFLWSHRHYCVTI PEILPKLLLSVKWNSRDEVAQMYCLVKDWPPIKP, or (iii) LFINLFSMMLGSGMPE LQSFDDIAYIRKTLALDKTEQEALEYFMKQMNDARHGGWTTKMDWIFHTI KQHALN; (r) POLE, wherein AxByCz is Q RGGVITDEEETSKKIADQLDNIVDMREYDVPYHIRLSIDIETTKLPLKFRDAETDQIMMISYMIDGQG YLITNREIVSE-DIEDFEFTPKPEYEGPFCVFN; (s) PTEN, wherein AxByCz is KFNCRVAQYPFEDHNPPQLELIKPFCEDLDQWLSED DNHVAAIHCKAGKGQTGVMICAYLLHRGKF LKAQEALDFYGEVRTRDKKGVTIPSQRRYVYYYSY; (t) RAC1, wherein AxByCz is MQAIKCVVVGDGAVGKTCLLISYTTNAFSGEYIPTVFDNYSANVMVDGKPVNLGLWDTAGQEDYD RLRPLSYPQTVGET; and (u) TP53, wherein AxByCz is (i) IRVEGNLRVEYLDDRNTFRHSVVVPYEPPEVGSDCTTIH YNYMCNSSCMGSMNRRPILTIITLEDSSG NLLGRNSFEVRVCACPGRDRRTEEEENLRKKGEP, (ii) TYSPALNKMFCQ LAKTCPVQLWVDSTPPPGTRVRAMAIYKQSQHMTEVVRHCPHHERCSDSDGLAP PQHLIRVEGNLR-VEYLDDRNTFRHSVVVPYEPPEV, (iii) EGNLRVEYLDDRNTFRHSVVVPYEPPEVGSDCTTIHYNYMCNSSCMGGMN QRPILTIITLEDSSGNLL GRNSFEVRVCACPGRDRRTEEEENLRKKGEPHHE, (iv) EGNLRVEYLDDRNTFRHSVVVPY EPPEVGSDCTTIHYNYMCNSSCMGGMNWRPILTIITLEDSSGNLL GRNSFEVRVCACPGRDRRTEEEENLRKK-GEPHHE, or (v) PEVGSDCTTIHYNYMCNSSCMGGMNRRPILTIITLEDSSGNLLGRNSFEVCVCACPGRDRRTEEEENL R KKGEPHHELPPGSTKRALPNNTSSSPQPKKKPL.

In some cases, the native polypeptide is encoded by the EGFR, ERBB3 or FGFR3 gene and at least one By is expressed extracellularly.

Provided herein is an isolated neoantigenic peptide comprising a tumor-specific neoepitope, wherein the isolated neoantigenic peptide is not a native polypeptide, wherein the neoepitope comprises at least 8 contiguous amino acids of an amino acid sequence represented by: AxByCz, wherein each A is an amino acid corresponding to the native polypeptide; By is absent; each C is an amino acid encoded by a frameshift of a sequence encoding the native polypeptide; x + y + z is at least 8; the at least 8 contiguous amino acids comprises at least one Cz; and the native polypeptide is encoded by a gene selected from the group consisting of: (a) APC, wherein Cz is (i) AKFQQCHSTLEPNPADCRVLVYLQNQPGTKLLNFL QERNLPPKVVLRHPKVHLNTMFRRPHSCLAD VLLSVHLIVLRVVVRLPAPFRVNHAVEW, (ii) APVIFQIALDKPCHQAE VKHLHHLLKQLKPSEKYLKIKHLLLKRERVDLSKLQ, or (iii) MLQFRGSRFFQMLILYYILPRKVLQMDFLVHPA; (b) ARID1A, wherein Cz is (i) ALGPHSRISCLPTQTRGCILLAATPRSSSSSSSSNDMIPMAISSPPKAPLLAAPSPASRLQCINS NSRITSGQ WMAHMALLPSGTKGRCTACHTALGRGSLSSSSCPQPSPSLPASNKLPSLPLSKMYTTSMAMPILPLPQ LLLLSADQQAAPRTNFHSSLAETVSLHPLAPMPSKTCHHK, (ii) AHQGFPAAKESRVIQLSLLSLLIPPLTCLASEALPRPL LALPPVLLSLAQDHSRLLQCQATRCHLGHPV ASRTASCILP, (iii) PILAATGTSVRTAARTWVPRAAIRVPDPAAVPDDH AGPGAECHGRPLLYTADSSLWTTRPQRVWST GPDSILQPAKSSPSAAAATLLPATTVPDPSCPTFVSAAATVSTTTAP VLSASILPAAIPASTSAVPGSIPL PAVDDTAAPPEPAPLLTATGSVSLPAAATSAASTLDALPAGCVSSAPVSAVPANCLF PAALPSTAGAIS RFIWVSGILSPLNDLQ, (iv) PCRAGRRVPWAASLIHSRFLLMDNKAPAGMVNRARLHITTSKVLTLSS SSHPTPSNHRPRPLMPNLRI SSSHSLNHHSSSPLSLHTPSSHPSLHISSPRLHTPPSSRRHSSTPRASPPTHSHRLSL LTSSSSNLSSQHPRR SPSRLRILSPSLSSPSKLPIPSSASLHRRSYLKIHLGLRHPQPPQ, (v) RTNPTVRMRPHCVPFW TGRILLPSAASVCPIPFEACHLCQAMTLRCPNTQGCCSSWAS, or (vi) TNQALPKIEVICRGTPRCPSTVPPSPAQPYL RVSLPEDRYTQAWAPTSRTPWGAMVPRGVSMAHKVA TPGSQTIMPCPMPTTPVQAWLEA; (c) β2M, wherein Cz is (i) RMERELKKWSIQTCLSARTGLSISCTTLNSPPLKKMSMPAV, or (ii) LCSRYSLFLAWRLSSVLQRFRFTHVIQQRMESQIS; (d) CDH1, wherein Cz is (i) RSACVTVKGPLASVGRHSLSKQDCKFLPFWGFLEEFLLC, (ii) IQWGTTTAPR PIRPPFLESKQNCSHFPTPLLASEDRRETGLFLPSAAQKMKKAHFLKTWFRSNPTKTK KARFSTASLA-KELTHPLLVSLLLKEKQDG, (iii) PTDPFLGLRLGLHLQKVFHQSHAEYSGAPPPPPAPSGLRFWNPSRIAHISQLLSWP QKTEERLGYSSHQ LPRK, (iv) FCCSCCFFGGERWSKSPYCPQRMTPGTTFITMMKKEAEKRTRTLT, or (v) WRRNC KAPVSLRKSVQTPARSSPARPDRTRRLPSLGVPGQPWALGAAASRRCCCCCRSPLGSARSRS PATLALT-PRATRSRCPGATWREAASWAE; (e) GATA3, wherein Cz is (i) PGRPLQTHVLPEPHLALQPLQPHADHAHADAPAIQP VLWTTPPLQHGHRHGLEPCSMLTGPPARVPA VPFDLHFCRSSIMKPKRDGYMFLKAESKIMFATLQRSSLWCLCSNH; or (ii) PRPRRCTRHPACPLDHTTPPAWSPPWVRALLDAHRAPSESPCSPFRLAFLQEQYHEA; (f) MLL2, wherein Cz is TRRCHCCPHLRSHPCPHHLRNHPRPHHLRHHACHHHLRNCPHPHFLRHCTCPGRWRNRPSLRRLRSL LCLPHLNH HLFLHWRSRPCLHRKSHPHLLHLRRLYPHHLKHRPCPHHLKNLLCPRHLRNCPLPRHLK HLACLHHLRSHPCPLHLK SHPCLHHRRHLVCSHHLKSLLCPLHLRSLPFPHHLRHHACPHHLRTRLCP HHLKNHLCPPHLRYRAYPPCLWCHACL HRLRNLPCPHRLRSLPRPLHLRLHASPHHLRTPPHPHHLR THLLPHHRRTRSCPCRWRSHPCCHYLRSRNSAPGPR GRTCHPGLRSRTCPPGLRSHTYLRRLRSHTCP PSLRSHAYALCLRSHTCPPRLRDHICPLSLRNCTCPPRLRSRTCL LCLRSHACPPNLRNHTCPPSLRSHA CPPGLRNRICPLSLRSHPCPLGLKSPLRSQANALHLRSCPCSLPLGNHPYLP CLESQPCLSLGNHLCPLC PRSCRCPHLGSHPCRLS; (g) PTEN, wherein Cz is (i) SWKGTNWCNDMCIFITSGQIFK GTRGPRFLWGSKDQRQKGSNYSQSEALCVLL, (ii) KRTKCFTFG, (iii) PIFIQTLLLWDFLQKDLKAYTGTILMM, (iv) QKMILTKQIKTKPTDTFLQILR, (v) GFWIQSIKTITRYTIFVLKDIMTPPNLIAELHNILLKTITHHS, (vi) NYSNVQWRNLQSSVCGLPAKGEDIFLQFRTHTTGRQVHVL, or (vii) YQSRVLPQTEQDAKKGQNVSLLGKYILHTRTRGNLRKSRKWKSM; (h) TP53, wherein Cz is (i) SSQNARGCSPRGPCTSSSYTGGPCTSPLLAPVIFCPF PENLPGQLRFPSGLLAFWDSQVCDLHVLPCPQ QDVLPTGQDLPCAAVG, (ii) GAAPTMSAAQIAMVWPLLSILSEWK EICVWSIWMTETLFDIVWWCPMSRLRLALTVPPSTTTTCVTV PAWAA, (iii) TGGPSSPSSHWKTPVVIYWDGTALRCV FVPVLGETGAQRKRISARKGSLTTSCPQGALSEHCPTTPAP LPSQRRNHWMENISPFRSVGVSASRCSES, (iv) FHTPARHPRPRHGHLQAVTAHDGGCEALPPP, (v) CCPRTILNNGSLKTQVQMKLPECQRLLPPWPLHQQLLHRRPLH QPPPGPCHLLSLPRKPTRAATVSV WASCILGQPSL, (vi) VRKHFQTYGNYFLKTTFCPPCRPKQWMI, or (vii) LARTPLPSTRCFANWPRPALCSCGLIPHPRPAPASAPWPSTSSHST; or (i) VHL wherein Cz is (i) ELQETGHRQVALR RSGRPPKCAERPGAADTGAHCTSTDGRLKISVETYTVSSQLLMVLMSLDLDTGL VPSLVSKCLILRVK, (ii) KSDASRLSGA, (iii) RTAYFCQYHTASVYSERAMPPGCPEPSQA, (iv) TRASPPRSSSATAVRASCCPYGSTSTASRSP TQRCRLARAAASTATEVTFGSSEMQGHTMGFWLTKLN YLCHLSMLTDSLFLPISHCQCIL, (v) SSLRITGDWTSSGRSTKIWKTTQMCRKTWSG, or (vi) RRRRGGVGRRGVRPGRVRPGGTGRRGGDGGRAAAARAA LGELARALPGHLLQSQSARRAARMAQ LRRRAAALPNAAAWHGPPHPQLPRSPLALQRCRDTRWASG; (j) ACVR2A, wherein AxByCz is (i) GVEPCYGDKDKRRHCFATWKNISGSIEIVKQGCWLDDINCYDRTDCVEKKRQP, or (ii) GVEP CYGDKDKRRHCFATWKNISGSIEIVKQGCWLDDINCYDRTDCVEKKTALKYIFVAVRAICVM KSFLIFRRWKSHSPL-QIQLHLSHPITTSCSIPWCHLC; (k) C15ORF40, wherein AxByCz is TAEAVNVAIAAPPSEGEANAELCRYLSKVLELR KSDVVLDKVGLALFFFFFETKSCSVAQAGVQWRS LGSLQPPPPGFKLFSCLSFLSSWDYRRMPPCLANFCIFNRDGV SPCWSGWS; (1) CNOT1, wherein AxByCz is (i) LSVIIFFFVYIWHWALPLILNNHHICLMSSIILDCNSVRQSIMSVCFF FFSVIFSTRCLTDSRYPNICWFK, or (ii) LSVIIFFFVYIWHWALPLILNNHHICLMSSIILDCNSVRQSIMSVCFFFFCYILN TMFDR; (m) EIF2B3, wherein AxByCz or Cz is VLVLSCDLITDVALHEVVDLFRAYDASLAMLMRKGQDSIEPVPGQKG

KKKQWSSVTSLEWTAQERG CSSWLMKQTWMKSWSLRDPSYRSILEYVSTRVLWMPTSTV; (n) EPHB2, wherein Ax-ByCz or Cz is SIQVMRAQMNQIQSVEGQPLARRPRATGRTKRCQPRDVTKKTCNSNDGKKREWEKRKQILGGGGK YKEYFLKRILIRKAMTVLAGDKKGLGRFMRCVQSETKAVSLQLPLGR; (o) ESRP1, wherein AxByCz is (i) LDFLGEFA TDIRTHGVHMVLNHQGRPSGDAFIQMKSADRAFMAAQKCHKKKHEGQIC, or (ii) LDFLGEFATDIRTHGVHMVLNHQ GRPSGDAFIQMKSADRAFMAAQKCHKKT; (p) FAM11B, wherein AxByCz is GALCKDGRFRSDIGEFEWKLKEGHKK IYGKQSMVDEVSGKVLEMDISKKKHYNRKISIKKLNRMKV PLMKLITRV; (q) GBP3, wherein AxByCz is RERAQLLEE QEKTLTSKLQEQARVLKERCQGESTQLQNEIQKLQKTLKKKPRDICRIS; (r) JAK1, wherein AxByCz is (i) VNTLKEGK RLPCPPNCPDEVYQLMRKCWEFQPSNRTSFQNLIEGFEALLKTSN or (ii) CRPVTPSCKELADLMTRCMNYDPNQRP FFRAIMRDINKLEEQNPDIVSEKNQQLKWTPHILKSAS; (s) LMAN1, wherein AxByCz is (i) DDHDVLSFLTFQLTEPGK EPPTPDKEISEKEKEKYQEEFEHFQQELDKKKRGIPEGPPRPPRAACGGNI, or (ii) DDHDVLSFLTFQLTEPGKEPPTP DKEISEKEKEKYQEEFEHFQQELDKKKRNSRRATPTSKGSLRRKY LRV; (t) MSH3, wherein AxByCz is (i) TKSTLIG EDVNPLIKLDDAVNVDEIMTDTSTSYLLCISENKENVRDKKKGQHFYWHCGSAACHRRGC V, or (ii) LYTKSTLIGEDV NPLIKLDDAVNVDEIMTDTSTSYLLCISENKENVRDKKRATFLLALWECSLPQARL CLIVSRTLLLVQS; (u) NDUFC2, wherein AxByCz is (i) LPPPKLTDPRLLYIGFLGYCSGLIDNLIRRRPIATAGLHRQLLYITAFFFCWILSCKT, or (ii) SLPPP KLTDPRLLYIGFLGYCSGLIDNLIRRRPIATAGLHRQLLYITAFFLLDIIL; (v) RBM27, wherein AxByCz is NQSGGAGED CQIFSTPGHPKMIYSSSNLKTPSKLCSGSKSHDVQEVLKKKTGSNEVTTRYEEKKTGSV RKANRMPKDVNIQVRKKQ-KHETRRKSKYNEDFERAWREDLTIKR; (w) RPL22, wherein AxByCz is (i) MAPVKKLVVKGGKKKEASSEVHS, or (ii) MAPVKKLVVKGGKKRSKF; (x) SEC31A, wherein AxByCz is (i) MPSHQGAEQQQQQHHVFISQVVTEKEFLSRSDQL QQAVQSQGFINYCQKKN, or (ii) MPSHQGAEQQQQQHHVFISQVVTEKEFLSRSDQLQQAVQSQGFINYCQKKLMLL RLNLRKMCGPF; (y) SEC63, wherein AxByCz is (i) AEVFEKEQSICAAEEQPAEDGQGETNKNRTKGGWQQKSKGP KKTAKSKKKETFKKKTYTCAITTVK ATETKAGKWSRWE, or (ii) MAEVFEKEQSICAAEEQPAEDGQGETNKNRTKGG WQQKSKGPKKTAKSKKRNL; (z) SLC35F5, wherein AxByCz is NIMEIRQLPSSHALEAKLSRMSYPVKEQESILKTVG KLTATQVAKISFFFALCGFWQICHIKKHFQTHK LL; (aa) SMAP1, wherein AxByCz is (i) YEKKKYYDKNAIAITNISSSD APLQPLVSSPSLQAAVDKNKLEKEKEKKKGREKERKGARKAGKTTY S, or (ii) KYEKKKYYDKNAIAITNISSSDAPLQP LVSSPSLQAAVDKNKLEKEKEKKRKRKREKRSQKSRQNHL QLKSCRRKISNWSLKKVPALKKLRSPLWIF; (bb) TFAM, wherein AxByCz is (i) IYQDAYRAEWQVYKEEISRFKEQLTPSQIMSLEKEIMDKHLKRKAMTKKKRVNTAWKTKKTSFS L, or (ii) IYQDAYRAEWQVYKEEISRFKEQLTPSQIMSLEKEIMDKHLKRKAMTKKKS; (cc) TGFBR2, wherein AxByCz is (i) KPQEVCVAVWRKNDENITLETVCHDPKLPYHDFILEDAASPKCIMKEKKKAW, or (ii) EKPQEVCVAVWRKNDENITL ETVCHDPKLPYHDFILEDAASPKCIMKEKKSLVRLSSCVPVALMSAM TTSSSQKNITPAILTCC; (dd) THAP5, wherein AxByCz is VPSKYQFLCSDHFTPDSLDIRWGIRYLKQTAVPTIFSLPEDNQGKDPSKKNPRRKTWKMRKKYAQKP SQKNHLY; (ee) TTK, wherein AxByCz is GTTEEMKYVLGQLVGLNSPNSILKAAKTLYEHYSGGESHNSSSSKTFEKK GEKNDLQLFVMSDTTYK IYWTVILLNPCGNLHLKTTSL; and (ff) XPOT, wherein AxByCz is QQLIRETLISWLQAQML NPQPEKTFIRNKAAQVFALLFVTEYLTKWPKFFLTFSQ.

Provided herein is an isolated neoantigenic peptide comprising a tumor-specific neoepitope, wherein the isolated neoantigenic peptide is not a native polypeptide, wherein the neoepitope comprises at least 8 contiguous amino acids of an amino acid sequence represented by: AxByCz, wherein each A is an amino acid corresponding to a first native polypeptide; each C is an amino acid corresponding to a second native polypeptide, or a cryptic exon or exon of a splice variant of the first native polypeptide, each B is an amino acid that is not an amino acid corresponding to the first native polypeptide, the second native polypeptide, or the cryptic exon of the first native polypeptide, and x + y + z is at least 8, wherein y is absent and the at least 8 contiguous amino acids comprises at least one Ax and at least one Cz, or y is at least 1 and the at least 8 contiguous amino acids comprises at least one By, wherein: (a) the first native polypeptide is encoded by a BCR gene, the second native polypeptide is encoded by an ABL gene, and (i) y is 0, and AxByCz is ERAEWRENIREQQKKCFRSFSL TSVELQML TNSCVKLQTVHSIPL TINKEEALQRPV ASDFEPQGLSEA ARWNSKENLLAGPSENDPNLFVALYDFVASG, or (ii) y is 1, and AxByCz is ELQMLTNSCVKLQTVHSIPLTINKEDDESPGLYGFLNVIVHS ATGFKQSSKALQRPVASDFEPQGLSE AARWNSKENLLAGPSENDPNLFVALYDFVASGD; (b) the first native polypeptide is encoded by a C11orf95 gene, the second native polypeptide is encoded by an RELA gene, y is 1, and AxByCz is ISNSWDAHLGLGACGEAEGLGVQGAEEEEEEEEEEEEGAGVPACPPKGPELFPLIFPAEPAQASGPY VEIIEQPKQRGMRFRYKCEGRSAGSIPGERSTD; (c) the first native polypeptide is encoded by a CBFB gene and the second native polypeptide is encoded by an MYH11 gene, y is 0, and AxByCz is LQRLDGMGCLEFDEERAQQEDAL AQQAFEEARRRTREFEDRDRSHREEMEVHELEKSKRALETQME EMKTQLEELEDELQATEDAKLRLEVNM-QALKGQF; (d) the first native polypeptide is encoded by a CD74 gene and the second native polypeptide is encoded by an ROS1 gene, y is 0, and AxByCz is KGSFPENLRHLKNTMETIDWKVFESWMHHWLLFEMSRHSLEQKPTDAPP KAGVPNKPGIPKLLEGS KNSIQWEKAEDNGCRITYYILEIRKSTSNNLQNQ; (e) the first native polypeptide is encoded by an EGFR gene and the second native polypeptide is encoded by (i) an SEPT14 gene, y is 0, and AxByCz is LPQP PICTIDVYMIMVKCWMIDADSRPKFRELIIEFSKMARDPQRYLVIQLQDKFEHLKMIQQEEIRKL EEEKKQLEGEIID-FYKMKAASEALQTQLSTD; or (ii) an EGFR gene, y is 1, and AxByCz is MRPSGTAGAALLALLAALCPASRALEEKKG NYVVTDHGSCVRACGADSYEMEEDGVRKCKKCEGP CRKVCNGIGIGEFKD; (f) the first native polypeptide is encoded by a EML4 gene, the second native polypeptide is encoded by an ALK gene, y is 1, and AxByCz is SWENSDD

SRNKLSKIPSTPKLIPKVTKTADKHKDVIINQAKMSTREKNSQVYRRKHQELQAMQMEL QSPEYKLSKLRTSTIMT-DYNPNYCFAGKTSSISDL (g) the first native polypeptide is encoded by a FGFR3 gene, the second native polypeptide is encoded by an TACC3 gene, y is 0, and AxByCz is EGHRMDKPANCTHDLYMIMRECWHAAPSQRPTFKQLVEDLD RVLTVTSTDVKATQEENRELRSRCE ELHGKNLELGKIMDRFEEVVYQAMEEVQKQKELS, (h) the first native polypeptide is encoded by a NAB gene, the second native polypeptide is encoded by an STAT6 gene, y is at least 1, and AxByCz is RDNTLLLRRVELFSLSRQVARESTYLSSLKGSRLHPEELGGPPLKKLKQEATSKSQIMSLWGLVSKMP PEKVQRLYVDFPQHLRHLLGDWLESQPWEFLVGSDAFCC; (i) the second native polypeptide is encoded by an ERG, y is 0, and (i) the first native polypeptide is encoded by a NDRG1 gene, and AxByCz is MSREMQDVDLAEVKPLVEKGETIT GLLQEFDVQEALSVVSEDQSLFECAYGTPHLAKTEMTASSSSD YGQTSKMSPRVPQQDW or (ii) the first native polypeptide is encoded by a TMPRSS2 gene, and AxByCz is MALNSEALSVVSEDQSLFECAYGTPHLAKTEMTASSSSD YGQTSKMSPRVPQQDW; (j) the first native polypeptide is encoded by a PML gene, the second native polypeptide is encoded by an RARA gene, y is 1, and AxByCz is (i) VLDMHGFLRQALCRLRQEEPQSLQAAVRTDGFDEFKVRLQD LSSCITQGKAIETQSSSSEEIVPSPPSP PPLPRIYKPCFVCQDKSSGYHYGVSACEGCKG, or (ii) RSSPEQPRPSTSK AVSPPHLDGPPSPRSPVIGSEVFLPNSNHVASGAGEAAIETQSSSSEEIVPSPPSPPPL PRIYKPCFVCQDKSSGY-HYGVSACEGCKG; (k) the first native polypeptide is encoded by a RUNX1 gene, the second native polypeptide is encoded by an CBFA2T1 (RUNX1T1) gene, y is 1, and AxByCz is VARFNDLRFVGRSGRGKSFTLTITVFTNPPQVAT YHRAIKITVDGPREPRNRTEKHSTMPDSPVDVKT QSRLTPPTMPPPPTTQGAPRTSSFTPTTLTNGT; (1) the first native polypeptide is encoded by a AR-v7 gene, the cryptic exon or the exon of a splice variant is encoded by the AR-v7 gene, y is 0, and AxByCz is SCKVFFKRAAEGKQKYLCASRNDCTIDKFRRKNCPSCRLRKCYEAGMTLGEKFRVGNCKHLK MTRP

Provided herein is an isolated neoantigenic peptide comprising a tumor-specific neoepitope, wherein the isolated neoantigenic peptide is not a native polypeptide, wherein the neoepitope comprises at least 8 contiguous amino acids of an amino acid sequence represented by: AxByCz wherein each A is an amino acid corresponding to a first native polypeptide; each B is an amino acid that is not an amino acid corresponding to the first native polypeptide or the second native polypeptide, each C is an amino acid encoded by a frameshift of a sequence encoding a second native polypeptide; x + y + z is at least 8, wherein y is absent and the at least 8 contiguous amino acids comprises at least one Cz, or y is at least 1 and the at least 8 contiguous amino acids comprises at least one By and/or at least one Cz; and (a) the first native polypeptide is encoded by an AC011997.1 gene, the second native polypeptide is encoded by a LRRC69 gene, y is 1, and AxByCz is MAGAPPPASLPPCSLISDCCASNQRDSVGVGPSEPGNNIKICNESASRK (b) the first native polypeptide is encoded by an EEF1DP3 gene, the second native polypeptide is encoded by a FRY gene, y is 1, and AxByCz is HGWRPFLPVR ARSRWNRRLDVTVANGRSWKYGWSLLRVPQVNGIQVLNVSLKSSSNVISY, (c) the first native polypeptide is encoded by a MAD1L1 gene, the second native polypeptide is encoded by a MAFK gene, y is 0, and AxByCz is RLKEVFQTKI QEFRKACYTLTGYQIDITTENQYRLTSLYAEHPGDCLIFKLRVPGSSVLVTVPGL, or (d) the first native polypeptide is encoded by a PPP1R1B gene, the second native polypeptide is encoded by a STARD3 gene, y is 1, and AxByCz is AEVLKVIRQSAGQKTTCGQGLEGPWERPPPLDESERDGGSEDQVEDPALSALLLRPRPPRPEVGAHQ DEQAAQGADPRLGAQPACRGLPGLLTVPQPEPLLAPPSAA.

In some cases, the isolated neoantigenic peptide comprises a sequence according to Table 1. In cases, x + y + z is at most 500, at most 250, at most 150, at most 125, or at most 100 In cases, x + y + z is at least 8, at least 50, at least 100, at least 200, or at least 300. In cases, z is at most 500, at most 250, at most 150, at most 125, or at most 100. In cases, z is at least 8, at least 50, at least 100, at least 200, or at least 300. In cases, the isolated neoantigenic peptide is from about 8 to about 500 amino acids in length. In cases, the isolated neoantigenic peptide is from about 8 to about 100 amino acids in length. In cases, the isolated neoantigenic peptide is from about 8 to about 50 amino acids in length. In cases, the isolated neoantigenic peptide is from about 15 to about 35 amino acids in length. In cases, the isolated neoantigenic peptide is from about 8 and about 15 amino acids in length. In cases, the isolated neoantigenic peptide is from about 8 and about 11 amino acids in length. In cases, the isolated neoantigenic peptide is 9 or 10 amino acids in length. In cases, the isolated neoantigenic peptide binds major histocompatibility complex (MHC) class I. In cases, the isolated neoantigenic peptide binds MHC class I with a binding affinity of about 500 nM or less. In cases, the isolated neoantigenic peptide binds MHC class I with a binding affinity of about 250 nM or less. In cases, the isolated neoantigenic peptide binds MHC class I with a binding affinity of about 50 nM or less. In cases, the isolated neoantigenic peptide is from about 8 and about 30 amino acids in length. In cases, the isolated neoantigenic peptide is from about 8 to about 25 amino acids in length. In cases, the isolated neoantigenic peptide is from about 15 to about 24 amino acids in length. In cases, the isolated neoantigenic peptide is from about 9 to about 15 amino acids in length. In cases, the isolated neoantigenic peptide binds MHC class II. In cases, the isolated neoantigenic peptide binds MHC class II with a binding affinity of 1000 nM or less. In cases, the isolated neoantigenic peptide binds MHC class I with a binding affinity of about 500 nM or less. In cases, the isolated neoantigenic peptide further comprises flanking amino acids. In cases, the flanking amino acids are not native flanking amino acids In cases, the isolated neoantigenic peptide has a total length of at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 40, at least 50, at least 60, at least 70, at least 80, at least 90, at least 100, at least 150, at least 200, at least 250, at least 300, at least 350, at least 400, at least 450, or at least 500 amino acids. In cases, the isolated neoantigenic peptide has a total length of at most 8, at most 9, at most 10, at most 11, at most 12, at most 13, at most 14, at most 15, at most 16, at most 17, at most 18, at most 19, at most 20, at most 21, at most 22, at most 23, at most 24, at most 25, at most 26, at most 27, at most 28, at most 29, at most 30, at most 40, at most 50, at most 60, at most 70, at most 80, at most 90, at most 100, at most 150, at most 200, at most 250, at most 300, at most 350, at most 400, at most 450, or at most 500 amino acids. In cases, the isolated neoantigenic peptide is a first neoantigenic peptide linked to at least a second neoantigenic peptide. In cases, the isolated neoantigenic peptide is linked to the at least second neoantigenic peptide by a poly-glycine or poly-serine linker. In cases, the second neoantigenic peptide binds MHC class I or class II with a binding affinity of less than about 1000 nM. In cases, the second neoantigenic peptide binds MHC class I or class II with a binding affinity of less than about 500 nM. In cases, isolated neoantigenic peptide and the second neoantigenic peptide bind to human leukocyte antigen (HLA) -A, -B, -C, -DP, -DQ, or -DR. In cases, the isolated neoantigenic peptide binds a class I HLA and the second neoantigenic peptide binds a class II HLA. In cases, the isolated neoantigenic peptide binds a class II HLA and the second neoantigenic peptide binds a class I HLA. In cases, the isolated neoantigenic peptide further comprises a modification which increases in vivo half-life, cellular targeting, antigen uptake, antigen processing, MHC affinity, MHC stability, antigen presentation, or a combination thereof. In cases, the modification is conjugation to a carrier protein, conjugation to a ligand, conjugation to an antibody, PEGylation, polysialylation HESylation, recombinant PEG mimetics, Fc fusion, albumin fusion, nanoparticle attachment, nanoparticulate encapsulation, cholesterol fusion, iron fusion, acylation, amidation, glycosylation, side chain oxidation, phosphorylation, biotinylation, the addition of a surface active material, the addition of amino acid mimetics, or the addition of unnatural amino acids. In cases, the isolated neoantigenic peptide further comprises a modification which increases cellular targeting to antigen presenting cells. In cases, the antigen presenting cells are dendritic cells. In cases, the dendritic cells are targeted using DEC205, XCR1, CD197, CD80, CD86, CD123, CD209, CD273, CD283, CD289, CD184, CD85h, CD85j, CD85k, CD85d, CD85g, CD85a, CD141, CD11c, CD83, TSLP receptor, Clec9a, or CD1a marker. In cases, the dendritic cells are targeted using the CD141, DEC205, Clec9a, or XCR1 marker. In cases, the dendritic cells are autologous cells. In cases, one or more of the dendritic cells are bound to a T cell. In cases, the T cell is an autologous T cell. In cases, the isolated neoantigenic peptide is not a isolated neoantigenic peptide listed in Table 2. In cases, the isolated neoantigenic peptide is linked to at least one additional neoantigenic peptide listed in Table 1 or 2.

Provided herein is an in vivo delivery system comprising an isolated neoantigenic peptide described herein. In cases, the delivery system includes cell-penetrating peptides, nanoparticulate encapsulation, virus like particles, liposomes, or any combination thereof. In cases, the cell-penetrating peptide is TAT peptide, herpes simplex virus VP22, transportan, Antp, or any combination thereof.

Provided herein is a cell comprising an isolated neoantigenic peptide described herein. In cases, the cell is an antigen presenting cell. In cases, the cell is a dendritic cell. In cases, the cell is an autologous cell. In cases, the cell is bound to a T cell. In cases, the T cell is an autologous T cell.

Provided herein is a composition comprising an isolated neoantigenic peptide described herein.

In cases, the composition comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 40, at least 50, at least 60, at least 70, at least 80, at least 90, or at least 100 of the isolated neoantigenic peptides comprising a tumor-specific neoepitope according to Table 1 or 2. In cases, the composition comprises from about 2 to about 20 neoantigenic peptides, or from about 2 to about 30 neoantigenic peptides. In cases, the neoantigen is specific for an individual subject's tumor. In cases, the composition further comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, or at least 25, at least 26, at least 27, at least 28, at least 29, at least 30, at least 40, at least 50, at least 60, at least 70, at least 80, at least 90, or at least 100 additional neoantigenic peptides. In cases, the composition comprises from about 4 to about 20 additional neoantigenic peptides, from about 4 to about 30 additional neoantigenic peptides. In cases, at least on of the additional neoantigenic peptides is specific for an individual subject's tumor. In cases, the subject specific neoantigenic peptide is selected by identifying sequence differences between the genome, exome, and/or transcriptome of the subject's tumor sample and the genome, exome, and/or transcriptome of a non-tumor sample. In cases, the samples are fresh or formalin-fixed paraffin embedded tumor tissues, freshly isolated cells, or circulating tumor cells. In cases, the sequence differences are determined by Next Generation Sequencing.

Provided herein is an isolated polynucleotide encoding an isolated neoantigenic peptide described herein. In cases, the polynucleotide is DNA. In cases, the polynucleotide is RNA. In cases, the RNA is a self-amplifying RNA. In cases, the RNA is modified to increase stability, increase cellular targeting, increase translation efficiency, adjuvanticity, cytosol accessibility, and/or decrease cytotoxicity. In cases, the modification is conjugation to a carrier protein, conjugation to a ligand, conjugation to an antibody, codon optimization, increased GC-content, incorporation of modified nucleosides, incorporation of 5'-cap or cap analog, and/or incorporation of an unmasked poly-A sequence.

Provided herein is a cell comprising the polynucleotide described herein.

Provided herein is a vector comprising the polynucleotide described herein. In cases, the polynucleotide is operably linked to a promoter. In cases, the vector is a self-amplifying RNA replicon, plasmid, phage, transposon, cosmid, virus, or virion. In cases, the vector is derived from an adeno-associated virus, herpesvirus, lentivirus, or a pseudotype thereof.

Provided herein is an in vivo delivery system comprising the isolated polynucleotide described herein. In cases, the delivery system includes spherical nucleic acids, viruses, virus-like particles, plasmids, bacterial plasmids, or nanoparticles.

Provided herein is a cell comprising a vector or delivery system described herein. In cases, the cell is an antigen presenting cell. In cases, the cell is a dendritic cell. In cases, the cell is an immature dendritic cell.

Provided herein is a composition comprising at least one polynucleotide described herein. In cases, the composition comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 40, at least 50, at least 60, at least 70, at least 80, at least 90, or at least 100 of the isolated polynucleotides. In cases, the composition comprises from about 2 and about 20 of the isolated polynucleotides, or from about 2 to about 30 of the isolated polynucleotides. In cases, the neoantigenic peptides are encoded by a vector comprising one or more of the the isolated polynucleotides. In cases, the composition further comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 40, at least 50, at least 60, at least 70, at least 80, at least 90, or at least 100 additional neoantigenic polynucleotides encoding for additional neoantigenic peptides. In cases, one or more of the additional neoantigenic peptides are encoded by a vector comprising one or more of the additional neoantigenic polynucleotides. In cases, the composition comprises from about 4 to about 20 additional neoantigenic polynucleotides, or from about 4 to about 30 additional neoantigenic polynucleotides. In cases, the isolated polynucleotides and the additional neoantigenic polynucleotides are linked. In cases, the polynucleotides are linked using nucleic acids that encode a poly-glycine or poly-serine linker. In cases, at least one of the additional neoantigenic peptide is specific for an individual subject's tumor. In cases, the subject specific neoantigenic peptide is selected by identifying sequence differences between the genome, exome, and/or transcriptome of the subject's tumor sample and the genome, exome, and/or transcriptome of a non-tumor sample. In cases, the samples are fresh or formalin-fixed paraffin embedded tumor tissues, freshly isolated cells, or circulating tumor cells. In cases, the sequence differences are determined by Next Generation Sequencing.

Provided herein is a T cell receptor (TCR) capable of binding at least one neoantigenic peptide described herein or an MHC-peptide complex comprising at least one neoantigenic peptide described herein. The In cases, the MHC of the MHC-peptide is MHC class I or class II. In cases, TCR is a bispecific TCR further comprising a domain comprising an antibody or antibody fragment capable of binding an antigen. In cases, the antigen is a T cell-specific antigen. In cases, the antigen is CD3. In cases, the antibody or antibody fragment is an anti-CD3 scFv.

Provided herein is a chimeric antigen receptor comprising: (i) a T cell activation molecule; (ii) a transmembrane region; and (iii) an antigen recognition moiety capable of binding at least one neoantigenic peptide described herein or an MHC-peptide complex comprising at least one neoantigenic peptide described herein. In cases, CD3-zeta is the T cell activation molecule. In cases, the chimeric antigen receptor further comprises at least one costimulatory signaling domain. The In cases, the signaling domain is CD28, 4-1BB, ICOS, OX40, ITAM, or Fc epsilon RI-gamma. In cases, the antigen recognition moiety is capable of binding the isolated neoantigenic peptide in the context of MHC class I or class II. In cases, the CD3-zeta, CD28, CTLA-4, ICOS, BTLA, KIR, LAG3, CD137, OX40, CD27, CD40L, Tim-3, A2aR, or PD-1 transmembrane region. In cases, the neoantigenic peptide is located in the extracellular domain of a tumor associated polypeptide. In cases, the MHC of the MHC-peptide is MHC class I or class II.

Provided herein is a T cell comprising the T cell receptor or chimeric antigen receptor described herein, optionally wherein the T cell is a helper or cytotoxic T cell. In cases, the T cell is a T cell of a subject.

Provided herein is a T cell comprising a T cell receptor (TCR) capable of binding at least one neoantigenic peptide described herein or an MHC-peptide complex comprising at least one neoantigenic peptide described herein, wherein the T cell is a T cell isolated from a population of T cells from a subject that has been incubated with antigen presenting cells and one or more of the at least one neoantigenic peptide described herein for a sufficient time to activate the T cells. In cases, the T cell is a CD8+ T cell, a helper T cell or cytotoxic T cell. In cases, the population of T cells from a subject is a population of CD8+ T cells from the subject. In cases, the one or more of the at least one neoantigenic peptide described herein is a subject-specific neoantigenic peptide. In cases, the subject-specific neoantigenic peptide has a different tumor neo-epitope that is an epitope specific to a tumor of the subject. In cases, the subject-specific neoantigenic peptide is an expression product of a tumor-specific non-silent mutation that is not present in a non-tumor sample of the subject. In cases, the subject-specific neoantigenic peptide binds to a HLA protein of the subject. In cases, the subject-specific neoantigenic peptide binds to a HLA protein of the subject with an IC50 less than 500 nM. In cases, the activated CD8+ T cells are separated from the antigen presenting cells. In cases, the antigen presenting cells are dendritic cells or CD40L-expanded B cells. In cases, the antigen presenting cells are non-transformed cells. In cases, the antigen presenting cells are non-infected cells. In cases, the antigen presenting cells are autologous. In cases, the antigen presenting cells have been treated to strip endogenous MHC-associated peptides from their surface. In cases, the treatment to strip the endogenous MHC-associated peptides comprises culturing the cells at about 26°C. In cases, the treatment to strip the endogenous MHC-associated peptides comprises treating the cells with a mild acid solution. In cases, the antigen presenting cells have been pulsed with at least one neoantigenic peptide described herein. In cases, pulsing comprises incubating the antigen presenting cells in the presence of at least about 2 μg/ml of each of the at least one neoantigenic peptide described herein. In cases, ratio of isolated T cells to antigen presenting cells is between about 30:1 and 300:1. In cases, the incubating the isolated population of T cells is in the presence of IL-2 and IL-7. In cases, the MHC of the MHC-peptide is MHC class I or class II.

Provided herein is a method for activating tumor specific T cells comprising: isolating a population of T cells from a subject; and incubating the isolated population of T cells with antigen presenting cells and at least one neoantigenic peptide described herein for a sufficient time to activate the T cells. In cases, the T cell is a CD8+ T cell, a helper T cell or cytotoxic T cell. In cases, the population of T cells from a subject is a population of CD8+ T cells from the subject. In cases, the one or more of the at least one neoantigenic peptide described herein is a subject-specific neoantigenic peptide. In cases, the subject-specific neoantigenic peptide has a different tumor neo-epitope that is an epitope specific to a tumor of the subject. In cases, the subject-specific neoantigenic peptide is an expression product of a tumor-specific non-silent mutation that is not present in a non-tumor sample of the subject. In cases, the subject-specific neoantigenic peptide binds to a HLA protein of the subject. In cases, the subject-specific neoantigenic peptide binds to a HLA protein of the subject with an IC50 less than 500 nM. In cases, the method further comprises separating the activated T cells from the antigen presenting cells. In cases, the method further comprises testing the activated T cells for evidence of reactivity against at least one of neoantigenic peptide of described herein. In cases, the antigen presenting cells are dendritic cells or CD40L-expanded B cells. In cases, the antigen presenting cells are non-transformed cells. In cases, the antigen presenting cells are non-infected cells. In cases, the antigen presenting cells are autologous. In cases, the antigen presenting cells have been treated to strip endogenous MHC-associated peptides from their surface. In cases, the treatment to strip the endogenous MHC-associated peptides comprises culturing the cells at about 26°C. In cases, the treatment to strip the endogenous MHC-associated peptides comprises treating the cells with a mild acid solution. In cases, the antigen presenting cells have been pulsed with at least one neoantigenic peptide described herein. In cases, pulsing comprises incubating the antigen presenting cells in the presence of at least about 2 μg/ml of each of at least one neoantigenic peptide described herein. In cases, ratio of isolated T cells to antigen presenting cells is between about 30:1 and 300:1. In cases, the incubating the isolated population of T cells is in the presence of IL-2 and IL-7. In cases, the MHC of the MHC-peptide is MHC class I or class II.

Provided herein is a composition comprising activated tumor specific T cells produced by a method described herein.

Provided herein is a method of treating cancer in a subject comprising administering to the subject a therapeutically effective amount of activated tumor specific T cell described herein, or produced by a method described herein. In cases, the administering comprises administering from about $10^6$ to $10^{12}$, from about $10^8$ to $10^{11}$, or from about $10^9$ to $10^{10}$ of the activated tumor specific T cells.

Provided herein is a nucleic acid comprising a promoter operably linked to a polynucleotide encoding the T cell receptor described herein. In cases, the TCR is capable of binding the at least one neoantigenic peptide in the context of major histocompatibility complex (MHC) class I or class II.

Provided herein is a nucleic acid comprising a promoter operably linked to a polynucleotide encoding the chimeric antigen receptor described herein. In cases, the antigen recognition moiety is capable of binding the at least one neoantigenic peptide in the context of major histocompatibility complex (MHC) class I or class II. In cases, the neoantigenic peptide is located in the extracellular domain of a tumor associated polypeptide. In cases, the nucleic acid comprises the CD3-zeta, CD28, CTLA-4, ICOS, BTLA, KIR, LAG3, CD137, OX40, CD27, CD40L, Tim-3, A2aR, or PD-1 transmembrane region.

Provided herein is an antibody or antibody fragment capable of binding at least one neoantigenic peptide described herein or an MHC-peptide complex comprising at least one neoantigenic peptide described herein, optionally wherein the antibody fragment is a bi-specific T cell engager (BiTE). In cases, the antibody or antibody fragment binds to an extracellular portion of the at least one neoantigenic peptide. In cases, the native polypeptide is encoded by a gene selected from the group consisting of: β2M, wherein Cz is RMERELKKWSIQTCLSARTGLSISCTTLNSPPLKKMSMPAV, or LCSRYSLFLAWRLSSVLQRFRFTHVIQQRMESQIS; EGFR, wherein AxByCz is IPVAIKELREATSPKANKEILDEAY VMASVDNPHVCRLLGICLTSTVQLIMQLMPFGCLLDYVREHKD NIGSQYLLNWCVQIAKGMNYLEDRRLVHRDLAA; BTK, wherein AxByCz is MIKEGSMSEDEFIEEAKVMMNLSHEKLVQLYGVCTKQRPIFIITEYMANGSLLNYLREMRHR FQTQQ LLEMCKDVCEAMEYLESKQFLHRDLAARNCLVND; or ESR1, wherein AxByCz is HLMAKAGLTLQQQHQRL

AQLLLILSHIRHMSNKGMEHLYSMKCKNVVPLYGLLLEMLDAHRLHAP TSRGGASVEETDQSHLATAGSTSSHSLQ-KYYITGEA, NQGKCVEGMVEIFDMLLATSSRFRMMNLQGEEFVCLKSIILLNSGVYTFLPSTLKSLEEKDHIHRVLD KITDTLIHLMAKAGLTLQQQHQRLAQLLLILSH, IHLMAKAGLTLQQQHQRLAQLLLILSHIRHMSNKGMEHLYSMKCKN VVPLCDLLLEMLDAHRLHAP TSRGGASVEETDQSHLATAGSTSSHSLQKYYITGE, IHLMAKAGLTLQQQHQRLAQLL LILSHIRHMSNKGMEHLYSMKCKNVVPLNDLLLEMLDAHRLHAP TSRGGASVEETDQSHLATAGSTSSHSLQ-KYYITGE, or IHLMAKAGLTLQQQHQRLAQLLLILSHIRHMSNKGMEHLYSMKCKNVVPLSDLLLEMLDAHRLHAP TSRGGASVEETDQSHLATAGSTSSHSLQKYYITGE; or HER2, wherein AxByCz is GSGAFGTVYKGIWIPDGENVKIP VAIKVLRENTSPKANKEILDEAYVMAGLGSPYVSRLLGICLTSTV QLVTQLMPYGCLLDHV-RENRGRLGSQDLLNWCM. In cases, the antibody or antibody fragment is a bispecific antibody or antibody fragment. In cases, one antigen binding domain of the bispecific antibody or antibody fragment is an anti-CD3 binding domain.

Provided herein is a modified cell transfected or transduced with a nucleic acid described herein. In cases, the modified cell is a T cell, tumor infiltrating lymphocyte, NK-T cell, TCR-expressing cell, CD4+ T cell, CD8+ T cell, or NK cell.

Provided herein is a composition comprising the T cell receptor or chimeric antigen receptor described herein.

Provided herein is a composition comprising autologous subject T cells containing the T cell receptor or chimeric antigen receptor described herein. In cases, the composition further comprises an immune checkpoint inhibitor. In cases, the composition further comprises at least two immune checkpoint inhibitors. In cases, each of the immune checkpoint inhibitors inhibits a checkpoint protein selected from the group consisting of CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, and B-7 family ligands or a combination thereof. In cases, each of the immune checkpoint inhibitors interacts with a ligand of a checkpoint protein selected from the group consisting of CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, and B-7 family ligands or a combination thereof. In cases, the T cells are PD-1 and/or CTLA4 knockout T cells, optionally, wherein the PD-1 and/or CTLA4 knockout T cells are created using a CRISPR system. In cases, the composition further comprises an immune modulator or adjuvant. In cases, the immune modulator is a co-stimulatory ligand, a TNF ligand, an Ig superfamily ligand, CD28, CD80, CD86, ICOS, CD40L, OX40, CD27, GITR, CD30, DR3, CD69, or 4-1BB. In cases, the immune modulator is at least one cancer cell or cancer cell extract. In cases, the cancer cell is autologous to the subject in need of the composition. In cases, the cancer cell has undergone lysis or been exposed to UV radiation. In cases, the composition further comprises an adjuvant. In cases, the adjuvant is selected from the group consisting of: Poly(I:C), Poly-ICLC, STING agonist, 1018 ISS, aluminium salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312 VG, Montanide ISA 206 VG, Montanide ISA 50 V2, Montanide ISA 51 VG, OK-432, OM-174, OM-197-MP-EC, ISA-TLR2 agonist, ONTAK, PepTel®. vector system, PLG microparticles, resiquimod, SRL172, virosomes and other virus-like particles, YF-17D, VEGF trap, R848, beta-glucan, Pam3Cys, Pam3CSK4, acrylic or methacrylic polymers, copolymers of maleic anhydride, and QS21 stimulon. In cases, the composition induces a humoral response when administered to a subject. In cases, the composition induces a T helper cell type 1 when administered to a subject.

Provided herein is a method of inhibiting growth of a tumor cell expressing a tumor-specific neoepitope, comprising contacting the tumor cell with the peptide, polynucleotide, delivery system, vector, composition, antibody, or cells described herein.

Provided herein is a method of prophylaxis of a subject, comprising contacting a cell of the subject with the peptide, polynucleotide, delivery system, vector, composition, antibody, or cells described herein. In cases, the native polypeptide is encoded by a gene selected from the group consisting of: β2M, wherein Cz is RMERELKKWSIQTCLSARTGLSISCTTLNSPPLKKMSMPAV, or LCSRYSLFLAWRLSSVLQRFRFTHVIQQRMESQIS; EGFR, wherein Ax-ByCz is IPVAIKELREATSPKANKEILDEAYVMASVDNPHVCRLLGICLTSTVQLIMQLMPFGCLLDYVREHKD NIGS-QYLLNWCVQIAKGMNYLEDRRLVHRDLAA; BTK, wherein AxByCz is MIKEGSMSEDEFIEEAKVMMNLSHEKLVQLY GVCTKQRPIFIITEYMANGSLLNYLREMRHRFQTQQ LLEMCKDVCEAMEYLESKQFLHRDLAARNCLVND; or ESR1, wherein AxByCz is HLMAKAGLTLQQQHQRLAQLLLILSHIRHMSNKGMEHLYSMKCKNVVPLYGLLLEMLDAHRLHAP TSRGGASVEETDQSHLATAGSTSSHSLQKYYITGEA, NQGKCVEGMVEIFDMLLATSSRFRMMNLQGEEFVCLKSIILL NSGVYTFLPSTLKSLEEKDHIHRVLD KITDTLIHLMAKAGLTLQQQHQRLAQLLLILSH, IHLMAKAGLTLQQQHQRLAQ LLLILSHIRHMSNKGMEHLYSMKCKNVVPLCDLLLEMLDAHRLHAP TSRGGASVEETDQSHLATAGSTSSHSLQ-KYYITGE, IHLMAKAGLTLQQQHQRLAQLLLILSHIRHMSNKGMEHLYSMKCKNVVPLNDLLLEMLDAHRLHAP TSRGGASVEETDQSHLATAGSTSSHSLQKYYITGE, or IHLMAKAGLTLQQQHQRLAQLLLILSHIRHMSNKGMEHLYS MKCKNVVPLSDLLLEMLDAHRLHAP TSRGGASVEETDQSHLATAGSTSSHSLQKYYITGE; and HER2, wherein Ax-ByCz is GSGAFGTVYKGIWIPDGENVKIPVAIKVLRENTSPKANKEILDEAYVMAGLGSPYVSRLLGICLTSTV QLVTQLMPYGCLLDHVRENRGRLGSQDLLNWCM.

Provided herein is a method of treating cancer or initiating, enhancing, or prolonging an anti-tumor response in a subject in need thereof comprising administering to the subject the peptide, polynucleotide, vector, composition, antibody, or cells described herein. In cases, the subject is a human. In cases, the subject has cancer. In cases, the cancer is selected from the group consisting of urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, haematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, oesophageal cancers, hepatocellular cancers, cholangiocellular cancers, head and neck squamous cell cancer soft tissue sarcoma, and small cell lung cancer. In cases, the peptide, polynucleotide, vector, composition, antibody, or cells described herein is for use in treating a corresponding cancer according to Table 1 or Table 2. In cases, the peptide, polynucleotide, vector, composition, antibody, or cells described herein is for use in treating a subject with an HLA type that is a corresponding HLA type according to Table 1 or Table 2. In cases, the subject has undergone surgical removal of the tumor. In cases, the peptide, polynucleotide, vector, composition, or cells is administered via intravenous, intraperitoneal, intratumoral, intradermal, or subcutaneous administration. In cases, the peptide, polynucleotide, vector, composition, or cells is administered into an anatomic site that drains into a lymph node basin. In cases, administration is into multiple lymph node basins. In cases, administration is by a subcutaneous or intradermal route. In cases, peptide is administered. In cases, administration is intratumorally. In cases, polynucleotide, optionally RNA, is administered. In cases, the polynucleotide is administered intravenously. In cases, the cell is a T cell or dendritic cell. In cases, the peptide or polynucleotide comprises an antigen presenting cell targeting moiety. In cases, the cell is an autologous cell. In cases, the method further comprises administering at least one immune checkpoint inhibitor to the subject. In cases, the checkpoint inhibitor is a biologic therapeutic or a small molecule. In cases, the checkpoint inhibitor is selected from the group consisting of a monoclonal antibody, a humanized antibody, a fully human antibody and a fusion protein or a combination thereof In cases, the checkpoint inhibitors is a PD-1 antibody or a PD-L1 antibody. In cases, the checkpoint inhibitor is selected from the group consisting of ipilimumab, tremelimumab, nivolumab, avelumab, durvalumab, atezolizumab, pembrolizumab, and any combination thereof. In cases, the checkpoint inhibitor inhibits a checkpoint protein selected from the group consisting of CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, and B-7 family ligands, and any combination thereof. In cases, the checkpoint inhibitor interacts with a ligand of a checkpoint protein selected from the group consisting of CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, and B-7 family ligands or a combination thereof. In cases, two or more checkpoint inhibitors are administered. In cases, at least one of the two or more checkpoint inhibitors is a PD-1 antibody or a PD-L1 antibody. In cases, at least one of the two or more checkpoint inhibitors is selected from the group consisting of ipilimumab, tremelimumab, nivolumab, avelumab, durvalumab, atezolizumab, and pembrolizumab. In cases, the checkpoint inhibitor and the composition are administered simultaneously or sequentially in any order. In cases, the peptide, polynucleotide, vector, composition, or cells is administered prior to the checkpoint inhibitor. In cases, the peptide, polynucleotide, vector, composition, or cells is administered after the checkpoint inhibitor. In cases, administration of the checkpoint inhibitor is continued throughout neoantigen peptide, polynucleotide, vector, composition, or cell therapy. In cases, the neoantigen peptide, polynucleotide, vector, composition, or cell therapy is administered to subjects that only partially respond or do not respond to checkpoint inhibitor therapy. In cases, the composition is administered intravenously or subcutaneously. In cases, the checkpoint inhibitor is administered intravenously or subcutaneously. In cases, the checkpoint inhibitor is administered subcutaneously within about 2 cm of the site of administration of the composition. In cases, the composition is administered into the same draining lymph node as the checkpoint inhibitor. In cases, the method further comprises administering an additional therapeutic agent to the subject either prior to, simultaneously with, or after treatment with the peptide, polynucleotide, vector, composition, or cells. In cases, the additional agent is a chemotherapeutic agent, an immunomodulatory drug, an immune metabolism modifying drug, a targeted therapy, radiation an anti-angiogenesis agent, or an agent that reduces immune-suppression. In cases, the chemotherapeutic agent is an alkylating agent, a topoisomerase inhibitor, an anti-metabolite, or an anti-mitotic agent. In cases, the additional agent is an anti-glucocorticoid induced tumor necrosis factor family receptor (GITR) agonistic antibody or antibody fragment, ibrutinib, docetaxeol, cisplatin, a CD40 agonistic antibody or antibody fragment, an IDO inhibitor, or cyclophosphamide. In cases, the method elicits a CD4+ T cell immune response or a CD8+ T cell immune response. In cases, the method elicits a CD4+ T cell immune response and a CD8+ T cell immune response.

Provided herein is a method for stimulating an immune response in a subject, comprising administering an effective amount of modified cells or composition described herein. In cases, the immune response is cytotoxic and/or humoral immune response. In cases, the method stimulates a T cell-mediated immune response in a subject. In cases, the T cell-mediated immune response is directed against a target cell. In cases, the target cell is a tumor cell. In cases, the modified cells are transfected or transduced in vivo. In cases, the modified cells are transfected or transduced ex vivo. In cases, the modified cells are autologous subject T cells. In cases, the autologous subject T cells are obtained from a subject that has received a neoantigen peptide or nucleic acid vaccine. In cases, the neoantigen peptide or nucleic acid vaccine comprises at least one personalized neoantigen. In cases, the neoantigen peptide or nucleic acid vaccine comprises at least one additional neoantigenic peptide listed in Table 1 or 2. In cases, the subject received a chemotherapeutic agent, an immunomodulatory drug, an immune metabolism modifying drug, targeted therapy or radiation prior to and/or during receipt of the neoantigen peptide or nucleic acid vaccine. In cases, the subject receives treatment with at least one checkpoint inhibitor. In cases, the autologous T cells are obtained from a subject that has already received at least one round of T cell therapy containing a neoantigen. In cases, the method further comprises adoptive T cell therapy. In cases, the adoptive T cell therapy comprises autologous T cells. In cases, the autologous T cells are targeted against tumor antigens. In cases, the adoptive T cell therapy further comprises allogenic T cells. In cases, the allogenic T cells are targeted against tumor antigens. In cases, the adoptive T cell therapy is administered before the checkpoint inhibitor, after the checkpoint inhibitor, or simultaneously eith the checkpoint inhibitor.

Provided herein is a method for evaluating the efficacy of any of the cells described herein, comprising: (i) measuring the number or concentration of target cells in a first sample obtained from the subject before administering the modified cell, (ii) measuring the number concentration of target cells in a second sample obtained from the subject after administration of the modified cell, and (iii) determining an increase or decrease of the number or concentration of target cells in the second sample compared to the number or concentration of target cells in the first sample. In cases, treatment efficacy is determined by monitoring a clinical outcome; an increase, enhancement or prolongation of anti-tumor activity by T cells; an increase in the number of anti-tumor T cells or activated T cells as compared with the number prior to treatment; B cell activity; CD4+ T cell activity; or a combination thereof. In cases, treatment efficacy is determined by monitoring a biomarker. In cases, the biomarker is selected from the group consisting of CEA, Her-2/neu, bladder tumor antigen, thyroglobulin, alpha-fetoprotein, PSA, CA 125, CA19.9, CA 15.3, leptin, prolactin, osteopontin, IGF-II, CD98, fascin, sPIgR, 14-3-3 eta, troponin I, circulating tumor cell RNA or DNA, and b-type natriuretic peptide. In cases, clinical outcome is selected from the group consisting of tumor regression; tumor shrinkage; tumor necrosis; anti-tumor response by the immune system; tumor expansion, recurrence or spread; or a combination thereof. In cases, the treatment effect is predicted by presence of T cells or by presence of a gene signature indicating T cell inflammation or a combination thereof.

Provided herein is a method of treating cancer or initiating, enhancing, or prolonging an anti-tumor response in a subject in need thereof comprising administering to the subject: the peptide, polynucleotide, vector, composition, antibody, or cells described herein; and at least one checkpoint inhibitor. In cases, the method further comprises administration of an immunomodulator or adjuvant. In cases, the immunomodulator or adjuvant is selected from the group consisting of Poly(I:C), Poly-ICLC, STING agonist, 1018 ISS, aluminium salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312 VG, Montanide ISA 206 VG, Montanide ISA 50 V2, Montanide ISA 51 VG, OK-432, OM-174, OM-197-MP-EC, ISA-TLR2 agonist, ONTAK, PepTel® vector system, PLG microparticles, resiquimod, SRL172, virosomes and other virus-like particles, YF-17D, VEGF trap, R848, beta-glucan, Pam3Cys, Pam3CSK4, acrylic or methacrylic polymers, copolymers of maleic anhydride, and QS21 stimulon. a co-stimulatory ligand, a TNF ligand, an Ig superfamily ligand, CD28, CD80, CD86, ICOS, CD40L, OX40, CD27, GITR, CD30, DR3, CD69, or 4-1BB. In cases, the immunomodulator or adjuvant is Poly-ICLC. In cases, the checkpoint inhibitor is an anti-PDI antibody or antibody fragment. In cases, the anti-PDI antibody or antibody fragment is nivolumab or pembolizumab. In cases, the checkpoint inhibitor is an anti-PD-L1 antibody or antibody fragment. In cases, the anti-PD-L1 antibody or antibody fragmentis avelumab, durvalumab or atezolizumab. In cases, the checkpoint inhibitor is an anti-CTLA4 antibody or antibody fragment. In cases, the anti-CTLA4 antibody is ipilimumab or tremelimumab. In cases, the method comprises administering both an anti-PD1 antibody and an anti-CTLA4 antibody. In cases, administration of the checkpoint inhibitor is initiated before initiation of administration of the peptide, polynucleotide, vector, composition, antibody, or cell. In cases, administration of the checkpoint inhibitor is initiated after initiation of administration of the peptide, polynucleotide, vector, composition, antibody, or cell. In cases, administration of the checkpoint inhibitor is initiated simultaneously with the initiation of administration of the peptide, polynucleotide, vector, composition, antibody, or cell. In cases, the peptide, polynucleotide, vector, composition, antibody, or cell is administered intravenously or subcutaneously. In cases, the checkpoint inhibitor is administered intravenously or subcutaneously. In cases, the checkpoint inhibitor is administered subcutaneously within about 2 cm of the site of administration of the peptide, polynucleotide, vector, composition, antibody, or cell. In cases, the peptide, polynucleotide, vector, composition, antibody, or cell is administered into the same draining lymph node as the checkpoint inhibitor.

Provided herein is a kit comprising the peptide, polynucleotide, vector, composition, antibody, cells, or composition described herein. In cases, the cancer is selected from the group consisting of: adrenal, bladder, breast, cervical, colorectal, glioblasoma, head and neck, kidney chromophobe, kidney clear cell, kidney papillary, liver, lung adenocarcinoma, lung squamous, ovarian, pancreatic, melanoma, stomach, uterine corpus endometrial,and uterine carcinosarcoma. In cases, the cancer is selected from the group consisting of: prostate cancer, bladder, lung squamous, NSCLC, breast, head and neck, lung adenocarcinoma, GBM, Glioma, CML, AML, supretentorial ependyomas, acute promyelocytic leukemia, solitary fibrous tumors, and crizotinib resistant cancer. In cases, the cancer is selected from the group consisting of: CRC, head and neck, stomach, lung squamous, lung adenocarcinoma, Prostate, Bladder. stomach, renal cell carcinoma, and uterine. In cases, the cancer is selected from the group consisting of: melanoma, lung squamous, DLBCL, uterine, head and neck, uterine, liver, and CRC. In cases, the cancer is selected from the group consisting of:

lymphoid cancer; Burkitt lymphoma, neuroblastoma, prostate adenocarcinoma, colorectal adenocarcinoma; Uterine/Endometrium Adenocarcinoma; MSI+; endometrium serous carcinoma; endometrium carcinosarcoma-malignant mesodermal mixed tumour; glioma; astrocytoma; GBM, acute myeloid leukaemia associated with MDS; chronic lymphocytic leukaemia-small lymphocytic lymphoma; myelodysplastic syndrome; acute myeloid leukaemia; luminal NS carcinoma of breast; chronic myeloid leukaemia; ductal carcinoma of pancreas; chronic myelomonocytic leukaemia; myelofibrosis; myelodysplastic syndrome; prostate adenocarcinoma; essential thrombocythaemia; and medullomyoblastoma. In cases, the cancer is selected from the group consisting of: colorectal, uterine, endometrial, and stomach. In cases, the cancer is selected from the group consisting of: cervical, head and neck, anal, stomach, Burkitt's lymphoma, and nasopharyngeal carcinoma. In cases, the cancer is selected from the group consisting of: bladder, colorectal, and stomach. In cases, the cancer is selected from the group consisting of: lung, CRC, melanoma, breast, NSCLC, and CLL. In cases, the subject is a partial or non-responder to checkpoint inhibitor therapy. In cases, the cancer is selected from the group consisting of: bladder urothelial carcinoma (BLCA), breast invasive carcinoma (BRCA), breast cancer, cervical squamous cell carcinoma and endocervical adenocarcinoma (CESC), chronic lymphocytic leukaemia (CLL), colorectal cancer (CRC), glioblastoma multiforme (GBM), head and neck squamous cell carcinoma (HNSC), kidney renal papillary cell carcinoma (KIRP), liver hepatocellular carcinoma (LIHC), lung adenocarcinoma (LUAD), lung squamous cell carcinoma (LUSC), pancreatic adenocarcinoma (PAAD), Prostate Cancer, skin cutaneous melanoma (SKCM), stomach adenocarcinoma (STAD), thyroid adenocarcinoma (THCA), and uterine corpus endometrioid carcinoma (UCEC). In cases, the cancer is selected from the group consisting of: colorectal cancer, uterine cancer, endometrium cancer, stomach cancer, and Lynch syndrome. In cases, the cancer is an MSI+ cancer.

The present disclosure is directed to an isolated neoantigenic peptide comprising a tumor-specific neoepitope defined in Table 1, wherein the isolated neoantigenic peptide is not a native polypeptide. The present disclosure is also directed to an isolated neoantigenic peptide which comprises a tumor-specific neoepitope and is defined in Table 1.

In some cases, the isolated neoantigenic peptide is between about 8 to about 50 amino acids in length. In another case, the isolated neoantigenic peptide is between about 15 to about 35 amino acids in length. In another case, the isolated neoantigenic peptide is about 15 amino acids or less in length. In another case, the isolated neoantigenic peptide is between about 8 and about 11 amino acids in length. In another case, the isolated neoantigenic peptide is 9 or 10 amino acids in length. In another case, the isolated neoantigenic peptide binds major histocompatibility complex (MHC) class I. In another case, the isolated neoantigenic peptide binds MHC class I with a binding affinity of less than about 500 nM.

In some cases, the isolated neoantigenic peptide is about 30 amino acids or less in length. In another case, the isolated neoantigenic peptide is between about 6 and about 25 amino acids in length. In another case, the isolated neoantigenic peptide is between about 15 and about 24 amino acids in length. In another case, the isolated neoantigenic peptide is between about 9 and about 15 amino acids in length. In another case, the isolated neoantigenic peptide binds MHC class II. In another case, the isolated neoantigenic peptide binds MHC class II with a binding affinity of less than about 1000 nM.

In some cases, the isolated neoantigenic peptide further comprises flanking amino acids. In another case, the flanking amino acids are not native flanking amino acids. In another case, the isolated neoantigenic peptide is linked to at least a second neoantigenic peptide. In another case, the peptides are linked using a poly-glycine or poly-serine linker. In another case, the second neoantigenic peptide binds MHC class I or class II with a binding affinity of less than about 1000 nM. In another case, the second neoantigenic peptide binds MHC class I or class II with a binding affinity of less than about 500 nM. In another case, both of the neoepitopes bind to human leukocyte antigen (HLA) -A, -B, -C, -DP, -DQ, or -DR. In another case, the isolated neoantigenic peptide binds a class I HLA and the second neoantigenic peptide binds a class II HLA. In another case, the isolated neoantigenic peptide binds a class II HLA and the second neoantigenic peptide binds a class I HLA.

In some cases, the isolated neoantigenic peptide further comprises modifications which increase in vivo half-life, cellular targeting, antigen uptake, antigen processing, MHC affinity, MHC stability, or antigen presentation. In another case, the modification is conjugation to a carrier protein, conjugation to a ligand, conjugation to an antibody, PEGylation, polysialylation HESylation, recombinant PEG mimetics, Fc fusion, albumin fusion, nanoparticle attachment, nanoparticulate encapsulation, cholesterol fusion, iron fusion, acylation, amidation, glycosylation, side chain oxidation, phosphorylation, biotinylation, the addition of a surface active material, the addition of amino acid mimetics, or the addition of unnatural amino acids. In another case, the cells that are targeted are antigen presenting cells. In another case, the antigen presenting cells are dendritic cells. In another case, the dendritic cells are targeted using DEC205, XCR1, CD197, CD80, CD86, CD123, CD209, CD273, CD283, CD289, CD184, CD85h, CD85j, CD85k, CD85d, CD85g, CD85a, CD141, CD11c, CD83, TSLP receptor, Clec9a or CD1a marker. In another case, the dendritic cells are targeted using the CD141, DEC205, or XCR1 marker.

In some cases, the disclosure provides an in vivo delivery system comprising an isolated neoantigenic peptide described herein. In another case, the delivery system includes cell-penetrating peptides, nanoparticulate encapsulation, virus like particles, or liposomes. In another case, the cell-penetrating peptide is TAT peptide, herpes simplex virus VP22, transportan, or Antp.

In some cases, the disclosure is directed to a cell comprising an isolated neoantigenic peptide described herein. In another case, the cell is an antigen presenting cell. In another case, the cell is a dendritic cell.

In some cases, the disclosure is directed to a composition comprising an isolated neoantigenic peptide described herein. In another case, the composition comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 of the isolated neoantigenic peptides comprising a tumor-specific neoepitope defined in Table 1 or 2. In another case, the composition comprises between 2 and 20 neoantigenic peptides. In another case, the composition further comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, or at least 25, at least 26, at least 27, at least 28, at least 29, or at least 30 additional neoantigenic peptides. In another case, the composition comprises between about 4 and about 20 additional neoantigenic peptides. In another case, the additional neoantigenic peptide is specific for an individual patient's tumor. In another case, the patient specific neoantigenic peptide is selected by identifying sequence differences between the genome, exome, and/or transcriptome of the patient's tumor sample and the genome, exome, and/or transcriptome of a non-tumor sample. In another case, the samples are fresh or formalin-fixed paraffin embedded tumor tissues, freshly isolated cells, or circulating tumor cells. In another case, the sequence differences are determined by Next Generation Sequencing.

In some cases, the disclosure is directed to an isolated polynucleotide encoding an isolated neoantigenic peptide described herein. In another case, the isolated polynucleotide is RNA, optionally a self-amplifying RNA. In another case, the RNA is modified to increase stability, increase cellular targeting, increase translation efficiency, adjuvanticity, cytosol accessibility, and/or decrease cytotoxicity. In another case, the modification is conjugation to a carrier protein, conjugation to a ligand, conjugation to an antibody, codon optimization, increased GC-content, incorporation of modified nucleosides, incorporation of 5'-cap or cap analog, and/or incorporation of an unmasked poly-A sequence.

In some cases, the disclosure is directed to a cell comprising a polynucleotide described herein.

In some cases, the disclosure is directed to a vector comprising a polynucleotide described herein. In another case, the polynucleotide is operably linked to a promoter. In another case, the vector comprises a self-amplifying RNA replicon, plasmid, phage, transposon, cosmid, virus, or virion. In another case, the vector is an adeno-associated virus, herpesvirus, lentivirus, or pseudotypes thereof.

In some cases, the disclosure is directed to an in vivo delivery system comprising an isolated polynucleotide described herein. In another case, the delivery system includes spherical nucleic acids, viruses, virus-like particles, plasmids, bacterial plasmids, or nanoparticles.

In some cases, the disclosure is directed to a cell comprising a vector or delivery system described herein. In another case, the cell is an antigen presenting cell. In another case, the cell is a dendritic cell. In another case, the cell is an immature dendritic cell.

In some cases, the disclosure is directed to a composition comprising at least one polynucleotide described herein. In another case, the composition comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 of the isolated polynucleotides. In another case, the composition comprises between about 2 and about 20 polynucleotides that encode neoantigenic peptides. In another case, the composition further comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 additional neoantigenic polynucleotides encoding for additional neoantigenic peptides. In some cases, the composition comprises between about 4 and about 20 additional neoantigenic polynucleotides. In some cases, the isolated polynucleotides and the additional neoantigenic polynucleotides are linked. In some cases, the polynucleotides are linked using nucleic acids that encode a poly-glycine or poly-serine linker. In some cases, at least one of the additional neoantigenic peptide is specific for an individual patient's tumor. In some cases, the patient specific neoantigenic peptide is selected by identifying sequence differences between the genome, exome, and/or transcriptome of the patient's tumor sample and the genome, exome, and/or transcriptome of a non-tumor sample. In some cases, the samples are fresh or formalin-fixed paraffin embedded tumor tissues, freshly isolated cells, or circulating tumor cells. In some cases, the sequence differences are determined by Next Generation Sequencing.

In some cases, the disclosure is directed to a T cell receptor (TCR) capable of binding at least one neoantigenic peptide described herein. In some cases, the TCR is capable of binding the isolated neoantigenic peptide in the context of MHC class I or class II.

In some cases, the disclosure is directed to a chimeric antigen receptor comprising: (i) a T cell activation molecule; (ii) a transmembrane region; and (iii) an antigen recognition moiety capable of binding an isolated neoantigenic peptide described herein. In some cases, the chimeric antigen receptor contains CD3-zeta as the T cell activation molecule. In some cases, the chimeric antigen receptor further comprises at least one costimulatory signaling domain. In some cases, the signaling domain is CD28, 4-1BB, ICOS, OX40, ITAM, or Fc epsilon RI-gamma. In some cases, the antigen recognition moiety is capable of binding the isolated neoantigenic peptide in the context of MHC class I or class II. In some cases, the chimeric antigen receptor comprises the CD3-zeta, CD28, CTLA-4, ICOS, BTLA, KIR, LAG3, CD137, OX40, CD27, CD40L, Tim-3, A2aR, or PD-1 transmembrane region. In some cases, the tumor-specific epitope is located in the extracellular domain of a tumor associated polypeptide.

In some cases, the disclosure is directed to a T cell comprising the T cell receptor or chimeric antigen receptor described herein. In some cases, the T cell is a helper or cytotoxic T cell.

In some cases, the disclosure is directed to a nucleic acid comprising a promoter operably linked to a polynucleotide encoding a T cell receptor described herein. In some cases, the TCR is capable of binding the at least one neoantigenic peptide in the context of major histocompatibility complex (MHC) class I or class II. In another case, the nucleic acid comprises a promoter operably linked to a polynucleotide encoding a chimeric antigen receptor described herein. In another case, the antigen recognition moiety is capable of binding the at least one neoantigenic peptide in the context of major histocompatibility complex (MHC) class I or class II. In some cases, the tumor-specific epitope is located in the extracellular domain of a tumor associated polypeptide. In some cases, the nucleic acid comprises the CD3-zeta, CD28, CTLA-4, ICOS, BTLA, KIR, LAG3, CD137, OX40, CD27, CD40L, Tim-3, A2aR, or PD-1 transmembrane region.

In some cases, the disclosure is directed to an antibody capable of binding at least one neoantigenic peptide described herein.

In some cases, the disclosure is directed to a modified cell transfected or transduced with a nucleic acid described herein. In some cases, the modified cell is a T cell, tumor infiltrating lymphocyte, NK-T cell, TCR-expressing cell, CD4$^+$ T cell, CD8$^+$ T cell, or NK cell.

In some cases, the disclosure is directed to a composition comprising a T cell receptor or chimeric antigen receptor described herein. In some cases, the composition comprises autologous patient T cells containing a T cell receptor or chimeric antigen receptor. In some cases, the composition further comprises an immune checkpoint inhibitor. In some cases, the composition further comprises at least two immune checkpoint inhibitors. In some cases, each of the immune checkpoint inhibitors inhibits a checkpoint protein selected from the group consisting of CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, and B-7 family ligands or a combination thereof. In some cases, each of the immune checkpoint inhibitors interacts with a ligand of a checkpoint protein selected from the group consisting of CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, and B-7 family ligands or a combination thereof.

In some cases, the composition further comprises an immune modulator or adjuvant. In another case, the immune modulator is a co-stimulatory ligand, a TNF ligand, an Ig superfamily ligand, CD28, CD80, CD86, ICOS, CD40L, OX40, CD27, GITR, CD30, DR3, CD69, or 4-1BB. In another case, the immune modulator is at least one cancer cell or cancer cell extract. In another case, the cancer cell is autologous to the subject in need of the composition. In another case, the cancer cell has undergone lysis or been exposed to UV radiation. In another case, the adjuvant induces a humoral when administered to a subject. In another case, the adjuvant induces a T helper cell type 1 when administered to a subject.

In some cases, the disclosure is directed to a method of inhibiting growth of a tumor cell expressing a tumor-specific neoepitope described herein, comprising contacting the tumor cell with the peptide, polynucleotide, delivery system, vector, composition, antibody, or cells of the disclosure.

In some cases, the disclosure is directed to a method of treating cancer or initiating, enhancing, or prolonging an anti-tumor response in a subject in need thereof comprising administering to the subject the peptide, polynucleotide, vector, composition, antibody, or cells described herein.

In some cases, the cancer is selected from adrenal, bladder, breast, cervical, colorectal, glioblasoma, head and neck, kidney chromophobe, kidney clear cell, kidney papillary, liver, lung adeno, lung squamous, ovarian, pancreatic, melanoma, stomach, uterine corpus endometrial,and uterine carcinosarcoma.

In some cases, the cancer is selected from the group of: prostate cancer, bladder, lung squamous, NSCLC, breast, head and neck, lung adenocarcinoma, GBM, Glioma, CML, AML, supretentorial ependyomas, acute promyelocytic leukemia, solitary fibrous tumors, and crizotinib resistant cancer.

In some cases, the cancer is selected from the group consisting of CRC, head and neck, stomach, lung squamous, lung adeno., Prostate, Bladder. stomach, renal cell carcinoma, and uterine.

In some cases, the cancer is selected from the group consisting of melanoma, lung squamous, DLBCL, uterine, head and neck, uterine, liver, and CRC.

In some cases, the cancer is selected from the group consisting of lymphoid cancer; Burkitt lymphoma, neuroblastoma, prostate adenocarcinoma, colorectal adenocarcinoma; Uterine/Endometrium Adenocarcinoma; MSI$^+$; endometrium serous carcinoma; endometrium carcinosarcoma-malignant mesodermal mixed tumour; glioma; astrocytoma; GBM, acute myeloid leukaemia associated with MDS; chronic lymphocytic leukaemia-small lymphocytic lymphoma; myelodysplastic syndrome; acute myeloid leukaemia; luminal NS carcinoma of breast; chronic myeloid leukaemia; ductal carcinoma of pancreas; chronic myelomonocytic leukaemia; myelofibrosis; myelodysplastic syndrome; prostate adenocarcinoma; essential thrombocythaemia; and medullomyoblastoma..

In some cases, the cancer is selected from the group consisting of colorectal, uterine, endometrial, and stomach.

In some cases, the cancer is selected from the group consisting of cervical, head and neck, anal, stomach, Burkitt's lymphoma, and nasopharyngeal carcinoma.

In some cases, the cancer is selected from the group consisting of bladder, colorectal, and stomach.

In some cases, the cancer is selected from the group consisting of lung, CRC, melanoma, breast, NSCLC, and CLL.

In some cases, the cancer is selected from the group consisting of adrenocortical carcinoma (ACC), acute promyelocytic leukemia, acute myeloid leukemia (AML), AML associated with myelodysplastic syndromes (MDS), anal cancer, astrocytoma, bladder cancer, bladder urothelial carcinoma (BLCA), breast cancer, breast invasive carcinoma (BRCA), Burkitt's Lymphoma, castration-resistant prostate cancer, cervical cancer, cervical squamous cell carcinoma and endocervical adenocarcinoma (CESC), chronic lymphocytic leukaemia-small lymphocytic lymphoma, chronic lymphocytic leukaemia (CLL), chronic myeloid leukaemia (CML), chronic myelomonocytic leukaemia, colorectal adenocarcinoma, colorectal cancer (CRC), Crizotinib resistant non-small cell lung cancer (NSCLC), diffuse large B-cell lymphoma (DLBCL), ductal carcinoma of pancreas, endometrium carcinosarcoma-malignant mesodermal mixed tumour, endometrium serous carcinoma, essential thrombocythaemia, glioblastoma multiforme (GBM), Glioma, head and neck cancer, head and neck squamous cell carcinoma (HNSC), invasive lobular carcinoma (ILC) LumA breast cancer, kidney chromophobe (KICH), kidney renal clear cell carcinoma (KIRC), kidney renal papillary cell carcinoma (KIRP), acute myeloid leukemia (LAML), liver hepatocellular carcinoma (LIHC), liver cancer, lung adenocarcinoma (LUAD), lung squamous cell carcinoma (LUSC), Luminal NS carcinoma of breast, lung cancer, lymphoid cancer, medullomyoblastoma, melanoma, microsatellite instability (MSI)$^+$ colorectal cancer (CRC), MSI$^+$ endometrioid carcinoma, MSI$^+$ stomach cancer, MSI$^+$ uterine/endometrium cancer, myelodysplastic syndrome, myelofibrosis, nasopharyngeal carcinoma, neuroblastoma, non-small cell lung cancer (NSCLC), ovarian serous cystadenocarcinoma (OV), pancreatic adenocarcinoma (PAAD), prostate adenocarcinoma (PRAD), prostate cancer, renal cell carcinoma, skin cutaneous melanoma (SKCM), solitary fibrous tumors, stomach adenocarcinoma (STAD), stomach cancer, supretentorial ependyomas, thyroid adenocarcinoma (THCA), uterine corpus endometrioid carcinoma (UCEC), or uterine carcinosarcoma (UCS), uterine cancer, and uterine/endometrium adenocarcinoma.

In some cases, the cancer is selected from the group consisting of bladder urothelial carcinoma (BLCA), breast invasive carcinoma (BRCA), breast cancer, cervical squamous cell carcinoma and endocervical adenocarcinoma (CESC), chronic lymphocytic leukaemia (CLL), colorectal cancer (CRC), glioblastoma multiforme (GBM), head and neck squamous cell carcinoma (HNSC), kidney renal papillary cell carcinoma (KIRP), liver hepatocellular carcinoma (LIHC), lung adenocarcinoma (LUAD), lung squamous cell carcinoma (LUSC), pancreatic adenocarcinoma (PAAD), Prostate Cancer, skin cutaneous melanoma (SKCM), stomach adenocarcinoma (STAD), thyroid adenocarcinoma (THCA), and uterine corpus endometrioid carcinoma (UCEC).

In some cases, the subject is a human. In another case, the subject has cancer. In another case, the cancer is selected from the group consisting of urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, haematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, oesophageal cancers, hepatocellular cancers, cholangiocellular cancers, head and neck squamous cell cancer soft tissue sarcoma, and small cell lung cancer. In another case, the subject has undergone surgical removal of the tumor. In another case, the peptide, polynucleotide, vector, composition, or cells is administered via intravenous, intraperitoneal, intratumoral, intradermal, or subcutaneous administration. In another case, the peptide, polynucleotide, vector, composition, or cells is administered into an anatomic site that drains into a lymph node basin. In another case, the administration is into multiple lymph node basins. In another case, the administration is by a subcutaneous or intradermal route.

In some cases of the method, a peptide is administered. In another case, the administration is intratumorally. In another case of the method, a polynucleotide, optionally RNA, is administered. In another case, the polynucleotide is administered intravenously. In some cases of the method, a cell is administered. In another case, the cell is a T cell or dendritic cell. In another case, the peptide or polynucleotide comprises an antigen presenting cell targeting moiety.

In another case of the method, at least one immune checkpoint inhibitor is also administered to the subject. In another case, the checkpoint inhibitor is a biologic therapeutic or a small molecule. In another case, the checkpoint inhibitor is selected from the group consisting of a monoclonal antibody, a humanized antibody, a fully human antibody and a fusion protein or a combination thereof. In another case, the checkpoint inhibitor inhibits a checkpoint protein selected from the group consisting of CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, and B-7 family ligands or a combination thereof. In another case, the checkpoint inhibitor interacts with a ligand of a checkpoint protein selected from the group consisting of CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, and B-7 family ligands or a combination thereof. In another case, two or more checkpoint inhibitors are administered. In another case, the checkpoint inhibitors are: (i) ipilimumab or tremelimumab, and (ii) nivolumab. In another case, the checkpoint inhibitor and the composition are administered simultaneously or sequentially in any order. In another case, a neoantigenic peptide, polynucleotide, vector, composition, or cells is administered prior to the checkpoint inhibitor. In another case, a peptide, polynucleotide, vector, composition, or cells is administered after the checkpoint inhibitor. In another case, the checkpoint inhibitor is continued throughout neoantigen peptide, polynucleotide, vector, composition, or cell therapy. In another case, the neoantigen peptide, polynucleotide, vector, composition, or cell therapy is administered to subjects that only partially respond or do not respond to checkpoint inhibitor therapy. In another case, the composition is administered intravenously or subcutaneously. In another case, the checkpoint inhibitor is administered intravenously or subcutaneously. In another case, the checkpoint inhibitor is administered subcutaneously within about 2 cm of the site of administration of the composition. In another case, the composition is administered into the same draining lymph node as the checkpoint inhibitor.

In some cases of the method, an additional agent is administered. In another case, the agent is a chemotherapeutic agent, an immunomodulatory drug, an immune metabolism modifying drug, a targeted therapy, radiation an anti-angiogenesis agent, or an agent that reduces immune-suppression. In another case, the chemotherapeutic agent is an alkylating agent, a topoisomerase inhibitor, an anti-metabolite, or an anti-mitotic agent. In another case, the additional agent is an anti-glucocorticoid induced tumor necrosis factor family receptor (GITR) agonistic antibody or antibody fragment, ibrutinib, docetaxeol, cisplatin, or cyclophosphamide. In another case, the administration elicits a $CD4^+$ T cell immune response. In another case, the administration elicits a $CD4^+$ T cell immune response and a $CD8^+$ T cell immune response.

In some cases, the disclosure is directed to a method for stimulating an immune response in a subject, comprising administering an effective amount of modified cells or composition described herein. In another case, the immune response is cytotoxic and/or humoral immune response. In another case, the method stimulates a T cell-mediated immune response in a subject. In another case, the T cell-mediated immune response is directed against a target cell. In another case, the target cell is a tumor cell. In another case, the modified cells are transfected or transduced in vivo. In another case, the modified cells are transfected or transduced ex vivo. In another case, the modified cells are autologous patient T cells. In another case, the autologous patient T cells are obtained from a patient that has received a neoantigen peptide or nucleic acid vaccine. In another case, the neoantigen peptide or nucleic acid vaccine comprises at least one personalized neoantigen. In another case, the neoantigen peptide or nucleic acid vaccine comprises at least one additional neoantigenic peptide described herein. In another case, the patient received a chemotherapeutic agent, an immunomodulatory drug, an immune metabolism modifying drug, targeted therapy or radiation prior to and/or during receipt of the neoantigen peptide or nucleic acid vaccine. In another case, the patient receives treatment with at least one checkpoint inhibitor. In another case, the autologous T cells are obtained from a patient that has already received at least one round of T cell therapy containing a neoantigen. In another case, the method further comprises adoptive T cell therapy. In another case, the adoptive T cell therapy comprises autologous T cells. In another case, the autologous T cells are targeted against tumor antigens. In another case, the adoptive T cell therapy further comprises allogenic T cells. In another case, the allogenic T cells are targeted against tumor antigens. In another case, the adoptive T cell therapy is administered before the checkpoint inhibitor.

In some cases, the disclosure is directed to a method for evaluating the efficacy of treatment comprising: (i) measuring the number or concentration of target cells in a first sample obtained from the subject before administering the modified cell, (ii) measuring the number concentration of target cells in a second sample obtained from the subject after administration of the modified cell, and (iii) determining an increase or decrease of the number or concentration of target cells in the second sample compared to the number or concentration of target cells in the first sample. In another case, the treatment efficacy is determined by monitoring a clinical outcome; an increase, enhancement or prolongation of anti-tumor activity by T cells; an increase in the number of anti-tumor T cells or activated T cells as compared with the number prior to treatment; B cell activity; CD4 T cell activity; or a combination thereof. In another case, the treatment efficacy is determined by monitoring a biomarker. In another case, the biomarker is selected from the group consisting of CEA, Her-2/neu, bladder tumor antigen, thyroglobulin, alpha-fetoprotein, PSA, CA 125, CA19.9, CA 15.3, leptin, prolactin, osteopontin, IGF-II, CD98, fascin, sPIgR, 14-3-3 eta, troponin I, and b-type natriuretic peptide. In another case, the clinical outcome is selected from the group consisting of tumor regression; tumor shrinkage; tumor necrosis; anti-tumor response by the immune system; tumor expansion, recurrence or spread; or a combination thereof. In another case, the treatment effect is predicted by presence of T cells or by presence of a gene signature indicating T cell inflammation or a combination thereof.

In some cases, the disclosure is directed to a method of treating cancer or initiating, enhancing, or prolonging an anti-tumor response in a subject in need thereof comprising administering to the subject: (a) the peptide, polynucleotide, vector, composition, antibody, or cells described herein; and (b) at least one checkpoint inhibitor. In another case, the method further comprises administration of an immunomodulator or adjuvant. In another case, the immunomodulator or adjuvant is selected from the group consisting of Poly(I:C), Poly-ICLC, STING agonist, 1018 ISS, aluminium salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312 VG, Montanide ISA 206 VG, Montanide ISA 50 V2, Montanide ISA 51 VG, OK-432, OM-174, OM-197-MP-EC, ISA-TLR2 agonist, ONTAK, PepTel® vector system, PLG microparticles, resiquimod, SRL172, virosomes and other virus-like particles, YF-17D, VEGF trap, R848, beta-glucan, Pam3Cys, Pam3CSK4, acrylic or methacrylic polymers, copolymers of maleic anhydride, and QS21 stimulon. a co-stimulatory ligand, a TNF ligand, an Ig superfamily ligand, CD28, CD80, CD86, ICOS, CD40L, OX40, CD27, GITR, CD30, DR3, CD69, or 4-1BB. In another case, the immunomodulator or adjuvant is Poly-ICLC. In another case, the checkpoint inhibitor is an anti-PDI antibody or antibody fragment. In another case, the inhibitor of the PD-1 pathway is nivolumab. In another case, the checkpoint inhibitor is an anti-CTLA4 antibody or antibody fragment. In another case, the anti-CTLA4 antibody is ipilimumab or tremelimumab. In another case, the method comprises administering both an anti-PD1 antibody and an anti-CTLA4 antibody. In another case, the administration of the checkpoint inhibitor is initiated before initiation of administration of the peptide, polynucleotide, vector, composition, antibody, or cell. In another case, the administration of the checkpoint inhibitor is initiated after initiation of administration of the peptide, polynucleotide, vector, composition, antibody, or cell. In another case, the administration of the checkpoint inhibitor is initiated simultaneously with the initiation of administration of the peptide, polynucleotide, vector, composition, antibody, or cell. In another case, the peptide, polynucleotide, vector, composition, antibody, or cell is administered intravenously or subcutaneously. In another case, the checkpoint inhibitor is administered intravenously or subcutaneously. In another case, the checkpoint inhibitor is administered subcutaneously within about 2 cm of the site of administration of the peptide, polynucleotide, vector, composition, antibody, or cell. In another case, the peptide, polynucleotide, vector, composition, antibody, or cell is administered into the same draining lymph node as the checkpoint inhibitor.

In some cases of the therapeutic methods, the additional therapeutic agent is for example, a chemotherapeutic or biotherapeutic agent, radiation, or immunotherapy. Any suitable therapeutic treatment for a particular cancer may be administered. Examples of chemotherapeutic and biotherapeutic agents include, but are not limited to, an angiogenesis inhibitor, such as hydroxy angiostatin K 1-3, DL-a-Difluoromethy!-oroithine, endostatiii, fumagillin, genistein, minocycline, staurosporine, and thalidomide; a DNA intercaitor/cross-linker, such as Bleomycin, Carboplatin, Carrmistme, Chlorambucil, Cyclophosphamide, cis-Diammineplat num(D) dichloride (Cispiatin), Melphalan, Mitoxantrone, and Oxaliplatin; a DNA synthesis inhibitor, such as (±)-Amethopterin (Methotrexate), 3-Amino-1,2,4-beiizotriazine 1,4-dioxide, Aminopterin, Cytosine β-D-arabinofuraiioside, 5-Fmoro-5'~ deoxyuridine, 5-Fhsorouracil, Ganciclovir, Hydroxyurea, and Mitomycin C; a DNA-RNA transcription regulator, such as Aetinomycin D, Dau orubicin, Doxorubicin, Homoharringtonine, and Idarubicin; an oη/.γηx; inhibitor, such as S(-i-)-Camptothecm, Curcumin, (-)-Deguelm, 5,6-Dichiorobenzimidazole 1 -β-D-ribofuranoside, Etoposide, Formestane, Fostriecin, Hispidin, 2-Immo-l-imidazoli-dineacetic acid (Cyclocreatine), Mevmolin, Trichostatin A, Tyrphostin AG 34, and Tyrphostin AG 879; a gene regulator, such as 5-Aza-2'-deoxycytidine, 5-Azacytidine, Cholecalciferol (Vitamin D3), 4-Hydroxytamoxifen, Melatonin, Mifepristone, Raloxifene, all trans-Retinal (Vitamin A aldehyde), Retinoic acid all trans (Vitamin A acid), 9-cis-Retinoic Acid, 13-cis-Retinoic acid, Retinol (Vitamin A), Tamoxifen, and Troglitazone; a microtubule inhibitor, such as Colchicine, docetaxel, Dolastatirs 15, Nocodazole, Paclitaxel, Podophyl!otoxin, Rhizoxin, Vinblastine, Vi cristine, Vindesiiie, and Vinorelbine (Navelbine); and an unclassified therapeutic agent, such as 17-(Allyiamino)-1 7-demethoxygeldanamycin, 4-Amino-1,8-naphthalimide, Apigenin, Brefeldin A, Cimetidine, Dichioromethylene-diphosphonic acid, Leuprolide (Leuprorelin), Luteinizing Hormone-Releasing Hormone, Pifithrin-a, Rapamycin, Sex hormone-binding globulin, Thapsigargin, and Urinary trypsin inhibitor fragment (Bikunin). The therapeutic agent may be altretamine, amifostine, asparaginase, capecitabine, cladribine, cisapride, cyiarahirse, dacarbazine (DT1C), dactinomycin, dronabinol, epoetin alpha, "filgrastim, fludarabine, gemcitabine, granisetron, ifosfamide, irinotecan, lansoprazole, levamisole, leucovorin, megestrol, mesna, metoclopramide, mitotane, omeprazole, ondansetron, pilocarpine, prochloroperazine, or topotecan hydrochloride. The therapeutic agent may be a monoclonal antibody such as rituximab (Rituxan®), alemtuzumab (Campath®), Bevacizumab (Avastin®), Cetuximab (Erbitux®), panitumumab (Vectibix®), and trastuzumab (Herceptin®), Vemurafenib (Zelboraf®) imatinib mesylate (Gleevec®), erlotinib (Tarceva®), gefitinib (Iressa®), Vismodegib (Erivedge™), 90Y-ibritumomab tiuxetan, 1311-tosit.umomab, ado-trastuzumab emtansine, lapatinib (Tykerb®), pertuzumab (Perjeta™), ado-trastuzumab emtansine ( adcyla™), regorafenib (Stivarga®), sunitinib (Sutent®), Denosumab (Xgeva®), sorafenib (Nexavar®), pazopanib (Votrient®), axitinib (Inita®), dasatinib (Sprycel®), nilotinib (Tasigna®), bosutinib (Bosulif®), ofatumumab (Arzerra®), obinutuzumab (Gazyva™), ibrutinib (Imbruvica™), idelalisib (Zydelig®), crizotinib (Xalkori®), erlotinib (Tarceva®), afatimb dimaleate (Giiotrif®), ceritinib (LDK378/Zykadia), Tositumomab and 1311-tositumomab (Bexxar®), ibritumomab tiuxetan (Zevalin®), brentuximab vedotin (Adcetris®), bortezomib (Velcade®), siltuximab (Sylvant™), trametinib ( ekinist®), dabrafenib (Tafmlar®), pembrolizimiab (Keytruda®), carfilzomib (Kyprolis®), Ramucirumab (Cyramza™), Cabozantinib (Cometriq™), vandetanib (Caprelsa®), Optionally, the therapeutic agent is a neoantigen. The therapeutic agent may be a cytokine such as interferons (INFs), interlcukins (ILs), or hematopoietic growth factors. The therapeutic agent may be INF-α, IL-2, Aldesleukin, IL-2, Erythropoietin, Granulocyte-macrophage colony-stimulating factor (GM-CSF) or granulocyte colony-stimulating factor. The therapeutic agent may be a targeted therapy such as toremifene (Fareston®), fulvestrant (Faslodex®), anastrozole (Arimidex®), exemestane (Aromasin®), letrozole (Femara®), zivaflibercept (Zaltrap®), Aiitretinoin (Panretin®), temsirolimus (Torisel®), Tretinoin (Vesanoid®), denileukin diftitox (Ontak®), vorinostat (Zoiinza®), romidepsin (Istodax®), bexarotene (Targretin®), pralatrexate (Foiotyn®), !enaliomide (Revlimid®), belinostat (Beleodaq™), lenaliomide (Revlimid®), pomalidomide (Pomalyst®), Cabazitaxel (Jevtana®), enzaluiamide (Xtandi®), abiraterone acetate (Zytiga®), radium 223 chloride (Xofigo®), or everolimus (Afiniior®). Aditionally, the therapeutic agent may be an epigenetic targeted drug such as HDAC inhibitors, kinase inhibitors, DNA methyltransferase inhibitors, histone demethylase inhibitors, or histone methylation inhibitors. The epigenetic drugs may be Azacitidine (Vidaza), Decitabine (Dacogen), Vorinostat (Zoiinza), Romidepsin (Istodax), or Ruxolitinib (Jakafi). For prostate cancer treatment, a preferred chemotherapeutic agent with which anti- CTLA-4 can be combined is paclitaxel (TAXOL).

In some cases, the disclosure is directed to a kit comprising any neoantigen therapeutic described herein.

Where aspects or cases of the disclosure are described in terms of a Markush group or other grouping of alternatives, the present disclosure encompasses not only the entire group listed as a whole, but also each member of the group individually and all possible subgroups of the main group, and also the main group absent one or more of the group members. The present invention also envisages the explicit exclusion of one or more of any of the group members in the claimed invention.

The documents discussed herein are provided solely for their disclosure prior to the filing date of the present application. Nothing herein is to be construed as an admission that the inventors described herein are not entitled to antedate such disclosure by virtue of prior invention or for any other reason.

## BRIEF DESDCRIPTION OF THE DRAWINGS

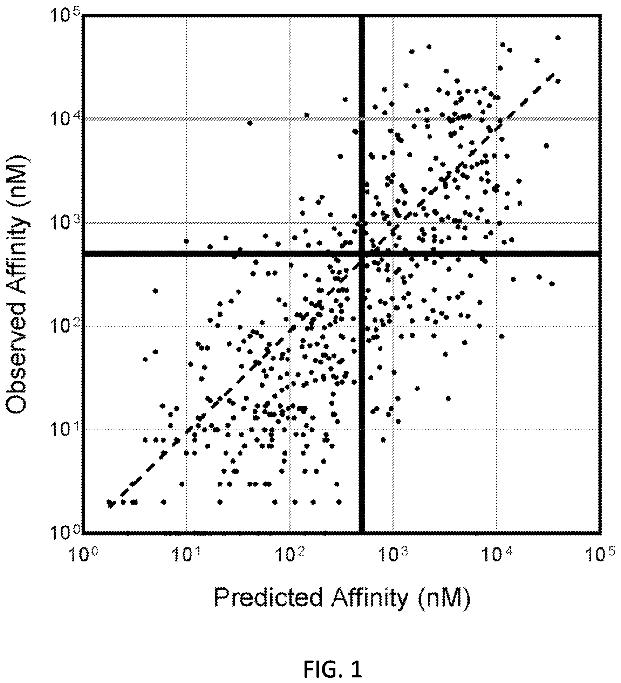

Figure 1 depicts and exemplary graph of 562 peptides with predicted affinity for select HLA Class I molecule between 1 nM and 100,000 nM (plotted on x-axis) that were synthesized. Actual affinity ($IC_{50}$ (nM)) was measured as described (y-axis). Thick vertical and horizontal lines denote 500 nM cutoff between weak and very weak predicted and observed binders, respectively. Diagonal dotted line depicts line-of-best fit (Graphpad Prism) with $R^2$ of 0.45.

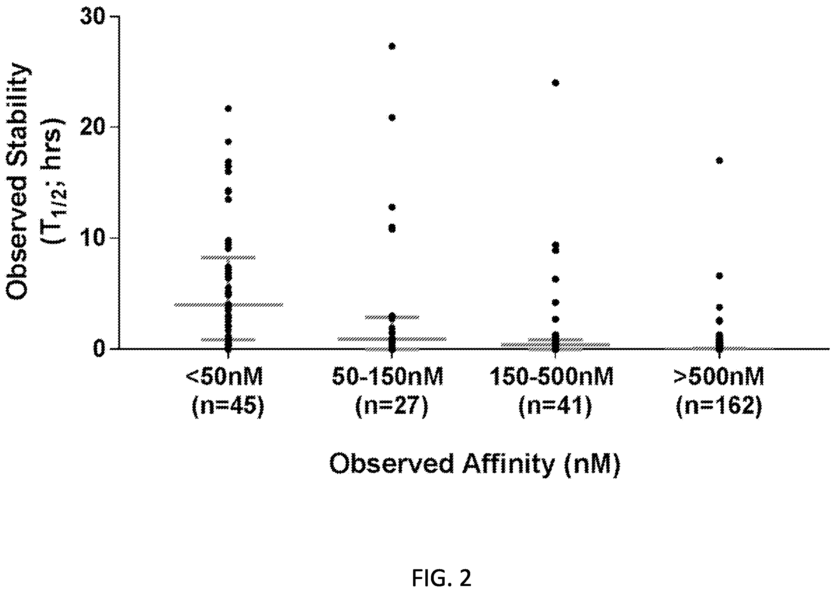

Figure 2 depicts and exemplary graph of 275 peptides from Figure 1 that were tested for stability ($T_{1/2}$; hrs) on their respective HLA Class I molecules. Peptides were binned according to observed affinity from Figure 1. Median and interquartile range is shown for each bin.

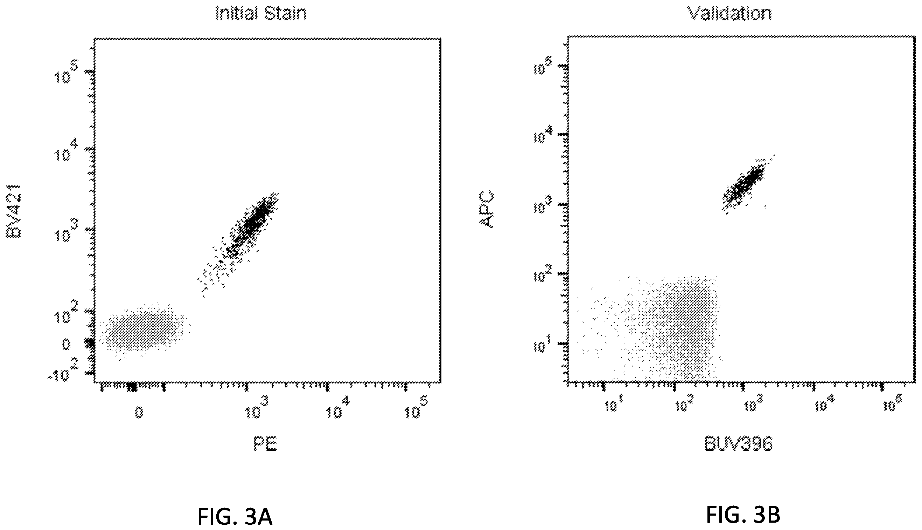

Figure 3A depicts and exemplary graph of HLA-A02:01+ T cells co-cultured with monocyte-derived dendritic cells loaded with TMPRSS2::ERG fusion neoepitope (ALNSEALSV; HLA-A02:01) for 10 days. CD8+ T cells were analyzed for antigen-specificity for TMPRSS2::ERG fusion neoepitope using multimers (initial: BV421 and PE).

Figure 3B depicts and exemplary graph of HLA-A02:01+ T cells co-cultured with monocyte-derived dendritic cells loaded with TMPRSS2::ERG fusion neoepitope (ALNSEALSV; HLA-A02:01) for 10 days. CD8+ T cells were analyzed for antigen-specificity for TMPRSS2::ERG fusion neoepitope using multimers (validation: APC and BUV396).

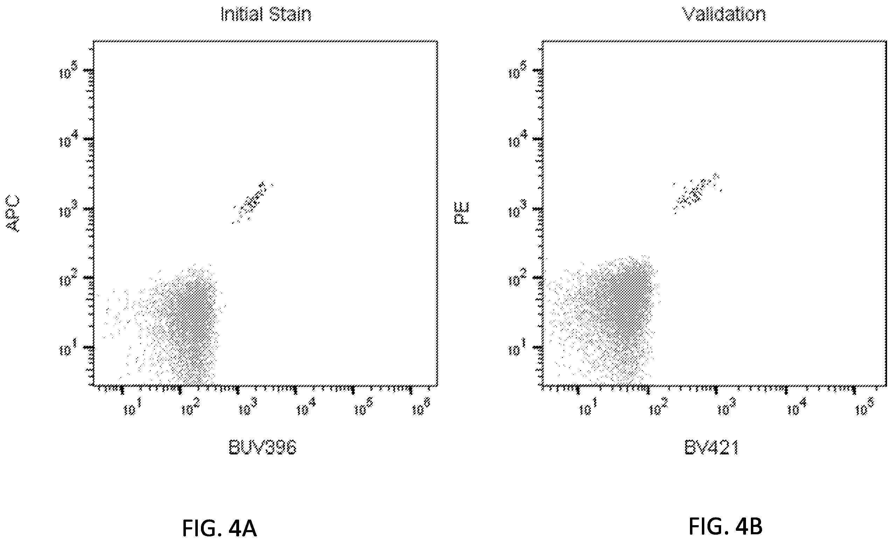

Figure 4A depicts and exemplary graph of HLA-A02:01+ T cells co-cultured with monocyte-derived dendritic cells loaded with GATA3 frameshift neoepitope (SMLTGPPARV; HLA-A02:01) for 10 days. CD8+ T cells were analyzed for antigen-specificity for GATA3 frameshift neoepitope using multimers (initial: APC and BUV396).

Figure 4B depicts and exemplary graph of HLA-A02:01+ T cells co-cultured with monocyte-derived dendritic cells loaded with GATA3 frameshift neoepitope (SMLTGPPARV; HLA-A02:01) for 10 days. CD8+ T cells were analyzed for antigen-specificity for GATA3 frameshift neoepitope using multimers (validation: PE and BV421).

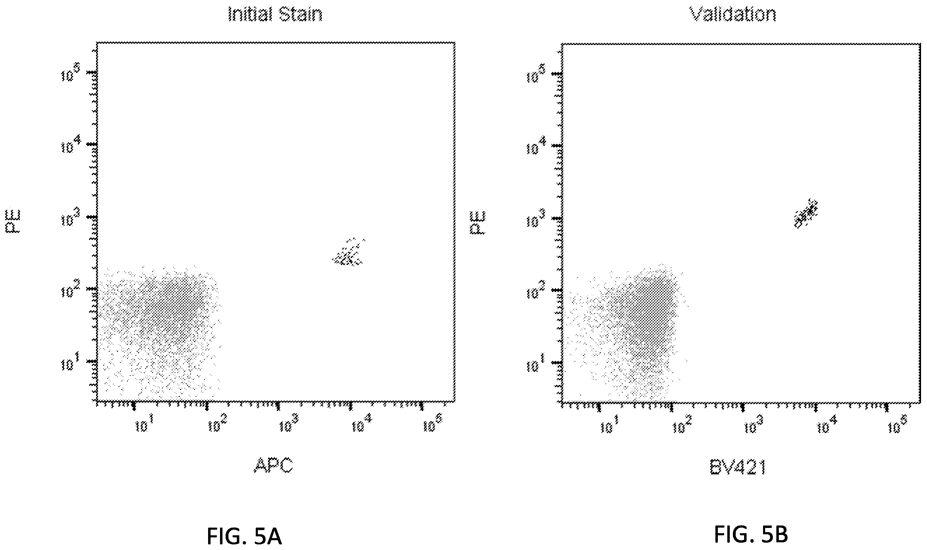

Figure 5A depicts and exemplary graph of HLA-A02:01+ T cells co-cultured with monocyte-derived dendritic cells loaded with β2M frameshift neoepitope (LLCVWVSSI; HLA-A02:01) for 10 days. CD8+ T cells were analyzed for antigen-specificity for β2M frameshift neoepitope using multimers (initial: PE and APC).

Figure 5B depicts and exemplary graph of HLA-A02:01+ T cells co-cultured with monocyte-derived dendritic cells loaded with β2M frameshift neoepitope (LLCVWVSSI; HLA-A02:01) for 10 days. CD8+ T cells were analyzed for antigen-specificity for β2M frameshift neoepitope using multimers (validation: PE and BV421).

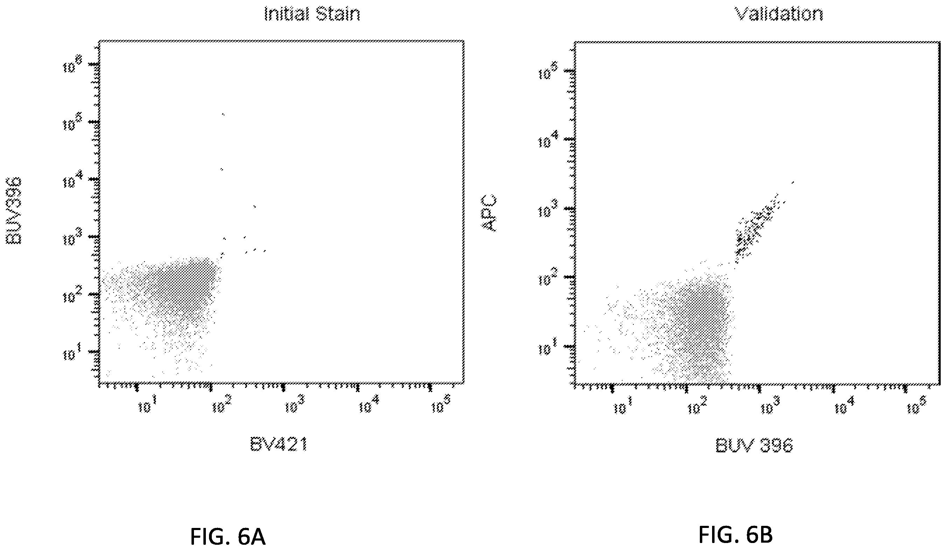

Figure 6A depicts and exemplary graph of HLA-A02:01+ T cells co-cultured with monocyte-derived dendritic cells loaded with KRAS G12C neoepitope (KLVVVGACGV; HLA-A02:01) for 10 days. CD8+ T cells were analyzed for antigen-specificity for KRAS G12C frameshift neoepitope using multimers (initial: BUV396 and BV421).

Figure 6B depicts and exemplary graph HLA-A02:01+ T cells co-cultured with monocyte-derived dendritic cells loaded with KRAS G12C neoepitope (KLVVVGACGV; HLA-A02:01) for 10 days. CD8+ T cells were analyzed for antigen-specificity for KRAS G12C frameshift neoepitope using multimers (validation: APC and BUV396).

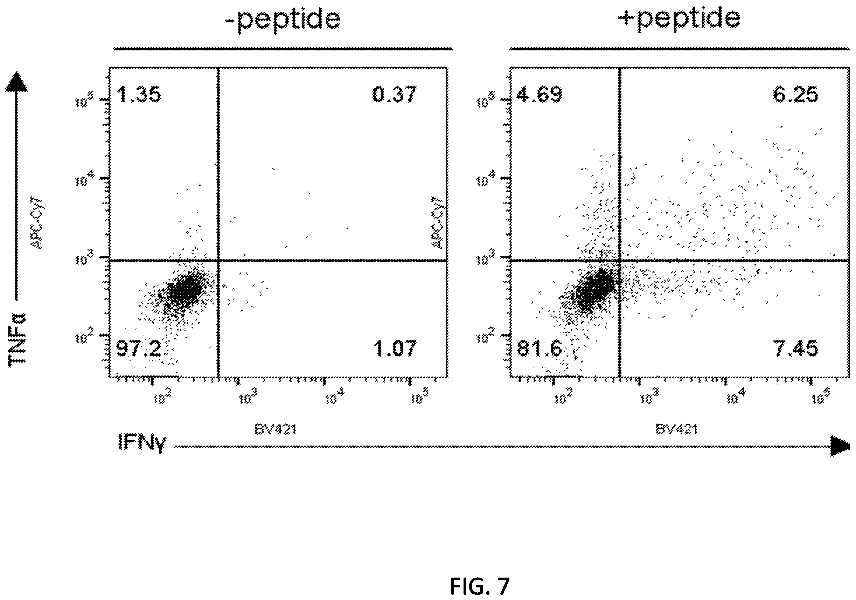

Figure 7 depicts and exemplary graph of T cells co-cultured with monocyte-derived dendritic cells loaded β2M frameshift neopeptides for 20 days (restimulation with fresh monocyte-derived dendritic cells on day 20). CD4+ T cells were analyzed for antigen-specificity by intracellular cytokine staining after restimulation with monocyte-derived dendritic cells loaded with β2M frameshift peptide for 24 hours (right), compared to controls without peptide (left).

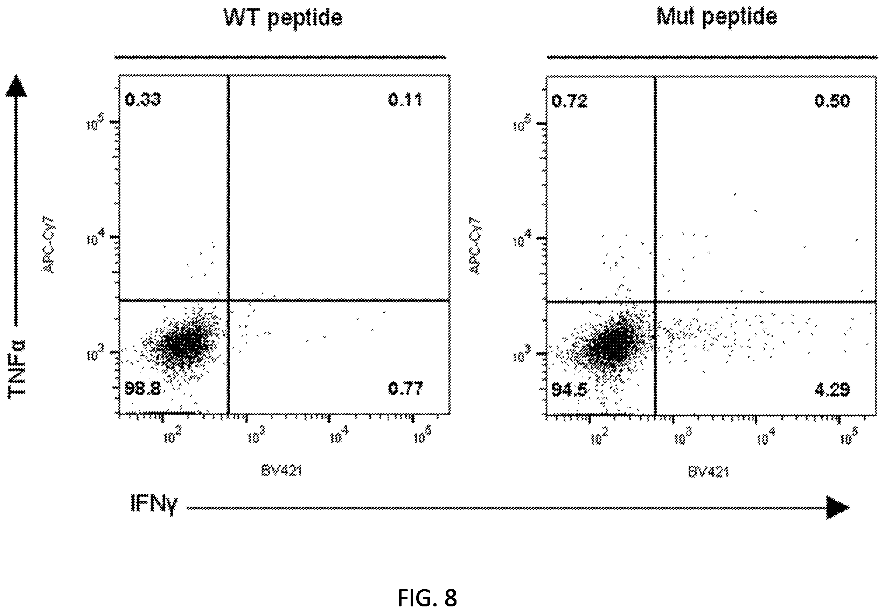

**Figure 8** depicts and exemplary graph of T cells co-cultured with monocyte-derived dendritic cells loaded BTK C481S neopeptide for 20 days (restimulation with fresh monocyte-derived dendritic cells on day 20). CD4+ T cells were analyzed for antigen-specificity by intracellular cytokine staining after restimulation with monocyte-derived dendritic cells loaded with BTK C481S neopeptide for 24 hours (right), compared to controls wild-type BTK peptide (left).

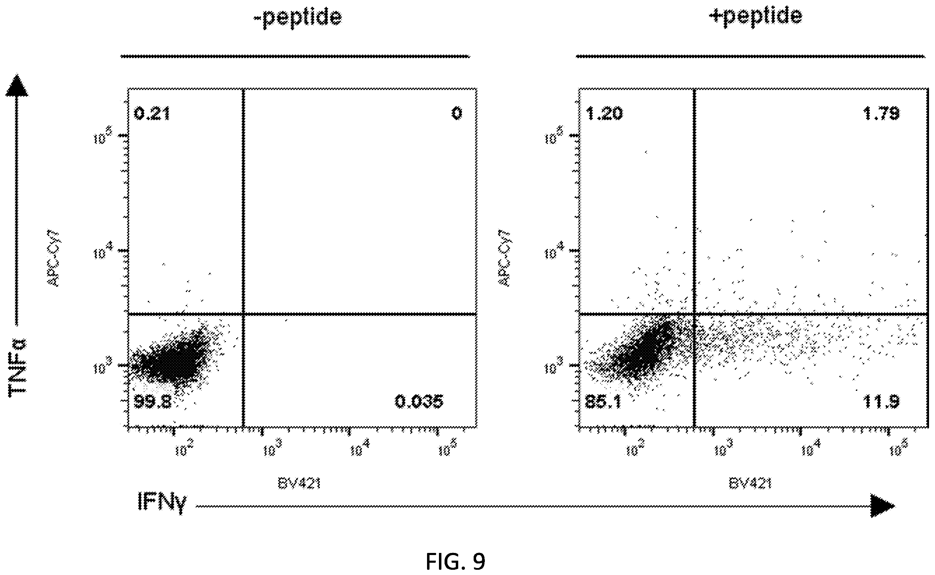

**Figure 9** depicts and exemplary graph of T cells co-cultured with monocyte-derived dendritic cells loaded GATA3 frameshift neopeptides for 20 days (restimulation with fresh monocyte-derived dendritic cells on day 20). CD4+ T cells were analyzed for antigen-specificity by intracellular cytokine staining after restimulation with monocyte-derived dendritic cells loaded with GATA3 frameshift peptide for 24 hours (right), compared to controls without peptide (left).

## DETAILED DESCRIPTION

Described herein are novel immunotherapeutic agents and uses thereof based on the discovery of neoantigens arising from mutational events unique to an individual's tumor. Accordingly, the disclosure described herein provides peptides, polynucleotides encoding the peptides, and peptide binding agents, that can be used, for example, to stimulate an immune response to a tumor associated antigen, to create an immunogenic composition or cancer vaccine for use in treating disease.

## I. Definitions

The terminology used herein is for the purpose of describing particular cases only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" can mean within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, within 5-fold, and more preferably within 2-fold, of a value. Where particular values are described in the application and claims, unless otherwise stated the term "about" meaning within an acceptable error range for the particular value should be assumed.

To facilitate an understanding of the present invention, a number of terms and phrases are defined below.

"Neoantigen" means a class of tumor antigens which arise from tumor-specific changes in proteins. Neoantigens encompass, but are not limited to, tumor antigens which arise from, for example, substitution in the protein sequence, frame shift mutation, fusion polypeptide, in-frame deletion, insertion, expression of endogenous retroviral polypeptides, and tumor-specific overexpression of polypepitdes.

"Tumor specific neoepitope" refers to an epitope that is not present in a reference such as a normal non-cancerous or germline cell but is found in cancer cells. This includes, in particular, situations wherein in a normal non-cancerous or germline cell a corresponding epitope is found, however, due to one or more mutations in a cancer cell the sequence of the epitope is changed so as to result in the neo-epitope.

A "reference" can be used to correlate and compare the results obtained in the methods of the disclosure from a tumor specimen. Typically the "reference" may be obtained on the basis of one or more normal specimens, in particular specimens which are not affected by a cancer disease, either obtained from a patient or one or more different individuals, for example, healthy individuals, in particular individuals of the same species. A "reference" can be determined empirically by testing a sufficiently large number of normal specimens.

The term "mutation" refers to a change of or difference in the nucleic acid sequence (nucleotide substitution, addition or deletion) compared to a reference. A "somatic mutation" can occur in any of the cells of the body except the germ cells (sperm and egg) and therefore are not passed on to children. These alterations can (but do not always) cause cancer or other diseases. In some cases, a mutation is a non-synonymous mutation. The term "non-synonymous mutation" refers to a mutation, for example, a nucleotide substitution, which does result in an amino acid change such as an amino acid substitution in the translation product. A "frameshift" occurs when a mutation disrupts the normal phase of a gene's codon periodicity (also known as "reading frame"), resulting in the translation of a non-native protein sequence. It is possible for different mutations in a gene to achieve the same altered reading frame.

As used herein, the term "affinity" refers to a measure of the strength of binding between two members of a binding pair, for example, an HLA-binding peptide and a class I or II HLA. $K_D$ is the dissociation constant and has units of molarity. The affinity constant is the inverse of the dissociation constant. An affinity constant is sometimes used as a generic term to describe this chemical entity. It is a direct measure of the energy of binding. Affinity may be determined experimentally, for example by surface plasmon resonance (SPR) using commercially available Biacore SPR units. Affinity may also be expressed as the inhibitory concentration 50 ($IC_{50}$), that concentration at which 50% of the peptide is displaced. Likewise, $ln(IC_{50})$ refers to the natural log of the $IC_{50}$. $K_{off}$ refers to the off-rate constant, for example, for dissociation of an HLA-binding peptide and a class I or II HLA.

Throughout this disclosure, "binding data" results can be expressed in terms of "$IC_{50}$." $IC_{50}$ is the concentration of the tested peptide in a binding assay at which 50% inhibition of binding of a labeled reference peptide is observed. Given the conditions in which the assays are run (i.e., limiting HLA protein and labeled reference peptide concentrations), these values approximate $K_D$ values. Assays for determining binding are well known in the art and are described in detail, for example, in PCT publications WO 94/20127 and WO 94/03205, and other publications such Sidney et al., Current Protocols in Immunology 18.3.1 (1998); Sidney, et al., J. Immunol. 154:247 (1995); and Sette, et al., Mol. Immunol. 31:813 (1994). Alternatively, binding can be expressed relative to binding by a reference standard peptide. For example, can be based on its $IC_{50}$, relative to the $IC_{50}$ of a reference standard peptide.

Binding can also be determined using other assay systems including those using: live cells (e.g., Ceppellini et al., Nature 339:392 (1989); Christnick et al., Nature 352:67 (1991); Busch et al., Int. Immunol. 2:443 (1990); Hill et al., J. Immunol. 147:189 (1991); del Guercio et al., J. Immunol. 154:685 (1995)), cell free systems using detergent lysates (e.g., Cerundolo et al., J. Immunol. 21:2069 (1991)), immobilized purified MHC (e.g., Hill et al., J. Immunol. 152, 2890 (1994); Marshall et al., J. Immunol. 152:4946 (1994)), ELISA systems (e.g., Reay et al., EMBO J. 11:2829 (1992)), surface plasmon resonance (e.g., Khilko et al., J. Biol. Chem. 268:15425 (1993)); high flux soluble phase assays (Hammer et al., J. Exp. Med. 180:2353 (1994)), and measurement of class I MHC stabilization or assembly (e.g., Ljunggren et al., Nature 346:476 (1990); Schumacher et al., Cell 62:563 (1990); Townsend et al., Cell 62:285 (1990); Parker et al., J. Immunol. 149:1896 (1992)).

"Cross-reactive binding" indicates that a peptide is bound by more than one HLA molecule; a synonym is degenerate binding.

The term "derived" when used to discuss an epitope is a synonym for "prepared." A derived epitope can be isolated from a natural source, or it can be synthesized according to standard protocols in the art. Synthetic epitopes can comprise artificial amino acid residues "amino acid mimetics," such as D isomers of natural occurring L amino acid residues or non-natural amino acid residues such as cyclohexylalanine. A derived or prepared epitope can be an analog of a native epitope.

A "diluent" includes sterile liquids, such as water and oils, including those of petroleum, animal, vegetable or synthetic origin, such as peanut oil, soybean oil, mineral oil, sesame oil and the like. Water is also a diluent for pharmaceutical compositions. Saline solutions and aqueous dextrose and glycerol solutions can also be employed as diluents, for example, in injectable solutions.

An "epitope" is the collective features of a molecule, such as primary, secondary and tertiary peptide structure, and charge, that together form a site recognized by, for example, an immunoglobulin, T cell receptor, HLA molecule, or chimeric antigen receptor. Alternatively, an epitope can be defined as a set of amino acid residues which is involved in recognition by a particular immunoglobulin, or in the context of T cells, those residues necessary for recognition by T cell receptor proteins, chimeric antigen receptors, and/or Major Histocompatibility Complex (MHC) receptors. Epitopes can be prepared by isolation from a natural source, or they can be synthesized according to standard protocols in the art. Synthetic epitopes can comprise artificial amino acid residues, "amino acid mimetics," such as D isomers of naturally-occurring L amino acid residues or non-naturally-occurring amino acid residues such as cyclohexylalanine. Throughout this disclosure, epitopes may be referred to in some cases as peptides or peptide epitopes.

It is to be appreciated that proteins or peptides that comprise an epitope or an analog described herein as well as additional amino acid(s) are still within the bounds of the invention. In certain cases, the peptide comprises a fragment of an antigen.

In certain cases, there is a limitation on the length of a peptide. The case that is length-limited occurs when the protein or peptide comprising an epitope described herein comprises a region (i.e., a contiguous series of amino acid residues) having 100% identity with a native sequence. In order to avoid the definition of epitope from reading, e.g., on whole natural molecules, there is a limitation on the length of any region that has 100% identity with a native peptide sequence. Thus, for a peptide comprising an epitope described herein and a region with 100% identity with a native peptide sequence, the region with 100% identity to a native sequence generally has a length of: less than or equal to 600 amino acid residues, less than or equal to 500 amino acid residues, less than or equal to 400 amino acid residues, less than or equal to 250 amino acid residues, less than or equal to 100 amino acid residues, less than or equal to 85 amino acid residues, less than or equal to 75 amino acid residues, less than or equal to 65 amino acid residues, and less than or equal to 50 amino acid residues. In certain cases, an "epitope" described herein is comprised by a peptide having a region with less than 51 amino acid residues that has 100% identity to a native peptide sequence, in any increment down to 5 amino acid residues; for example 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 amino acid residues.

"Human Leukocyte Antigen" or "HLA" is a human class I or class II Major Histocompatibility Complex (MHC) protein (see, e.g., Stites, et al., IMMUNOLOGY, 8TH ED., Lange Publishing, Los Altos, Calif. (1994).

An "HLA supertype or HLA family", as used herein, describes sets of HLA molecules grouped on the basis of shared peptide-binding specificities. HLA class I molecules that share somewhat similar binding affinity for peptides bearing certain amino acid motifs are grouped into such HLA supertypes. The terms HLA superfamily, HLA supertype family, HLA family, and HLA xx-like molecules (where "xx" denotes a particular HLA type), are synonyms.

The terms "identical" or percent "identity," in the context of two or more peptide sequences or antigen fragments, refer to two or more sequences or subsequences that are the same or have a specified percentage of amino acid residues that are the same, when compared and aligned for maximum correspondence over a comparison window, as measured using a sequence comparison algorithm or by manual alignment and visual inspection.

An "immunogenic" peptide or an "immunogenic" epitope or "peptide epitope" is a peptide that comprises an allele-specific motif such that the peptide will bind an HLA molecule and induce a cell-mediated or humoral response, for example, cytotoxic T lymphocyte (CTL), helper T lymphocyte (HTL) and/or B lymphocyte response. Thus, immunogenic peptides described herein are capable of binding to an appropriate HLA molecule and thereafter inducing a CTL (cytotoxic) response, or a HTL (and humoral) response, to the peptide.

As used herein, a "chimeric antigen receptor" or "CAR" refers to an antigen binding protein in that includes an immunoglobulin antigen binding domain (e.g., an immunoglobulin variable domain) and a T cell receptor (TCR) constant domain. As used herein, a "constant domain" of a TCR polypeptide includes a membrane-proximal TCR constant domain, and may also include a TCR transmembrane domain and/or a TCR cytoplasmic tail. For example, in some cases, the CAR is a dimer that includes a first polypeptide comprising a immunoglobulin heavy chain variable domain linked to a TCR.beta. constant domain and a second polypeptide comprising an immunoglobulin light chain variable domain (e.g., a κ or λ variable domain) linked to a TCRα constant domain. In some cases, the CAR is a dimer that includes a first polypeptide comprising a immunoglobulin heavy chain variable domain linked to a TCRα constant domain and a second polypeptide comprising an immunoglobulin light chain variable domain (e.g., a κ or λ variable domain) linked to a TCRβ constant domain.

The phrases "isolated" or "biologically pure" refer to material which is substantially or essentially free from components which normally accompany the material as it is found in its native state. Thus, isolated peptides described herein do not contain some or all of the materials normally associated with the peptides in their in situ environment. An "isolated" epitope refers to an epitope that does not include the whole sequence of the antigen from which the epitope was derived. Typically the "isolated" epitope does not have attached thereto additional amino acid residues that result in a sequence that has 100% identity over the entire length of a native sequence. The native sequence can be a sequence such as a tumor-associated antigen from which the epitope is derived. Thus, the term "isolated" means that the material is removed from its original environment (e.g., the natural environment if it is naturally occurring). For example, a naturally-occurring polynucleotide or peptide present in a living animal is not isolated, but the same polynucleotide or peptide, separated from some or all of the coexisting materials in the natural system, is isolated. Such a polynucleotide could be part of a vector, and/or such a polynucleotide or peptide could be part of a composition, and still be "isolated" in that such vector or composition is not part of its natural environment. Isolated RNA molecules include in vivo or in vitro RNA transcripts of the DNA molecules described herein, and further include such molecules produced synthetically.

"Major Histocompatibility Complex" or "MHC" is a cluster of genes that plays a role in control of the cellular interactions responsible for physiologic immune responses. In humans, the MHC complex is also known as the human leukocyte antigen (HLA) complex. For a detailed description of the MHC and HLA complexes, see, Paul, FUNDAMENTAL IMMUNOLOGY, 3.sup.RD ED., Raven Press, New York (1993).

A "native" or a "wild type" sequence refers to a sequence found in nature. Such a sequence can comprise a longer sequence in nature.

A "T cell epitope" is to be understood as meaning a peptide sequence which can be bound by the MHC molecules of class I or II in the form of a peptide-presenting MHC molecule or MHC complex and then, in this form, be recognized and bound by cytotoxic T-lymphocytes or T-helper cells, respectively.

A "receptor" is to be understood as meaning a biological molecule or a molecule grouping capable of binding a ligand. A receptor may serve, to transmit information in a cell, a cell formation or an organism. The receptor comprises at least one receptor unit, for example, where each receptor unit may consist of a protein molecule. The receptor has a structure which complements that of a ligand and may complex the ligand as a binding partner. The information is transmitted in particular by conformational changes of the receptor following complexation of the ligand on the surface of a cell. In some cases, a receptor is to be understood as meaning in particular proteins of MHC classes I and II capable of forming a receptor/ligand complex with a ligand, in particular a peptide or peptide fragment of suitable length.

A "ligand" is to be understood as meaning a molecule which has a structure complementary to that of a receptor and is capable of forming a complex with this receptor. In some cases, a ligand is to be understood as meaning a peptide or peptide fragment which has a suitable length and suitable binding motifs in its amino acid sequence, so that the peptide or peptide fragment is capable of forming a complex with proteins of MHC class I or MHC class II.

In some cases, a "receptor/ligand complex" is also to be understood as meaning a "receptor/peptide complex" or "receptor/peptide fragment complex", including a peptide- or peptide fragment-presenting MHC molecule of class I or of class II.

"Proteins or molecules of the major histocompatibility complex (MHC)", "MHC molecules", "MHC proteins" or "HLA proteins" are to be understood as meaning proteins capable of binding peptides resulting from the proteolytic cleavage of protein antigens and representing potential lymphocyte epitopes, (e.g., T cell epitope and B cell epitope) transporting them to the cell surface and presenting them there to specific cells, in particular cytotoxic T-lymphocytes, T-helper cells, or B cells. The major histocompatibility complex in the genome comprises the genetic region whose gene products expressed on the cell surface are important for binding and presenting endogenous and/or foreign antigens and thus for regulating immunological processes. The major histocompatibility complex is classified into two gene groups coding for different proteins, namely molecules of MHC class I and molecules of MHC class II. The cellular biology and the expression patterns of the two MHC classes are adapted to these different roles.

The terms "peptide" and "peptide epitope" are used interchangeably with "oligopeptide" in the present specification to designate a series of residues connected one to the other, typically by peptide bonds between the $\alpha$-amino and carboxyl groups of adjacent amino acid residues.

"Synthetic peptide" refers to a peptide that is obtained from a non-natural source, e.g., is man-made. Such peptides can be produced using such methods as chemical synthesis or recombinant DNA technology. "Synthetic peptides" include "fusion proteins."

A "PanDR binding" peptide, a "PanDR binding epitope" is a member of a family of molecules that binds more than one HLA class II DR molecule.

"Pharmaceutically acceptable" refers to a generally non-toxic, inert, and/or physiologically compatible composition or component of a composition.

A "pharmaceutical excipient" or "excipient" comprises a material such as an adjuvant, a carrier, pH-adjusting and buffering agents, tonicity adjusting agents, wetting agents, preservatives, and the like. A "pharmaceutical excipient" is an excipient which is pharmaceutically acceptable.

The term "motif" refers to a pattern of residues in an amino acid sequence of defined length, for example, a peptide of less than about 15 amino acid residues in length, or less than about 13 amino acid residues in length, for example, from about 8 to about 13 amino acid residues (e.g., 8, 9, 10, 11, 12, or 13) for a class I HLA motif and from about 6 to about 25 amino acid residues (e.g., 6, 7, 8, 9, 10, 11, 12, 13, 14, 15, 16, 17, 18, 19, 20, 21, 22, 23, 24, or 25) for a class II HLA motif, which is recognized by a particular HLA molecule. Motifs are typically different for each HLA protein encoded by a given human HLA allele. These motifs differ in their pattern of the primary and secondary anchor residues. In some cases, an MHC class I motif identifies a peptide of 9, 10, or 11 amino acid residues in length.

A "supermotif" is a peptide binding specificity shared by HLA molecules encoded by two or more HLA alleles. In some cases, a supermotif-bearing peptide described herein is recognized with high or intermediate affinity (as defined herein) by two or more HLA antigens.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring.

According to the disclosure, the term "vaccine" relates to a pharmaceutical preparation (pharmaceutical composition) or product that upon administration induces an immune response, for example, a cellular or humoral immune response, which recognizes and attacks a pathogen or a diseased cell such as a cancer cell. A vaccine may be used for the prevention or treatment of a disease. The term "individualized cancer vaccine" or "personalized cancer vaccine" concerns a particular cancer patient and means that a cancer vaccine is adapted to the needs or special circumstances of an individual cancer patient.

A "protective immune response" or "therapeutic immune response" refers to a CTL and/or an HTL response to an antigen derived from an pathogenic antigen (e.g., a tumor antigen), which in some way prevents or at least partially arrests disease symptoms, side effects or progression. The immune response can also include an antibody response which has been facilitated by the stimulation of helper T cells.

"Antigen processing" or "processing" refers to the degradation of a polypeptide or antigen into procession products, which are fragments of said polypeptide or antigen (e.g., the degradation of a polypeptide into peptides) and the association of one or more of these fragments (e.g., via binding) with MHC molecules for presentation by cells, for example, antigen presenting cells, to specific T cells.

"Antigen presenting cells" (APC) are cells which present peptide fragments of protein antigens in association with MHC molecules on their cell surface. Some APCs may activate antigen specific T cells. Professional antigen-presenting cells are very efficient at internalizing antigen, either by phagocytosis or by receptor-mediated endocytosis, and then displaying a fragment of the antigen, bound to a class II MHC molecule, on their membrane. The T cell recognizes and interacts with the antigen-class II MHC molecule complex on the membrane of the antigen presenting cell. An additional co-stimulatory signal is then produced by the antigen presenting cell, leading to activation of the T cell. The expression of co-stimulatory molecules is a defining feature of professional antigen-presenting cells.

The main types of professional antigen-presenting cells are dendritic cells, which have the broadest range of antigen presentation, and are probably the most important antigen presenting cells, macrophages, B-cells, and certain activated epithelial cells.

Dendritic cells (DCs) are leukocyte populations that present antigens captured in peripheral tissues to T cells via both MHC class II and I antigen presentation pathways. It is well known that dendritic cells are potent inducers of immune responses and the activation of these cells is a critical step for the induction of antitumoral immunity.

Dendritic cells are conveniently categorized as "immature" and "mature" cells, which can be used as a simple way to discriminate between two well characterized phenotypes. However, this nomenclature should not be construed to exclude all possible intermediate stages of differentiation.

Immature dendritic cells are characterized as antigen presenting cells with a high capacity for antigen uptake and processing, which correlates with the high expression of Fey receptor and mannose receptor. The mature phenotype is typically characterized by a lower expression of these markers, but a high expression of cell surface molecules responsible for T cell activation such as class I and class II MHC, adhesion molecules (e. g. CD54 and CD1 1) and costimulatory molecules (e. g., CD40, CD80, CD86 and 4-1 BB).

The term "residue" refers to an amino acid residue or amino acid mimetic residue incorporated into a peptide or protein by an amide bond or amide bond mimetic, or nucleic acid (DNA or RNA) that encodes the amino acid or amino acid mimetic.

The nomenclature used to describe peptides or proteins follows the conventional practice wherein the amino group is presented to the left (the amino- or N-terminus) and the carboxyl group to the right (the carboxy- or C-terminus) of each amino acid residue. When amino acid residue positions are referred to in a peptide epitope they are numbered in an amino to carboxyl direction with position one being the residue located at the amino terminal end of the epitope, or the peptide or protein of which it can be a part.

In the formulae representing selected specific cases of the present disclosure, the amino- and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acid residues having D-forms is represented by a lower case single letter or a lower case three letter symbol. However, when three letter symbols or full names are used without capitals, they can refer to L amino acid residues. Glycine has no asymmetric carbon atom and is simply referred to as "Gly" or "G". The amino acid sequences of peptides set forth herein are generally designated using the standard single letter symbol. (A, Alanine; C, Cysteine; D, Aspartic Acid; E, Glutamic Acid; F, Phenylalanine; G, Glycine; H, Histidine; I, Isoleucine; K, Lysine; L, Leucine; M, Methionine; N, Asparagine; P, Proline; Q, Glutamine; R, Arginine; S, Serine; T, Threonine; V, Valine; W, Tryptophan; and Y, Tyrosine.)

The terms "polynucleotide" and "nucleic acid" are used interchangeably herein and refer to polymers of nucleotides of any length, and include DNA and RNA, for example, mRNA. The nucleotides can be deoxyribonucleotides, ribonucleotides, modified nucleotides or bases, and/or their analogs, or any substrate that can be incorporated into a polymer by DNA or RNA polymerase. In some cases, the polynucleotide and nucleic acid can be in vitro transcribed mRNA. In some cases, the polynucleotide that is administered using the methods of the disclosure is mRNA.

The terms "identical" or percent "identity" in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software that can be used to obtain alignments of amino acid or nucleotide sequences are well-known in the art. These include, but are not limited to, BLAST, ALIGN, Megalign, BestFit, GCG Wisconsin Package, and variations thereof. In some cases, two nucleic acids or polypeptides described herein are substantially identical, meaning they have at least 70%, at least 75%, at least 80%, at least 85%, at least 90%, and in some cases at least 95%, 96%, 97%, 98%, 99% nucleotide or amino acid residue identity, when compared and aligned for maximum correspondence, as measured using a sequence comparison algorithm or by visual inspection. In some cases, identity exists over a region of the sequences that is at least about 10, at least about 20, at least about 40-60 residues, at least about 60-80 residues in length or any integral value 2between. In some cases, identity exists over a longer region than 60-80 residues, such as at least about 80-100 residues, and in some cases the sequences are substantially identical over the full length of the sequences being compared, such as the coding region of a nucleotide sequence.

A "conservative amino acid substitution" is one in which one amino acid residue is replaced with another amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). For example, substitution of a phenylalanine for a tyrosine is a conservative substitution. Methods of identifying nucleotide and amino acid conservative substitutions which do not eliminate peptide function are well-known in the art.

The term "vector" as used herein means a construct, which is capable of delivering, and usually expressing, one or more gene(s) or sequence(s) of interest in a host cell. Examples of vectors include, but are not limited to, viral vectors, naked DNA or RNA expression vectors, plasmid, cosmid, or phage vectors, DNA or RNA expression vectors associated with cationic condensing agents, and DNA or RNA expression vectors encapsulated in liposomes.

A polypeptide, antibody, polynucleotide, vector, cell, or composition which is "isolated" is a polypeptide, antibody, polynucleotide, vector, cell, or composition which is in a form not found in nature. Isolated polypeptides, antibodies, polynucleotides, vectors, cells, or compositions include those which have been purified to a degree that they are no longer in a form in which they are found in nature. In some cases, a polypeptide, antibody, polynucleotide, vector, cell, or composition which is isolated is substantially pure. In some cases, an "isolated polynucleotide" encompasses a PCR or quantitative PCR reaction comprising the polynucleotide amplified in the PCR or quantitative PCR reaction.

The term "substantially pure" as used herein refers to material which is at least 50% pure (i.e., free from contaminants), at least 90% pure, at least 95% pure, at least 98% pure, or at least 99% pure.

The term "subject" refers to any animal (e.g., a mammal), including, but not limited to, humans, non-human primates, canines, felines, rodents, and the like, which is to be the recipient of a particular treatment. Typically, the terms "subject" and "patient" are used interchangeably herein in reference to a human subject.

The terms "effective amount" or "therapeutically effective amount" or "therapeutic effect" refer to an amount of a therapeutic effective to "treat" a disease or disorder in a subject or mammal. The therapeutically effective amount of a drug has a therapeutic effect and as such can prevent the development of a disease or disorder; slow down the development of a disease or disorder; slow down the progression of a disease or disorder; relieve to some extent one or more of the symptoms associated with a disease or disorder; reduce morbidity and mortality; improve quality of life; or a combination of such effects.

The terms "treating" or "treatment" or "to treat" or "alleviating" or "to alleviate" refer to both 1) therapeutic measures that cure, slow down, lessen symptoms of, and/or halt progression of a diagnosed pathologic condition or disorder and 2) prophylactic or preventative measures that prevent or slow the development of a targeted pathologic condition or disorder. Thus those in need of treatment include those already with the disorder; those prone to have the disorder; and those in whom the disorder is to be prevented.

As used in the present disclosure and claims, the singular forms "a", "an" and "the" include plural forms unless the context clearly dictates otherwise.

It is understood that terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention. Nothing herein is intended as a promise.

The term "and/or" as used in a phrase such as "A and/or B" herein is intended to include both A and B; A or B; A (alone); and B (alone). Likewise, the term "and/or" as used in a phrase such as "A, B, and/or C" is intended to encompass each of the following cases: A, B, and C; A, B, or C; A or C; A or B; B or C; A and C; A and B; B and C; A (alone); B (alone); and C (alone).

## II. Neoantigens

One of the critical barriers to developing curative and tumor-specific immunotherapy is the identification and selection of highly specific and restricted tumor antigens to avoid autoimmunity. Tumor neoantigens, which arise as a result of genetic change (e.g., inversions, translocations, deletions, missense mutations, splice site mutations, etc.) within malignant cells, represent the most tumor-specific class of antigens. Neoantigens have rarely been used in cancer vaccine or immunogenic compositions due to technical difficulties in identifying them, selecting optimized antigens, and producing neoantigens for use in a vaccine or immunogenic composition. These problems may be addressed by: identifying mutations in neoplasias/tumors which are present at the DNA level in tumor but not in matched germline samples from a high proportion of subjects having cancer; analyzing the identified mutations with one or more peptide-MHC binding prediction algorithms to generate a plurality of neoantigen T cell epitopes that are expressed within the neoplasia/tumor and that bind to a high proportion of patient HLA alleles; and synthesizing the plurality of neoantigenic peptides selected from the sets of all neoantigen peptides and predicted binding peptides for use in a cancer vaccine or immunogenic composition suitable for treating a high proportion of subjects having cancer.

For example, translating peptide sequencing information into a therapeutic vaccine may include prediction of mutated peptides that can bind to HLA molecules of a high proportion of individuals. Efficiently choosing which particular mutations to utilize as immunogen requires the ability to predict which mutated peptides would efficiently bind to a high proportion of patient's HLA alleles. Recently, neural network based learning approaches with validated binding and non-binding peptides have advanced the accuracy of prediction algorithms for the major HLA-A and -B alleles. However, even using advanced neural network-based algorithms to encode HLA-peptide binding rules, several factors limit the power to predict peptides presented on HLA alleles.

For example, translating peptide sequencing information into a therapeutic vaccine may include formulating the drug as a multi-epitope vaccine of long peptides. Targeting as many mutated epitopes as practically possible takes advantage of the enormous capacity of the immune system, prevents the opportunity for immunological escape by down-modulation of an immune targeted gene product, and compensates for the known inaccuracy of epitope prediction approaches. Synthetic peptides provide a useful means to prepare multiple immunogens efficiently and to rapidly translate identification of mutant epitopes to an effective vaccine. Peptides can be readily synthesized chemically and easily purified utilizing reagents free of contaminating bacteria or animal substances. The small size allows a clear focus on the mutated region of the protein and also reduces irrelevant antigenic competition from other components (unmutated protein or viral vector antigens).

For example, translating peptide sequencing information into a therapeutic vaccine may include a combination with a strong vaccine adjuvant. Effective vaccines may require a strong adjuvant to initiate an immune response. For example, poly-ICLC, an agonist of TLR3 and the RNA helicase-domains of MDA5 and RIG3, has shown several desirable properties for a vaccine adjuvant. These properties include the induction of local and systemic activation of immune cells in vivo, production of stimulatory chemokines and cytokines, and stimulation of antigen-presentation by DCs. Furthermore, poly-ICLC can induce durable CD4+ and CD8+ responses in humans. Importantly, striking similarities in the upregulation of transcriptional and signal transduction pathways were seen in subjects vaccinated with poly-ICLC and in volunteers who had received the highly effective, replication-competent yellow fever vaccine. Furthermore, >90% of ovarian carcinoma patients immunized with poly-ICLC in combination with a NYESO-1 peptide vaccine (in addition to Montanide) showed induction of CD4+ and CD8+ T cell, as well as antibody responses to the peptide in a recent phase 1 study. At the same time, poly-ICLC has been extensively tested in more than 25 clinical trials to date and exhibited a relatively benign toxicity profile.

Applicants have discovered mutational events in the following genes:

ABL1, AC011997, ACVR2A, AFP, AKT1, ALK, ALPPL2, ANAPC1, APC, ARID1A, AR, AR-v7, ASCL2, β2M, BRAF, BTK, C15ORF40, CDH1, CLDN6, CNOT1, CT45A5, CTAG1B, DCT, DKK4, EEF1B2, EEF1DP3, EGFR, EIF2B3, env, EPHB2, ERBB3, ESR1, ESRP1, FAM111B, FGFR3, FRG1B, GAGE1, GAGE10, GATA3, GBP3, HER2, IDH1, JAK1, KIT, KRAS, LMAN1, MABEB16, MAGEA1, MAGEA10, MAGEA4, MAGEA8, MAGEB17, MAGEB4, MAGEC1, MEK, MLANA, MLL2, MMP13, MSH3, MSH6, MYC, NDUFC2, NRAS, PAGE2, PAGE5, PDGFRa, PIK3CA, PMEL, pol protein, POLE, PTEN, RAC1, RBM27, RNF43, RPL22, RUNX1, SEC31A, SEC63, SF3B1, SLC35F5, SLC45A2, SMAP1, SMAP1, SPOP, TFAM, TGFBR2, THAP5, TP53, TTK, TYR, UBR5, VHL, XPOT an EEF1DP3:FRY fusion polypeptide, an EGFR:SEPT14 fusion polypeptide, an EGFRVIII deletion polypeptide, an EML4:ALK fusion polypeptide, an NDRG1:ERG fusion polypeptide, an AC011997.1:LRRC69 fusion polypeptide, a RUNX1(ex5)-RUNX1T1 fusion polypeptide, a TMPRSS2:ERG fusion polypeptide, a NAB:STAT6 fusion polypeptide, a NDRG1:ERG fusion polypeptide, a PML:RARA fusion polypeptide, a PPP1R1B:STARD3 fusion polypeptide, a MAD1L1:MAFK fusion polypeptide, a FGFR3:TAC fusion polypeptide, a FGFR3:TACC3 fusion polypeptide, a BCR:ABL fusion polypeptide, a C11orf95:RELA fusion polypeptide, a CBFB:MYH11 fusion polypeptide, a CBFB:MYH11 fusion polypeptide, a CD74:ROS1 fusion polypeptide, a CD74:ROS1 fusion polypeptide, ERVE-4: protease, ERVE-4: reverse transcriptase, ERVE-4: reverse transcriptase, ERVE-4: unknown, ERVH-2 matrix protein, ERVH-2: gag, ERVH-2: retroviral matrix, ERVH48-1: coat protein, ERVH48-1: syncytin, ERVI-1 envelope protein, ERVK-5 gag, ERVK-5 env, ERVK-5 pol, EBV A73, EBV BALF3, EBV BALF4, EBV BALF5, EBV BARF0, EBV LF2, EBV RPMS1, HPV-16, HPV-16 E7, and HPV-16 E6.

In some cases, a neoantigen described herein is due to a mutational event in β2M, BTK, EGFR, GATA3, KRAS, MLL2, a TMPRSS2:ERG fusion polypeptide, or TP53.

Neoantigen polypeptides

In aspects, the disclosure provides isolated peptides that comprise a tumor specific mutation from Table 1 or 2. These peptides and polypeptides are referred to herein as "neoantigenic peptides" or "neoantigenic polypeptides". The term "peptide" is used interchangeably with "mutant peptide" and "neoantigenic peptide" in the present specification to designate a series of residues, typically L-amino acids, connected one to the other, typically by peptide bonds between the α-amino and carboxyl groups of adjacent amino acids. Similarly, the term "polypeptide" is used interchangeably with "mutant polypeptide" and "neoantigenic polypeptide" in the present specification to designate a series of residues, e.g., L-amino acids, connected one to the other, typically by peptide bonds between the α-amino and carboxyl groups of adjacent amino acids. The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

In some cases, sequencing methods are used to identify tumor specific mutations. Any suitable sequencing method can be used according to the disclosure, for example, Next Generation Sequencing (NGS) technologies. Third Generation Sequencing methods might substitute for the NGS technology in the future to speed up the sequencing step of the method. For clarification purposes: the terms "Next Generation Sequencing" or "NGS" in the context of the present disclosure mean all novel high throughput sequencing technologies which, in contrast to the "conventional" sequencing methodology known as Sanger chemistry, read nucleic acid templates randomly in parallel along the entire genome by breaking the entire genome into small pieces. Such NGS technologies (also known as massively parallel sequencing technologies) are able to deliver nucleic acid sequence information of a whole genome, exome, transcriptome (all transcribed sequences of a genome) or methylome (all methylated sequences of a genome) in very short time periods, e.g. within 1-2 weeks, for example, within 1-7 days or within less than 24 hours and allow, in principle, single cell sequencing approaches. Multiple NGS platforms which are commercially available or which are mentioned in the literature can be used in the context of the invention e.g. those described in detail in WO 2012/159643.

In certain cases a neoantigenic peptide described herein molecule can comprise, but is not limited to, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 60, about 70, about 80, about 90, about 100, about 110, about 120 or greater amino acid residues, and any range derivable therein. In specific cases, a neoantigenic peptide molecule is equal to or less than 100 amino acids.

In some cases, neoantigenic peptides and polypeptides described herein for MHC Class I are 13 residues or less in length and usually consist of between about 8 and about 11 residues, particularly 9 or 10 residues. In some cases, neoantigenic peptides and polypeptides described herein for MHC Class II are 9-24 residues in length.

A longer neoantigenic peptide can be designed in several ways. In some cases, when HLA-binding peptides are predicted or known, a longer neoantigenic peptide could consist of (1) individual binding peptides with extensions of 2-5 amino acids toward the N- and C-terminus of each corresponding gene product; or (2) a concatenation of some or all of the binding peptides with extended sequences for each. In other cases, when sequencing reveals a long (>10 residues) neoepitope sequence present in the tumor (e.g. due to a frameshift, read-through or intron inclusion that leads to a novel peptide sequence), a longer neoantigenic peptide could consist of the entire stretch of novel tumor-specific amino acids as either a single longer peptide or several overlapping longer peptides. In some cases, use of a longer peptide is presumed to allow for endogenous processing by patient cells and can lead to more effective antigen presentation and induction of T cell responses. In some cases, two or more peptides can be used, where the peptides overlap and are tiled over the long neoantigenic peptide.

In some cases, the neoantigenic peptides and polypeptides bind an HLA protein (e.g., HLA class I or HLA class II). In specific cases the neoantigenic peptides and polypeptides bind an HLA protein with greater affinity than the corresponding wild-type peptide. In specific cases the neoantigenic peptide or polypeptide has an $IC_{50}$ of at least less than 5000 nM, at least less than 500 nM, at least less than 100 nM, at least less than 50 nM or less.

In some cases, the neoantigenic peptides can be from about 8 and about 50 amino acid residues in length, or from about 8 and about 30, from about 8 and about 20, from about 8 and about 18, from about 8 and about 15, or from about 8 and about 12 amino acid residues in length. In some cases, the neoantigenic peptides can be from about 8 and about 500 amino acid residues in length, or from about 8 and about 450, from about 8 and about 400, from about 8 and about 350, from about 8 and about 300, from about 8 and about 250, from about 8 and about 200, from about 8 and about 150, from about 8 and about 100, from about 8 and about 50, or from about 8 and about 30 amino acid residues in length.

In some cases, the neoantigenic peptides can be at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more amino acid residues in length. In some cases, the neoantigenic peptides can be at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 70, 80, 90, 100, 150, 200, 250, 300, 350, 400, 450, 500 or more amino acid residues in length. In some cases, the neoantigenic peptides can be at most 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or less amino acid residues in length. In some cases, the neoantigenic peptides can be at most 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 70, 80, 90, 100, 150, 200, 250, 300, 350, 400, 450, 500, or less amino acid residues in length.

In some cases, the neoantigenic peptides has a total length of at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 40, at least 50, at least 60, at least 70, at least 80, at least 90, at least 100, at least 150, at least 200, at least 250, at least 300, at least 350, at least 400, at least 450, or at least 500 amino acids.

In some cases, the neoantigenic peptides has a total length of at most 8, at most 9, at most 10, at most 11, at most 12, at most 13, at most 14, at most 15, at most 16, at most 17, at most 18, at most 19, at most 20, at most 21, at most 22, at most 23, at most 24, at most 25, at most 26, at most 27, at most 28, at most 29, at most 30, at most 40, at most 50, at most 60, at most 70, at most 80, at most 90, at most 100, at most 150, at most 200, at most 250, at most 300, at most 350, at most 400, at most 450, or at most 500 amino acids.

In some cases, the neoantigenic peptides can have a pI value of about 0.5 and about 12, about 2 and about 10, or about 4 and about 8. In some cases, the neoantigenic peptides can have a pI value of at least 4.5, 5, 5.5, 6, 6.5, 7, 7.5, or more. In some cases, the neoantigenic peptides can have a pI value of at most 4.5, 5, 5.5, 6, 6.5, 7, 7.5, or less.

In some cases, the neoantigenic peptides can have an HLA binding affinity of between about 1pM and about 1mM, about 100pM and about 500$\mu$M, about 500pM and about 10$\mu$M, about 1nM and about 1$\mu$M, or about 10nM and about 1$\mu$M In some cases, the neoantigenic peptides can have an HLA binding affinity of at least 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 700, 800, 900 $\mu$M, or more. In some cases, the neoantigenic peptides can have an HLA binding affinity of at most 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 700, 800, 900 $\mu$M.

In some cases, a neoantigenic peptide described herein can comprise carriers such as those well known in the art, e.g., thyroglobulin, albumins such as human serum albumin, tetanus toxoid, polyamino acid residues such as poly L-lysine, poly L-glutamic acid, influenza virus proteins, hepatitis B virus core protein, and the like.

In some cases, a neoantigenic peptide described herein can be modified by terminal-$NH_2$ acylation, e.g., by alkanoyl ($C_1$-$C_{20}$) or thioglycolyl acetylation, terminal-carboxyl amidation, e.g., ammonia, methylamine, etc. In some cases these modifications can provide sites for linking to a support or other molecule.

In some cases, a neoantigenic peptide described herein can contain modifications such as but not limited to glycosylation, side chain oxidation, biotinylation, phosphorylation, addition of a surface active material, e.g. a lipid, or can be chemically modified, e.g., acetylation, etc. Moreover, bonds in the peptide can be other than peptide bonds, e.g., covalent bonds, ester or ether bonds, disulfide bonds, hydrogen bonds, ionic bonds, etc.

In some cases, a neoantigenic peptide described herein can contain substitutions to modify a physical property (e.g., stability or solubility) of the resulting peptide. For example, neoantigenic peptides can be modified by the substitution of a cysteine (C) with $\alpha$-amino butyric acid ("B"). Due to its chemical nature, cysteine has the propensity to form disulfide bridges and sufficiently alter the peptide structurally so as to reduce binding capacity. Substituting $\alpha$-amino butyric acid for C not only alleviates this problem, but actually improves binding and crossbinding capability in certain instances. Substitution of cysteine with $\alpha$-amino butyric acid can occur at any residue of aneoantigenic peptide, e.g., at either anchor or non-anchor positions of an epitope or analog within a peptide, or at other positions of a peptide.

In some cases, a neoantigenic peptide described herein can comprise amino acid mimetics or unnatural amino acid residues, e.g. D- or L-naphylalanine; D- or L-phenylglycine; D- or L-2-thieneylalanine; D- or L-1, -2, 3-, or 4-pyreneylalanine; D- or L-3 thieneylalanine; D- or L-(2-pyridinyl)-alanine; D- or L-(3-pyridinyl)-alanine; D- or L-(2-pyrazinyl)-alanine; D- or L-(4-isopropyl)-phenylglycine; D-(trifluoromethyl)-phenylglycine; D-(trifluoro-methyl)-phenylalanine; D-.rho.-fluorophenylalanine; D- or L-.rho.-biphenyl-phenylalanine; D- or L-.rho.-methoxybiphenylphenylalanine; D- or L-2-indole(allyl)alanines; and, D- or L-alkylalanines, where the alkyl group can be a substituted or unsubstituted methyl, ethyl, propyl, hexyl, butyl, pentyl, isopropyl, iso-butyl, sec-isotyl, iso-pentyl, or a non-acidic amino acid residues. Aromatic rings of a non-natural amino acid include, e.g., thiazolyl, thiophenyl, pyrazolyl, benzimidazolyl, naphthyl, furanyl, pyrrolyl, and pyridyl aromatic rings. Modified peptides that have various amino acid mimetics or unnatural amino acid residues are particularly useful, as they tend to manifest increased stability in vivo. Such peptides can also possess improved shelf-life or manufacturing properties.

Peptide stability can be assayed in a number of ways. For instance, peptidases and various biological media, such as human plasma and serum, have been used to test stability. See, e.g., Verhoef, et al., Eur. J. Drug Metab. Pharmacokinetics 11:291 (1986). Half-life of the peptides described herein is conveniently determined using a 25% human serum (v/v) assay. The protocol is as follows: pooled human serum (Type AB, non-heat inactivated) is dilapidated by centrifugation before use. The serum is then diluted to 25% with RPMI-1640 or another suitable tissue culture medium. At predetermined time intervals, a small amount of reaction solution is removed and added to either 6% aqueous trichloroacetic acid (TCA) or ethanol. The cloudy reaction sample is cooled (4°C) for 15 minutes and then spun to pellet the precipitated serum proteins. The presence of the peptides is then determined by reversed-phase HPLC using stability-specific chromatography conditions.

In some cases, a neoantigenic peptide described herein can be in solution, lyophylized, or can be in crystal form.

In some cases, a neoantigenic peptide described herein can be prepared synthetically, by recombinant DNA technology or chemical synthesis, or can be isolated from natural sources such as native tumors or pathogenic organisms. Epitopes can be synthesized individually or joined directly or indirectly in a peptide. Although a neoantigenic peptide described herein will be substantially free of other naturally occurring host cell proteins and fragments thereof, in some cases the peptide can be synthetically conjugated to be joined to native fragments or particles.

In some cases, a neoantigenic peptide described herein can be prepared in a wide variety of ways. In some cases, the peptides can be synthesized in solution or on a solid support according to conventional techniques. Various automatic synthesizers are commercially available and can be used according to known protocols. (See, for example, Stewart & Young, SOLID PHASE PEPTIDE SYNTHESIS, 2D. ED., Pierce Chemical Co., 1984). Further, individual peptides can be joined using chemical ligation to produce larger peptides.

Alternatively, recombinant DNA technology can be employed wherein a nucleotide sequence which encodes a peptide inserted into an expression vector, transformed or transfected into an appropriate host cell and cultivated under conditions suitable for expression. These procedures are generally known in the art, as described generally in Sambrook et al., MOLECULAR CLONING, A LABORATORY MANUAL, Cold Spring Harbor Press, Cold Spring Harbor, N.Y. (1989). Thus, recombinant peptides, which comprise or consist of one or more epitopes described herein, can be used to present the appropriate T cell epitope.

In one aspect, the disclosure described herein also provides compositions comprising one, at least two, or more than two neoantigenic peptides. In some cases a composition described herein contains at least two distinct peptides. In some cases, the at least two distinct peptides are derived from the same polypeptide. By distinct polypeptides is meant that the peptide vary by length, amino acid sequence or both. The peptides are derived from any polypeptide known to or have been found to contain a tumor specific mutation.

In some cases, the isolated neoantigenic peptide is encoded by a gene with a point mutation resulting in an amino acid substitution of the native peptide.

In some cases, the isolated neoantigenic peptide is encoded by a ABL gene. In related cases, $A_xB_yC_z$ is VA DGLITTLHYPAPKRNKPTVYGVSPNYDKWEMERTDITMKHKLGGGQYGKVYEGVWKKYSLTV AVKTLKEDTME-VEEFLKEAAVMKEIKHPNLVQLLGVC, VADGLITTLHYPAPKRNKPTVYGVSPNYDKWEMERTDITMKHKLGGGQYGV VYEGVWKKYSLTV AVKTLKEDTMEVEEFLKEAAVMKEIKHPNLVQLLGVC, LLGVCTREPPFYIITEFMTYGNLLDYLR ECNRQEVNAVVLLYMATQISSATEYLEKKNFIHRDLAARN CLVGENHLVKVADFGLSRLMTGDTYTAHAGAKF, SLTVA VKTLKEDTMEVEEFLKEAAVMKEIKHPNLVQLLGVCTREPPFYIIIEFMTYGNLLDYLRECNR QEVNAVVLLYMATQIS-SAMEYLEKKNFIHRDLA, or STVADGLITTLHYPAPKRNKPTVYGVSPNYDKWEMERTDITMKHKLGGGQHGEVYEGV WKKYSLT VAVKTLKEDTMEVEEFLKEAAVMKEIKHPNLVQLLG.

In some cases, the isolated neoantigenic peptide is encoded by a ALK gene. In related cases, $A_xB_yC_z$ is SS LAMLDLLHVARDIACGCQYLEENHFIHRDIAARNCLLTCPGPGRVAKIADFGMARDIYRASYYRK GGCAMLPVKWMP-PEAFMEGIFTSKTDTWSFGVLL or QVAVKTLPEVCSEQDELDFLMEALIISKFNHQNIVRCIGVSLQSLPRFILMELMAG GDLKSFLRETRPR PSQPSSLAMLDLLHVARDIACGCQYLEENHFI.

In some cases, the isolated neoantigenic peptide is encoded by a BRAF gene. In related cases, $A_xB_yC_z$ is MIKLIDIARQTAQGMDYLHAKSIIHRDLKSNNIFLHEDLTVKIGDFGLATEKSRWSGSHQFEQLSGSIL WMAPE-VIRMQDKNPYSFQSDVYAFGIVLYELM. In some related cases, the neoantigenic peptide is not DFGLATEKSR or FGLATEKSRW.

In some cases, the isolated neoantigenic peptide is encoded by a BTK gene. In related cases, $A_xB_yC_z$ is MI KEGSMSEDEFIEEAKVMMNLSHEKLVQLYGVCTKQRPIFIITEYMANGSLLNYLREMRHRFQTQQ LLEMCKDVCEA-MEYLESKQFLHRDLAARNCLVND.

In some cases, the isolated neoantigenic peptide is encoded by a EEF1B2 gene. In related cases, $A_xB_yC_z$ is MGFGDLKSPAGLQVLNDYLADKSYIEGYVPSQADVAVFEAVSGPPPADLCHALRWYNHIKSYEKEK ASLPGVKKALG-KYGPADVEDTTGSGAT. In some related cases, the neoantigenic peptide is not EAVSGPPPA, FEAVSGPPP or FEAVSGPPPA.

In some cases, the isolated neoantigenic peptide is encoded by a EGFR gene. In related cases, $A_xB_yC_z$ is MSLNITSLGLRSLKEISDGDVIISGNKNLCYANTINWKKLFGTSGQKTKIIRNRGENSCKATGQVCHAL CSPEGCWG-PEPRDCVSCRNVSRGRECVDKCNLL or IPVAIKELREATSPKANKEILDEAYVMASVDNPHVCRLLGICLTSTVQLIMQ LMPFGCLLDYVREHKD NIGSQYLLNWCVQIAKGMNYLEDRRLVHRDLAA.

In some cases, the isolated neoantigenic peptide is encoded by a ERBB3 gene. In related cases, $A_xB_yC_z$ is EFSTLPLPNLRMVRGTQVYDGKF. In some related cases, the neoantigenic peptide is not RMVRGTQVY, LPLPNLRMV, LRMVRGTQV, TLPLPNLRMV, NLRMVRGTQV, or LRMVRGTQVY.

In some cases, the isolated neoantigenic peptide is encoded by a ESR1 gene. In related cases, $A_xB_yC_z$ is H LMAKAGLTLQQQHQRLAQLLLILSHIRHMSNKGMEHLYSMKCKNVVPLYGLLLEMLDAHRLHAP TSRGGAS-VEETDQSHLATAGSTSSHSLQKYYITGEA, NQGKCVEGMVEIFDMLLATSSRFRMMNLQGEEFVCLKSIILLNSGVYTF LPSTLKSLEEKDHIHRVLD KITDTLIHLMAKAGLTLQQQHQRLAQLLLILSH, IHLMAKAGLTLQQQHQRLAQLLLILSHIR HMSNKGMEHLYSMKCKNVVPLCDLLLEMLDAHRLHAP TSRGGASVEETDQSHLATAGSTSSHSLQKYYITGE, IHLM AKAGLTLQQQHQRLAQLLLILSHIRHMSNKGMEHLYSMKCKNVVPLNDLLLEMLDAHRLHAP TSRGGASVEETDQSH-LATAGSTSSHSLQKYYITGE, or IHLMAKAGLTLQQQHQRLAQLLLILSHIRHMSNKGMEHLYSMKCKNVVPLSDLLLEM LDAHRLHAP TSRGGASVEETDQSHLATAGSTSSHSLQKYYITGE.

In some cases, the isolated neoantigenic peptide is encoded by a FGFR3 gene. In related cases, $A_xB_yC_z$ is

HRIGGIKLRHQQWSLVMESVVPSDRGNYTCVVENKFGSIRQTYTLDVLERCPHRPILQAGLPANQTA VLGSDVEFHCKVYSDAQPHIQWLKHVEVNGSKVG. In some related cases, the neoantigenic peptide is not DVLERCPHR, LERCPHRPI, CPHRPILQA, or LERCPHRPIL.

In some cases, the isolated neoantigenic peptide is encoded by a FRG1B gene. In related cases, $A_xB_yC_z$ is AVKLSDSRIALKSGYGKYLGINSDELVGHSDAIGPREQWEPVFQNGKMALSASNSCFIRCNEAGDIEA KSKTAGEEE-MIKIRSCAEKETKKKDDIPEEDKG. In some related cases, the neoantigenic peptide is not SASNSCFIR, LSASNSCFI, ALSASNSCF or FQNGKMALSA.

In some cases, the isolated neoantigenic peptide is encoded by a HER2 gene. In related cases, $A_xB_yC_z$ is GSGAFGTVYKGIWIPDGENVKIPVAIKVLRENTSPKANKEILDEAYVMAGLGSPYVSRLLGICLTSTV QLVTQLMPYGCLLDHVRENRGRLGSQDLLNWCM.

In some cases, the isolated neoantigenic peptide is encoded by a IDH1 gene. In related cases, $A_xB_yC_z$ is R VEEFKLKQMWKSPNGTIRNILGGTVFREAIICKNIPRLVSGWVKPIIIGHHAYGDQYRATDFVVPGP GKVEI-TYTPSDGTQKVTYLVHNFEEGGGVAMGM, RVEEFKLKQMWKSPNGTIRNILGGTVFREAIICKNIPRLVSGWVKPIIIG CHAYGDQYRATDFVVPGPG KVEITYTPSDGTQKVTYLVHNFEEGGGVAMGM, RVEEFKLKQMWKSPNGTIRNILGG TVFREAIICKNIPRLVSGWVKPIIIGGHAYGDQYRATDFVVPGP GKVEITYTPSDGTQKVTYLVHNFEEGGGVAMGM, or RVEEFKLKQMWKSPNGTIRNILGGTVFREAIICKNIPRLVSGWVKPIIIGSHAYGDQYRATDFVVPGPG KVEI-TYTPSDGTQKVTYLVHNFEEGGGVAMGM. In some related cases, the neoantigenic peptide is not PIIIGHHAY, GHHAYGDQY, KPIIIGHHAY, IGHHAYGDQY, PIIIGCHAY, GCHAYGDQY, KPIIIGCHAY, IGCHAYGDQY, PIIIGGHAY, GGHAYGDQY, KPIIIGGHAY, or IGGHAYGDQY.

In some cases, the isolated neoantigenic peptide is encoded by a KIT gene. In related cases, $A_xB_yC_z$ is VEA TAYGLIKSDAAMTVAVKMLKPSAHLTEREALMSELKVLSYLGNHMNIANLLGACTIGGPTLVIT EYC-CYGDLLNFLRRKRDSFICSKQEDHAEAALYK or VEATAYGLIKSDAAMTVAVKMLKPSAHLTEREALMSELKVLSYLGN HMNIANLLGACTIGGPTLVIT EYCCYGDLLNFLRRKRDSFICSKQEDHAEAALYK.

In some cases, the isolated neoantigenic peptide is MEK gene. In related cases, $A_xB_yC_z$ is ISELGAGNGGV VFKVSHKPSGLVMARKLIHLEIKPAIRNQIIRELQVLHESNSPYIVGFYGAFYSDGEIS ICMEHMDGGSLDQVLKKAGRI-PEQILGKVSI or LGAGNGGVVFKVSHKPSGLVMARKLIHLEIKPAIRNQIIRELQVLHECNSLYIVGFYGAFYSDGEISIC MEHMDGGSLDQVLKKAGRIPEQILGKVSIAVI.

In some cases, the isolated neoantigenic peptide is encoded by a MYC gene. In related cases, $A_xB_yC_z$ is M PLNVSFTNRNYDLDYDSVQPYFYCDEEENFYQQQQQSDLQPPAPSEDIWKKFELLPTPPLSPSRRSG LCSPSY-VAVTPFSLRGDNDGG, FTNRNYDLDYDSVQPYFYCDEEENFYQQQQQSELQPPAPSEDIWKKFELLSTPPLSPSRRS GLCSPSY VAVTPFSLRGDNDGGGGSFSTADQLEMVTELLG, or TNRNYDLDYDSVQPYFYCDEEENFYQQQQQSEL QPPAPSEDIWKKFELLPIPPLSPSRRSGLCSPSYVA VTPFSLRGDNDGGGGSFSTADQLEMVTELLGG.

In some cases, the isolated neoantigenic peptide is encoded by a PDGFRa gene. In related cases, $A_xB_yC_z$ is VAVKMLKPTARSSEKQALMSELKIMTHLGPHLNIVNLLGACTKSGPIYIIIEYCFYGDLVNYLHKNRD SFLSHHPEKPK-KELDIFGLNPADESTRSYVILS.

In some cases, the isolated neoantigenic peptide is encoded by a PIK3CA gene. In related cases, $A_xB_yC_z$ is IEEHANWSVSREAGFSYSHAGLSNRLARDNELRENDKEQLKAISTRDPLSKITEQEKDFLWSHRHYC VTI-PEILPKLLLSVKWNSRDEVAQMYCLVKDWPP, HANWSVSREAGFSYSHAGLSNRLARDNELRENDKEQLKAISTRDP LSEITKQEKDFLWSHRHYCVTI PEILPKLLLSVKWNSRDEVAQMYCLVKDWPPIKP, or LFINLFSMMLGSGMPELQSF DDIAYIRKTLALDKTEQEALEYFMKQMNDARHGGWTTKMDWIFHTI KQHALN. In some related cases, the neoantigenic peptide is not ISTRDPLSK, STRDPLSKI, LSKITEQEK, AISTRDPLSK, SKITEQEKDF, SEITKQEKDF, KQEKDFLWSH, FMKQMNDAR, KQMNDARHG, RHGGWTTKM, YFMKQMNDAR, FMKQMNDARH, KQMNDARHGG, QMNDARHGGW, or ARHGGWTTKM.

In some cases, the isolated neoantigenic peptide is encoded by a POLE gene. In related cases, $A_xB_yC_z$ is QRGGVITDEEETSKKIADQLDNIVDMREYDVPYHIRLSIDIETTKLPLKFRDAETDQIMMISYMIDGQG YLITNREIVSE-DIEDFEFTPKPEYEGPFCVFN. In some related cases, the neoantigenic peptide is not TTKLPLKFR, RDAETDQIM, KFRDAETDQI, ETTKLPLKFR, or RDAETDQIMM.

In some cases, the isolated neoantigenic peptide is encoded by a PTEN gene. In related cases, $A_xB_yC_z$ is KFNCRVAQYPFEDHNPPQLELIKPFCEDLDQWLSEDDNHVAAIHCKAGKGQTGVMICAYLLHRGKF LKAQEALDFY-GEVRTRDKKGVTIPSQRRYVYYYSY. In some related cases, the neoantigenic peptide is not QTGVMICAY, GKGQTGVMI, GQTGVMICAY, or KAGKGQTGVM.

In some cases, the isolated neoantigenic peptide is encoded by a RAC1 gene. In related cases, $A_xB_yC_z$ is MQAIKCVVVGDGAVGKTCLLISYTTNAFSGEYIPTVFDNYSANVMVDGKPVNLGLWDTAGQEDYD RLRPLSYPQTV-GET. In some related cases, the neoantigenic peptide is not TTNAFSGEY, FSGEYIPTV, SGEYIPTVF, YTTNAFSGEY, TTNAFSGEYI, or FSGEYIPTVF.

In some cases, the isolated neoantigenic peptide is encoded by a TP53 gene. In related cases, $A_xB_yC_z$ is IR VEGENLRVEYLDDRNTFRHSVVVPYEPPEVGSDCTTIHYNYMCNSSCMGSMNRRPILTIITLEDSSG NLLGRNSFEVRVCACPGRDRRTEEENLRKKGEP, TYSPALNKMFCQLAKTCPVQLWVDSTPPPGTRVRAMAIYKQS

QHMTEVVRHCPHHERCSDSDGLAP PQHLIRVEGNLRVEYLDDRNTFRHSVVVPYEPPEV, EGNLRVEYLDDRNTFR HSVVVPYEPPEVGSDCTTIHYNYMCNSSCMGGMNQRPILTIITLEDSSGNLL GRNSFEVRVCACPGRDRR- TEEEENLRKKGEPHHE, EGNLRVEYLDDRNTFRHSVVVPYEPPEVGSDCTTIHYNYMCNSSCMGGMNWRPILTIITLED SSGNLL GRNSFEVRVCACPGRDRRTEEEENLRKKGEPHHE, or PEVGSDCTTIHYNYMCNSSCMGGMNRRPILTIITL EDSSGNLLGRNSFEVCVCACPGRDRRTEEEENLR KKGEPHHELPPGSTKRALPNNTSSSPQPKKKPL. In some related cases, the neoantigenic peptide is not SSCMGSMNR, GSMNRRPIL, MGSMNRRPI, CNSSCMGSM, SMNRRPILTI, SSCMGSMNRR, NSSCMGSMNR, MGSMNRRPIL, MCNSSCMGS, CMGSMNRRPI, TEVVRHCPH, VVRHCPHHER, SQHMTEVVRH, MNQRPILTI, NQRPILTII, CMGGMNQRPI, GMNQRPILTI, SSCMGGMNQR, NQRPILTIIT, NWRPILTII, SSCMGGMNW, MGGMNWRPI, MNWRPILTI, CMGGMNWRPI, GMNWRPILTI, SSCMGGMNWR, MNWRPILTII, NSSCMGGMNW, NSFEVCVCA, EVCVCACPGR, or FEVCVCACPG.

In some cases, the isolated neoantigenic peptide is encoded by a gene with a frameshift mutation In some cases, the isolated neoantigenic peptide is encoded by a ACVR2A gene. In related cases, $A_xB_yC_z$ is GVEPC YGDKDKRRHCFATWKNISGSIEIVKQGCWLDDINCYDRTDCVEKKRQP or GVEPCYGDKDKRRHCFATWKNISGSIEI VKQGCWLDDINCYDRTDCVEKKTALKYIFVAVRAICVM KSFLIFRRWKSHSPLQIQLHLSHPITTSCSIPWCHLC.

In some cases, the isolated neoantigenic peptide is C15ORF40 gene. In related cases, $A_xB_yC_z$ is TAEAVNV AIAAPPSEGEANAELCRYLSKVLELRKSDVVLDKVGLALFFFFFETKSCSVAQAGVQWRS LGSLQPPPPGFKLFSCLS FLSSWDYRRMPPCLANFCIFNRDGVSPCWSGWS.

In some cases, the isolated neoantigenic peptide is encoded by a CNOT1 gene. In related cases, $A_xB_yC_z$ is LSVIIFFFVYIWHWALPLILNNHHICLMSSIILDCNSVRQSIMSVCFFFFSVIFSTRCLTDSRYPNICWFK or LSVIIFFFVYI WHWALPLILNNHHICLMSSIILDCNSVRQSIMSVCFFFFCYILNTMFDR.

In some cases, the isolated neoantigenic peptide is encoded by a EIF2B3 gene. In related cases, $A_xB_yC_z$ is VLVLSCDLITDVALHEVVDLFRAYDASLAMLMRKGQDSIEPVPGQKGKKKQWSSVTSLEWTAQERG CSSWLMKQTWMKSWSLRDPSYRSILEYVSTRVLWMPTSTV.

In some cases, the isolated neoantigenic peptide is encoded by a EPHB2 gene. In related cases, $A_xB_yC_z$ is SIQVMRAQMNQIQSVEGQPLARRPRATGRTKRCQPRDVTKKTCNSNDGKKREWEKRKQILGGGGK YKEYFLKRI- LIRKAMTVLAGDKKGLGRFMRCVQSETKAVSLQLPLGR.

In some cases, the isolated neoantigenic peptide is encoded by a ESRP1 gene. In related cases, $A_xB_yC_z$ is LDFLGEFATDIRTHGVHMVLNHQGRPSGDAFIQMKSADRAFMAAQKCHKKKHEGQIC or LDFLGEFATDIRTHGVHM VLNHQGRPSGDAFIQMKSADRAFMAAQKCHKKT.

In some cases, the isolated neoantigenic peptide is encoded by a FAM11B gene. In related cases, $A_xB_yC_z$ is GALCKDGRFRSDIGEFEWKLKEGHKKIYGKQSMVDEVSGKVLEMDISKKKHYNRKISIKKLNRMKV PLMKLITRV. In some related cases, the neoantigenic peptide is not KLNRMKVPL, PLMKLITRV, RMKVPLMKL, ISKKKHYNR, SKKKHYNRK, KHYNRKISI, HYNRKISIK, YNRKISIKK, KISIKKLNR, SIKKLNRMK, LNRMKVPLM, ISIKKLNRM, MKVPLMKLI, KLNRMKVPLM, RMKVPLMKLI, ISIKKLNRMK, ISKKKHYNRK, KHYNRKISIK, HYNRKISIKK, KISIKKLNRM, SIKKLNRMKV, LNRMKVPLMK, KVPLMKLITR, DISKKKHYNR, KKHYNRKISI, KKLNRMKVPL, or VPLMKLITRV.

In some cases, the isolated neoantigenic peptide is encoded by a GBP3 gene. In related cases, $A_xB_yC_z$ is RERAQLLEEQEKTLTSKLQEQARVLKERCQGESTQLQNEIQKLQKTLKKKPRDICRIS.

In some cases, the isolated neoantigenic peptide is encoded by a JAK1 gene. In related cases, $A_xB_yC_z$ is V NTLKEGKRLPCPPNCPDEVYQLMRKCWEFQPSNRTSFQNLIEGFEALLKTSN or CRPVTPSCKELADLMTRCMNYD PNQRPFFRAIMRDINKLEEQNPDIVSEKNQQLKWTPHILKSAS.

In some cases, the isolated neoantigenic peptide is encoded by a LMAN1 gene. In related cases, $A_xB_yC_z$ is DDHDVLSFLTFQLTEPGKEPPTPDKEISEKEKEKYQEEFEHFQQELDKKKRGIPEGPPRPPRAACGGNI or DDHDVLS FLTFQLTEPGKEPPTPDKEISEKEKEKYQEEFEHFQQELDKKKRNSRRATPTSKGSLRRKY LRV.

In some cases, the isolated neoantigenic peptide is encoded by a MSH3 gene. In related cases, $A_xB_yC_z$ is TKSTLIGEDVNPLIKLDDAVNVDEIMTDTSTSYLLCISENKENVRDKKKGQHFYWHCGSAACHRRGC V or LYTKSTLIG EDVNPLIKLDDAVNVDEIMTDTSTSYLLCISENKENVRDKKRATFLLALWECSLPQARL CLIVSRTLLLVQS.

In some cases, the isolated neoantigenic peptide is encoded by a NDUFC2 gene. In related cases, $A_xB_yC_z$ is LPPPKLTDPRLLYIGFLGYCSGLIDNLIRRRPIATAGLHRQLLYITAFFFCWILSCKT, or SLPPPKLTDPRLLYIGFLGYCS GLIDNLIRRRPIATAGLHRQLLYITAFFLLDIIL.

In some cases, the isolated neoantigenic peptide is encoded by a RBM27 gene. In related cases, $A_xB_yC_z$ is NQSGGAGEDCQIFSTPGHPKMIYSSSNLKTPSKLCSGSKSHDVQEVLKKKTGSNEVTTRYEEKKTGSV RKANRMPKDVNIQVRKKQKHETRRKSKYNEDFERAWREDLTIKR.

In some cases, the isolated neoantigenic peptide is encoded by a RPL22 gene. In related cases, $A_xB_yC_z$ is MAPVKKLVVKGGKKKEASSEVHS or MAPVKKLVVKGGKKRSKF. In some related cases, the neoantigenic peptide is not VVKGGKKRSK or VKGGKKRSK

In some cases, the isolated neoantigenic peptide is encoded by a SEC31A gene. In related cases, $A_xB_yC_z$ is MPSHQGAEQQQQQQHHVFISQVVTEKEFLSRSDQLQQAVQSQGFINYCQKKN or MPSHQGAEQQQQQQHHVFISQVV

TEKEFLSRSDQLQQAVQSQGFINYCQKKLMLLRLNLRKMCGPF.

In some cases, the isolated neoantigenic peptide is encoded by a SEC63 gene. In related cases, $A_xB_yC_z$ is AEVFEKEQSICAAEEQPAEDGQGETNKNRTKGGWQQKSKGPKKTAKSKKKETFKKKTYTCAITTVK ATET-KAGKWSRWE or MAEVFEKEQSICAAEEQPAEDGQGETNKNRTKGGWQQKSKGPKKTAKSKKRNL.

In some cases, the isolated neoantigenic peptide is encoded by a SLC35F5 gene. In related cases, $A_xB_yC_z$ is NIMEIRQLPSSHALEAKLSRMSYPVKEQESILKTVGKLTATQVAKISFFFALCGFWQICHIKKHFQTHK LL.

In some cases, the isolated neoantigenic peptide is encoded by a SMAP1 gene. In related cases, $A_xB_yC_z$ is YEKKKYYDKNAIAITNISSSDAPLQPLVSSPSLQAAVDKNKLEKEKEKKKGREKERKGARKAGKTTY S or KYEKKKYY DKNAIAITNISSSDAPLQPLVSSPSLQAAVDKNKLEKEKEKKRKRKREKRSQKSRQNHL QLKSCRRKISNWSLKKV-PALKKLRSPLWIF. In some related cases, the neoantigenic peptide is not ALKKLRSPL, KISNWSLKK, SLKKVPALK, KLRSPLWIF, KKRKRKREK, RKREKRSQK, RSQKSRQNH, SQKSRQNHL, KSRQNHLQL, RRKISNWSL, RKISNWSLK, KVPALKKLR, HLQLKSCRR, WSLKKVPAL, RQNHLQLKS, KKVPALKKL, LKKLRSPLW, KKLRSPLWI, KISNWSLKKV, KSRQNHLQLK, SLKKVPALKK, WSLKKVPALK, KRKREKRSQK, RSQKSRQNHL, HLQLKSCRRK, RRKISNWSLK, CRRKISNWSL, NWSLKKVPAL, QKSRQNHLQL, RQNHLQLKSC, LQLKSCRRKI, ALKKLRSPLW, or KKLRSPLWI

In some cases, the isolated neoantigenic peptide is encoded by a TFAM gene. In related cases, $A_xB_yC_z$ is IYQDAYRAEWQVYKEEISRFKEQLTPSQIMSLEKEIMDKHLKRKAMTKKKRVNTAWKTKKTSFSL or IYQDAYRAEWQ VYKEEISRFKEQLTPSQIMSLEKEIMDKHLKRKAMTKKKS.

In some cases, the isolated neoantigenic peptide is encoded by a TGFBR2 gene. In related cases, $A_xB_yC_z$ is KPQEVCVAVWRKNDENITLETVCHDPKLPYHDFILEDAASPKCIMKEKKKAW or EKPQEVCVAVWRKNDENITLETVC HDPKLPYHDFILEDAASPKCIMKEKKSLVRLSSCVPALMSAM TTSSSQKNITPAILTCC.

In some cases, the isolated neoantigenic peptide is encoded by a THAP5 gene. In related cases, $A_xB_yC_z$ is VPSKYQFLCSDHFTPDSLDIRWGIRYLKQTAVPTIFSLPEDNQGKDPSKKNPRRKTWKMRKKYAQKP SQKNHLY.

In some cases, the isolated neoantigenic peptide is encoded by a TTK gene. In related cases, $A_xB_yC_z$ is GT TEEMKYVLGQLVGLNSPNSILKAAKTLYEHYSGGESHNSSSSKTFEKKGEKNDLQLFVMSDTTYK IYWT-VILLNPCGNLHLKTTSL

In some cases, the isolated neoantigenic peptide is encoded by a XPOT gene. In related cases, $A_xB_yC_z$ is QQLIRETLISWLQAQMLNPQPEKTFIRNKAAQVFALLFVTEYLTKWPKFFLTFSQ.

In some cases, the isolated neoantigenic peptide is encoded by a APC gene. In related cases, $C_z$ is AKFQQ CHSTLEPNPADCRVLVYLQNQPGTKLLNFLQERNLPPKVVLRHPKVHLNTMFRRPHSCLAD VLLSVHLIVLRVVRL-PAPFRVNHAVEW, APVIFQIALDKPCHQAEVKHLHHLLKQLKPSEKYLKIKHLLLKRERVDLSKLQ, or MLQFRGSRFFQMLILYYILPRKVLQMDFLVHPA. In some related cases, the neoantigenic peptide is not ADVLLSVHL, ADVLLSVHLI, APFRVNHAV, ARHKAVEFL, CLADVLLSV, CLADVLLSVH, DVLLSVHLI, DVLLSVHLIV, FLQERNLPPK, FRVNHAVEW, HLIVLRVVR, HLIVLRVVRL, HLNTMFRRPH, HPKVHLNTM, HPKVHLNTMF, KAVEFLQER, KVHLNTMFR, KVHLNTMFRR, KVVLRHPKV, LLSVHLIVL, LLSVHLIVLR, LPAPFRVNH, LPAPFRVNHA, LQERNLPPKV, LRVVRLPAPF, LSVHLIVLR, LSVHLIVLRV, MFRRPHSCL, MFRRPHSCLA, NLPPKVVLR, NTMFRRPHSC, QERNLPPKV, RHPKVHLNTM, RNLPPKVVL, RNLPPKVVLR, RPHSCLADV, RPHSCLADVL, RVVRLPAPF, RVVRLPAPFR, SARHKAVEFL, SVHLIVLRV, SVHLIVLRVV, TMFRRPHSC, TMFRRPHSCL, VEFLQERNL, VLLSVHLIV, VLLSVHLIVL, VLRHPKVHL, VLRVVRLPA, VVRLPAPFR, or VVRLPAPFRV.

In some cases, the isolated neoantigenic peptide is encoded by a ARID1A gene. In related cases, $C_z$ is ALG PHSRISCLPTQTRGCILLAATPRSSSSSSSSNDMIPMAISSPPKAPLLAAPSPASRLQCINSNSRITSGQ WMAHMALLPS GTKGRCTACHTALGRGSLSSSSCPQPSPSLPASNKLPSLPLSKMYTTSMAMPILPLPQ LLLLSADQQAAPRTNFHS-SLAETVSLHPLAPMPSKTCHHK, AHQGFPAAKESRVIQLSLLSLLIPPLTCLASEALPRPLLALPPVLLSLAQDHSRLLQ CQATRCHLGHPV ASRTASCILP, PILAATGTSVRTAARTWVPRAAIRVPDPAAVPDDHAGPGAECHGRPLLYTADSSL WTTRPQRVWST GPDSILQPAKSSPSAAAATLLPATTVPDPSCPTFVSAAATVSTTTAPVLSASILPAAIPASTSAVPGSI PL PAVDDTAAPPEPAPLLTATGSVSLPAAATSAASTLDALPAGCVSSAPVSAVPANCLFPAALPSTAGAIS RFIWVS-GILSPLNDLQ, PCRAGRRVPWAASLIHSRFLLMDNKAPAGMVNRARLHITTSKVLTLSSSSHPTPSNHRPRPLMPNLRI SSSHSLNHHSSSPLSLHTPSSHPSLHISSPRLHTPPSSRRHSSTPRASPPTHSHRLSLLTSSSNLSSQHPRR SPSRLRILSPSLSSPSKLPIPSSASLHRRSYLKIHLGLRHPQPPQ, RTNPTVRMRPHCVPFWTGRILLPSAASVCPIPFE ACHLCQAMTLRCPNTQGCCSSWAS, or TNQALPKIEVICRGTPRCPSTVPPSPAQPYLRVSLPEDRYTQAWAPTSRT PWGAMVPRGVSMAHKVA TPGSQTIMPCPMPTTPVQAWLEA. In some related cases, the neoantigenic peptide is not AAWRSCIAL, AAWRSCIALW, AETVSLHPL, AETVSLHPLA, AHMALLPSG, ALWCASSVT, AMPILPLPQL, APL-LAAPSPA, APRTNFHSS, APRTNFHSSL, ASNKLPSLP, AWRSCIALW, CAGRWLWYCW, CIALWCASSV, CPQPSPSLPA, CRRAVSATSW, CTACHTALGR, DQQAAPRTNF, ETVSLHPLA, FHSSLAETV, GMYSPSRYPR, GQWMAHMAL, GQWMAHMALL, GRCTACHTAL, GTAWQLVPL, HMALLPSGT, HMALLPSGTK, HPLAPMPSK, HSSLAETVSL, HTALGRGSL, IALWCASSV, ILATPPSAA, ILATPPSAAW, IPMAISSPP, IPMAISSPPK, ISSPPKAPL, ISSPPKAPLL, ITSGQWMAHM, KGRCTACHT, KGRCTACHTA, KLPSLPLSK, KLPSLPLSKM, KMYTTSMAM, KMYTTSMAMP, LAAPSPASR, LAAPSPASRL, LAETVSLHPL, LATPPSAAW, LATPPSAAWR, LLAAPSPASR, LLLLSADQQAA, LLSADQQAA, LPASNKLPS, LPASNKLPSL, LPLPQLLLS, LPLPQLLLSA, LPSLPLSKM, LPSLPLSKMY, LQCINSNSRI, LQCRRAVSA, LQCRRAVSAT, LSADQQAAPR, LSKMYTTSM, LSKMYTTSMA, LWCASSVTER, LWYCWPTWL, LWYCWPTWLR, MAHMALLPS, MALLPSGTK, MPILPLPQL, MPILPLPQLL, MYSPSRYPR, MYTTSMAMPI, PMAISSPPK, QLVPLQCRR, QQAAPRTNF, QQAAPRTNFH, QWMAHMALL, RAVSATSWA, RAVSATSWAS, RCAGRWLWY, RCTACHTAL, RGTAWQLVPL, RLQCINSNSR, RRAVSATSW, RTNFHSSLA, RTNFHSSLAE, RTRCAGRWL, RTRCAGRWLW, RWLWYCWPT, RWLWYCWPTW, SAAWRSCIA, SAAWRSCIAL, SGQWMAHMAL, SKMYTTSMA, SKMYTTSMAM, SMAMPILPL, SNKLPSLPL, SPASRLQCI, SPPKAPLLAA, SPSLPASNKL, SRITSGQWM, SSCPQPSPSL, SSLAETVSL, SSNDMIPMAI, SSSNDMIPM, SSSSNDMIPM, SSSSSSNDM, SSSSSSSNDM, TACHTALGR, TERTRCAGRW, TSMAMPILPL, TTSMAMPIL, TVSLHPLAPM, TWLRGTAWQL, VPLQCRRAV, VSLHPLAPM, VTERTRCAGR, WLRGTAWQL, WLRGTAWQLV, WLWYCWPTW, WLWYCWPTWL, WMAHMALLPS, WPTWLRGTA, WPTWLRGTAW, WYCWPTWLR, YPRSSSSSS, YPRSSSSSSS, YTTSMAMPI, or YTTSMAMPIL.

In some cases, the isolated neoantigenic peptide is encoded by a β2M gene. In related cases, $C_z$ is RMERELKKWSIQTCLSARTGLSISCTTLNSPPLKKMSMPAV, or LCSRYSLFLAWRLSSVLQRFRFTHVIQQRMESQIS. In some related cases, the neoantigenic peptide is not ALAVLALLSF, ALLSFWPGGY, DSGLLTSSSR, ELLCVWVSSI, EWKVKFPEL, FPELLCVWV, FWPGGYPAY, GLLTSSSREW, KFPELLCVW, KFPELLCVWV, KVKFPELLC, KVKFPELLCV, LAVLALLSF, LAVLALLSFW, LLCVWVSSI, LLCVWVSSIR, LLSFWPGGY, LLTSSSREW, LLTSSSREWK, LSFWPGGYPA, LTSSSREWK, LTSSSREWKV, REWKVKFPE, REWKVKFPEL, SFWPGGYPA, SFWPGGYPAY, SSREWKVKF, SSSREWKVK, SSSREWKVKF, TSSSREWKV, TSSSREWKVK, VKFPELLCV, VKFPELLCVW, WKVKFPELL, or YPAYSKDSGL.

In some cases, the isolated neoantigenic peptide is encoded by a CDH1 gene. In related cases, $C_z$ is RSACVTVKGPLASVGRHSLSKQDCKFLPFWGFLEEFLLC, IQWGTTTAPRPIRPPFLESKQNCSHFPTPLLASEDRRE TGLFLPSAAQKMKKAHFLKTWFRSNPTKTK KARFSTASLAKELTHPLLVSLLLKEKQDG, PTDPFLGLRLGLHLQKVF HQSHAEYSGAPPPPPAPSGLRFWNPSRIAHISQLLSWPQKTEERLGYSSHQ LPRK, FCCSCCFFGGERWSK-SPYCPQRMTPGTTFITMMKKEAEKRTRTLT, or WRRNCKAPVSLRKSVQTPARSSPARPDRTRRLPSLGVPGQPWAL GAAASRRCCCCCRSPLGSARSRS PATLALTPRATRSRCPGATWREAASWAE.

In some cases, the isolated neoantigenic peptide is encoded by a GATA3 gene. In related cases, $C_z$ is PGR PLQTHVLPEPHLALQPLQPHADHAHADAPAIQPVLWTTPPLQHGHRHGLEPCSMLTGPPARVPA VPFDLHFCRSSIMKPKRDGYMFLKAESKIMFATLQRSSLWCLCSNH or PRPRRCTRHPACPLDHTTPPAWSPPWVRA LLDAHRAPSESPCSPFRLAFLQEQYHEA. In some related cases, the neoantigenic peptide is not AALSRHNVL, ADAPAIQPV, ADAPAIQPVL, AESKIMFAT, AESKIMFATL, AIQPVLWTT, ALQPLQPHA, APAIQPVLW, ARVPAVPFDL, ATLQRSSLW, AVPFDLHFCR, CSMLTGPPA, CSMLTGPPAR, DLHFCRSSI, DLHFCRSSIM, EPHLALQPL, ESKIMFATL, FATLQRSSL, FATLQRSSLW, FCRSSIMKPK, FDLHFCRSSI, FLKAESKIM, FLKAESKIMF, GPPARVPAV, GYMFLKAESK, HADAPAIQPV, HAHADAPAI, HFCRSSIMK, HLALQPLQPH, HMSSLSHISA, HPLQHGHRH, HPPSSLSFW, HRHGLEPCSM, IMFATLQRS, IMFATLQRSS, IMKPKRDGY, IMKPKRDGYM, KAESKIMFA, KIMFATLQR, KPKRDGYMF, KPKRDGYMFL, LALQPLQPH, LHFCRSSIM, LHFCRSSIMK, LKAESKIMF, LQHGHRHGL, LQPHADHAH, LQRSSLWCL, LQRSSLWCLC, LSFGPHHPL, LSFGPHPPL, LSFWTTPPL, LSHISALQPL, MFATLQRSSL, MFLKAESKI, MFLKAESKIM, MHPPSSLSFW, MKPKRDGYMF, MLTGPPARV, MSSLSHISA, MSSLSHISAL, NPAALSRHNV, PARVPAVPF, PAVPFDLHF, PEPHLALQPL, PPARVPAVPF, QPVLWTTPPL, RHGLEPCSM, RHNVLPEPHL, RSSIMKPKR, SALQPLQPH, SHISALQPL, SIMKPKRDGY, SLSFGPHHPL, SLSFGPHPPL, SLSFWTTPPL, SMLTGPPAR, SMLTGPPARV, SSLSHISAL, TLQRSSLWCL, TTPPLQHGHR, VLPEPHLAL, VPAVPFDLHF, VPFDLHFCR, YMFLKAESK or YMFLKAESKI.

In some cases, the isolated neoantigenic peptide is encoded by a MLL2 gene. In related cases, $C_z$ is TRRC HCCPHLRSHPCPHHLRNHPRPHHLRHHACHHHLRNCPHPHFLRHCTCPGRWRNRPSLRRLRSL LCLPHLNHHLFL HWRSRPCLHRKSHPHLLHLRRLYPHHLKHRPCPHHLKNLLCPRHLRNCPLPRHLK HLACLHHLRSHPCPLHLKSHP CLHHRRHLVCSHHLKSLLCPLHLRSLPFPHHLRHHACPHHLRTRLCP HHLKNHLCPPHLRYRAYPPCLWCHACLHRL RNLPCPHRLRSLPRPLHLRLHASPHHLRTPPHPHHLR THLLPHHRRTRSCPCRWRSHPCCHYLRSRNSAPGPRGR TCHPGLRSRTCPPGLRSHTYLRRLRSHTCP PSLRSHAYALCLRSHTCPPRLRDHICPLSLRNCTCPPRLRSRTCLLCL RSHACPPNLRNHTCPPSLRSHA CPPGLRNRICPLSLRSHPCPLGLKSPLRSQANALHLRSCPCSLPLGNHPYLPCLE SQPCLSLGNHLCPLC PRSCRCPHLGSHPCRLS. In some related cases, the neoantigenic peptide is not APGPRGRTC, CHYLRSRNSA, CLRSHTCPPR, CLWCHACLHR, CPHLGSHPC, CPHRLRSLPR, CPLGLKSPL, CPPGLRNRI, CPPGLRSHTY, CPPRLRDHI, CPPSLRSHAY, CPRSCRCPH, CPRSCRCPHL, CSLPLGNHPY, CTCPPRLRSR, DHICPLSLR, EESPMSPHL, ESPMSPHLR, ESPMSPHLRY, EVSRLSPCL, GLKSPLRSQA, GLRNRICPL, GLRNRICPLS, GLRSHTYLR, GLRSHTYLRR, GLRSRTCPPG, HACPPGLRNR, HAYALCLRSH, HHLRTHLLPH, HLGSHPCRL, HLLPHHRRTR, HLRLHASPH, HLRLHASPHH, HLRSCPCSL, HLRTHLLPH, HLRTHLLPHH, HLRTPPHPH, HLRTPPHPHH, HLRYRAYPP, HLRYRAYPPC, HPCCHYLRSR, HPHHLRTHL, HPHHLRTHLL, HRLRSLPRPL, HTYLRRLRS, HTYLRRLRSH, HYLRSRNSA, KSPLRSQANA, LESQPCLSL, LHLRLHASPH, LHLRSCPCSL, LLPHHRRTR, LPCPHRLRSL, LPHHRRTRS, LPHHRRTRSC, LPLGNHPYL, LPRPLHLRL, LRLHASPHHL, LRNCTCPPRL, LRNHTCPPSL, LRNRICPLSL, LRSCPCSLPL, LRSHACPPGL, LRSHACPPNL, LRSHAYALCL, LRSHTCPPRL, LRSHTCPPSL, LRSHTYLRRL, LRSLPRPLHL, LRSQANALHL, LRTPPHPHHL, LRYRAYPPCL, LSLGNHLCPL, LSLRSHPCPL, LWCHACLHRL, MSPHLRYRA, MSPHLRYRAY, NLPCPHRLR, NLRNHTCPP, PMSPHLRYR, PPRLRSRTCL, PPSLRSHAY, RAYPPCLWCH, RDHICPLSL, RGRTCHPGL, RGRTCHPGLR, RLHASPHHL, RLHASPHHLR, RLRDHICPL, RLRDHICPLS, RLRNLPCPH, RLRNLPCPHR, RLRSHTCPP, RLRSHTCPPS, RLRSLPRPL, RLRSLPRPLH, RLRSRTCLL, RLRSRTCLLC, RLSPCLWCHA, RNHTCPPSL, RNHTCPPSLR, RNLPCPHRLR, RNRICPLSL, RNRICPLSLR, RPLHLRLHA, RPLHLRLHAS, RSCPCRWRSH, RSCPCSLPL, RSHACPPGL, RSHACPPGLR, RSHACPPNL, RSHACPPNLR, RSHAYALCL, RSHAYALCLR, RSHPCCHYL, RSHPCCHYLR, RSHPCPLGL, RSHPCPLGLK, RSHTCPPRL, RSHTCPPRLR, RSHTCPPSL, RSHTCPPSLR, RSHTYLRRL, RSHTYLRRLR, RSLPRPLHL, RSLPRPLHLR, RSQANALHL, RSQANALHLR, RSRNSAPGP, RSRNSAPGPR, RSRTCLLCL, RSRTCLLCLR, RSRTCPPGL, RSRTCPPGLR, RTCHPGLRSR, RTHLLPHHR, RTHLLPHHRR, RTPPHPHHL, RTPPHPHHLR, RTRSCPCRW, RTRSCPCRWR, RWRSHPCCH, RWRSHPCCHY, RYRAYPPCL, RYRAYPPCLW, SHAYALCLR, SLGNHLCPL, SLPLGNHPY, SLPLGNHPYL, SLPRPLHLR, SLPRPLHLRL, SLRNCTCPPR, SLRSHACPPG, SLRSHAYAL, SLRSHAYALC, SLRSHPCPL, SLRSHPCPLG, SPHHLRTPP, SPHHLRTPPH, SPHLRYRAY, SPLRSQANAL, SPMSPHLRY, SPMSPHLRYR, SQANALHLR, SQANALHLRS, VSRLSPCLW, WRSHPCCHY, WRSHPCCHYL, YLPCLESQPC, YLRRLRSHTC, YLRSRNSAP, or YLRSRNSAPG.

In some cases, the isolated neoantigenic peptide is encoded by a PTEN gene. In related cases, $C_z$ is SWK GTNWCNDMCIFITSGQIFKGTRGPRFLWGSKDQRQKGSNYSQSEALCVLL, KRTKCFTFG, PIFIQTLLLWDFLQKDLKAYTGTILMM, QKMILTKQIKTKPTDTFLQILR, GFWIQSIKTITRYTIFVLKDIMTPPNLIAELHNILLKTITHHS, NYSNVQWRNLQSSVCGLPAKGEDIFLQFRTHTTGRQVHVL, or YQSRVLPQTEQDAKKGQNVSLLGKYILHTRTRGNLRKSRKWKSM. In some related cases, the neoantigenic peptide is not KMLKRTKCF, MLKRTKCFT, LKRTKCFTF, MLKRTKCFTF, KQNKMLKRTK, KMLKRTKCFT, or NKMLKRTKCF.

In some cases, the isolated neoantigenic peptide is encoded by a TP53 gene. In related cases, $C_z$ is SSQN ARGCSPRGPCTSSSYTGGPCTSPLLAPVIFCPFPENLPGQLRFPSGLLAFWDSQVCDLHVLPCPQ QDVLPTGQDLPCAAVG, GAAPTMSAAQIAMVWPLLSILSEWKEICVWSIWMTETLFDIVWWCPMSRLRLALTVPPSTT TTCVTV PAWAA, TGGPSSPSSHWKTPVVIYWDGTALRCVFVPVLGETGAQRKRISARKGSLTTSCPQGALSEHCPTT PAP LPSQRRNHWMENISPFRSVGVSASRCSES, FHTPARHPRPRHGHLQAVTAHDGGCEALPPP, CCPRTILNNGSL KTQVQMKLPECQRLLPPWPLHQQLLHRRPLHQPPPGPCHLLSLPRKPTRAATVSV WASCILGQPSL, VRKHFQTYGNYFLKTTFCPPCRPKQWMI, or LARTPLPSTRCFANWPRPALCSCGLIPHPRPAPASAPWPSTSSHST. In some related cases, the neoantigenic peptide is not APASAPWPST, APPWPLHQQL, APWPSTSSH, ASCILGQPSL, ATVSVWASCI, CQRLLPPWPL, HQPPPGPCHL, HQQLLHRRPL, IEQWFTEDQV, KLPECQRLL, KPTRAATVSV, KTYQGSYGFV, KTYQGSYVS, KTYQGSYVSV, LLSLPRKPTR, LPPWPLHQQL, LPRKPTRAA, LPRKPTRAAT, LSLPRKPTR, MKLPECQRL, MKLPECQRLL, MPEAAPPWPL, PEAAPPWPL, PTRAATVSV, QMKLPECQR, QQLLHRRPL, QQLLHRRPLH, QRLLPPWPL, QWFTEDQVQM, RAATVSVWA, RLLPPWPLH, RMPEAAPPW, RPAPASAPW, SLPRKPTRA, SLPRKPTRAA, SQKTYQGSYV, STPRPAPASA, SYGFVWASC, SYGFVWASCI, SYVSVWASC, SYVSVWASCI, TEDQVQMKL, TPRPAPASA, TPRPAPASAP, TRAATVSVW, TVSVWASCI, TVSVWASCIL, TYQGSYGFV, TYQGSYGFVW, TYQGSYVSV, TYQGSYVSVW, VSVWASCIL, WPLHQQLLH, WPSTSSHST, YGFVWASCI, YGFVWASCIL, YQGSYGFVW, YQGSYGFVWA, YQGSYVSVW, YQGSYVSVWA, YVSVWASCI, or YVSVWASCIL.

In some cases, the isolated neoantigenic peptide is encoded by a VHL gene. In related cases, $C_z$ is ELQET GHRQVALRRSGRPPKCAERPGAADTGAHCTSTDGRLKISVETYTVSSQLLMVLMSLDLDTGL VPSLVSKCLILRVK, KSDASRLSGA, RTAYFCQYHTASVYSERAMPPGCPEPSQA, TRASSPPRSSSAIAVRASCCPYGSTSTASRSPTQRCR LARAAASTATEVTFGSSEMQGHTMGFWLTKLN YLCHLSMLTDSLFLPISHCQCIL, SSLRITGDWTSSGRSTKIWKTTQMCRKTWSG, or RRRRGGVGRRGVRPGRVRPGGTGRRGGDGGRAAAARAALGELARALPGHLLQSQSA RRAARMAQ LRRRAAALPNAAAWHGPPHPQLPRSPLALQRCRDTRWASG. In some related cases, the neoantigenic peptide is not CHLSMLTDSL, EMQGHTMGF, FLPISHCQC, FLPISHCQCI, FRDAGHTMGF, FWLTKLNYL, GFWLTKLNY, GFWLTKLNYL, GSSEMQGHT, HLSMLTDSL, HLSMLTDSLF, HSYRGHLGSS, HTMGFWLTK, HTMGFWLTKL, KERCLQLSGA, KLNYLCHLSM, LFRDAGHTM, LNYLCHLSM, LNYLCHLSML, LPISHCQCI, LPISHCQCIL, LSMLTDSLF, LSMLTDSLFL, LTDSLFLPI, LTKLNYLCHL, MGFWLTKLNY, MLTDSLFLPI, MQGHTMGFW, MQGHTMGFWL, NYLCHLSML, RDAGHTMGF, RDAGHTMGFW, RGHLGSSEM, RIHSYRGHLG, SEMQGHTMG, SEMQGHTMGF, SLFLPISHC, SMLTDSLFL, SSEMQGHTM, TKLNYLCHL, TLKERCLQL, TMGFWLTKL, WLFRDAGHT, WLFRDAGHTM, YLCHLSMLT, or YRGHLGSSEM.

In some cases, the isolated neoantigenic peptide is encoded by a fusion of a first gene with a second gene. In some cases, the isolated neoantigenic peptide is encoded by an in-frame fusion of a first gene with a second gene.

In some cases, the isolated neoantigenic peptide is encoded by a BCR gene and an ABL gene. In related cases, $A_xB_yC_z$ is ERAEWRENIREQQKKCFRSFSLTSVELQMLTNSCVKLQTVHSIPLTINKEEALQRPVASDFEPQGLSEA ARWNSKENLLAGPSENDPNLFVALYDFVASG or ELQMLTNSCVKLQTVHSIPLTINKEDDESPGLYGFLNVIVHSATGF KQSSKALQRPVASDFEPQGLSE AARWNSKENLLAGPSENDPNLFVALYDFVASGD.

In some cases, the isolated neoantigenic peptide is encoded by a C11orf95 gene and a RELA gene. In related

cases, $A_xB_yC_z$ is ISNSWDAHLGLGACGEAEGLGVQGAEEEEEEEEEEEEEGAGVPACPPKGPELFPLIFPAEPAQASG PY VEIIEQPKQRGMRFRYKCEGRSAGSIPGERSTD.

In some cases, the isolated neoantigenic peptide is encoded by a CBFB gene and an MYH11 gene. LQRLD GMGCLEFDEERAQQEDALAQQAFEEARRRTREFEDRDRSHREEMEVHELEKSKRALETQME EMKTQLEELEDEL-QATEDAKLRLEVNMQALKGQF.

In some cases, the isolated neoantigenic peptide is encoded by a CD74 gene and an ROS1 gene. In related cases, $A_xB_yC_z$ is KGSFPENLRHLKNTMETIDWKVFESWMHHWLLFEMSRHSLEQKPTDAPPKAGVPNKPGIPKLLEGS KNSIQWEKAEDNGCRITYYILEIRKSTSNNLQNQ.

In some cases, the isolated neoantigenic peptide is encoded by a EGFR gene and an SEPT14 gene. In related cases, $A_xB_yC_z$ is the first native polypeptide is encoded by an gene and the second native polypeptide is encoded by LPQPPICTIDVYMIMVKCWMIDADSRPKFRELIIEFSKMARDPQRYLVIQLQDKFEHLKMIQQEEIRKL EEEKKQLEGEIIDFYKMKAASEALQTQLSTD.

In some cases, the isolated neoantigenic peptide is encoded by a EGFR gene and an EGFR gene. In related cases, $A_xB_yC_z$ is MRPSGTAGAALLALLAALCPASRALEEKKGNYVVTDHGSCVRACGADSYEMEEDGVRKCKKCEGP CRKVCNGIGIGEFKD.

In some cases, the isolated neoantigenic peptide is encoded by a EML4 gene and an ALK gene. In related cases, $A_xB_yC_z$ is SWENSDDSRNKLSKIPSTPKLIPKVTKTADKHKDVIINQAKMSTREKNSQVYRRKHQELQAMQMEL QSPEYKLSKLRTSTIMTDYNPNYCFAGKTSSISDL.

In some cases, the isolated neoantigenic peptide is encoded by a FGFR3 gene and an TACC3 gene. In related cases, $A_xB_yC_z$ is EGHRMDKPANCTHDLYMIMRECWHAAPSQRPTFKQLVEDLDRVLTVTSTDVKATQEENRELRSRCE ELHGKNLELGKIMDRFEEVVYQAMEEVQKQKELS,

In some cases, the isolated neoantigenic peptide is encoded by a NAB gene and an STAT6 gene. In related cases, $A_xB_yC_z$ is RDNTLLLRRVELFSLSRQVARESTYLSSLKGSRLHPEELGGPPLKKLKQEATSKSQIMSLWGLVSKM P PEKVQRLYVDFPQHLRHLLGDWLESQPWEFLVGSDAFCC.

In some cases, the isolated neoantigenic peptide is encoded by a NDRG1 gene and an ERG gene. In related cases, $A_xB_yC_z$ is MSREMQDVDLAEVKPLVEKGETITGLLQEFDVQEALSVVSEDQSLFECAYGTPHLAKTEMTASSSSD YGQTSKMSPRVPQQDW.

In some cases, the isolated neoantigenic peptide is encoded by a TMPRSS2 gene and an ERG gene. In related cases, $A_xB_yC_z$ is MALNSEALSVVSEDQSLFECAYGTPHLAKTEMTASSSSDYGQTSKMSPRVPQQDW.

In some cases, the isolated neoantigenic peptide is encoded by a PML gene and an RARA gene. In related cases, $A_xB_yC_z$ is VLDMHGFLRQALCRLRQEEPQSLQAAVRTDGFDEFKVRLQDLSSCITQGKAIETQSSSSEEIVPSPPSP PPLPRIYKPCFVCQDKSSGYHYGVSACEGCKG or RSSPEQPRPSTSKAVSPPHLDGPPSPRSPVIGSEVFLPNSNHV ASGAGEAAIETQSSSSEEIVPSPPSPPPL PRIYKPCFVCQDKSSGYHYGVSACEGCKG.

In some cases, the isolated neoantigenic peptide is encoded by a RUNX1 gene and an CBFA2T1 (RUNX1T1) gene. In related cases, $A_xB_yC_z$ is VARFNDLRFVGRSGRGKSFTLTITVFTNPPQVATYHRAIKITVDGPREPRNRTEKH STMPDSPVDVKT QSRLTPPTMPPPPTTQGAPRTSSFTPTTLTNGT

In some cases, the isolated neoantigenic peptide is encoded by a fusion of a first gene with an exon of a splice variant of the first gene. In some cases, the isolated neoantigenic peptide is encoded by a fusion of a first gene with a cryptic exon of the first gene.

In some cases, the isolated neoantigenic peptide is encoded by a AR-v7 gene and a cryptic exon encoded by the AR-v7 gene. In some cases, the isolated neoantigenic peptide is encoded by a AR-v7 gene comprising an exon of a splice variant of an AR gene. In related cases, $A_xB_yC_z$ is SCKVFFKRAAEGKQKYLCASRNDCTIDKFRRKNCPSCRLRKCY EAGMTLGEKFRVGNCKHLKMTRP

In some cases, the isolated neoantigenic peptide is encoded by a fusion of a first gene with a second gene, wherein the peptide comprises an amino acid sequence encoded by an out-of frame sequence resulting from the fusion.

In some cases, the isolated neoantigenic peptide is encoded by an AC011997.1 gene and a LRRC69 gene. In related cases, y is 1. In related cases, $A_xB_yC_z$ is MAGAPPPASLPPCSLISDCCASNQRDSVGVGPSEPGNNIKICNE-SASRK.

In some cases, the isolated neoantigenic peptide is encoded by an EEF1DP3 gene and a FRY gene. In related cases, y is 1. In related cases, $A_xB_yC_z$ is HGWRPFLPVRARSRWNRRLDVTVANGRSWKYGWSLLRVPQVNGIQVLN VSLKSSSNVISY.

In some cases, the isolated neoantigenic peptide is encoded by an MAD1L1 gene and a MAFK gene. In related cases, y is 0. In related cases, $A_xB_yC_z$ is RLKEVFQTKIQEFRKACYTLTGYQIDITTENQYRLTSLYAEHPGDCLIFKLRV PGSSVLVTVPGL.

In some cases, the isolated neoantigenic peptide is encoded by an PPP1R1B gene and a STARD3 gene. In related cases, y is 1. In related cases, $A_xB_yC_z$ is AEVLKVIRQSAGQKTTCGQGLEGPWERPPPLDESERDGGSEDQV EDPALSALLLRPRPPRPEVGAHQ DEQAAQGADPRLGAQPACRGLPGLLTVPQPEPLLAPPSAA.

In some cases, the isolated neoantigenic peptide comprises one or more of the peptide sequences depicted in Table 1. In some cases, the isolated neoantigenic peptide comprises one or more of the peptide sequences depicted in Table 1A, Table 1B, Table 1C, Table 1D, Table 1E, and/or Table 1F. In some cases, the isolated neoantigenic peptide does not comprise one or more of the peptide sequences depicted in Table 2. In some cases, the isolated neoantigenic peptide does not comprise one or more of the peptide sequences depicted in Table 2A, Table 2B, Table 2C, and/or Table 2D.

Neoantigen polynucleotides

Polynucleotides encoding each of the peptides described herein are also part of the disclosure. As appreciated by one of ordinary skill in the art, various nucleic acid sequences can encode the same peptide due to the redundancy of the genetic code. Each of these nucleic acids falls within the scope of the present disclosure. Nucleic acids encoding peptides can be DNA or RNA, for example, mRNA, or a combination of DNA and RNA. In some cases, a nucleic acid encoding a peptide is a self-amplifying mRNA. (Brito et al., Adv. Genet. 2015; 89:179-233). Any suitable polynucleotide that encodes a peptide described herein falls within the scope of this disclosure.

The term "RNA" includes and in some cases relates to "mRNA". The term "mRNA" means "messenger-RNA" and relates to a "transcript" which is generated by using a DNA template and encodes a peptide or polypeptide. Typically, an mRNA comprises a 5'-UTR, a protein coding region, and a 3'-UTR. mRNA only possesses limited half-life in cells and in vitro. In some cases, the mRNA is self-amplifying mRNA. In the context of the present disclosure, mRNA may be generated by in vitro transcription from a DNA template. The in vitro transcription methodology is known to the skilled person. For example, there is a variety of in vitro transcription kits commercially available.

The stability and translation efficiency of RNA may be modified as required. For example, RNA may be stabilized and its translation increased by one or more modifications having a stabilizing effects and/or increasing translation efficiency of RNA. Such modifications are described, for example, in PCT/EP2006/009448. In order to increase expression of the RNA used according to the present disclosure, it may be modified within the coding region, i.e. the sequence encoding the expressed peptide or protein, without altering the sequence of the expressed peptide or protein, so as to increase the GC-content to increase mRNA stability and to perform a codon optimization and, thus, enhance translation in cells.

The term "modification" in the context of the RNA used in the present disclosure includes any modification of an RNA which is not naturally present in said RNA. In some cases of the disclosure, the RNA used according to the disclosure does not have uncapped 5'-triphosphates. Removal of such uncapped 5'-triphosphates can be achieved by treating RNA with a phosphatase. The RNA according to the disclosure may have modified ribonucleotides in order to increase its stability and/or decrease cytotoxicity. For example, In some cases, in the RNA used according to the disclosure 5-methylcytidine is substituted partially or completely, for example, completely, for cytidine. Alternatively or additionally, In some cases, in the RNA used according to the disclosure pseudouridine is substituted partially or completely, for example, completely, for uridine.

In some cases the term "modification" relates to providing an RNA with a 5'-cap or 5'- cap analog. The term "5'-cap" refers to a cap structure found on the 5'-end of an mRNA molecule and generally consists of a guanosine nucleotide connected to the mRNA via an unusual 5' to 5' triphosphate linkage. In some cases, this guanosine is methylated at the 7-position. The term "conventional 5'-cap" refers to a naturally occurring RNA 5'-cap, to the 7-methylguanosine cap (m G). In the context of the present disclosure, the term "5'-cap" includes a 5'-cap analog that resembles the RNA cap structure and is modified to possess the ability to stabilize RNA and/or enhance translation of RNA if attached thereto, in vivo and/or in a cell.

In certain cases, an mRNA encoding a neoantigen peptide of the disclosure is administered to a subject in need thereof. In some cases, the disclosure provides RNA, oligoribonucleotide, and polyribonucleotide molecules comprising a modified nucleoside, gene therapy vectors comprising same, gene therapy methods and gene transcription silencing methods comprising same. In some cases, the mRNA to be administered comprises at least one modified nucleoside.

The polynucleotides encoding peptides described herein can be synthesized by chemical techniques, for example, the phosphotriester method of Matteucci, et al., J. Am. Chem. Soc. 103:3185 (1981). Polynucleotides encoding peptides comprising or consisting of an analog can be made simply by substituting the appropriate and desired nucleic acid base(s) for those that encode the native epitope.

A large number of vectors and host systems suitable for producing and administering a neoantigenic peptide described herein are known to those of skill in the art, and are commercially available. The following vectors are provided by way of example. Bacterial: pQE70, pQE60, pQE-9 (Qiagen), pBS, pD 10, phage script, psiX174, pBluescript SK, pbsks, pNH8A, pNH16a, pNH18A, pNH46A (Stratagene); ptrc99a, pKK223-3, pKK233-3, pDR540, pRIT5 (Pharmacia); pCR (Invitrogen). Eukaryotic: pWLNEO, pSV2CAT, pOG44, pXT1, pSG (Stratagene) pSVK3, pBPV, pMSG, pSVL (Pharmacia); p75.6 (Valentis); pCEP (Invitrogen); pCEI (Epimmune). However, any other plasmid or vector can be used as long as it is replicable and viable in the host.

As representative examples of appropriate hosts, there can be mentioned: bacterial cells, such as E. *coli, Bacillus subtilis, Salmonella typhimurium* and various species within the genera *Pseudomonas, Streptomyces,* and *Staphylo-*

*coccus;* fungal cells, such as yeast; insect cells such as Drosophila and Sf9; animal cells such as COS-7 lines of monkey kidney fibroblasts, described by Gluzman, Cell 23:175 (1981), and other cell lines capable of expressing a compatible vector, for example, the C127, 3T3, CHO, HeLa and BHK cell lines or Bowes melanoma; plant cells, etc. The selection of an appropriate host is deemed to be within the scope of those skilled in the art from the teachings herein.

Thus, the present disclosure is also directed to vectors, and expression vectors useful for the production and administration of the neoantigenic peptides described herein, and to host cells comprising such vectors.

Host cells are genetically engineered (transduced or transformed or transfected) with the vectors which can be, for example, a cloning vector or an expression vector. The vector can be, for example, in the form of a plasmid, a viral particle, a phage, etc. The engineered host cells can be cultured in conventional nutrient media modified as appropriate for activating promoters, selecting transformants or amplifying the polynucleotides. The culture conditions, such as temperature, pH and the like, are those previously used with the host cell selected for expression, and will be apparent to the ordinarily skilled artisan.

For expression of the neoantigenic peptides described herein, the coding sequence will be provided operably linked start and stop codons, promoter and terminator regions, and in some cases, and a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), acid phosphatase, or heat shock proteins, among others. The heterologous structural sequence is assembled in appropriate phase with translation initiation and termination sequences, and in some cases, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Yeast, insect or mammalian cell hosts can also be used, employing suitable vectors and control sequences. Examples of mammalian expression systems include the COS-7 lines of monkey kidney fibroblasts, described by Gluzman, Cell 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. Such promoters can also be derived from viral sources, such as, e.g., human cytomegalovirus (CMV-IE promoter) or herpes simplex virus type-1 (HSV TK promoter). Nucleic acid sequences derived from the SV40 splice, and polyadenylation sites can be used to provide the required nontranscribed genetic elements.

Polynucleotides encoding neoantigenic peptides described herein can also comprise a ubiquitination signal sequence, and/or a targeting sequence such as an endoplasmic reticulum (ER) signal sequence to facilitate movement of the resulting peptide into the endoplasmic reticulum.

Polynucleotides described herein can be administered and expressed in human cells (e.g., immune cells, including dendritic cells). A human codon usage table can be used to guide the codon choice for each amino acid. Such polynucleotides comprise spacer amino acid residues between epitopes and/or analogs, such as those described above, or can comprise naturally-occurring flanking sequences adjacent to the epitopes and/or analogs (and/or CTL, HTL, and B cell epitopes).

In some cases, a neoantigenic peptide described herein can also be administered/expressed by viral or bacterial vectors. Examples of expression vectors include attenuated viral hosts, such as vaccinia or fowlpox. As an example of this approach, vaccinia virus is used as a vector to express nucleotide sequences that encode the neoantigenic peptides described herein. Vaccinia vectors and methods useful in immunization protocols are described in, e.g., U.S. Pat. No. 4,722,848. Another vector is BCG (Bacille Calmette Guerin). BCG vectors are described by Stover et al., Nature 351:456-460 (1991). A wide variety of other vectors useful for therapeutic administration or immunization of the neoantigenic polypeptides described herein, e.g. adeno and adeno-associated virus vectors, retroviral vectors, Salmonella typhi vectors, detoxified anthrax toxin vectors, Sendai virus vectors, poxvirus vectors, canarypox vectors, and fowlpox vectors, and the like, will be apparent to those skilled in the art from the description herein. In some cases, the vector is Modified Vaccinia Ankara (VA) (e.g. Bavarian Noridic (MVA-BN)).

Standard regulatory sequences well known to those of skill in the art can be included in the vector to ensure expression in the human target cells. Several vector elements are desirable: a promoter with a downstream cloning site for polynucleotide, e.g., minigene insertion; a polyadenylation signal for efficient transcription termination; an E. coli origin of replication; and an E. coli selectable marker (e.g. ampicillin or kanamycin resistance). Numerous promoters can be used for this purpose, e.g., the human cytomegalovirus (hCMV) promoter. See, e.g., U.S. Pat. Nos. 5,580,859 and 5,589,466 for other suitable promoter sequences. In some cases, the promoter is the CMV-IE promoter.

Polynucleotides described herein can comprise one or more synthetic or naturally-occurring introns in the transcribed region. The inclusion of mRNA stabilization sequences and sequences for replication in mammalian cells can also be considered for increasing polynucleotide expression.

In addition, a polynucleotide described herein can comprise immunostimulatory sequences (ISSs or CpGs). These sequences can be included in the vector, outside the polynucleotide coding sequence to enhance immunogenicity.

In some cases, the size of at least one antigenic peptide molecule may comprise, but is not limited to, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 60, about 70, about 80, about 90, about 100, about 110, about 120 or greater amino molecule residues, and any range derivable therein.

In some cases, the antigenic peptide molecules are equal to or less than 50 amino acids. In some cases, the antigenic peptide molecules are equal to about 20 to about 30 amino acids. A longer peptide may be designed in several ways. For example, when the HLA-binding regions are predicted or known, a longer peptide may consist of either: individual binding peptides with an extension of 0-10 amino acids toward the N- and C-terminus of each corresponding gene product. A longer peptide may also consist of a concatenation of some or all of the binding peptides with extended sequences for each. In another case, when sequencing reveals a long (>10 residues) epitope sequence present in the diseased tissue (e.g. due to a frameshift, read-through or intron inclusion that leads to a novel peptide sequence), a longer peptide may consist of the entire stretch of novel disease-specific amino acids. In both cases, use of a longer peptide requires endogenous processing by professional antigen presenting cells such as dendritic cells and may lead to more effective antigen presentation and induction of T cell responses. In some cases, the extended sequence is altered to improve the biochemical properties of the polypeptide (properties such as solubility or stability) or to improve the likelihood for efficient proteasomal processing of the peptide.

The antigenic peptides and polypeptides may bind an HLA protein. In some cases, the antigenic peptides may bind an HLA protein with greater affinity than a corresponding native / wild-type peptide. The antigenic peptide may have an IC50 of about less than 1000 nM, about less than 500 nM, about less than 250 nM, about less than 200 nM, about less than 150 nM, about less than 100 nM, or about less than 50 nM. In some cases, the antigenic peptides do not induce an autoimmune response and/or invoke immunological tolerance when administered to a subject.

The disclosure also provides compositions comprising a plurality of antigenic peptides. Reference to antigenic peptides includes any suitable delivery modality that can result in introduction of the peptide into a subject's cell (e.g., nucleic acid). In some cases, the composition comprises at least 2 or more antigenic peptides. In some cases, the composition contains at least about 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 70, 80, 90, 100, 150, 200, 250, 300, 350, 400, 450, or 500 distinct peptides. In some cases, the composition contains at least one peptide from Table 1. In some cases, the composition contains at least one peptide from Table 1 and at least one other peptide from Table 1. In some cases, the composition contains at least one peptide from Table 1 and at least one other peptide from Table 2. In some cases the composition contains at least 20 distinct peptides.. In some cases the composition contains at most 20 distinct peptides. According to the disclosure, 2 or more of the distinct peptides may be derived from the same polypeptide. For example, if an antigenic mutation encodes a polypeptide, two or more of the antigenic peptides may be derived from the polypeptide. In some cases, the two or more antigenic peptides derived from the polypeptide may comprise a tiled array that spans the polypeptide (e.g., the antigenic peptides may comprise a series of overlapping antigenic peptides that spans a portion, or all, of the polypeptide). Antigenic peptides can be derived from any protein coding gene. The antigenic peptides can be derived from mutations in human cancer or from an infectious agent or an autoimmune disease.

The antigenic peptides, polypeptides, and analogs can be further modified to contain additional chemical moieties not normally part of the protein. Those derivatized moieties can improve the solubility, the biological half-life, absorption of the protein, or binding affinity. The moieties can also reduce or eliminate any desirable side effects of the proteins and the like. An overview for those moieties can be found in Remington's Pharmaceutical Sciences, 20th ed., Mack Publishing Co., Easton, PA (2000). For example, antigenic peptides and polypeptides having the desired activity may be modified as necessary to provide certain desired attributes, e.g. improved pharmacological characteristics, while increasing or at least retaining substantially all of the biological activity of the unmodified peptide to bind the desired MHC molecule and activate the appropriate T cell. For instance, the antigenic peptide and polypeptides may be subject to various changes, such as substitutions, either conservative or non-conservative, where such changes might provide for certain advantages in their use, such as improved MHC binding. Such conservative substitutions may encompass replacing an amino acid residue with another amino acid residue that is biologically and/or chemically similar, e.g., one hydrophobic residue for another, or one polar residue for another. The effect of single amino acid substitutions may also be probed using D- amino acids. Such modifications may be made using well known peptide synthesis procedures, as described in e.g., Merrifield, Science 232:341-347 (1986), Barany & Merrifield, The Peptides, Gross & Meienhofer, eds.

(N.Y., Academic Press), pp. 1-284 (1979); and Stewart & Young, Solid Phase Peptide Synthesis, (Rockford, III., Pierce), 2d Ed. (1984).

The antigenic peptide may also be modified by extending or decreasing the compound's amino acid sequence, e.g., by the addition or deletion of amino acids. The antigenic peptides, polypeptides, or analogs can also be modified by altering the order or composition of certain residues. It will be appreciated by the skilled artisan that certain amino acid residues essential for biological activity, e.g., those at critical contact sites or conserved residues, may generally not be altered without an adverse effect on biological activity. The noncritical amino acids need not be limited to those naturally occurring in proteins, such as L-a- amino acids, or their D-isomers, but may include non-natural amino acids as well, such as β-γ-δ- amino acids, as well as many derivatives of L-a-amino acids.

An antigen peptide may be optimized by using a series of peptides with single amino acid substitutions to determine the effect of electrostatic charge, hydrophobicity, etc. on MHC binding. For instance, a series of positively charged (e.g., Lys or Arg) or negatively charged (e.g., Glu) amino acid substitutions may be made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding. Amino acid substitutions are typically of single residues. Substitutions, deletions, insertions or any combination thereof may be combined to arrive at a final peptide.

An antigenic peptide may be modified to provide desired attributes. For instance, the ability of the peptides to induce CTL activity can be enhanced by linkage to a sequence which contains at least one epitope that is capable of inducing a T helper cell response. In some cases, immunogenic peptides/T helper conjugates are linked by a spacer molecule. In some cases, a spacer comprises relatively small, neutral molecules, such as amino acids or amino acid mimetics, which are substantially uncharged under physiological conditions. Spacers can be selected from, e.g., Ala, Gly, or other neutral spacers of nonpolar amino acids or neutral polar amino acids. It will be understood that the optionally present spacer need not be comprised of the same residues and thus may be a hetero- or homo-oligomer. The -antigenic peptide may be linked to the T helper peptide either directly or via a spacer either at the amino or carboxy terminus of the peptide. The amino terminus of either the antigenic peptide or the T helper peptide may be acylated. Exemplary T helper peptides include tetanus toxoid 830-843, influenza 307-319, malaria circumsporozoite 382-398 and 378- 389.

The present disclosure is based, at least in part, on the ability to present the immune system of the patient with one or more disease-specific antigens. One of skill in the art from this disclosure and the knowledge in the art will appreciate that there are a variety of ways in which to produce such disease specific antigens. In general, such disease specific antigens may be produced either in vitro or in vivo. Disease specific antigens may be produced in vitro as peptides or polypeptides, which may then be formulated into a vaccine or immunogenic composition and administered to a subject. As described in further detail herein, such in vitro production may occur by a variety of methods known to one of skill in the art such as, for example, peptide synthesis or expression of a peptide/polypeptide from a DNA or RNA molecule in any of a variety of bacterial, eukaryotic, or viral recombinant expression systems, followed by purification of the expressed peptide/polypeptide. Alternatively, disease specific antigens may be produced in vivo by introducing molecules (e.g., DNA, RNA, viral expression systems, and the like) that encode disease specific antigens into a subject, whereupon the encoded disease specific antigens are expressed. The methods of in vitro and in vivo production of antigens is also further described herein as it relates to pharmaceutical compositions and methods of delivery of the therapy.

In some cases, the present disclosure includes modified antigenic peptides. A modification can include a covalent chemical modification that does not alter the primary amino acid sequence of the antigenic peptide itself. Modifications can produce peptides with desired properties, for example, prolonging the in vivo half-life, increasing the stability, reducing the clearance, altering the immunogenicity or allergenicity, enabling the raising of particular antibodies, cellular targeting, antigen uptake, antigen processing, MHC affinity, MHC stability, or antigen presentation. Changes to an antigenic peptide that may be carried out include, but are not limited to, conjugation to a carrier protein, conjugation to a ligand, conjugation to an antibody, PEGylation, polysialylation HESylation, recombinant PEG mimetics, Fc fusion, albumin fusion, nanoparticle attachment, nanoparticulate encapsulation, cholesterol fusion, iron fusion, acylation, amidation, glycosylation, side chain oxidation, phosphorylation, biotinylation, the addition of a surface active material, the addition of amino acid mimetics, or the addition of unnatural amino acids.

Issues associated with short plasma half- life or susceptibility to protease degradation may be overcome by various modifications, including conjugating or linking the polypeptide sequence to any of a variety of non-proteinaceous polymers, e.g., polyethylene glycol (PEG), polypropylene glycol, or polyoxyalkylenes (see, for example, typically via a linking moiety covalently bound to both the protein and the nonproteinaceous polymer, e.g., a PEG). Such PEG conjugated biomolecules have been shown to possess clinically useful properties, including better physical and thermal stability, protection against susceptibility to enzymatic degradation, increased solubility, longer in vivo circulating half-life and decreased clearance, reduced immunogenicity and antigenicity, and reduced toxicity.

PEGs suitable for conjugation to a polypeptide sequence are generally soluble in water at room temperature, and have the general formula R(0-CH2-CH2)nO-R, where R is hydrogen or a protective group such as an alkyl or an alkanol group, and where n is an integer from 1 to 1000. When R is a protective group, it generally has from 1 to 8 carbons. The PEG conjugated to the polypeptide sequence can be linear or branched. Branched PEG derivatives, "star-PEGs" and multi-armed PEGs are contemplated by the present disclosure.

The present disclosure also contemplates compositions of conjugates wherein the PEGs have different n values and thus the various different PEGs are present in specific ratios. For example, some compositions comprise a mixture of conjugates where n=1, 2, 3 and 4. In some compositions, the percentage of conjugates where n=1 is 18-25%, the percentage of conjugates where n=2 is 50-66%, the percentage of conjugates where n=3 is 12-16%, and the percentage of conjugates where n=4 is up to 5%. Such compositions can be produced by reaction conditions and purification methods know in the art. For example, cation exchange chromatography may be used to separate conjugates, and a fraction is then identified which contains the conjugate having, for example, the desired number of PEGs attached, purified free from unmodified protein sequences and from conjugates having other numbers of PEGs attached.

PEG may be bound to a polypeptide of the present disclosure via a terminal reactive group (a "spacer"). The spacer is, for example, a terminal reactive group which mediates a bond between the free amino or carboxyl groups of one or more of the polypeptide sequences and polyethylene glycol. The PEG having the spacer which may be bound to the free amino group includes N-hydroxysuccinylimide polyethylene glycol which may be prepared by activating succinic acid ester of polyethylene glycol with N-hydroxy succinylimide. Another activated polyethylene glycol which may be bound to a free amino group is 2,4-bis(0- methoxypolyethyleneglycol)-6-chloro-s-triazine which may be prepared by reacting polyethylene glycol monomethyl ether with cyanuric chloride. The activated polyethylene glycol which is bound to the free carboxyl group includes polyoxyethylenediamine.

Conjugation of one or more of the polypeptide sequences of the present disclosure to PEG having a spacer may be carried out by various conventional methods. For example, the conjugation reaction can be carried out in solution at a pH of from 5 to 10, at temperature from 4°C to room temperature, for 30 minutes to 20 hours, utilizing a molar ratio of reagent to protein of from 4: 1 to 30: 1. Reaction conditions may be selected to direct the reaction towards producing predominantly a desired degree of substitution. In general, low temperature, low pH (e.g., pH=5), and short reaction time tend to decrease the number of PEGs attached, whereas high temperature, neutral to high pH (e.g., pH>7), and longer reaction time tend to increase the number of PEGs attached. Various means known in the art may be used to terminate the reaction. In some cases the reaction is terminated by acidifying the reaction mixture and freezing at, e.g., -20°C.

The present disclosure also contemplates the use of PEG mimetics. Recombinant PEG mimetics have been developed that retain the attributes of PEG (e.g., enhanced serum half- life) while conferring several additional advantageous properties. By way of example, simple polypeptide chains (comprising, for example, Ala, Glu, Gly, Pro, Ser and Thr) capable of forming an extended conformation similar to PEG can be produced recombinantly already fused to the peptide or protein drug of interest (e.g., Amunix' XTEN technology; Mountain View, CA). This obviates the need for an additional conjugation step during the manufacturing process. Moreover, established molecular biology techniques enable control of the side chain composition of the polypeptide chains, allowing optimization of immunogenicity and manufacturing properties.

Glycosylation can affect the physical properties of proteins and can also be important in protein stability, secretion, and subcellular localization. Proper glycosylation can be important for biological activity. In fact, some genes from eukaryotic organisms, when expressed in bacteria (e.g., E. coli) which lack cellular processes for glycosylating proteins, yield proteins that are recovered with little or no activity by virtue of their lack of glycosylation. Addition of glycosylation sites can be accomplished by altering the amino acid sequence. The alteration to the polypeptide may be made, for example, by the addition of, or substitution by, one or more serine or threonine residues (for O-linked glycosylation sites) or asparagine residues (for N-linked glycosylation sites). The structures of N-linked and O- linked oligosaccharides and the sugar residues found in each type may be different. One type of sugar that is commonly found on both is N-acetylneuraminic acid (hereafter referred to as sialic acid). Sialic acid is usually the terminal residue of both N-linked and O-linked oligosaccharides and, by virtue of its negative charge, may confer acidic properties to the glycoprotein. Some cases of the present disclosure comprise the generation and use of N-glycosylation variants.

The polypeptide sequences of the present disclosure may optionally be altered through changes at the DNA level, particularly by mutating the DNA encoding the polypeptide at preselected bases such that codons are generated that will translate into the desired amino acids. Another means of increasing the number of carbohydrate moieties on the polypeptide is by chemical or enzymatic coupling of glycosides to the polypeptide. Removal of carbohydrates may be accomplished chemically or enzymatically, or by substitution of codons encoding amino acid residues that are glycosylated. Chemical deglycosylation techniques are known, and enzymatic cleavage of carbohydrate moieties on polypeptides can be achieved by the use of a variety of endo- and exo-glycosidases.

Additional suitable components and molecules for conjugation include, for example, molecules for targeting to the lymphatic system, thyroglobulin; albumins such as human serum albumin (HAS); tetanus toxoid; Diphtheria toxoid; polyamino acids such as poly(D-lysine:D-glutamic acid); VP6 polypeptides of rotaviruses; influenza virus hemaglutinin, influenza virus nucleoprotein; Keyhole Limpet Hemocyanin (KLH); and hepatitis B virus core protein and surface antigen; or any combination of the foregoing.

Fusion of albumin to one or more polypeptides of the present disclosure can, for example, be achieved by genetic manipulation, such that the DNA coding for HSA, or a fragment thereof, is joined to the DNA coding for the one or more polypeptide sequences. Thereafter, a suitable host can be transformed or transfected with the fused nucleotide sequences in the form of, for example, a suitable plasmid, so as to express a fusion polypeptide. The expression may be effected in vitro from, for example, prokaryotic or eukaryotic cells. In some cases of the present disclosure, the expression of the fusion protein is performed in mammalian cell lines, for example, CHO cell lines. Transformation is used broadly herein to refer to the genetic alteration of a cell resulting from the direct uptake, incorporation and expression of exogenous genetic material (exogenous DNA) from its surroundings and taken up through the cell membrane(s). Transformation occurs naturally in some species of bacteria, but it can also be effected by artificial means in other cells. Furthermore, albumin itself may be modified to extend its circulating half-life. Fusion of the modified albumin to one or more polypeptides can be attained by the genetic manipulation techniques described above or by chemical conjugation; the resulting fusion molecule has a half- life that exceeds that of fusions with non-modified albumin. (See WO2011/051489). Several albumin-binding strategies have been developed as alternatives for direct fusion, including albumin binding through a conjugated fatty acid chain (acylation). Because serum albumin is a transport protein for fatty acids, these natural ligands with albumin - binding activity have been used for half-life extension of small protein therapeutics. For example, insulin detemir (LEVEMIR), an approved product for diabetes, comprises a myristyl chain conjugated to a genetically-modified insulin, resulting in a long- acting insulin analog.

Another type of modification is to conjugate (e.g., link) one or more additional components or molecules at the N- and/or C-terminus of a polypeptide sequence, such as another protein (e.g., a protein having an amino acid sequence heterologous to the subject protein), or a carrier molecule. Thus, an exemplary polypeptide sequence can be provided as a conjugate with another component or molecule.

A conjugate modification may result in a polypeptide sequence that retains activity with an additional or complementary function or activity of the second molecule. For example, a polypeptide sequence may be conjugated to a molecule, e.g., to facilitate solubility, storage, in vivo or shelf half-life or stability, reduction in immunogenicity, delayed or controlled release in vivo, etc. Other functions or activities include a conjugate that reduces toxicity relative to an unconjugated polypeptide sequence, a conjugate that targets a type of cell or organ more efficiently than an unconjugated polypeptide sequence, or a drug to further counter the causes or effects associated with a disorder or disease as set forth herein (e.g., diabetes).

A polypeptide may also be conjugated to large, slowly metabolized macromolecules such as proteins; polysaccharides, such as sepharose, agarose, cellulose, cellulose beads; polymeric amino acids such as polyglutamic acid, polylysine; amino acid copolymers; inactivated virus particles; inactivated bacterial toxins such as toxoid from diphtheria, tetanus, cholera, leukotoxin molecules; inactivated bacteria; and dendritic cells.

Additional candidate components and molecules for conjugation include those suitable for isolation or purification. Particular non-limiting examples include binding molecules, such as biotin (biotin-avidin specific binding pair), an antibody, a receptor, a ligand, a lectin, or molecules that comprise a solid support, including, for example, plastic or polystyrene beads, plates or beads, magnetic beads, test strips, and membranes. Purification methods such as cation exchange chromatography may be used to separate conjugates by charge difference, which effectively separates conjugates into their various molecular weights. The content of the fractions obtained by cation exchange chromatography may be identified by molecular weight using conventional methods, for example, mass spectroscopy, SDS-PAGE, or other known methods for separating molecular entities by molecular weight.

In some cases, the amino- or carboxyl- terminus of a polypeptide sequence of the present disclosure can be fused with an immunoglobulin Fc region (e.g., human Fc) to form a fusion conjugate (or fusion molecule). Fc fusion conjugates have been shown to increase the systemic half-life of biopharmaceuticals, and thus the biopharmaceutical product may require less frequent administration.

Fc binds to the neonatal Fc receptor (FcRn) in endothelial cells that line the blood vessels, and, upon binding, the Fc fusion molecule is protected from degradation and re- released into the circulation, keeping the molecule in circulation longer. This Fc binding is believed to be the mechanism by which endogenous IgG retains its long plasma half-life. More recent Fc-fusion technology links a single copy of a biopharmaceutical to the Fc region of an antibody to optimize the pharmacokinetic and pharmacodynamic properties of the biopharmaceutical as compared to traditional Fc-fusion conjugates.

The present disclosure contemplates the use of other modifications, currently known or developed in the future, of the polypeptides to improve one or more properties. One such method for prolonging the circulation half-life, increasing the stability, reducing the clearance, or altering the immunogenicity or allergenicity of a polypeptide of the present disclosure involves modification of the polypeptide sequences by hesylation, which utilizes hydroxyethyl starch derivatives linked to other molecules in order to modify the molecule's characteristics. Various aspects of hesylation are described in, for example, U.S. Patent Appln. Nos. 2007/134197 and 2006/0258607.

Proteins or peptides may be made by any technique known to those of skill in the art, including the expression of proteins, polypeptides or peptides through standard molecular biological techniques, the isolation of proteins or peptides from natural sources, in vitro translation, or the chemical synthesis of proteins or peptides.

Peptides can be readily synthesized chemically utilizing reagents that are free of contaminating bacterial or animal substances (Merrifield RB: Solid phase peptide synthesis. I. The synthesis of a tetrapeptide. J. Am. Chem. Soc.85:2149-54, 1963). In some cases, antigenic peptides are prepared by (1) parallel solid-phase synthesis on multi-channel instruments using uniform synthesis and cleavage conditions; (2) purification over a RP-HPLC column with column stripping; and re-washing, but not replacement, between peptides; followed by (3) analysis with a limited set of the most informative assays. The Good Manufacturing Practices (GMP) footprint can be defined around the set of peptides for an individual patient, thus requiring suite changeover procedures only between syntheses of peptides for different patients.

Alternatively, a nucleic acid (e.g., a polynucleotide) encoding an antigenic peptide of the disclosure may be used to produce the antigenic peptide in vitro. The polynucleotide may be, e.g., DNA, cDNA, PNA, CNA, RNA, either single- and/or double-stranded, or native or stabilized forms of polynucleotides, such as e.g. polynucleotides with a phosphorothiate backbone, or combinations thereof and it may or may not contain introns so long as it codes for the peptide. In some cases in vitro translation is used to produce the peptide. An expression vector capable of expressing a polypeptide can also be prepared. Expression vectors for different cell types are well known in the art and can be selected without undue experimentation. Generally, the DNA is inserted into an expression vector, such as a plasmid, in proper orientation and correct reading frame for expression. If necessary, the DNA may be linked to the appropriate transcriptional and translational regulatory control nucleotide sequences recognized by the desired host (e.g., bacteria), although such controls are generally available in the expression vector. The vector is then introduced into the host bacteria for cloning using standard techniques (see, e.g., Sambrook et al. (1989) Molecular Cloning, A Laboratory Manual, Cold Spring Harbor Laboratory, Cold Spring Harbor, N.Y.).

Expression vectors comprising the isolated polynucleotides, as well as host cells containing the expression vectors, are also contemplated. The antigenic peptides may be provided in the form of RNA or cDNA molecules encoding the desired antigenic peptides. One or more antigenic peptides of the invention may be encoded by a single expression vector.

In some cases, the polynucleotides may comprise the coding sequence for the disease specific antigenic peptide fused in the same reading frame to a polynucleotide which aids, for example, in expression and/or secretion of a polypeptide from a host cell (e.g., a leader sequence which functions as a secretory sequence for controlling transport of a polypeptide from the cell). The polypeptide having a leader sequence is a preprotein and can have the leader sequence cleaved by the host cell to form the mature form of the polypeptide.

In some cases, the polynucleotides can comprise the coding sequence for the disease specific antigenic peptide fused in the same reading frame to a marker sequence that allows, for example, for purification of the encoded polypeptide, which may then be incorporated into a personalized disease vaccine or immunogenic composition. For example, the marker sequence can be a hexa-histidine tag supplied by a pQE-9 vector to provide for purification of the mature polypeptide fused to the marker in the case of a bacterial host, or the marker sequence can be a hemagglutinin (HA) tag derived from the influenza hemagglutinin protein when a mammalian host (e.g., COS-7 cells) is used. Additional tags include, but are not limited to, Calmodulin tags, FLAG tags, Myc tags, S tags, SBP tags, Softag 1, Softag 3, V5 tag, Xpress tag, Isopeptag, SpyTag, Biotin Carboxyl Carrier Protein (BCCP) tags, GST tags, fluorescent protein tags (e.g., green fluorescent protein tags), maltose binding protein tags, Nus tags, Strep-tag, thioredoxin tag, TC tag, Ty tag, and the like.

In some cases, the polynucleotides may comprise the coding sequence for one or more of the disease specific antigenic peptides fused in the same reading frame to create a single concatamerized antigenic peptide construct capable of producing multiple antigenic peptides.

In some cases, isolated nucleic acid molecules having a nucleotide sequence at least 60% identical, at least 65% identical, at least 70% identical, at least 75% identical, at least 80% identical, at least 85% identical, at least 90% identical, at least 95% identical, or at least 96%, 97%, 98% or 99% identical to a polynucleotide encoding a disease specific antigenic peptide of the present invention, can be provided.

The isolated disease specific antigenic peptides described herein can be produced in vitro (e.g., in the laboratory) by any suitable method known in the art. Such methods range from direct protein synthetic methods to constructing a DNA sequence encoding isolated polypeptide sequences and expressing those sequences in a suitable transformed host. In some cases, a DNA sequence is constructed using recombinant technology by isolating or synthesizing a DNA sequence encoding a wild-type protein of interest. Optionally, the sequence can be mutagenized by site-specific mutagenesis to provide functional analogs thereof. See, e.g. Zoeller et al., Proc. Nat'l. Acad. Sci. USA 81:5662-5066 (1984) and U.S. Pat. No.4,588,585.

In some cases, a DNA sequence encoding a polypeptide of interest would be constructed by chemical synthesis using an oligonucleotide synthesizer. Such oligonucleotides can be designed based on the amino acid sequence of the desired polypeptide and selecting those codons that are favored in the host cell in which the recombinant polypeptide of interest is produced. Standard methods can be applied to synthesize an isolated polynucleotide sequence encoding an isolated polypeptide of interest. For example, a complete amino acid sequence can be used to construct a back-translated gene. Further, a DNA oligomer containing a nucleotide sequence coding for the particular isolated polypeptide can be synthesized. For example, several small oligonucleotides coding for portions of the desired polypeptide can be synthesized and then ligated. The individual oligonucleotides typically contain 5' or 3' overhangs for complementary assembly

Once assembled (e.g., by synthesis, site-directed mutagenesis, or another method), the polynucleotide sequences encoding a particular isolated polypeptide of interest is inserted into an expression vector and optionally operatively linked to an expression control sequence appropriate for expression of the protein in a desired host. Proper assembly can be confirmed by nucleotide sequencing, restriction mapping, and expression of a biologically active polypeptide in a suitable host. As well known in the art, in order to obtain high expression levels of a transfected gene in a host, the gene can be operatively linked to transcriptional and translational expression control sequences that are functional in the chosen expression host.

Recombinant expression vectors may be used to amplify and express DNA encoding the disease specific antigenic peptides. Recombinant expression vectors are replicable DNA constructs which have synthetic or cDNA-derived DNA fragments encoding a disease specific antigenic peptide or a bioequivalent analog operatively linked to suitable transcriptional or translational regulatory elements derived from mammalian, microbial, viral or insect genes. A transcriptional unit generally comprises an assembly of (1) a genetic element or elements having a regulatory role in gene expression, for example, transcriptional promoters or enhancers, (2) a structural or coding sequence which is transcribed into mRNA and translated into protein, and (3) appropriate transcription and translation initiation and termination sequences, as described in detail herein. Such regulatory elements can include an operator sequence to control transcription. The ability to replicate in a host, usually conferred by an origin of replication, and a selection gene to facilitate recognition of transformants can additionally be incorporated. DNA regions are operatively linked when they are functionally related to each other. For example, DNA for a signal peptide (secretory leader) is operatively linked to DNA for a polypeptide if it is expressed as a precursor which participates in the secretion of the polypeptide; a promoter is operatively linked to a coding sequence if it controls the transcription of the sequence; or a ribosome binding site is operatively linked to a coding sequence if it is positioned so as to permit translation. Generally, operatively linked means contiguous, and in the case of secretory leaders, means contiguous and in reading frame. Structural elements intended for use in yeast expression systems include a leader sequence enabling extracellular secretion of translated protein by a host cell. Alternatively, where recombinant protein is expressed without a leader or transport sequence, it can include an N-terminal methionine residue. This residue can optionally be subsequently cleaved from the expressed recombinant protein to provide a final product.

Useful expression vectors for eukaryotic hosts, especially mammals or humans include, for example, vectors comprising expression control sequences from SV40, bovine papilloma virus, adenovirus and cytomegalovirus. Useful expression vectors for bacterial hosts include known bacterial plasmids, such as plasmids from Escherichia coli, including pCR 1, pBR322, pMB9 and their derivatives, wider host range plasmids, such as M13 and filamentous single-stranded DNA phages.

Suitable host cells for expression of a polypeptide include prokaryotes, yeast, insect or higher eukaryotic cells under the control of appropriate promoters. Prokaryotes include gram negative or gram positive organisms, for example E. coli or bacilli. Higher eukaryotic cells include established cell lines of mammalian origin. Cell-free translation systems could also be employed. Appropriate cloning and expression vectors for use with bacterial, fungal, yeast, and mammalian cellular hosts are well known in the art (see Pouwels et al., Cloning Vectors: A Laboratory Manual, Elsevier, N.Y., 1985).

Various mammalian or insect cell culture systems are also advantageously employed to express recombinant protein. Expression of recombinant proteins in mammalian cells can be performed because such proteins are generally correctly folded, appropriately modified and completely functional. Examples of suitable mammalian host cell lines include the COS-7 lines of monkey kidney cells, described by Gluzman (Cell 23:175, 1981), and other cell lines capable of expressing an appropriate vector including, for example, L cells, C127, 3T3, Chinese hamster ovary (CHO), 293, HeLa and BHK cell lines. Mammalian expression vectors can comprise nontranscribed elements such as an origin of replication, a suitable promoter and enhancer linked to the gene to be expressed, and other 5' or 3' flanking nontranscribed sequences, and 5' or 3' nontranslated sequences, such as necessary ribosome binding sites, a polyadenylation site, splice donor and acceptor sites, and transcriptional termination sequences. Baculovirus systems for production of heterologous proteins in insect cells are reviewed by Luckow and Summers, Bio/Technology 6:47 (1988).

The proteins produced by a transformed host can be purified according to any suitable method. Such standard methods include chromatography (e.g., ion exchange, affinity and sizing column chromatography, and the like), centrifugation, differential solubility, or by any other standard technique for protein purification. Affinity tags such as hexahistidine, maltose binding domain, influenza coat sequence, glutathione-S-transferase, and the like can be attached to the protein to allow easy purification by passage over an appropriate affinity column. Isolated proteins can also be physically characterized using such techniques as proteolysis, nuclear magnetic resonance and x-ray crystallography. For example, supernatants from systems which secrete recombinant protein into culture media can be first concentrated using a commercially available protein concentration filter, for example, an Amicon or Millipore Pellicon ultrafiltration unit. Following the concentration step, the concentrate can be applied to a suitable purification matrix. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. Finally, one or more reversed-phase high performance liquid chromatography (RP-HPLC) steps employing hydrophobic RP-HPLC media, e.g., silica gel having pendant methyl or other aliphatic groups, can be employed to further purify a cancer stem cell protein-Fc composition. Some or all of the foregoing purification steps, in various combinations, can also be employed to provide a homogeneous recombinant protein.

Recombinant protein produced in bacterial culture can be isolated, for example, by initial extraction from cell pellets, followed by one or more concentration, salting-out, aqueous ion exchange or size exclusion chromatography steps. High performance liquid chromatography (HPLC) can be employed for final purification steps. Microbial cells employed in expression of a recombinant protein can be disrupted by any convenient method, including freeze-thaw cycling, sonication, mechanical disruption, or use of cell lysing agents.

The present disclosure also contemplates the use of nucleic acid molecules as vehicles for delivering antigenic peptides/polypeptides to the subject in need thereof, in vivo, in the form of, e.g., DNA/RNA vaccines (see, e.g., WO2012/159643, and WO2012/159754).

In some cases antigens may be administered to a patient in need thereof by use of a plasmid. These are plasmids which usually consist of a strong viral promoter to drive the in vivo transcription and translation of the gene (or complementary DNA) of interest (Mor, et al., (1995). The Journal of Immunology 155 (4): 2039-2046). Intron A may sometimes be included to improve mRNA stability and hence increase protein expression (Leitner et al. (1997).The Journal of Immunology 159 (12): 6112-6119). Plasmids also include a strong polyadenylation/transcriptional termination signal, such as bovine growth hormone or rabbit beta-globulin polyadenylation sequences (Alarcon et al., (1999). Adv. Parasitol. Advances in Parasitology 42: 343-410; Robinson et al., (2000). Adv. Virus Res. Advances in Virus Research 55: 1-74; Böhmet al., (1996). Journal of Immunological Methods 193 (1): 29-40.). Multicistronic vectors are sometimes constructed to express more than one immunogen, or to express an immunogen and an immunostimulatory protein (Lewis et al., (1999). Advances in Virus Research (Academic Press) 54: 129-88)

Plasmids may be introduced into animal tissues by a number of different methods. The two most popular approaches are injection of DNA in saline, using a standard hypodermic needle, and gene gun delivery. A schematic outline of the construction of a DNA vaccine plasmid and its subsequent delivery by these two methods into a host is illustrated at Scientific American (Weiner et al., (1999) Scientific American 281 (1): 34-41). Injection in saline is normally conducted intramuscularly (IM) in skeletal muscle, or intradermally (ID), with DNA being delivered to the extracellular spaces. This can be assisted by electroporation by temporarily damaging muscle fibres with myotoxins such as bupivacaine; or by using hypertonic solutions of saline or sucrose (Alarcon et al., (1999). Adv. Parasitol. Advances in Parasitology 42: 343-410). Immune responses to this method of delivery can be affected by many factors, including needle type, needle alignment, speed of injection, volume of injection, muscle type, and age, sex and physiological condition of the animal being injected(Alarcon et al., (1999). Adv. Parasitol. Advances in Parasitology 42: 343-410).

Gene gun delivery, the other commonly used method of delivery, ballistically accelerates plasmid DNA (pDNA) that has been adsorbed onto gold or tungsten microparticles into the target cells, using compressed helium as an accelerant (Alarcon et al., (1999). Adv. Parasitol. Advances in Parasitology 42: 343-410; Lewis et al., (1999). Advances in Virus Research (Academic Press) 54: 129-88).

Alternative delivery methods may include aerosol instillation of naked DNA on mucosal surfaces, such as the nasal and lung mucosa, (Lewis et al., (1999). Advances in Virus Research (Academic Press) 54: 129-88) and topical administration of pDNA to the eye and vaginal mucosa (Lewis et al., (1999) Advances in Virus Research (Academic Press) 54: 129-88). Mucosal surface delivery has also been achieved using cationic liposome-DNA preparations, biodegradable microspheres, attenuated Shigella or Listeria vectors for oral administration to the intestinal mucosa, and recombinant adenovirus vectors. DNA or RNA may also be delivered to cells following mild mechanical disruption of the cell membrane, temporarily permeabilizing the cells. Such a mild mechanical disruption of the membrane can be accomplished by gently forcing cells through a small aperture (Ex Vivo Cytosolic Delivery of Functional Macromolecules to Immune Cells, Sharei et al, PLOS ONE | DOI:10.1371/journal.pone.0118803 April 13, 2015).

In some cases, a disease specific vaccine or immunogenic composition may include separate DNA plasmids encoding, for example, one or more antigenic peptides/polypeptides. As discussed herein, the exact choice of expression vectors can depend upon the peptide/polypeptides to be expressed, and is well within the skill of the ordinary artisan. The expected persistence of the DNA constructs (e.g., in an episomal, non-replicating, non-integrated form in the muscle cells) is expected to provide an increased duration of protection.

One or more antigenic peptides of the disclosure may be encoded and expressed in vivo using a viral based system (e.g., an adenovirus system, an adeno associated virus (AAV) vector, a poxvirus, or a lentivirus). In some cases, the disease vaccine or immunogenic composition may include a viral based vector for use in a human patient in need thereof, such as, for example, an adenovirus. Plasmids that can be used for adeno associated virus, adenovirus, and lentivirus delivery have been described previously (see e.g., U.S. Patent Nos. 6,955,808 and 6,943,019, and U.S. Patent application No. 20080254008).

The peptides and polypeptides of the invention can also be expressed by a vector, e.g., a nucleic acid molecule as herein-discussed, e.g., RNA or a DNA plasmid, a viral vector such as a poxvirus, e.g., orthopox virus, avipox virus, or adenovirus, AAV or lentivirus. This approach involves the use of a vector to express nucleotide sequences that encode the peptide of the invention. Upon introduction into an acutely or chronically infected host or into a noninfected host, the vector expresses the immunogenic peptide, and thereby elicits a host CTL response.

Among vectors that may be used in the practice of the disclosure, integration in the host genome of a cell is possible with retrovirus gene transfer methods, often resulting in long term expression of the inserted transgene. In some cases, the retrovirus is a lentivirus. Additionally, high transduction efficiencies have been observed in many different cell types and target tissues. The tropism of a retrovirus can be altered by incorporating foreign envelope proteins, expanding the potential target population of target cells. A retrovirus can also be engineered to allow for conditional expression of the inserted transgene, such that only certain cell types are infected by the lentivirus. Cell type specific promoters can be used to target expression in specific cell types. Lentiviral vectors are retroviral vectors (and hence both lentiviral and retroviral vectors may be used in the practice of the disclosure). Moreover, lentiviral vectors are able to transduce or infect non-dividing cells and typically produce high viral titers. Selection of a retroviral gene transfer system may therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the desired nucleic acid into the target cell to provide permanent expression. Widely used retroviral vectors that may be used in the practice of the disclosure include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., (1992) J. Virol. 66:2731-2739; Johann et al., (1992) J. Virol.66:1635-1640; Sommnerfelt et al., (1990) Virol.176:58-59; Wilson et al., (1998) J. Virol.63:2374-2378; Miller et al., (1991) J. Virol.65:2220-2224; PCT/US94/05700).

Also useful in the practice of the disclosure is a minimal non-primate lentiviral vector, such as a lentiviral vector based on the equine infectious anemia virus (EIAV). The vectors may have cytomegalovirus (CMV) promoter driving expression of the target gene. Accordingly, the disclosure contemplates amongst vector(s) useful in the practice of the disclosure: viral vectors, including retroviral vectors and lentiviral vectors.

In a case herein the delivery is via a lentivirus. Dosages, e.g., 10 $\mu$l of a recombinant lentivirus having a titer of 1 x $10^9$ transducing units (TU)/mL, can be adapted or extrapolated to use of a retroviral or lentiviral vector in the present disclosure. For transduction in tissues such as the brain, it is necessary to use very small volumes, so the viral preparation is concentrated by ultracentrifugation. Other methods of concentration such as ultrafiltration or binding to and elution from a matrix may be used. In other cases the amount of lentivirus administered may be $1 \times 10^5$ or about $1 \times 10^5$ plaque forming units (PFU), $5 \times 10^5$ or about $5 \times 10^5$ PFU, $1 \times 10^6$ or about $1. \times 10^6$ PFU, $5 \times 10^6$ or about $5 \times 10^6$ PFU, $1 \times 10^7$ or about $1 \times 107$ PFU, $5 \times 10^7$ or about $5 \times 10^7$ PFU, $1 \times 10^8$ or about $1 \times 10^8$ PFU, $5 \times 10^8$ or about $5 \times 10^8$ PFU, $1 \times 10^9$ or about $1 \times 10^9$ PFU, $5 \times 10^9$ or about $5 \times 10^9$ PFU, $1 \times 10^{10}$ or about $1 \times 10^{10}$ PFU or $5 \times 10^{10}$ or about $5 \times 10^{10}$ PFU as total single dosage for an average human of 75 kg or adjusted for the weight and size and species of the subject. One of skill in the art can determine suitable dosage. Suitable dosages for a virus can be determined empirically.

Also useful in the practice of the disclosure is an adenovirus vector. One advantage is the ability of recombinant adenoviruses to efficiently transfer and express recombinant genes in a variety of mammalian cells and tissues in vitro and in vivo, resulting in the high expression of the transferred nucleic acids. Further, the ability to productively infect quiescent cells, expands the utility of recombinant adenoviral vectors. In addition, high expression levels ensure that the products of the nucleic acids will be expressed to sufficient levels to generate an immune response (see e.g., U.S. Patent No.7,029,848). As to adenovirus vectors useful in the practice of the disclosure, mention is made of US Patent No.6,955,808. The adenovirus vector used can be selected from the group consisting of the Ad5, Ad35, Ad11, C6, and C7 vectors. The sequence of the Adenovirus 5 ("Ad5") genome has been published. (Chroboczek, J., Bieber, F., and Jacrot, B. (1992) The Sequence of the Genome of Adenovirus Type 5 and Its Comparison with the Genome of Adenovirus Type 2, Virology 186, 280-285). Ad35 vectors are described in U.S. Pat. Nos.6,974,695, 6,913,922, and 6,869,794. Ad11 vectors are described in U.S. Pat. No. 6,913,922. C6 adenovirus vectors are described in U.S. Pat. Nos. 6,780,407; 6,537,594; 6,309,647; 6,265,189; 6,156,567; 6,090,393; 5,942,235 and 5,833,975. C7 vectors are described in U.S. Pat. No. 6,277,558. Adenovirus vectors that are E1-defective or deleted, E3- defective or deleted, and/or E4-defective or deleted may also be used. Certain adenoviruses having mutations in the E1 region have improved safety margin because E1-defective adenovirus mutants are replication-defective in non-permissive cells, or, at the very least, are highly attenuated. Adenoviruses having mutations in the E3 region may have enhanced the immunogenicity by disrupting the mechanism whereby adenovirus down-regulates MHC class I molecules. Adenoviruses having E4 mutations may have reduced immunogenicity of the adenovirus vector because of suppression of late gene expression. Such vectors may be particularly useful when repeated re-vaccination utilizing the same vector is desired. Adenovirus vectors that are deleted or mutated in E1, E3, E4, E1 and E3, and E1 and E4 can be used in accordance with the present disclosure. Furthermore, "gutless" adenovirus vectors, in which all viral genes are deleted, can also be used in accordance with the present disclosure. Such vectors require a helper virus for their replication and require a special human 293 cell line expressing both E1a and Cre, a condition that does not exist in natural environment. Such "gutless" vectors are non-immunogenic and thus the vectors may be inoculated multiple times for re-vaccination. The "gutless" adenovirus vectors can be used for insertion of heterologous inserts/genes such as the transgenes of the present disclosure, and can even be used for co-delivery of a large number of heterologous inserts/genes. In some cases, the delivery is via an adenovirus, which may be at a single booster dose. In some cases, the adenovirus is delivered via multiple doses. In terms of in vivo delivery, AAV is advantageous over other viral vectors due to low toxicity and low probability of causing insertional mutagenesis because it doesn't integrate into the host genome. AAV has a packaging limit of 4.5 or 4.75 Kb. Constructs larger than 4.5 or 4.75 Kb result in significantly reduced virus production. There are many promoters that can be used to drive nucleic acid molecule expression. AAV ITR can serve as a promoter and is advantageous for eliminating the need for an additional promoter element. For ubiquitous expression, the following promoters can be used: CMV, CAG, CBh, PGK, SV40, Ferritin heavy or light chains, etc. For brain expression, the following promoters can be used: SynapsinI for all neurons, CaMKIIalpha for excitatory neurons, GAD67 or GAD65 or VGAT for GABAergic neurons, etc. Promoters used to drive RNA synthesis can include: Pol III promoters such as U6 or H1. The use of a Pol II promoter and intronic cassettes can be used to express guide RNA (gRNA). With regard to AAV vectors useful in the practice of the disclosure, mention is made of US Patent Nos. 5658785, 7115391, 7172893, 6953690, 6936466, 6924128, 6893865, 6793926, 6537540, 6475769 and 6258595, and documents cited therein. As to AAV, the AAV can be AAV1, AAV2, AAV5 or any combination thereof. One can select the AAV with regard to the cells to be targeted; e.g., one can select AAV serotypes 1, 2, 5 or a hybrid capsid AAV1, AAV2, AAV5 or any combination thereof for targeting brain or neuronal cells; and one can select AAV4 for targeting cardiac tissue. AAV8 is useful for delivery to the liver. In some cases the delivery is via an AAV. The dosage may be adjusted to balance the therapeutic benefit against any side effects.

In some cases, effectively activating a cellular immune response for a disease vaccine or immunogenic composition can be achieved by expressing the relevant antigens in a vaccine or immunogenic composition in a non-pathogenic microorganism. Well-known examples of such microorganisms are Mycobacterium bovis BCG, Salmonella and Pseudomona (See, U.S. Patent No.6,991,797).

In some cases, a Poxvirus is used in the disease vaccine or immunogenic composition. These include orthopoxvirus, avipox, vaccinia, MVA, NYVAC, canarypox, ALVAC, fowlpox, TROVAC, etc. (see e.g., Verardi et al., Hum Vaccin Immunother. 2012 Jul;8(7):961-70; and Moss, Vaccine. 2013; 31(39): 4220-4222). Poxvirus expression vectors were described in 1982 and quickly became widely used for vaccine development as well as research in numerous fields. Advantages of the vectors include simple construction, ability to accommodate large amounts of foreign DNA and high expression levels. Information concerning poxviruses that may be used in the practice of the disclosure, such as Chordopoxvirinae subfamily poxviruses (poxviruses of vertebrates), for instance, orthopoxviruses and avipoxviruses, e.g., vaccinia virus (e.g., Wyeth Strain, WR Strain (e.g., ATCC® VR-1354), Copenhagen Strain, NYVAC, NYVAC.1, NYVAC.2, MVA, MVA-BN), canarypox virus (e.g., Wheatley C93 Strain, ALVAC), fowlpox virus (e.g., FP9 Strain, Webster Strain, TROVAC), dovepox, pigeonpox, quailpox, and raccoon pox, inter alia, synthetic or non- naturally occurring recombinants thereof, uses thereof, and methods for making and using such recombinants may be found in scientific and patent literature.

In some cases, the vaccinia virus is used in the disease vaccine or immunogenic composition to express a antigen. (Rolph et al., Recombinant viruses as vaccines and immunological tools. Curr Opin Immunol 9:517-524, 1997). The recombinant vaccinia virus is able to replicate within the cytoplasm of the infected host cell and the polypeptide of interest can therefore induce an immune response. Moreover, Poxviruses have been widely used as vaccine or immunogenic composition vectors because of their ability to target encoded antigens for processing by the major histocompatibility complex class I pathway by directly infecting immune cells, in particular antigen-presenting cells, but also due to their ability to self-adjuvant.

In some cases, ALVAC is used as a vector in a disease vaccine or immunogenic composition. ALVAC is a canarypox virus that can be modified to express foreign transgenes and has been used as a method for vaccination against both prokaryotic and eukaryotic antigens (Horig H, Lee DS, Conkright W, et al. Phase I clinical trial of a recombinant canarypoxvirus (ALVAC) vaccine expressing human carcinoembryonic antigen and the B7.1 co-stimulatory molecule. Cancer Immunol Immunother 2000;49:504-14; von Mehren M, Arlen P, Tsang KY, et al. Pilot study of a dual gene recombinant avipox vaccine containing both carcinoembryonic antigen (CEA) and B7.1 transgenes in patients with recurrent CEA-expressing adenocarcinomas. Clin Cancer Res 2000;6:2219-28; Musey L, Ding Y, Elizaga M, et al. HIV-1 vaccination administered intramuscularly can induce both systemic and mucosal T cell immunity in HIV-1-uninfected individuals. J Immunol 2003;171:1094-101; Paoletti E. Applications of pox virus vectors to vaccination: an update. Proc Natl Acad Sci U S A 1996;93:11349-53; U.S. Patent No.7,255,862). In a phase I clinical trial, an ALVAC virus expressing the tumor antigen CEA showed an excellent safety profile and resulted in increased CEA-specific T cell responses in selected patients; objective clinical responses, however, were not observed (Marshall JL, Hawkins MJ, Tsang KY, et al. Phase I study in cancer patients of a replication-defective avipox recombinant vaccine that expresses human carcinoembryonic antigen. J Clin Oncol 1999;17:332-7).

In some cases, a Modified Vaccinia Ankara (MVA) virus may be used as a viral vector for an antigen vaccine or immunogenic composition. MVA is a member of the Orthopoxvirus family and has been generated by about 570 serial passages on chicken embryo fibroblasts of the Ankara strain of Vaccinia virus (CVA) (for review see Mayr, A., et al., Infection 3, 6-14, 1975). As a consequence of these passages, the resulting MVA virus contains 31 kilobases less genomic information compared to CVA, and is highly hosT cell restricted (Meyer, H. et al., J. Gen. Virol. 72, 1031-1038, 1991). MVA is characterized by its extreme attenuation, namely, by a diminished virulence or infectious ability, but still holds an excellent immunogenicity. When tested in a variety of animal models, MVA was proven to be avirulent, even in immuno-suppressed individuals. Moreover, MVA-BN®-HER2 is a candidate immunotherapy designed for the treatment of HER-2-positive breast cancer and is currently in clinical trials. (Mandl et al., Cancer Immunol Immunother. Jan 2012; 61(1): 19-29). Methods to make and use recombinant MVA has been described (e.g., see U.S. Patent Nos. 8,309,098 and 5,185,146 hereby incorporated in its entirety).

In some cases, recombinant viral particles of the vaccine or immunogenic composition are administered to patients in need thereof.

## Neoantigen binding peptides

In certain cases, the present disclosure provides a binding protein (e.g., an antibody or antigen-binding fragment thereof), or a T cell receptor (TCR), or a chimeric antigen receptor (CAR) capable of binding with a high affinity to a neoantigen peptide:human leukocyte antigen (HLA) complex. In some cases, the present disclosure provides a CAR that is capable of binding with a high affinity to a neoantigenic peptide derived from the extracellular domain of a protein. In certain cases, a neoantigen-specific binding protein or TCR or CAR as described herein includes variant polypeptide species that have one or more amino acid substitutions, insertions, or deletions in the native amino acid sequence, provided that the binding protein retains or substantially retains its specific binding function. Conservative substitutions of amino acids are well known and may occur naturally or may be introduced when the binding protein or TCR is recombinantly produced. Amino acid substitutions, deletions, and additions may be introduced into a protein using mutagenesis methods known in the art (see, e.g., Sambrook et al., Molecular Cloning: A Laboratory Manual, 3d ed., Cold Spring Harbor Laboratory Press, N Y, 2001). Oligonucleotide-directed site-specific (or segment specific) mutagenesis procedures may be employed to provide an altered polynucleotide that has particular codons altered according to the substitution, deletion, or insertion desired. Alternatively, random or saturation mutagenesis techniques, such as alanine scanning mutagenesis, error prone polymerase chain reaction mutagenesis, and oligonucleotide-directed mutagenesis may be used to prepare immunogen polypeptide variants (see, e.g., Sambrook et al., supra).

A variety of criteria known to persons skilled in the art indicate whether an amino acid that is substituted at a particular position in a peptide or polypeptide is conservative (or similar). For example, a similar amino acid or a conservative amino acid substitution is one in which an amino acid residue is replaced with an amino acid residue having a similar side chain. Similar amino acids may be included in the following categories: amino acids with basic side chains (e.g., lysine, arginine, histidine); amino acids with acidic side chains (e.g., aspartic acid, glutamic acid); amino acids with uncharged polar side chains (e.g., glycine, asparagine, glutamine, serine, threonine, tyrosine, cysteine, histidine); amino acids with nonpolar side chains (e.g., alanine, valine, leucine, isoleucine, proline, phenylalanine, methionine, tryptophan); amino acids with beta-branched side chains (e.g., threonine, valine, isoleucine), and amino acids with aromatic side chains (e.g., tyrosine, phenylalanine, tryptophan). Proline, which is considered more difficult to classify, shares properties with amino acids that have aliphatic side chains (e.g., leucine, valine, isoleucine, and alanine) In certain circumstances, substitution of glutamine for glutamic acid or asparagine for aspartic acid may be considered a similar substitution in that glutamine and asparagine are amide derivatives of glutamic acid and aspartic acid, respectively. As understood in the art "similarity" between two polypeptides is determined by comparing the amino acid sequence and conserved amino acid substitutes thereto of the polypeptide to the sequence of a second polypeptide (e.g., using GENEWORKS, Align, the BLAST algorithm, or other algorithms described herein and practiced in the art).

In certain cases, a neoantigen specific binding protein, TCR or CAR is capable of (a) specifically binding to a neoantigen:HLA complex on a cell surface independent or in the absence of CD8. In certain cases, a neoantigen specific binding protein is a T cell receptor (TCR), a chimeric antigen receptor or an antigen-binding fragment of a TCR, any of which can be chimeric, humanized or human. In further cases, an antigen-binding fragment of the TCR comprises a single chain TCR (scTCR).

In certain cases, there is provided a composition comprising a neoantigen-specific binding protein or high affinity recombinant TCR according to any one of the above cases and a pharmaceutically acceptable carrier, diluent, or excipient.

Methods useful for isolating and purifying recombinantly produced soluble TCR, by way of example, can include obtaining supernatants from suitable host cell/vector systems that secrete the recombinant soluble TCR into culture media and then concentrating the media using a commercially available filter. Following concentration, the concentrate can be applied to a single suitable purification matrix or to a series of suitable matrices, such as an affinity matrix or an ion exchange resin. One or more reverse phase HPLC steps may be employed to further purify a recombinant polypeptide. These purification methods can also be employed when isolating an immunogen from its natural environment. Methods for large scale production of one or more of the isolated/recombinant soluble TCR described herein include batch cell culture, which is monitored and controlled to maintain appropriate culture conditions. Purification of the soluble TCR may be performed according to methods described herein and known in the art.

### III. Immunogenic and vaccine compositions

In some cases the present disclosure is directed to an immunogenic composition, e.g., a vaccine composition capable of raising a neoantigen-specific response (e.g., a humoral or cell-mediated immune response). In some cases, the immunogenic composition comprises neoantigen therapeutics (e.g., peptides, polynucleotides, TCR, CAR, cells containing TCR or CAR, dendritic cell containing polypeptide, dendritic cell containing polynucleotide, antibody, etc.) described herein corresponding to tumor specific neoantigen identified herein.

In some cases, immunogenic peptides are identified from one or more subjects with a disease or condition. In some cases, immunogenic peptides are specific to one or more subjects with a disease or condition. In some cases, immunogenic peptides can bind to an HLA that is matched to an HLA haplotype of one or more subjects with a disease or condition.

A person skilled in the art will be able to select neoantigenic therapeutics by testing, for example, the generation of T cells in vitro as well as their efficiency and overall presence, the proliferation, affinity and expansion of certain T cells for certain peptides, and the functionality of the T cells, e.g. by analyzing the IFN-$\gamma$ production or tumor killing by T cells. The most efficient peptides can then combined as an immunogenic composition.

In some cases of the present disclosure the different neoantigenic peptides and/or polypeptides are selected so that one immunogenic composition comprises neoantigenic peptides and/or polypeptides capable of associating with different MHC molecules, such as different MHC class I molecule. In some cases, an immunogenic composition comprises neoantigenic peptides and/or polypeptides capable of associating with the most frequently occurring MHC class I molecules. Hence immunogenic compositions described herein comprise different peptides capable of associating with at least 2, at least 3, or at least 4 MHC class I or class II molecules.

In some cases, an immunogenic composition described herein is capable of raising a specific cytotoxic T cells response, specific helper T cell response, or a B cell response.

In some cases, an immunogenic composition described herein can further comprise an adjuvant and/or a carrier. Examples of useful adjuvants and carriers are given herein below. Polypeptides and/or polynucleotides in the composition can be associated with a carrier such as e.g. a protein or an antigen-presenting cell such as e.g. a dendritic cell (DC) capable of presenting the peptide to a T cell or a B cell. In further cases, DC-binding peptides are used as carriers to target the neoantigenic peptides and polynucleotides encoding the neoantigen peptides to dendritic cells (Sioud et al. FASEB J 27: 3272-3283 (2013)).

In cases, the neoantigenic polypeptides or polynucleotides can be provided as antigen presenting cells (e.g., dendritic cells) containing such polypeptides or polynucleotides. In other cases, such antigen presenting cells are used to stimulate T cells for use in patients.

In some cases, the antigen presenting cells are dendritic cells. In related cases, the dendritic cells are autologous dendritic cells that are pulsed with the neoantigenic peptide or nucleic acid. The neoantigenic peptide can be any suitable peptide that gives rise to an appropriate T cell response. T cell therapy using autologous dendritic cells pulsed with peptides from a tumor associated antigen is disclosed in Murphy et al. (1996) The Prostate 29, 371-380 and Tjua et al. (1997) The Prostate 32, 272-278. In some cases, the T cell is a CTL. In some cases, the T cell is a HTL.

Thus, one case of the present disclosure an immunogenic composition containing at least one antigen presenting cell (e.g., a dendritic cell) that is pulsed or loaded with one or more neoantigenic polypeptides or polynucleotides described herein. In cases, such APCs are autologous (e.g., autologous dendritic cells). Alternatively, peripheral blood mononuclear cells (PBMCs) isolated from a patient can be loaded with neoantigenic peptides or polynucleotides ex vivo. In related cases, such APCs or PBMCs are injected back into the patient.

The polynucleotide can be any suitable polynucleotide that is capable of transducing the dendritic cell, thus resulting in the presentation of a neoantigenic peptide and induction of immunity. In some cases, the polynucleotide can be naked DNA that is taken up by the cells by passive loading. In another case, the polynucleotide is part of a delivery vehicle, for example, a liposome, virus like particle, plasmid, or expression vector. In another case, the polynucleotide is delivered by a vector-free delivery system, for example, high performance electroporation and high-speed cell deformation). In cases, such antigen presenting cells (APCs) (e.g., dendritic cells) or peripheral blood mononuclear cells (PBMCs) are used to stimulate a T cell (e.g., an autologous T cell). In related cases, the T cell is a CTL. In other related cases, the T cell is an HTL. Such T cells are then injected into the patient. In some cases, CTL is injected into the patient. In some cases, HTL is injected into the patient. In some cases, both CTL and HTL are injected into the patient. Administration of either therapeutic can be performed simultaneously or sequentially and in any order.

The pharmaceutical compositions (e.g., immunogenic compositions) described herein for therapeutic treatment are intended for parenteral, topical, nasal, oral or local administration. In some cases, the pharmaceutical compositions described herein are administered parenterally, e.g., intravenously, subcutaneously, intradermally, or intramuscularly. In cases, the composition can be administered intratumorally. The compositions can be administered at the site of surgical excision to induce a local immune response to the tumor. In some cases, described herein are compositions for parenteral administration which comprise a solution of the neoantigenic peptides and immunogenic compositions are dissolved or suspended in an acceptable carrier, for example, an aqueous carrier. A variety of aqueous carriers can be used, e.g., water, buffered water, 0.9% saline, 0.3% glycine, hyaluronic acid and the like. These compositions can be sterilized by conventional, well known sterilization techniques, or can be sterile filtered. The resulting aqueous solutions can be packaged for use as is, or lyophilized, the lyophilized preparation being combined with a sterile solution prior to administration. The compositions can contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of neoantigenic peptides and polynucleotides described herein in the pharmaceutical formulations can vary widely, i.e., from less than about 0.1%, usually at or at least about 2% to as much as 20% to 50% or more by weight, and will be selected by fluid volumes, viscosities, etc., according to the particular mode of administration selected.

The neoantigenic peptides and polynucleotides described herein can also be administered via liposomes, which target the peptides to a particular cells tissue, such as lymphoid tissue. Liposomes are also useful in increasing the half-life of the peptides. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the DEC205 antigen, or with other therapeutic or immunogenic compositions. Thus, liposomes filled with a desired peptide or polynucleotide described herein can be directed to the site of lymphoid cells, where the liposomes then deliver the selected therapeutic/immunogenic polypeptide/polynucleotide compositions. Liposomes can be formed from standard vesicle-forming lipids, which generally include neutral and negatively charged phospholipids and a sterol, for example, cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9; 467 (1980), U.S. Pat. Nos. 4,235,871, 4,501,728, 4,501,728, 4,837,028, and 5,019,369.

For targeting to the immune cells, a neoantigen polypeptides or polynucleotides to be incorporated into the liposome for cell surface determinants of the desired immune system cells. A liposome suspension containing a peptide can be administered intravenously, locally, topically, etc. in a dose which varies according to, inter alia, the manner of administration, the polypeptide or polynucleotide being delivered, and the stage of the disease being treated.

In some cases, neoantigen polypeptides and polynucleotides are targeted to dendritic cells. In some cases, the neoantigen polypeptides and polynucleotides are target to dendritic cells using the markers DEC205, XCR1, CD197, CD80, CD86, CD123, CD209, CD273, CD283, CD289, CD184, CD85h, CD85j, CD85k, CD85d, CD85g, CD85a, TSLP receptor, Clec9a or CD1a.

For solid compositions, conventional or nanoparticle nontoxic solid carriers can be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more neoantigenic polypeptides or polynucleotides described herein at a concentration of 25%-75%.

For aerosol administration, the neoantigenic polypeptides or polynucleotides can be supplied in finely divided form along with a surfactant and propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides can be employed. The surfactant can constitute 0.1%-20% by weight of the composition, or 0.25-5%. The balance of the composition can be propellant. A carrier can also be included as desired, as with, e.g., lecithin for intranasal delivery.

Additional methods for delivering the neoantigenic polynucleotides described herein are also known in the art. For instance, the nucleic acid can be delivered directly, as "naked DNA". This approach is described, for instance, in Wolff et al., Science 247: 1465-1468 (1990) as well as U.S. Pat. Nos. 5,580,859 and 5,589,466. The nucleic acids can also be administered using ballistic delivery as described, for instance, in U.S. Pat. No. 5,204,253. Particles comprised solely of DNA can be administered. Alternatively, DNA can be adhered to particles, such as gold particles.

For therapeutic or immunization purposes, mRNA encoding the neoantigenic peptides, or peptide binding agents can also be administered to the patient. In some cases, the mRNA is self-amplifying RNA. In a further case, the self-amplifying RNA is a part of a synthetic lipid nanoparticle formulation (Geall et al., Proc Natl Acad Sci U S A. 109: 14604-14609 (2012)).

The nucleic acids can also be delivered complexed to cationic compounds, such as cationic lipids. Lipid-mediated gene delivery methods are described, for instance, in WO 96/18372, WO 93/24640; Mannino & Gould-Fogerite, BioTechniques 6(7): 682-691 (1988); U.S. Pat. No. 5,279,833; WO 91/06309; and Felgner et al., Proc. Natl. Acad. Sci. USA 84: 7413-7414 (1987).

The neoantigenic peptides and polypeptides described herein can also be expressed by attenuated viruses, such as vaccinia or fowlpox. This approach involves the use of vaccinia virus as a vector to express nucleotide sequences that encode the peptide described herein. Upon introduction into an acutely or chronically infected host or into a noninfected host, the recombinant vaccinia virus expresses the immunogenic peptide, and thereby elicits a host CTL response. Vaccinia vectors and methods useful in immunization protocols are described in, e.g., U.S. Pat. No. 4,722,848. Another vector is BCG (Bacille Calmette Guerin). BCG vectors are described in Stover et al. (Nature 351:456-460 (1991)). A wide variety of other vectors useful for therapeutic administration or immunization of the peptides described herein will be apparent to those skilled in the art from the description herein.

Adjuvants are any substance whose admixture into the immunogenic composition increases or otherwise modifies the immune response to the therapeutic agent. Carriers are scaffold structures, for example a polypeptide or a polysaccharide, to which a neoantigenic polypeptide or polynucleotide, is capable of being associated. Optionally, adjuvants are conjugated covalently or non-covalently to the polypeptides or polynucleotides described herein.

The ability of an adjuvant to increase the immune response to an antigen is typically manifested by a significant increase in immune-mediated reaction, or reduction in disease symptoms. For example, an increase in humoral immunity can be manifested by a significant increase in the titer of antibodies raised to the antigen, and an increase in T cell activity can be manifested in increased cell proliferation, or cellular cytotoxicity, or cytokine secretion. An adjuvant can also alter an immune response, for example, by changing a primarily humoral or T helper 2 response into a primarily cellular, or T helper 1 response.

Suitable adjuvants are known in the art (see, WO 2015/095811) and include, but are not limited to poly(I:C), poly-ICLC, STING agonist, 1018 ISS, aluminium salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312, Montanide ISA 206, Montanide ISA 50V, Montanide ISA-51, OK-432, OM-174, OM-197-MP-EC, ONTAK, PepTel®. vector system, PLG microparticles, resiquimod, SRL172, virosomes and other virus-like particles, YF-17D, VEGF trap, R848, beta-glucan, Pam3Cys, Pam3CSK4, Aquila's QS21 stimulon (Aquila Biotech, Worcester, Mass., USA) which is derived from saponin, mycobacterial extracts and synthetic bacterial cell wall mimics, and other proprietary adjuvants such as Ribi's Detox. Quil or Superfos. Adjuvants also include incomplete Freund's or GM-CSF. Several immunological adjuvants (e.g., MF59) specific for dendritic cells and their preparation have been described previously (Dupuis M, et al., Cell Immunol. 1998; 186(1):18-27; Allison A C; Dev Biol Stand. 1998; 92:3-11) (Mosca et al. Frontiers in Bioscience, 2007; 12:4050-4060) (Gamvrellis et al. Immunol & Cell Biol. 2004; 82: 506-516).. Also cytokines can be used. Several cytokines have been directly linked to influencing dendritic cell migration to lymphoid tissues (e.g., TNF-alpha), accelerating the maturation of dendritic cells into efficient antigen-presenting cells for T-lymphocytes (e.g., GM-CSF, PGE1, PGE2, IL-1, IL-1b, IL-4, IL-6 and CD40L) (U.S. Pat. No. 5,849,589 incorporated herein by reference in its entirety) and acting as immunoadjuvants (e.g., IL-12) (Gabrilovich D I, et al., J Immunother Emphasis Tumor Immunol. 1996 (6):414-418).

CpG immunostimulatory oligonucleotides have also been reported to enhance the effects of adjuvants in a vaccine setting. Without being bound by theory, CpG oligonucleotides act by activating the innate (non-adaptive) immune system via Toll-like receptors (TLR), mainly TLR9. CpG triggered TLR9 activation enhances antigen-specific humoral and cellular responses to a wide variety of antigens, including peptide or protein antigens, live or killed viruses, dendritic cell immunogenic pharmaceutical compositions, autologous cellular immunogenic pharmaceutical compositions and polysaccharide conjugates in both prophylactic and therapeutic immunogenic pharmaceutical compositions. Importantly, it enhances dendritic cell maturation and differentiation, resulting in enhanced activation of TH1 cells and strong cytotoxic T-lymphocyte (CTL) generation, even in the absence of CD4 T cell help. The TH1 bias induced by TLR9 stimulation is maintained even in the presence of adjuvants such as alum or incomplete Freund's adjuvant (IFA) that normally promote a TH2 bias. CpG oligonucleotides show even greater adjuvant activity when formulated or co-administered with other adjuvants or in formulations such as microparticles, nano particles, lipid emulsions or similar formulations, which are especially necessary for inducing a strong response when the antigen is relatively weak. They also accelerate the immune response and enabled the antigen doses to be reduced with comparable antibody responses to the full-dose immunogenic pharmaceutical composition without CpG in some experiments (Arthur M. Krieg, Nature Reviews, Drug Discovery, 5, June 2006, 471-484). U.S. Pat. No. 6,406,705 B1 describes the combined use of CpG oligonucleotides, non-nucleic acid adjuvants and an antigen to induce an antigen-specific immune response. A commercially available CpG TLR9 antagonist is dSLIM (double Stem Loop Immunomodulator) by Mologen (Berlin, GERMANY), which is a component of the pharmaceutical composition described herein. Other TLR binding molecules such as RNA binding TLR 7, TLR 8 and/or TLR 9 can also be used.

Other examples of useful adjuvants include, but are not limited to, chemically modified CpGs (e.g. CpR, Idera), Poly(I:C)(e.g. polyi:CI2U), non-CpG bacterial DNA or RNA, ssRNA40 for TLR8, as well as immunoactive small molecules and antibodies such as cyclophosphamide, sunitinib, bevacizumab, celebrex, NCX-4016, sildenafil, tadalafil, vardenafil, sorafinib, XL-999, CP-547632, pazopanib, ZD2171, AZD2171, ipilimumab, tremelimumab, and SC58175, which can act therapeutically and/or as an adjuvant. The amounts and concentrations of adjuvants and additives useful in the context of the present disclosure can readily be determined by the skilled artisan without undue experimentation. Additional adjuvants include colony-stimulating factors, such as Granulocyte Macrophage Colony Stimulating Factor (GM-CSF, sargramostim).

In some cases, an immunogenic composition according to the present disclosure can comprise more than one different adjuvants. Furthermore, the disclosure encompasses a therapeutic composition comprising any adjuvant substance including any of the above or combinations thereof. It is also contemplated that the neoantigenic therapeutic (e.g., a humoral or cell-mediated immune response). In some cases, the immunogenic composition comprises neoantigen therapeutics (e.g., peptides, polynucleotides, TCR, CAR, cells containing TCR or CAR, dendritic cell containing polypeptide, dendritic cell containing polynucleotide, antibody, etc.) and the adjuvant can be administered separately in any appropriate sequence.

A carrier can be present independently of an adjuvant. The function of a carrier can for example be to increase the molecular weight of in particular mutant in order to increase their activity or immunogenicity, to confer stability, to increase the biological activity, or to increase serum half-life. Furthermore, a carrier can aid presenting peptides to T cells. The carrier can be any suitable carrier known to the person skilled in the art, for example a protein or an antigen presenting cell. A carrier protein could be but is not limited to keyhole limpet hemocyanin, serum proteins such as transferrin, bovine serum albumin, human serum albumin, thyroglobulin or ovalbumin, immunoglobulins, or hormones, such as insulin or palmitic acid. In some cases, the carrier comprises a human fibronection type III domain (Koide et al. Methods Enzymol. 2012;503:135-56). For immunization of humans, the carrier must be a physiologically acceptable carrier acceptable to humans and safe. However, tetanus toxoid and/or diptheria toxoid are suitable carriers In some cases of the disclosure. Alternatively, the carrier can be dextrans for example sepharose.

In some cases, the polypeptides can be synthesized as multiply linked peptides as an alternative to coupling a polypeptide to a carrier to increase immunogenicity. Such molecules are also known as multiple antigenic peptides (MAPS).

Neoantigens that induce an immune response can be used as a composition when combined with an acceptable carrier or excipient. Such compositions are useful for *in vitro* or *in vivo* analysis or for administration to a subject *in vivo* or *ex vivo* for treating a subject with a disease.

Thus pharmaceutical compositions can include, in addition to active ingredient, a pharmaceutically acceptable excipient, carrier, buffer, stabilizer or other materials well known to those skilled in the art. Such materials should be non-toxic and should not interfere with the efficacy of the active ingredient. The precise nature of the carrier or other material will depend on the route of administration.

Pharmaceutical formulations comprising a protein of interest, *e.g.*, a neoantigen described herein, can be prepared for storage by mixing the neoantigen having the desired degree of purity with optional physiologically acceptable carriers, excipients or stabilizers (Remington's Pharmaceutical Sciences 16th edition, Oslo, A. Ed. (1980)), in the form of lyophilized formulations or aqueous solutions. Acceptable carriers, excipients, or stabilizers are those that are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as TWEEN®, PLURONICS® or polyethylene glycol (PEG).

Acceptable carriers are physiologically acceptable to the administered patient and retain the therapeutic properties of the compounds with/in which it is administered. Acceptable carriers and their formulations are generally described in, for example, Remington' pharmaceutical Sciences (18th Edition, ed. A. Gennaro, Mack Publishing Co., Easton, PA 1990). One exemplary carrier is physiological saline. A pharmaceutically acceptable carrier is a pharmaceutically acceptable material, composition or vehicle, such as a liquid or solid filler, diluent, excipient, solvent or encapsulating material, involved in carrying or transporting the subject compounds from the administration site of one organ, or portion of the body, to another organ, or portion of the body, or in an *in vitro* assay system. Acceptable carriers are compatible with the other ingredients of the formulation and not injurious to a subject to whom it is administered. Nor should an acceptable carrier alter the specific activity of the neoantigens.

In one aspect, provided herein are pharmaceutically acceptable or physiologically acceptable compositions including solvents (aqueous or non-aqueous), solutions, emulsions, dispersion media, coatings, isotonic and absorption promoting or delaying agents, compatible with pharmaceutical administration. Pharmaceutical compositions or pharmaceutical formulations therefore refer to a composition suitable for pharmaceutical use in a subject. The pharmaceutical compositions and formulations include an amount of a neoantigen (or polynucleotide encoding a neoantigen) and a pharmaceutically or physiologically acceptable carrier. Compositions can be formulated to be compatible with a particular route of administration (i.e., systemic or local). Thus, compositions include carriers, diluents, or excipients suitable for administration by various routes.

In some cases, a composition further comprises an acceptable additive in order to improve the stability of the neoantigen in the composition and/or to control the release rate of the composition. Acceptable additives do not alter the specific activity of the neoantigens. Exemplary acceptable additives include, but are not limited to, a sugar such as mannitol, sorbitol, glucose, xylitol, trehalose, sorbose, sucrose, galactose, dextran, dextrose, fructose, lactose and mixtures thereof. Acceptable additives can be combined with acceptable carriers and/or excipients such as dextrose. Alternatively, exemplary acceptable additives include, but are not limited to, a surfactant such as polysorbate 20 or polysorbate 80 to increase stability of the peptide and decrease gelling of the solution. The surfactant can be added to the composition in an amount of 0.01% to 5% of the solution. Addition of such acceptable additives increases the stability and half-life of the composition in storage.

The pharmaceutical composition can be administered, for example, by injection. Compositions for injection include aqueous solutions (where water soluble) or dispersions and sterile powders for the extemporaneous preparation of sterile injectable solutions or dispersion. For intravenous administration, suitable carriers include physiological saline, bacteriostatic water, or phosphate buffered saline (PBS). The carrier can be a solvent or dispersion medium containing, for example, water, ethanol, polyol (for example, glycerol, propylene glycol, and liquid polyethylene glycol, and the like), and suitable mixtures thereof. Fluidity can be maintained, for example, by the use of a coating such as lecithin, by the maintenance of the required particle size in the case of dispersion and by the use of surfactants. Antibacterial and antifungal agents include, for example, parabens, chlorobutanol, phenol, ascorbic acid and thimerosal. Isotonic agents, for example, sugars, polyalcohols such as manitol, sorbitol, and sodium chloride can be included in the composition. The resulting solutions can be packaged for use as is, or lyophilized; the lyophilized preparation can later be combined with a sterile solution prior to administration. For intravenous, injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives can be included, as needed. Sterile injectable solutions can be prepared by incorporating an active ingredient in the required amount in an appropriate solvent with one or a combination of ingredients enumerated above, as required, followed by filtered sterilization. Generally, dispersions are prepared by incorporating the active ingredient into a sterile vehicle which contains a basic dispersion medium and the required other ingredients from those enumerated above. In the case of sterile powders for the preparation of sterile injectable solutions, the preferred methods of preparation are vacuum drying and freeze drying which yields a powder of the active ingredient plus any additional desired ingredient from a previously sterile-filtered solution thereof.

Compositions can be conventionally administered intravenously, such as by injection of a unit dose, for example. For injection, an active ingredient can be in the form of a parenterally acceptable aqueous solution which is substantially pyrogen-free and has suitable pH, isotonicity and stability. One can prepare suitable solutions using, for example, isotonic vehicles such as Sodium Chloride Injection, Ringer's Injection, Lactated Ringer's Injection. Preservatives, stabilizers, buffers, antioxidants and/or other additives can be included, as required. Additionally, compositions can be administered via aerosolization.

In some cases, the composition is lyophilized, for example, to increase shelf-life in storage. When the compositions are considered for use in medicaments or any of the methods provided herein, it is contemplated that the composition can be substantially free of pyrogens such that the composition will not cause an inflammatory reaction or an unsafe allergic reaction when administered to a human patient. Testing compositions for pyrogens and preparing compositions substantially free of pyrogens are well understood to one or ordinary skill of the art and can be accomplished using commercially available kits.

Acceptable carriers can contain a compound that stabilizes, increases or delays absorption, or increases or delays clearance. Such compounds include, for example, carbohydrates, such as glucose, sucrose, or dextrans; low molecular weight proteins; compositions that reduce the clearance or hydrolysis of peptides; or excipients or other stabilizers and/or buffers. Agents that delay absorption include, for example, aluminum monostearate and gelatin. Detergents can also be used to stabilize or to increase or decrease the absorption of the pharmaceutical composition, including liposomal carriers. To protect from digestion the compound can be complexed with a composition to render it resistant to acidic and enzymatic hydrolysis, or the compound can be complexed in an appropriately resistant carrier such as a liposome.

The compositions can be administered in a manner compatible with the dosage formulation, and in a therapeutically effective amount. The quantity to be administered depends on the subject to be treated, capacity of the subject's immune system to utilize the active ingredient, and degree of binding capacity desired. Precise amounts of active ingredient required to be administered depend on the judgment of the practitioner and are peculiar to each individual. Suitable regimes for initial administration and booster shots are also variable, but are typified by an initial administration followed by repeated doses at one or more hour intervals by a subsequent injection or other administration. Alternatively, continuous intravenous infusions sufficient to maintain concentrations in the blood are contemplated.

Peptide-based immunogenic pharmaceutical compositions can be formulated using any of the well-known techniques, carriers, and excipients as suitable and as understood in the art. The polypeptides can be a cocktail of multiple polypeptides containing the same sequence, or a cocktail of multiple copies of different polypeptides. The peptides can be modified, such as for example by lipidation, or attachment to a carrier protein. Lipidation can be the covalent attachment of a lipid group to a polypeptide. Lipidated peptides, or lipidated polypeptides, can stabilize structures and can enhance efficacy of the treatment.

Lipidation can be classified into several different types, such as N-myristoylation, palmitoylation, GPI-anchor addition, prenylation, and several additional types of modifications. N-myristoylation is the covalent attachment of myristate, a $C_{14}$ saturated acid, to a glycine residue. Palmitoylation is thioester linkage of long-chain fatty acids ($C_{16}$) to cysteine residues. GPI-anchor addition is glycosyl-phosphatidylinositol (GPI) linkage via amide bond. Prenylation is the thioether linkage of an isoprenoid lipid (e.g. farnesyl (C-15), geranylgeranyl (C-20)) to cysteine residues. Additional types of modifications can include attachment of S-diacylglycerol by a sulfur atom of cysteines, O-octanoyl conjugation via serine or threonine residues, S-archaeol conjugation to cysteine residues, and cholesterol attachment.

Fatty acids for generating a lipidated peptides can include $C_2$ to $C_{30}$ saturated, monounsaturated, or polyunsaturated fatty acyl groups. Exemplary fatty acids can include palmitoyl, myristoyl, stearoyl and decanoyl groups. In some instances, a lipid moiety that has adjuvant property is attached to a polypeptide of interest to elicit or enhance immunogenicity in the absence of an extrinsic adjuvant. A lipidated peptide or lipopeptide can be referred to as a self-adjuvant lipopeptide. Any of the fatty acids described above and elsewhere herein can elicit or enhance immunogenicity of a polypeptide of interest. A fatty acid that can elicit or enhance immunogenicity can include palmitoyl, myristoyl, stearoyl, lauroyl, octanoyl, and decanoyl groups.

Polypeptides such as naked peptides or lipidated peptides can be incorporated into a liposome. Sometimes, lipidated peptides can be incorporated into a liposome. For example, the lipid portion of the lipidated peptide can spontaneously integrate into the lipid bilayer of a liposome. Thus, a lipopeptide can be presented on the "surface" of a liposome.

Exemplary liposomes suitable for incorporation in the formulations include, and are not limited to, multilamellar vesicles (MLV), oligolamellar vesicles (OLV), unilamellar vesicles (UV), small unilamellar vesicles (SUV), medium-sized unilamellar vesicles (MUV), large unilamellar vesicles (LUV), giant unilamellar vesicles (GUV), multivesicular vesicles (MVV), single or oligolamellar vesicles made by reverse-phase evaporation method (REV), multilamellar vesicles made by the reverse-phase evaporation method (MLV-REV), stable plurilamellar vesicles (SPLV), frozen and thawed MLV (FATMLV), vesicles prepared by extrusion methods (VET), vesicles prepared by French press (FPV), vesicles prepared by fusion (FUV), dehydration-rehydration vesicles (DRV), and bubblesomes (BSV).

Depending on the method of preparation, liposomes can be unilamellar or multilamellar, and can vary in size with diameters ranging from about 0.02 $\mu$m to greater than about 10 $\mu$m. Liposomes can adsorb many types of cells and then release an incorporated agent (e.g., a peptide described herein). In some cases, the liposomes fuse with the target cell, whereby the contents of the liposome then empty into the target cell. A liposome can be endocytosed by cells that are phagocytic. Endocytosis can be followed by intralysosomal degradation of liposomal lipids and release of the encapsulated agents.

The liposomes provided herein can also comprise carrier lipids. In some cases the carrier lipids are phospholipids. Carrier lipids capable of forming liposomes include, but are not limited to dipalmitoylphosphatidylcholine (DPPC), phosphatidylcholine (PC; lecithin), phosphatidic acid (PA), phosphatidylglycerol (PG), phosphatidylethanolamine (PE), phosphatidylserine (PS). Other suitable phospholipids further include distearoylphosphatidylcholine (DSPC), dimyristoylphosphatidylcholine (DMPC), dipalmitoylphosphatidyglycerol (DPPG), distearoylphosphatidyglycerol (DSPG), dimyristoylphosphatidylglycerol (DMPG), dipalmitoylphosphatidic acid (DPPA); dimyristoylphosphatidic acid (DMPA), distearoylphosphatidic acid (DSPA), dipalmitoylphosphatidylserine (DPPS), dimyristoylphosphatidylserine (DMPS), distearoylphosphatidylserine (DSPS), dipalmitoylphosphatidyethanolamine (DPPE), dimyristoylphosphatidylethanolamine (DMPE), distearoylphosphatidylethanolamine (DSPE) and the like, or combinations thereof. In some cases, the liposomes further comprise a sterol (e.g., cholesterol) which modulates liposome formation. The carrier lipids can be any known non-phosphate polar lipids.

A pharmaceutical composition can be encapsulated within liposomes using well-known technology. Biodegradable microspheres can also be employed as carriers for the pharmaceutical compositions of this disclosure.

The pharmaceutical composition can be administered in liposomes or microspheres (or microparticles). Methods for preparing liposomes and microspheres for administration to a patient are well known to those of skill in the art. Essentially, material is dissolved in an aqueous solution, the appropriate phospholipids and lipids added, along with surfactants if required, and the material dialyzed or sonicated, as necessary.

Microspheres formed of polymers or proteins are well known to those skilled in the art, and can be tailored for passage through the gastrointestinal tract directly into the blood stream. Alternatively, the compound can be incorporated and the microspheres, or composite of microspheres, implanted for slow release over a period of time ranging from days to months.

A polypeptide can also be attached to a carrier protein for delivery. The carrier protein can be an immunogenic carrier element and can be attached by any recombinant technology. Exemplary carrier proteins include Mariculture keyhole limpet hemocyanin (mcKLH), PEGylated mcKLH, Blue Carrier* Proteins, bovine serum albumin (BSA), cationized BSA, ovalbumin, and bacterial proteins such as tetanus toxoid (TT).

A polypeptide can also be prepared as multiple antigenic peptides (MAPs). Peptides may be attached at the N-terminus or the C-terminus to small non-immunogenic cores. Peptides built upon this core can offer highly localized peptide density. The core can be a dendritic core residue or matrix composed of bifunctional units. Suitable core molecules for constructing MAPs can include ammonia, ethylenediamine, aspartic acid, glutamic acid, and lysine. For example, a lysine core molecule can be attached via peptide bonds through each of its amino groups to two additional lysines.

A polypeptide can be chemically synthesized, or recombinantly expressed in a cell system or a cell-free system. A peptide can be synthesized, such as by a liquid-phase synthesis, a solid-phase synthesis, or by microwave assisted peptide synthesis. A polypeptide can be modified, such as for example, by acylation, alkylation, amidation, arginylation, polyglutamylation, polyglycylation, butyrylation, gamma-carboxylation, glycosylation, malonylation, hydroxylation, iodination, nucleotide addition (e.g. ADP-ribosylation), oxidation, phosphorylation, adenylylation, propionylation, S-glutathionylation, S-nitrosylation, succinylation, sulfation, glycation, palmitoylation, myristoylation, isoprenylation or prenylation (e.g. farnesylation or geranylgeranylation), glypiation, lipoylation, attachement of flavin moiety (e.g. FMN or FAD), attachment of heme C, phosphopantetheinylation, retinylidene Schiff base formation, diphthamide formation, ethanolamine phosphoglycerol attachment, hypusine formuation, biotinylation, pegylation, ISGylation, SUMUylation, ubiquitination, Neddylation, Pupylation, citrullination, deamidation, eliminylation, carbamylation, or a combination thereof.

After generation of a polypeptide, the polypeptide can be subjected to one or more rounds of purification steps to remove impurities. The purification step can be a chromatographic step utilizing separation methods such as affinity-based, size-exclusion based, ion-exchange based, or the like. In some cases, the polypeptide is at most 30%, 40%, 50%, 60%, 70%, 80%, 90%, 95%, 99%, 99.9%, or 100% pure or without the presence of impurities. In some cases, the polypeptide is at least 30%, 40%, 50%, 60%, 70%, 80%, 90%, 95%, 99%, 99.9%, or 100% pure or without the presence of impurities.

A polypeptide can include natural amino acids, unnatural amino acids, or a combination thereof. An amino acid residue can refer to a molecule containing both an amino group and a carboxyl group. Suitable amino acids include, without limitation, both the D- and L-isomers of the naturally-occurring amino acids, as well as non-naturally occurring amino acids prepared by organic synthesis or other metabolic routes. The term amino acid, as used herein, includes, without limitation, $\alpha$-amino acids, natural amino acids, non-natural amino acids, and amino acid analogs.

The term "$\alpha$-amino acid" can refer to a molecule containing both an amino group and a carboxyl group bound to a carbon which is designated the $\alpha$-carbon.

The term "$\beta$-amino acid" can refer to a molecule containing both an amino group and a carboxyl group in a $\beta$ configuration.

"Naturally occurring amino acid" can refer to any one of the twenty amino acids commonly found in peptides synthesized in nature, and known by the one letter abbreviations A, R, N, C, D, Q, E, G, H, I, L, K, M, F, P, S, T, W, Y and V. A table showing a summary of the properties of natural amino acids can be found, e.g., in U.S. Patent Application Publication No. 20130123169.

A peptide provided herein can comprise one or more hydrophobic, polar, or charged amino acids. "Hydrophobic amino acids" include small hydrophobic amino acids and large hydrophobic amino acids. "Small hydrophobic amino acid" can be glycine, alanine, proline, and analogs thereof. "Large hydrophobic amino acids" can be valine, leucine, isoleucine, phenylalanine, methionine, tryptophan, and analogs thereof. "Polar amino acids" can be serine, threonine, asparagine, glutamine, cysteine, tyrosine, and analogs thereof. "Charged amino acids" can be lysine, arginine, histidine, aspartate, glutamate, and analogs thereof.

A peptide provided herein can comprise one or more amino acid analogs. An "amino acid analog" can be a molecule which is structurally similar to an amino acid and which can be substituted for an amino acid in the formation of a peptidomimetic macrocycle Amino acid analogs include, without limitation, β-amino acids and amino acids where the amino or carboxy group is substituted by a similarly reactive group (e.g., substitution of the primary amine with a secondary or tertiary amine, or substitution of the carboxy group with an ester).

A peptide provided herein can comprises one or more non-natural amino acids. A "non-natural amino acid" can be an amino acid which is not one of the twenty amino acids commonly found in peptides synthesized in nature, and known by the one letter abbreviations A, R, N, C, D, Q, E, G, H, I, L, K, M, F, P, S, T, W, Y and V. Non-natural amino acids or amino acid analogs include structures disclosed, e.g., in U.S. Patent Application Publication No. 20130123169.

Amino acid analogs can include β-amino acid analogs. Examples of β-amino acid analogs and analogs of alanine, valine, glycine, leucine, arginine, lysine, aspartic acids, glutamic acids, cysteine, methionine, phenylalanine, tyrosine, proline, serine, threonine, and tryptophan can include structures disclosed, e.g., in U.S. Patent Application Publication No. 20130123169.

Amino acid analogs can be racemic. In some instances, the D isomer of the amino acid analog is used. In some cases, the L isomer of the amino acid analog is used. In some instances, the amino acid analog comprises chiral centers that are in the R or S configuration. Sometimes, the amino group(s) of a β-amino acid analog is substituted with a protecting group, e.g., tert-butyloxycarbonyl (BOC group), 9-fluorenylmethyloxycarbonyl (FMOC), tosyl, and the like. Sometimes, the carboxylic acid functional group of a β-amino acid analog is protected, e.g., as its ester derivative. In some cases, the salt of the amino acid analog is used.

A "non-essential" amino acid residue can be a residue that can be altered from the wild-type sequence of a polypeptide without abolishing or substantially altering its essential biological or biochemical activity (e.g., receptor binding or activation). An "essential" amino acid residue can be a residue that, when altered from the wild-type sequence of the polypeptide, results in abolishing or substantially abolishing the polypeptide's essential biological or biochemical activity.

A "conservative amino acid substitution" can be one in which the amino acid residue is replaced with an amino acid residue having a similar side chain. Families of amino acid residues having similar side chains have been defined in the art. These families can include amino acids with basic side chains (e.g., K, R, H), acidic side chains (e.g., D, E), uncharged polar side chains (e.g., G, N, Q, S, T, Y, C), nonpolar side chains (e.g., A, V, L, I, P, F, M, W), beta-branched side chains (e.g., T, V, I) and aromatic side chains (e.g., Y, F, W, H). Thus, a predicted nonessential amino acid residue in a polypeptide, for example, can be replaced with another amino acid residue from the same side chain family. Other examples of acceptable substitutions can be substitutions based on isosteric considerations (e.g. norleucine for methionine) or other properties (e.g. 2-thienylalanine for phenylalanine, or 6-Cl-tryptophan for tryptophan).

Nucleic acid -based immunogenic pharmaceutical compositions can also be administered to a subject. Nucleic acid -based immunogenic pharmaceutical compositionscan be formulated using any of the well-known techniques, carriers, and excipients as suitable and as understood in the art. The nucleic acid can be DNA, genomic DNA or cDNA, RNA, or a hybrid, where the nucleic acid may contain combinations of deoxyribo- and ribo-nucleotides, and combinations of bases including uracil, adenine, thymine, cytosine, guanine, inosine, xanthine hypoxanthine, isocytosine and isoguanine. Nucleic acids can be obtained by chemical synthesis methods or by recombinant methods. The immunogenic pharmaceutical composition can be a DNA-based immunogenic pharmaceutical composition, an RNA-based immunogenic pharmaceutical composition, a hybrid DNA/RNA based immunogenic pharmaceutical composition, or a hybrid nucleic acid/peptide based immunogenic pharmaceutical composition. The peptide can be a peptide derived from a peptide in Table 1 or 2, a peptide that has a sequence that is at least 40%, 50%, 60%, 70%, 80%, 90%, 95%, or more in sequence homology to a peptide in Table 1 or 2, or a peptide that has a sequence that is at most 40%, 50%, 60%, 70%, 80%, 90%, 95%, or less in sequence homology to a peptide in Table 1 or 2.

A nucleic acid described herein can contain phosphodiester bonds, although in some cases, as outlined below (for example in the construction of primers and probes such as label probes), nucleic acid analogs are included that can have alternate backbones, comprising, for example, phosphoramide, phosphorothioate, O-methylphosphoroamidite linkages, and peptide nucleic acid backbones and linkages. Other analog nucleic acids include those with bicyclic structures including locked nucleic acids, positive backbones and non-ribose backbones. Nucleic acids containing one or more carbocyclic sugars are also included within the definition of nucleic acids. Locked nucleic acids (LNAs) are also included within the definition of nucleic acid analogs. LNAs are a class of nucleic acid analogues in which the ribose ring is "locked" by a methylene bridge connecting the 2'-O atom with the 4'-C atom. These modifications of the ribose-phosphate backbone can be done to increase the stability and half-life of such molecules in physiological environments. For example, PNA:DNA and LNA-DNA hybrids can exhibit higher stability and thus can be used in some cases. The nucleic acids can be single stranded or double stranded, as specified, or contain portions of both double stranded or single stranded sequence. Depending on the application, the nucleic acids can be DNA (including, e.g., genomic DNA, mitochondrial DNA, and cDNA), RNA (including, e.g., mRNA and rRNA) or a hybrid, where the nucleic acid contains any combination of deoxyribo- and ribo-nucleotides, and any combination of bases, including uracil, adenine, thymine, cytosine, guanine, inosine, xathanine hypoxathanine, isocytosine, isoguanine, etc.

A nucleic acid -based immunogenic pharmaceutical compositions can be in the form of a vector. A vector can be a circular plasmid or a linear nucleic acid. A circular plasmid or linear nucleic acid can be capable of directing expression of a particular nucleotide sequence in an appropriate subject cell. A vector can have a promoter operably linked to the polypeptide-encoding nucleotide sequence, which can be operably linked to termination signals. A vector can contain sequences required for proper translation of the nucleotide sequence. The vector comprising the nucleotide sequence of interest can be chimeric, meaning that at least one of its components can be heterologous with respect to at least one of its other components. The expression of the nucleotide sequence in an expression cassette can be under the control of a constitutive promoter or of an inducible promoter, which can initiate transcription only when the host cell is exposed to some particular internal or external stimulus.

The vector can be a plasmid. A plasmid can be useful for transfecting cells with nucleic acid encoding the polypeptide, and the transformed host cells can be cultured and maintained under conditions wherein expression of the polypeptide takes place.

A plasmid can comprise a nucleic acid sequence that encodes one or more of the various polypeptides disclosed herein. A single plasmid can contain coding sequence for a single polypeptide, or coding sequence for more than one polypeptide. Sometimes, the plasmid can further comprise coding sequence that encodes an adjuvant, such as an immune stimulating molecule, such as a cytokine.

A plasmid can further comprise an initiation codon, which can be upstream of the coding sequence, and a stop codon, which can be downstream of the coding sequence. The initiation and termination codon can be in frame with the coding sequence. A plasmid can also comprise a promoter that is operably linked to the coding sequence, and an enhancer upstream of the coding sequence. The enhancer can be human actin, human myosin, human hemoglobin, human muscle creatine or a viral enhancer such as one from CMV, FMDV, RSV or EBV.

A plasmid can also comprise a mammalian origin of replication in order to maintain the plasmid extrachromosomally and produce multiple copies of the plasmid in a cell. A plasmid can also comprise a regulatory sequence, which can be well suited for gene expression in a cell into which the plasmid is administered. The coding sequence can comprise a codon that can allow more efficient transcription of the coding sequence in the host cell.

The nucleic acid based immunogenic pharmaceutical compositions can also be a linear nucleic acid immunogenic pharmaceutical composition, or linear expression cassette, that is capable of being efficiently delivered to a subject via electroporation and expressing one or more polypeptides disclosed herein.

Cell-based immunogenic pharmaceutical compositions can also be administered to a subject. For example, an antigen presenting cell (APC) based immunogenic pharmaceutical composition can be formulated using any of the well-known techniques, carriers, and excipients as suitable and as understood in the art. APCs include monocytes, monocyte-derived cells, macrophages, and dendritic cells. Sometimes, an APC based immunogenic pharmaceutical composition can be a dendritic cell-based immunogenic pharmaceutical composition.

A dendritic cell-based immunogenic pharmaceutical composition can be prepared by any methods well known in the art. In some cases, dendritic cell-based immunogenic pharmaceutical compositions can be prepared through an *ex vivo* or *in vivo* method. The *ex vivo* method can comprise the use of autologous DCs pulsed *ex vivo* with the polypeptides described herein, to activate or load the DCs prior to administration into the patient. The *in vivo* method can comprise targeting specific DC receptors using antibodies coupled with the polypeptides described herein. The DC-based immunogenic pharmaceutical composition can further comprise DC activators such as TLR3, TLR-7-8, and CD40 agonists. The DC-based immunogenic pharmaceutical composition can further comprise adjuvants, and a pharmaceutically acceptable carrier.

An adjuvant can be used to enhance the immune response (humoral and/or cellular) elicited in a patient receiving the immunogenic pharmaceutical composition. Sometimes, adjuvants can elicit a Th1-type response. Other times, adjuvants can elicit a Th2-type response. A Th1-type response can be characterized by the production of cytokines such as IFN-$\gamma$ as opposed to a Th2-type response which can be characterized by the production of cytokines such as IL-4, IL-5 and IL-10.

In some aspects, lipid-based adjuvants, such as MPLA and MDP, can be used with the immunogenic pharmaceutical compositions disclosed herein. Monophosphoryl lipid A (MPLA), for example, is an adjuvant that causes increased presentation of liposomal antigen to specific T Lymphocytes. In addition, a muramyl dipeptide (MDP) can also be used as a suitable adjuvant in conjunction with the immunogenic pharmaceutical formulations described herein.

Adjuvant can also comprise stimulatory molecules such as cytokines. Non-limiting examples of cytokines include: CCL20, a-interferon(IFN- a), $\beta$-interferon (IFN-$\beta$), $\gamma$- interferon, platelet derived growth factor (PDGF), TNFa, TNFp, GM-CSF, epidermal growth factor (EGF), cutaneous T cell-attracting chemokine (CTACK), epithelial thymus-expressed chemokine (TECK), mucosae-associated epithelial chemokine (MEC), IL-12, IL-15,, IL-28, MHC, CD80, CD86, IL-1, IL-2, IL-4, IL-5, IL-6, IL-10, IL-18, MCP-1, MIP-la, MIP-1-, IL-8, L- selectin, P-selectin, E-selectin, CD34, GlyCAM-1, MadCAM-1, LFA-1, VLA-1, Mac-1, pl50.95, PECAM, ICAM-1, ICAM-2, ICAM-3, CD2, LFA-3, M-CSF, G-CSF, mutant forms of IL-18, CD40, CD40L, vascular growth factor, fibroblast growth factor, IL-7, nerve growth factor, vascular endothelial growth factor, Fas, TNF receptor, Fit, Apo-1, p55, WSL-1, DR3, TRAMP, Apo-3, AIR LARD, NGRF, DR4, DRS, KILLER, TRAIL-R2, TRICK2, DR6, Caspase ICE, Fos, c-jun, Sp-1, Ap-1, Ap-2, p38, p65Rel, MyD88, IRAK, TRAF6, IkB, Inactive NIK, SAP K, SAP-I, JNK, interferon response genes, NFkB, Bax, TRAIL, TRAILrec, TRAILrecDRC5, TRAIL-R3, TRAIL-R4, RANK, RANK LIGAND, Ox40, Ox40 LIGAND, NKG2D, MICA, MICB, NKG2A, NKG2B, NKG2C, NKG2E, NKG2F, TAPI, and TAP2.

Additional adjuvants include: MCP-1, MIP-la, MIP-lp, IL-8, RANTES, L-selectin, P-selectin, E-selectin, CD34, GlyCAM-1, MadCAM-1, LFA-1, VLA-1, Mac-1, pl50.95, PECAM, ICAM-1, ICAM-2, ICAM-3, CD2, LFA-3, M-CSF, G-CSF, IL-4, mutant forms of IL-18, CD40, CD40L, vascular growth factor, fibroblast growth factor, IL-7, IL-22, nerve growth factor, vascular endothelial growth factor, Fas, TNF receptor, Fit, Apo-1, p55, WSL-1, DR3, TRAMP, Apo-3, AIR LARD, NGRF, DR4, DR5, KILLER, TRAIL-R2, TRICK2, DR6, Caspase ICE, Fos, c-jun, Sp-1, Ap-1, Ap-2, p38, p65Rel, MyD88, IRAK, TRAF6, IkB, Inactive NIK, SAP K, SAP-1, JNK, interferon response genes, NFkB, Bax, TRAIL, TRAILrec, TRAILrecDRC5, TRAIL-R3, TRAIL-R4, RANK, RANK LIGAND, Ox40, Ox40 LIGAND, NKG2D, MICA, MICB, NKG2A, NKG2B, NKG2C, NKG2E, NKG2F, TAP1, TAP2 and functional fragments thereof.

In some aspects, an adjuvant can be a modulator of a toll like receptor. Examples of modulators of toll-like receptors include TLR-9 agonists and are not limited to small molecule modulators of toll-like receptors such as Imiquimod. Other examples of adjuvants that are used in combination with an immunogenic pharmaceutical composition described herein can include and are not limited to saponin, CpG ODN and the like. Sometimes, an adjuvant is selected from bacteria toxoids, polyoxypropylene-polyoxyethylene block polymers, aluminum salts, liposomes, CpG polymers, oil-in-water emulsions, or a combination thereof. Sometimes, an adjuvant is an oil-in-water emulsion. The oil-in-water emulsion can include at least one oil and at least one surfactant, with the oil(s) and surfactant(s) being biodegradable (metabolisable) and biocompatible. The oil droplets in the emulsion can be less than 5 $\mu$m in diameter, and can even have a submicron diameter, with these small sizes being achieved with a microfluidiser to provide stable emulsions. Droplets with a size less than 220 nm can be subjected to filter sterilization.

In some instances, an immunogenic pharmaceutical composition can include carriers and excipients (including but not limited to buffers, carbohydrates, mannitol, proteins, polypeptides or amino acids such as glycine, antioxidants, bacteriostats, chelating agents, suspending agents, thickening agents and/or preservatives), water, oils including those of petroleum, animal, vegetable or synthetic origin, such as peanut oil, soybean oil, mineral oil, sesame oil and the like, saline solutions, aqueous dextrose and glycerol solutions, flavoring agents, coloring agents, detackifiers and other acceptable additives, adjuvants, or binders, other pharmaceutically acceptable auxiliary substances as required to approximate physiological conditions, such as pH buffering agents, tonicity adjusting agents, emulsifying agents, wetting agents and the like. Examples of excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. In another instances, the pharmaceutical preparation is substantially free of preservatives. In other instances, the pharmaceutical preparation can contain at least one preservative. It will be recognized that, while any suitable carrier known to those of ordinary skill in the art can be employed to administer the pharmaceutical compositions described herein, the type of carrier will vary depending on the mode of administration.

An immunogenic pharmaceutical composition can include preservatives such as thiomersal or 2-phenoxyethanol. In some instances, the immunogenic pharmaceutical composition is substantially free from (e.g. <10 $\mu$g/ml) mercurial material e.g. thiomersal-free. $\alpha$-Tocopherol succinate may be used as an alternative to mercurial compounds.

For controlling the tonicity, a physiological salt such as sodium salt can be included in the immunogenic pharmaceutical composition. Other salts can include potassium chloride, potassium dihydrogen phosphate, disodium phosphate, and/or magnesium chloride, or the like.

An immunogenic pharmaceutical composition can have an osmolality of between 200 mOsm/kg and 400 mOsm/kg, between 240-360 mOsm/kg, or within the range of 290-310 mOsm/kg.

An immunogenic pharmaceutical composition can comprise one or more buffers, such as a Tris buffer; a borate buffer; a succinate buffer; a histidine buffer (particularly with an aluminum hydroxide adjuvant); or a citrate buffer. Buffers, in some cases, are included in the 5-20 mM range.

The pH of the immunogenic pharmaceutical composition can be between about 5.0 and about 8.5, between about 6.0 and about 8.0, between about 6.5 and about 7.5, or between about 7.0 and about 7.8.

An immunogenic pharmaceutical composition can be sterile. The immunogenic pharmaceutical composition can be non-pyrogenic e.g. containing <1 EU (endotoxin unit, a standard measure) per dose, and can be <0.1 EU per dose. The composition can be gluten free.

An immunogenic pharmaceutical composition can include detergent e.g. a polyoxyethylene sorbitan ester surfactant (known as 'Tweens'), or an octoxynol (such as octoxynol-9 (Triton X-100) or t-octylphenoxypolyethoxyethanol). The detergent can be present only at trace amounts. The immunogenic pharmaceutical composition can include less than 1 mg/ml of each of octoxynol-10 and polysorbate 80. Other residual components in trace amounts can be antibiotics (e.g. neomycin, kanamycin, polymyxin B).

An immunogenic pharmaceutical composition can be formulated as a sterile solution or suspension, in suitable vehicles, well known in the art. The pharmaceutical compositions can be sterilized by conventional, well-known sterilization techniques, or can be sterile filtered. The resulting aqueous solutions can be packaged for use as is, or lyophilized, the lyophilized preparation being combined with a sterile solution prior to administration.

An immunogenic pharmaceutical composition can be formulated with one or more pharmaceutically acceptable salts. Pharmaceutically acceptable salts can include those of the inorganic ions, such as, for example, sodium, potassium, calcium, magnesium ions, and the like. Such salts can include salts with inorganic or organic acids, such as hydrochloric acid, hydrobromic acid, phosphoric acid, nitric acid, sulfuric acid, methanesulfonic acid, p-toluenesulfonic acid, acetic acid, fumaric acid, succinic acid, lactic acid, mandelic acid, malic acid, citric acid, tartaric acid or maleic acid. In addition, if the agent(s) contain a carboxy group or other acidic group, it can be converted into a pharmaceutically acceptable addition salt with inorganic or organic bases. Examples of suitable bases include sodium hydroxide, potassium hydroxide, ammonia, cyclohexylamine, dicyclohexyl-amine, ethanolamine, diethanolamine, triethanolamine, and the like.

Pharmaceutical compositions comprising, for example, an active agent such as a peptide, a nucleic acid, an antibody or fragments thereof, and/or an APC described herein, in combination with one or more adjuvants can be formulated to comprise certain molar ratios. For example, molar ratios of about 99:1 to about 1:99 of an active agent such as a peptide, a nucleic acid, an antibody or fragments thereof, and/or an APC described herein, in combination with one or more adjuvants can be used. In some instances, the range of molar ratios of an active agent such as a peptide, a nucleic acid, an antibody or fragments thereof, and/or an APC described herein, in combination with one or more adjuvants can be selected from about 80:20 to about 20:80; about 75:25 to about 25:75, about 70:30 to about 30:70, about 66:33 to about 33:66, about 60:40 to about 40:60; about 50:50; and about 90:10 to about 10:90. The molar ratio of an active agent such as a peptide, a nucleic acid, an antibody or fragments thereof, and/or an APC described herein, in combination with one or more adjuvants can be about 1:9, and in some cases can be about 1:1. The active agent such as a peptide, a nucleic acid, an antibody or fragments thereof, and/or an APC described herein, in combination with one or more adjuvants can be formulated together, in the same dosage unit e.g., in one vial, suppository, tablet, capsule, an aerosol spray; or each agent, form, and/or compound can be formulated in separate units, e.g., two vials, suppositories, tablets, two capsules, a tablet and a vial, an aerosol spray, and the like.

In some instances, an immunogenic pharmaceutical composition can be administered with an additional agent. The choice of the additional agent can depend, at least in part, on the condition being treated. The additional agent can include, for example, any agents having a therapeutic effect for a pathogen infection (e.g. viral infection), including, e.g., drugs used to treat inflammatory conditions such as an NSAID, e.g., ibuprofen, naproxen, acetaminophen, ketoprofen, or aspirin. As another example, formulations can additionally contain one or more supplements, such as vitamin C, E or other anti-oxidants.

A pharmaceutical composition comprising an active agent such as a peptide, a nucleic acid, an antibody or fragments thereof, and/or an APC described herein, in combination with one or more adjuvants can be formulated in conventional manner using one or more physiologically acceptable carriers, comprising excipients, diluents, and/or auxiliaries, e.g., which facilitate processing of the active agents into preparations that can be administered. Proper formulation can depend at least in part upon the route of administration chosen. The agent(s) described herein can be delivered to a patient using a number of routes or modes of administration, including oral, buccal, topical, rectal, transdermal, transmucosal, subcutaneous, intravenous, and intramuscular applications, as well as by inhalation.

The active agents can be formulated for parenteral administration (e.g., by injection, for example bolus injection or continuous infusion) and can be presented in unit dose form in ampoules, pre-filled syringes, small volume infusion or in multi-dose containers with an added preservative. The compositions can take such forms as suspensions, solutions, or emulsions in oily or aqueous vehicles, for example solutions in aqueous polyethylene glycol.

For injectable formulations, the vehicle can be chosen from those known in art to be suitable, including aqueous solutions or oil suspensions, or emulsions, with sesame oil, corn oil, cottonseed oil, or peanut oil, as well as elixirs, mannitol, dextrose, or a sterile aqueous solution, and similar pharmaceutical vehicles. The formulation can also comprise polymer compositions which are biocompatible, biodegradable, such as poly(lactic-co-glycolic)acid. These materials can be made into micro or nanospheres, loaded with drug and further coated or derivatized to provide superior sustained release performance. Vehicles suitable for periocular or intraocular injection include, for example, suspensions of therapeutic agent in injection grade water, liposomes and vehicles suitable for lipophilic substances. Other vehicles for periocular or intraocular injection are well known in the art.

In some instances, pharmaceutical composition is formulated in accordance with routine procedures as a pharmaceutical composition adapted for intravenous administration to human beings. Typically, compositions for intravenous administration are solutions in sterile isotonic aqueous buffer. Where necessary, the composition can also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

When administration is by injection, the active agent can be formulated in aqueous solutions, specifically in physiologically compatible buffers such as Hanks solution, Ringer's solution, or physiological saline buffer. The solution can contain formulatory agents such as suspending, stabilizing and/or dispersing agents. Alternatively, the active compound can be in powder form for constitution with a suitable vehicle, e.g., sterile pyrogen-free water, before use. In another case, the pharmaceutical composition does not comprise an adjuvant or any other substance added to enhance the immune response stimulated by the peptide. In another case, the pharmaceutical composition comprises a substance that inhibits an immune response to the peptide.

In addition to the formulations described previously, the active agents can also be formulated as a depot preparation. Such long acting formulations can be administered by implantation or transcutaneous delivery (for example subcutaneously or intramuscularly), intramuscular injection or use of a transdermal patch. Thus, for example, the agents can be formulated with suitable polymeric or hydrophobic materials (for example as an emulsion in an acceptable oil) or ion exchange resins, or as sparingly soluble derivatives, for example, as a sparingly soluble salt.

In cases, pharmaceutical compositions comprising one or more agents exert local and regional effects when administered topically or injected at or near particular sites of infection. Direct topical application, e.g., of a viscous liquid, solution, suspension, dimethylsulfoxide (DMSO)-based solutions, liposomal formulations, gel, jelly, cream, lotion, ointment, suppository, foam, or aerosol spray, can be used for local administration, to produce for example local and/or regional effects. Pharmaceutically appropriate vehicles for such formulation include, for example, lower aliphatic alcohols, polyglycols (e.g., glycerol or polyethylene glycol), esters of fatty acids, oils, fats, silicones, and the like. Such preparations can also include preservatives (e.g., p-hydroxybenzoic acid esters) and/or antioxidants (e.g., ascorbic acid and tocopherol). See also Dermatological Formulations: Percutaneous absorption, Barry (Ed.), Marcel Dekker Incl, 1983. In another case, local/topical formulations comprising a transporter, carrier, or ion channel inhibitor are used to treat epidermal or mucosal viral infections.

Pharmaceutical compositions can contain a cosmetically or dermatologically acceptable carrier. Such carriers are compatible with skin, nails, mucous membranes, tissues and/or hair, and can include any conventionally used cosmetic or dermatological carrier meeting these requirements. Such carriers can be readily selected by one of ordinary skill in the art. In formulating skin ointments, an agent or combination of agents can be formulated in an oleaginous hydrocarbon base, an anhydrous absorption base, a water-in-oil absorption base, an oil-in-water water-removable base and/or a water-soluble base. Examples of such carriers and excipients include, but are not limited to, humectants (e.g., urea), glycols (e.g., propylene glycol), alcohols (e.g., ethanol), fatty acids (e.g., oleic acid), surfactants (e.g., isopropyl myristate and sodium lauryl sulfate), pyrrolidones, glycerol monolaurate, sulfoxides, terpenes (e.g., menthol), amines, amides, alkanes, alkanols, water, calcium carbonate, calcium phosphate, various sugars, starches, cellulose derivatives, gelatin, and polymers such as polyethylene glycols.

Ointments and creams can, for example, be formulated with an aqueous or oily base with the addition of suitable thickening and/or gelling agents. Lotions can be formulated with an aqueous or oily base and will in general also containing one or more emulsifying agents, stabilizing agents, dispersing agents, suspending agents, thickening agents, or coloring agents. The construction and use of transdermal patches for the delivery of pharmaceutical agents is well known in the art. Such patches can be constructed for continuous, pulsatile, or on demand delivery of pharmaceutical agents.

Lubricants which can be used to form pharmaceutical compositions and dosage forms can include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, or mixtures thereof. Additional lubricants include, for example, a syloid silica gel, a coagulated aerosol of synthetic silica, or mixtures thereof. A lubricant can optionally be added, in an amount of less than about 1 weight percent of the pharmaceutical composition.

The pharmaceutical compositions can be in any form suitable for topical application, including aqueous, aqueous-alcoholic or oily solutions, lotion or serum dispersions, aqueous, anhydrous or oily gels, emulsions obtained by dispersion of a fatty phase in an aqueous phase (O/W or oil in water) or, conversely, (W/O or water in oil), microemulsions or alternatively microcapsules, microparticles or lipid vesicle dispersions of ionic and/or nonionic type. These compositions can be prepared according to conventional methods. The amounts of the various constituents of the compositions are those conventionally used in the art. These compositions in particular constitute protection, treatment or care creams, milks, lotions, gels or foams for the face, for the hands, for the body and/or for the mucous membranes, or for cleansing the skin. The compositions can also consist of solid preparations constituting soaps or cleansing bars.

Pharmaceutical compositions can contain adjuvants such as hydrophilic or lipophilic gelling agents, hydrophilic or lipophilic active agents, preserving agents, antioxidants, solvents, fragrances, fillers, sunscreens, odor-absorbers and dyestuffs. The amounts of these various adjuvants are those conventionally used in the fields considered and, for example, are from about 0.01% to about 20% of the total weight of the composition. Depending on their nature, these adjuvants can be introduced into the fatty phase, into the aqueous phase and/or into the lipid vesicles.

In instances relating to topical/local application, the pharmaceutical compositions can include one or more penetration enhancers. For example, the formulations can comprise suitable solid or gel phase carriers or excipients that increase penetration or help delivery of agents or combinations of agents of the present disclosure across a permeability barrier, e.g., the skin. Many of these penetration-enhancing compounds are known in the art of topical formulation, and include, e.g., water, alcohols (e.g., terpenes like methanol, ethanol, 2-propanol), sulfoxides (e.g., dimethyl sulfoxide, decylmethyl sulfoxide, tetradecylmethyl sulfoxide), pyrrolidones (e.g., 2-pyrrolidone, N-methyl-2-pyrrolidone, N-(2-hydroxyethyl)pyrrolidone), laurocapram, acetone, dimethylacetamide, dimethylformamide, tetrahydrofurfuryl alcohol, L-$\alpha$-amino acids, anionic, cationic, amphoteric or nonionic surfactants (e.g., isopropyl myristate and sodium lauryl sulfate), fatty acids, fatty alcohols (e.g., oleic acid), amines, amides, clofibric acid amides, hexamethylene lauramide, proteolytic enzymes, $\alpha$-bisabolol, d-limonene, urea and N,N-diethyl-m-toluamide, and the like. Additional examples include humectants (e.g., urea), glycols (e.g., propylene glycol and polyethylene glycol), glycerol monolaurate, alkanes, alkanols, ORGELASE, calcium carbonate, calcium phosphate, various sugars, starches, cellulose derivatives, gelatin, and/or other polymers. In another case, the pharmaceutical compositions will include one or more such penetration enhancers.

The pharmaceutical compositions for local/topical application can include one or more antimicrobial preservatives such as quaternary ammonium compounds, organic mercurials, p-hydroxy benzoates, aromatic alcohols, chlorobutanol, and the like.

The pharmaceutical compositions can be formulated into aerosol solutions, suspensions or dry powders. The aerosol can be administered through the respiratory system or nasal passages. For example, one skilled in the art will recognize that a composition of the present disclosure can be suspended or dissolved in an appropriate carrier, e.g., a pharmaceutically acceptable propellant, and administered directly into the lungs using a nasal spray or inhalant. For example, an aerosol formulation comprising a transporter, carrier, or ion channel inhibitor can be dissolved, suspended or emulsified in a propellant or a mixture of solvent and propellant, e.g., for administration as a nasal spray or inhalant. Aerosol formulations can contain any acceptable propellant under pressure, such as a cosmetically or dermatologically or pharmaceutically acceptable propellant, as conventionally used in the art.

An aerosol formulation for nasal administration is generally an aqueous solution designed to be administered to the nasal passages in drops or sprays. Nasal solutions can be similar to nasal secretions in that they are generally isotonic and slightly buffered to maintain a pH of about 5.5 to about 6.5, although pH values outside of this range can additionally be used. Antimicrobial agents or preservatives can also be included in the formulation.

An aerosol formulation for inhalations and inhalants can be designed so that the agent or combination of agents is carried into the respiratory tree of the subject when administered by the nasal or oral respiratory route. Inhalation solutions can be administered, for example, by a nebulizer. Inhalations or insufflations, comprising finely powdered or liquid drugs, can be delivered to the respiratory system as a pharmaceutical aerosol of a solution or suspension of the agent or combination of agents in a propellant, e.g., to aid in disbursement. Propellants can be liquefied gases, including halocarbons, for example, fluorocarbons such as fluorinated chlorinated hydrocarbons, hydrochlorofluorocarbons, and hydrochlorocarbons, as well as hydrocarbons and hydrocarbon ethers.

Halocarbon propellants can include fluorocarbon propellants in which all hydrogens are replaced with fluorine, chlorofluorocarbon propellants in which all hydrogens are replaced with chlorine and at least one fluorine, hydrogen-containing fluorocarbon propellants, and hydrogen-containing chlorofluorocarbon propellants. Hydrocarbon propellants useful in the present disclosure include, for example, propane, isobutane, n-butane, pentane, isopentane and neopentane. A blend of hydrocarbons can also be used as a propellant. Ether propellants include, for example, dimethyl ether as well as the ethers. An aerosol formulation of the present disclosure can also comprise more than one propellant. For example, the aerosol formulation can comprise more than one propellant from the same class, such as two or more fluorocarbons; or more than one, more than two, more than three propellants from different classes, such as a fluorohydrocarbon and a hydrocarbon. Pharmaceutical compositions of the present disclosure can also be dispensed with a compressed gas, e.g., an inert gas such as carbon dioxide, nitrous oxide or nitrogen.

Aerosol formulations can also include other components, for example, ethanol, isopropanol, propylene glycol, as well as surfactants or other components such as oils and detergents. These components can serve to stabilize the formulation and/or lubricate valve components.

The aerosol formulation can be packaged under pressure and can be formulated as an aerosol using solutions, suspensions, emulsions, powders and semisolid preparations. For example, a solution aerosol formulation can comprise a solution of an agent of the present disclosure such as a transporter, carrier, or ion channel inhibitor in (substantially) pure propellant or as a mixture of propellant and solvent. The solvent can be used to dissolve the agent and/or retard the evaporation of the propellant. Solvents can include, for example, water, ethanol and glycols. Any combination of suitable solvents can be use, optionally combined with preservatives, antioxidants, and/or other aerosol components.

An aerosol formulation can be a dispersion or suspension. A suspension aerosol formulation can comprise a suspension of an agent or combination of agents of the instant disclosure, e.g., a transporter, carrier, or ion channel inhibitor, and a dispersing agent. Dispersing agents can include, for example, sorbitan trioleate, oleyl alcohol, oleic acid, lecithin and corn oil. A suspension aerosol formulation can also include lubricants, preservatives, antioxidant, and/or other aerosol components.

An aerosol formulation can similarly be formulated as an emulsion. An emulsion aerosol formulation can include, for example, an alcohol such as ethanol, a surfactant, water and a propellant, as well as an agent or combination of agents of the disclosure, e.g., a transporter, carrier, or ion channel. The surfactant used can be nonionic, anionic or cationic. One example of an emulsion aerosol formulation comprises, for example, ethanol, surfactant, water and propellant. Another example of an emulsion aerosol formulation comprises, for example, vegetable oil, glyceryl monostearate and propane.

The pharmaceutical compounds can be formulated for administration as suppositories. A low melting wax, such as a mixture of triglycerides, fatty acid glycerides, Witepsol S55 (trademark of Dynamite Nobel Chemical, Germany), or cocoa butter is first melted and the active component is dispersed homogeneously, for example, by stirring. The molten homogeneous mixture is then poured into convenient sized molds, allowed to cool, and to solidify.

The pharmaceutical compositions can be formulated for vaginal administration. Pessaries, tampons, creams, gels, pastes, foams or sprays containing in addition to the active ingredient such carriers as are known in the art to be appropriate.

The pharmaceutical compositions can be attached releasably to biocompatible polymers for use in sustained release formulations on, in or attached to inserts for topical, intraocular, periocular, or systemic administration. The controlled release from a biocompatible polymer can be utilized with a water soluble polymer to form a instillable formulation, as well. The controlled release from a biocompatible polymer, such as for example, PLGA microspheres or nanospheres, can be utilized in a formulation suitable for intra ocular implantation or injection for sustained release administration, as well. Any suitable biodegradable and biocompatible polymer can be used.

## IV. Combinations of CTL peptides and HTL peptides

Immunogenic or vaccine compositions comprising the neoantigenic polypeptides and polynucleotides described herein, or analogs thereof, which have immunostimulatory activity can be modified to provide desired attributes, such as improved serum half-life, or to enhance immunogenicity.

For instance, the ability of the neoantigenic peptides to induce CTL activity can be enhanced by linking the peptide to a sequence which contains at least one epitope that is capable of inducing a T helper cell response. In some cases, CTL epitope/HTL epitope conjugates are linked by a spacer molecule. The spacer is typically comprised of relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer need not be comprised of the same residues and thus can be a hetero- or homo-oligomer. When present, the spacer will usually be at least one or two residues, more usually three to six residues. Alternatively, the CTL peptide can be linked to the T helper peptide without a spacer.

Although the CTL peptide epitope can be linked directly to the T helper peptide epitope, CTL epitope/HTL epitope conjugates can be linked by a spacer molecule. The spacer is typically comprised of relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer need not be comprised of the same residues and thus can be a hetero- or homo-oligomer. When present, the spacer will usually be at least one or two residues, more usually three to six residues. The CTL peptide epitope can be linked to the T helper peptide epitope either directly or via a spacer either at the amino or carboxy terminus of the CTL peptide. The amino terminus of either the immunogenic peptide or the T helper peptide can be acylated.

HTL peptide epitopes can also be modified to alter their biological properties. For example, peptides comprising HTL epitopes can contain D-amino acids to increase their resistance to proteases and thus extend their serum half-life. Also, the epitope peptides can be conjugated to other molecules such as lipids, proteins or sugars, or any other synthetic compounds, to increase their biological activity. For example, the T helper peptide can be conjugated to one or more palmitic acid chains at either the amino or carboxyl termini.

In certain cases, the T helper peptide is one that is recognized by T helper cells present in the majority of the population. This can be accomplished by selecting amino acid sequences that bind to many, most, or all of the HLA class II molecules. These are known as "loosely HLA-restricted" or "promiscuous" T helper sequences. Examples of amino acid sequences that are promiscuous include sequences from antigens such as tetanus toxoid at positions 830-843 (QYIKANSKFIGITE), *Plasmodium falciparum* CS protein at positions 378-398 (DIEKKIAKMEKASSVFNVVNS), and Streptococcus 18kD protein at positions 116 (GAVDSILGGVATYGAA). Other examples include peptides bearing a DR 1-4-7 supermotif, or either of the DR3 motifs.

Alternatively, it is possible to prepare synthetic peptides capable of stimulating T helper lymphocytes, in a loosely HLA-restricted fashion, using amino acid sequences not found in nature (see, e.g., PCT publication WO 95/07707). These synthetic compounds called Pan-DR-binding epitopes (e.g., PADRE, Epimmune, Inc., San Diego, CA) are designed to bind most HLA-DR (human HLA class II) molecules. For instance, a pan-DR-binding epitope peptide having the formula: aKXVWANTLKAAa, where "X" is either cyclohexylalanine, phenylalanine, or tyrosine, and a is either D-alanine or L-alanine, has been found to bind to most HLA-DR alleles, and to stimulate the response of T helper lymphocytes from most individuals, regardless of their HLA type. An alternative of a pan-DR binding epitope comprises all "L" natural amino acids and can be provided in the form of nucleic acids that encode the epitope.

In some cases it can be desirable to include in a neoantigen therapeutic (e.g., peptides, polynucleotides, TCR, CAR, cells containing TCR or CAR, dendritic cell containing polypeptide, dendritic cell containing polynucleotide, antibody, etc.) in pharmaceutical compositions (e.g., immunogenic compositions) at least one component of which primes cytotoxic T lymphocytes. Lipids have been identified as agents capable of priming CTL in vivo against viral antigens. For example, palmitic acid residues can be attached to the ε-and α- amino groups of a lysine residue and then linked, e.g., via one or more linking residues such as Gly, Gly-Gly-, Ser, Ser-Ser, or the like, to an immunogenic neoantigenic peptide. The lipidated peptide can then be administered either directly in a micelle or particle, incorporated into a liposome, or emulsified in an adjuvant. In some cases, a particularly effective immunogenic construct comprises palmitic acid attached to ε- and α- amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl- serine (P3CSS) can be used to prime virus specific CTL when covalently attached to an appropriate peptide. (See, e.g., Deres, et al., Nature 342:561, 1989). Neoantigenic peptides described herein can be coupled to P3CSS, for example, and the lipopeptide administered to an individual to specifically prime a CTL response to the target antigen. Moreover, because the induction of neutralizing antibodies can also be primed with P3CSS-conjugated epitopes, two such compositions can be combined to more effectively elicit both humoral and cell-mediated responses to infection.

As noted herein, additional amino acids can be added to the termini of a neoantigenic peptide to provide for ease of linking peptides one to another, for coupling to a carrier support or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. However, it is to be noted that modification at the carboxyl terminus of a T cell epitope can, in some cases, alter binding characteristics of the peptide. In addition, the peptide or oligopeptide sequences can differ from the natural sequence by being modified by terminal-NH2 acylation, e.g., by alkanoyl (C1-C20) orthioglycolyl acetylation, terminal-carboxyl amidation, e.g., ammonia, methylamine, etc. In some instances these modifications can provide sites for linking to a support or other molecule.

A case of an immunogenic composition described herein comprises *ex vivo* administration of a cocktail of epitope-bearing neoantigenic polypeptide or polynucleotides to PBMC, or isolated DC therefrom, from the patient's blood. A pharmaceutical to facilitate harvesting of dendritic cells (DCs) can be used, including GM-CSF, IL-4, IL-6, IL-1β, and TNFα. After pulsing the DCs with peptides or polynucleotides encoding the peptides, and prior to reinfusion into patients, the DC are washed to remove unbound peptides. In this case, a vaccine or immunogenic composition comprises peptide-pulsed DCs which present the pulsed peptide epitopes complexed with HLA molecules on their surfaces. The composition is then administered to the patient. In other cases, such pulsed DCs are used to stimulate T cells suitable for use in T cell therapy.

### V. Multi-epitope immunogenic compositions

A number of different approaches are available which allow simultaneous delivery of multiple epitopes. Nucleic acids encoding the neoantigenic peptides described herein are a particularly useful case of the disclosure. In some cases, the nucleic acid is RNA. In some cases, minigene constructs encoding a neoantigenic peptide comprising one or multiple epitopes described herein are used to administer nucleic acids encoding the neoantigenic peptides described herein uses.

The use of multi-epitope minigenes is described An, L. and Whitton, J. L., J. Virol. 71:2292, 1997; Thomson, S. A. et al., J. Immunol. 157:822, 1996; Whitton, J. L. et al., J. Virol. 67:348, 1993; Hanke, R. et al., Vaccine 16:426, 1998. For example, a multi-epitope DNA plasmid encoding supermotif- and/or motif-bearing neoantigen peptides, a universal helper T cell epitope (or multiple tumor associated antigen HTL epitopes), and an endoplasmic reticulum-translocating signal sequence can be engineered.

The immunogenicity of a multi-epitopic minigene can be tested in transgenic mice to evaluate the magnitude of immune response induced against the epitopes tested. Further, the immunogenicity of DNA-encoded epitopes in vivo can be correlated with the in vitro responses of specific CTL lines against target cells transfected with the DNA plasmid. Thus, these experiments can show that the minigene serves to both: 1.) generate a cell mediated and/or humoral response and 2.) that the induced immune cells recognized cells expressing the encoded epitopes.

For example, to create a DNA sequence encoding the selected neoepitopes (minigene) for expression in human cells, the amino acid sequences of the epitopes can be reverse translated. A human codon usage table can be used to guide the codon choice for each amino acid. These neoepitope-encoding DNA sequences can be directly adjoined, so that when translated, a continuous polypeptide sequence is created. To optimize expression and/or immunogenicity, additional elements can be incorporated into the minigene design. Examples of amino acid sequences that can be reverse translated and included in the minigene sequence include: HLA class I epitopes, HLA class II epitopes, a ubiquitination signal sequence, and/or an endoplasmic reticulum targeting signal. In addition, HLA presentation of CTL and HTL epitopes can be improved by including synthetic (e.g. poly-alanine) or naturally-occurring flanking sequences adjacent to the CTL or HTL epitopes; these larger peptides comprising the epitope(s) are within the scope of the disclosure.

The minigene sequence can be converted to DNA by assembling oligonucleotides that encode the plus and minus strands of the minigene. Overlapping oligonucleotides (30-100 bases long) can be synthesized, phosphorylated, purified and annealed under appropriate conditions using well known techniques. The ends of the oligonucleotides can be joined, for example, using T4 DNA ligase. This synthetic minigene, encoding the epitope polypeptide, can then be cloned into a desired expression vector.

Standard regulatory sequences well known to those of skill in the art can be included in the vector to ensure expression in the target cells. For example, a promoter with a down-stream cloning site for minigene insertion; a polyadenylation signal for efficient transcription termination; an E. coli origin of replication; and an E. coli selectable marker (e.g. ampicillin or kanamycin resistance). Numerous promoters can be used for this purpose, e.g., the human cytomegalovirus (hCMV) promoter. See, e.g., U.S. Patent Nos. 5,580,859 and 5,589,466 for other suitable promoter sequences.

Additional vector modifications can be used to optimize minigene expression and immunogenicity. In some cases, introns are required for efficient gene expression, and one or more synthetic or naturally-occurring introns could be incorporated into the transcribed region of the minigene. The inclusion of mRNA stabilization sequences and sequences for replication in mammalian cells can also be considered for increasing minigene expression.

Once an expression vector is selected, the minigene can be cloned into the polylinker region downstream of the promoter. This plasmid is transformed into an appropriate E. coli strain, and DNA is prepared using standard techniques. The orientation and DNA sequence of the minigene, as well as all other elements included in the vector, can be confirmed using restriction mapping and DNA sequence analysis. Bacterial cells harboring the correct plasmid can be stored as a master cell bank and a working cell bank.

In addition, immunomodulatory sequences appear to play a role in the immunogenicity of DNA vaccines. These sequences can be included in the vector, outside the minigene coding sequence, if desired to enhance immunogenicity. In some cases, the sequences are immunostimulatory. In another case, the sequences are ISSs or CpGs.

In some cases, a bi-cistronic expression vector which allows production of both the minigene-encoded epitopes and a second protein (included to enhance or decrease immunogenicity) can be used. Examples of proteins or polypeptides that could beneficially enhance the immune response if co-expressed include cytokines (e.g., IL-2, IL-12, GM-CSF), cytokine-inducing molecules (e.g., LeIF), costimulatory molecules, or for HTL responses, pan-DR binding proteins. Helper (HTL) epitopes can be joined to intracellular targeting signals and expressed separately from expressed CTL epitopes; this allows direction of the HTL epitopes to a cell compartment different than that of the CTL epitopes. If required, this could facilitate more efficient entry of HTL epitopes into the HLA class II pathway, thereby improving HTL induction. In contrast to HTL or CTL induction, specifically decreasing the immune response by co-expression of immunosuppressive molecules (e.g. TGF-β) can be beneficial in certain diseases.

Therapeutic quantities of plasmid DNA can be produced for example, by fermentation in E. coli, followed by purification. Aliquots from the working cell bank are used to inoculate growth medium, and grown to saturation in shaker flasks or a bioreactor according to well-known techniques. Plasmid DNA can be purified using standard bioseparation technologies such as solid phase anion-exchange resins supplied by QIAGEN, Inc. (Valencia, California). If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). This approach, known as "naked DNA," is currently being used for intramuscular (IM) administration in clinical trials. To maximize the immunotherapeutic effects of minigene DNA vaccines, an alternative method for formulating purified plasmid DNA can be used. A variety of methods have been described, and new techniques can become available. Cationic lipids can also be used in the formulation (see, e.g., as described by WO 93/24640; Mannino & Gould-Fogerite, BioTechniques 6(7): 682 (1988); U.S. Pat No. 5,279,833; WO 91/06309; and Felgner, et al., Proc. Nat'l Acad. Sci. USA 84:7413 (1987). In addition, glycolipids, fusogenic liposomes, peptides and compounds referred to collectively as protective, interactive, non-condensing compounds (PINC) could also be complexed to purified plasmid DNA to influence variables such as stability, intramuscular dispersion, or trafficking to specific organs or cell types.

In another case, the nucleic acid is introduced into cells by use of high-speed cell deformation. During high-speed deformation, cells are squeezed such that temporary disruptions occur in the cell membrane, thus allowing the nucleic acid to enter the cell. Alternatively, protein can be produced from expression vectors - in a bacterial expression vector, for example, and the proteins can then be delivered to the cell.

Target cell sensitization can be used as a functional assay for expression and HLA class I presentation of minigene-encoded CTL epitopes. For example, the plasmid DNA is introduced into a mammalian cell line that is suitable as a target for standard CTL chromium release assays. The transfection method used will be dependent on the final formulation. Electroporation can be used for "naked" DNA, whereas cationic lipids allow direct in vitro transfection. A plasmid expressing green fluorescent protein (GFP) can be co-transfected to allow enrichment of transfected cells using fluorescence activated cell sorting (FACS). These cells are then chromium-51 ($^{51}$Cr) labeled and used as target cells for epitope-specific CTL lines; cytolysis, detected by $^{51}$Cr release, indicates both production of, and HLA presentation of, minigene-encoded CTL epitopes. Expression of HTL epitopes can be evaluated in an analogous manner using assays to assess HTL activity.

In vivo immunogenicity is a second approach for functional testing of minigene DNA formulations. Transgenic mice expressing appropriate human HLA proteins are immunized with the DNA product. The dose and route of administration are formulation dependent (e.g., IM for DNA in PBS, intraperitoneal (IP) for lipid-complexed DNA). An exemplary protocol is twenty-one days after immunization, splenocytes are harvested and restimulated for 1 week in the presence of peptides encoding each epitope being tested. Thereafter, for CTL effector cells, assays are conducted for cytolysis of peptide-loaded, $^{51}$Cr-labeled target cells using standard techniques. Lysis of target cells that were sensitized by HLA loaded with peptide epitopes, corresponding to minigene-encoded epitopes, demonstrates DNA vaccine function for in vivo induction of CTLs. Immunogenicity of HTL epitopes is evaluated in transgenic mice in an analogous manner.

Alternatively, the nucleic acids can be administered using ballistic delivery as described, for instance, in U.S. Patent No. 5,204,253. Using this technique, particles comprised solely of DNA are administered. In a further alternative case, DNA can be adhered to particles, such as gold particles.

VI. Cells for use in the invention

In one aspect, the present disclosure also provides cells expressing a neoantigen-recognizing receptor that activates an immunoresponsive cell (e.g., T cell receptor (TCR) or chimeric antigen receptor (CAR)), and methods of using such cells for the treatment of a disease that requires an enhanced immune response.

Such cells include genetically modified immunoresponsive cells (e.g., T cells, Natural Killer (NK) cells, cytotoxic T lymphocytes (CTL) cells, helper T lymphocyte (HTL) cells) expressing an antigen-recognizing receptor (e.g., TCR or CAR) that binds one of the neoantigenic peptides described herein, and methods of use therefore for the treatment of neoplasia and other pathologies where an increase in an antigen-specific immune response is desired. T cell activation is mediated by a TCR or a CAR targeted to an antigen.

The present disclosure provides cells expressing a combination of an antigen-recognizing receptor that activates an immunoresponsive cell (e.g., TCR, CAR) and a chimeric co-stimulating receptor (CCR), and methods of using such cells for the treatment of a disease that requires an enhanced immune response. In some cases, tumor antigen-specific T cells, NK cells, CTL cells or other immunoresponsive cells are used as shuttles for the selective enrichment of one or more co-stimulatory ligands for the treatment or prevention of neoplasia. Such cells are administered to a human subject in need thereof for the treatment or prevention of a particular cancer.

In some cases, the tumor antigen-specific human lymphocytes that can be used in the methods of the disclosure include, without limitation, peripheral donor lymphocytes genetically modified to express chimeric antigen receptors (CARs) (Sadelain, M., et al. 2003 Nat Rev Cancer 3:35-45), peripheral donor lymphocytes genetically modified to express a full-length tumor antigen-recognizing T cell receptor complex comprising the a and p heterodimer (Morgan, R. A., et al. 2006 Science 314:126-129), lymphocyte cultures derived from tumor infiltrating lymphocytes (TILs) in tumor biopsies (Panelli, M. C., et al. 2000 J Immunol 164:495-504; Panelli, M. C., et al. 2000 J Immunol 164:4382-4392), and selectively in vitro-expanded antigen-specific peripheral bloodleukocytes employing artificial antigen-presenting cells (AAPCs) or pulsed dendritic cells (Dupont, J., et al. 2005 Cancer Res 65:5417-5427; Papanicolaou, G. A., et al. 2003 Blood 102:2498-2505). The T cells may be autologous, allogeneic, or derived in vitro from engineered progenitor or stem cells.

In some cases, the immunotherapeutic is an engineered receptor. In some cases, the engineered receptor is a chimeric antigen receptor (CAR), a T cell receptor (TCR), or a B-cell receptor (BCR), an adoptive T cell therapy (ACT), or a derivative thereof. In other aspects, the engineered receptor is a chimeric antigen receptor (CAR). In some aspects, the CAR is a first generation CAR. In other aspects, the CAR is a second generation CAR. In still other aspects, the CAR is a third generation CAR.

In some aspects, the CAR comprises an extracellular portion, a transmembrane portion, and an intracellular portion. In some aspects, the intracellular portion comprises at least one T cell co-stimulatory domain. In some aspects, the T cell co-stimulatory domain is selected from the group consisting of CD27, CD28, TNFRS9 (4-1BB), TNFRSF4 (OX40), TNFRSF8 (CD30), CD40LG (CD40L), ICOS, ITGB2 (LFA-1), CD2, CD7, KLRC2 (NKG2C), TNFRS18 (GITR), TNFRSF14 (HVEM), or any combination thereof.

In some aspects, the engineered receptor binds a target. In some aspects, the binding is specific to a peptide specific to one or more subjects suffering from a disease or condition.

In some aspects, the immunotherapeutic is a cell as described in detail herein. In some aspects, the immunotherapeutic is a cell comprising a receptor that specifically binds a peptide. In some aspects, the immunotherapeutic is a cell used in combination with the peptides/nucleic acids of this invent.ion. In some cases, the cell is a patient cell. In some cases, the cell is a T cell. In some cases, the cell is tumor infiltrating lymphocyte.

In some aspects, a subject with a condition or disease is treated based on a T cell receptor repertoire of the subject. In some cases, an antigen vaccine is selected based on a T cell receptor repertoire of the subject. In some cases, a subject is treated with T cells expressing TCRs specific to an antigen or peptide. In some cases, a subject is treated with an antigen or peptide specific to TCRs, e.g., subject specific TCRs. In some cases, a subject is treated with an antigen or peptide specific to T cells expressing TCRs, e.g., subject specific TCRs. In some cases, a subject is treated with an antigen or peptide specific to subject specific TCRs.

In some cases, an immunogenic antigen composition or vaccine is selected based on TCRs identified in one or more subjects. In some cases identification of a T cell repertoire and testing in functional assays is used to determine an immunogenic composition or vaccine to be administered to one or more subjects with a condition or disease. In some cases, the immunogenic composition is an antigen vaccine. In some cases, the antigen vaccine comprises subject specific antigen peptides. In some cases, antigen peptides to be included in an antigen vaccine are selected based on a quantification of subject specific TCRs that bind to the antigens. In some cases, antigen peptides are selected based on a binding affinity of the peptide to a TCR. In some cases, the selecting is based on a combination of both the quantity and the binding affinity. For example, a TCR that binds strongly to an antigen in a functional assay, but that is not highly represented in a TCR repertoire may be a good candidate for an antigen vaccine because T cells expressing the TCR would be advantageously amplified.

In some cases, antigens are selected for administering to one or more subjects based on binding to TCRs. In some cases, T cells, such as T cells from a subject with a disease or condition, can be expanded. Expanded T cells that express TCRs specific to an immunogenic antigen peptide, can be administered back to a subject. In some cases, suitable cells, e.g., PBMCs, are transduced or transfected with polynucleotides for expression of TCRs specific to an immunogenic antigen peptide and administered to a subject. T cells expressing TCRs specific to an immunogenic antigen peptide can be expanded and administered back to a subject. In some cases, T cells that express TCRs specific to an immunogenic antigen peptide that result in cytolytic activity when incubated with autologous diseased tissue can be expanded and administered to a subject. In some cases, T cells used in functional assays result in binding to an immunogenic antigen peptide can be expanded and administered to a subject. In some cases, TCRs that have been determined to bind to subject specific immunogenic antigen peptides can be expressed in T cells and administered to a subject.

Co-Stimulatory Ligands

In some cases, the cells are provided with at least one co-stimulatory ligand which is a non-antigenspecific signal important for full activation of an immune cell. Co-stimulatory ligands include, without limitation, tumor necrosis factor (TNF) ligands, cytokines (such as IL-2, IL-12, IL-15 or IL21), and immunoglobulin (Ig) superfamily ligands.

Tumor necrosis factor (TNF) is a cytokine involved in systemic inflammation and stimulates the acute phase reaction. Its primary role is in the regulation of immune cells. Tumor necrosis factor (TNF) ligands share a number of common features. The majority of the ligands are synthesized as type II transmembrane proteins containing a short cytoplasmic segment and a relatively long extracellular region. TNF ligands include, without limitation, nerve growth factor (NGF), CD4OL (CD4OL)/CD154, CD137L/4-1BBL, tumor necrosis factor alpha (TNFα), CD134L/OX4OL/CD252, CD27L/CD70, Fas ligand (FasL), CD3OL/CD153, tumor necrosis factor β (TNF(3)/lymphotoxin-alpha (LTa), lymphotoxin-beta (ur(3), CD257/B cell-activating factor (BAFF)/Blys/THANK/Ta11-1, glucocorticoid-induced TNF Receptor ligand (GITRL), and TNF-related apoptosis-inducing ligand (TRAIL), LIGHT (TNFSF14). The immunoglobulin (Ig) superfamily is a large group of cell surface and soluble proteins that are involved in the recognition, binding, or adhesion processes of cells. These proteins share structural features with immunoglobulins--they possess an immunoglobulin domain (fold). Immunoglobulin superfamily ligands include, without limitation, CD80 and CD86, both ligands for CD28.

Compositions comprising genetically modified immunoresponsive cells of the disclosure can be provided systemically or directly to a subject for the treatment of a neoplasia. In some cases, cells of the disclosure are directly injected into an organ of interest (e.g., an organ affected by a tumor). Alternatively, compositions comprising genetically modified immunoresponsive cells are provided indirectly to the organ of interest, for example, by administration into the circulatory system (e.g., the tumor vasculature). Expansion and differentiation agents can be provided prior to, during or after administration of the cells to increase production of T cells, NK cells, or CTL cells in vitro or in vivo.

The modified cells can be administered in any physiologically acceptable vehicle, normally intravascularly, although they may also be introduced into bone or other convenient site where the cells may find an appropriate site for regeneration and differentiation (e.g., thymus). Genetically modified immunoresponsive cells of the disclosure can comprise a purified population of cells. Those skilled in the art can readily determine the percentage of genetically modified immunoresponsive cells in a population using various well-known methods, such as fluorescence activated cell sorting (FACS). Dosages can be readily adjusted by those skilled in the art (e.g., a decrease in purity may require an increase in dosage). The cells can be introduced by injection, catheter, or the like. If desired, factors can also be included, including, but not limited to, interleukins, e.g. IL-2, IL-3, IL-6, and IL-11, as well as the other interleukins, the colony stimulating factors, such as G-, M- and GM-CSF, interferons, e.g. γ-interferon and erythropoietin.

Compositions of the disclosure include pharmaceutical compositions comprising genetically modified immunoresponsive cells or their progenitors and a pharmaceutically acceptable carrier. Administration can be autologous or heterologous. For example, immunoresponsive cells, or progenitors can be obtained from one subject, and administered to the same subject or a different, compatible subject. Peripheral blood derived immunoresponsive cells of the disclosure or their progeny (e.g., in vivo, ex vivo or in vitro derived) can be administered via localized injection, including catheter administration, systemic injection, localized injection, intravenous injection, or parenteral administration. When administering a therapeutic composition of the present disclosure (e.g., a pharmaceutical composition containing a genetically modified immunoresponsive cell), it will generally be formulated in a unit dosage injectable form (solution, suspension, emulsion).

VII. Methods of use and pharmaceutical compositions

The neoantigen therapeutics (e.g., peptides, polynucleotides, TCR, CAR, cells containing TCR or CAR, dendritic cell containing polypeptide, dendritic cell containing polynucleotide, antibody, etc.) described herein are useful in a variety of applications including, but not limited to, therapeutic treatment methods, such as the treatment of cancer. In some cases, the therapeutic treatment methods comprise immunotherapy. In certain cases, a neoantigen peptide is useful for activating, promoting, increasing, and/or enhancing an immune response, redirecting an existing immune response to a new target, increasing the immunogenicity of a tumor, inhibiting tumor growth, reducing tumor volume, increasing tumor cell apoptosis, and/or reducing the tumorigenicity of a tumor. The methods of use can be in vitro, ex vivo, or in vivo methods.

In some aspects, the present disclosure provides methods for activating an immune response in a subject using a neoantigen therapeutic described herein. In some cases, the disclosure provides methods for promoting an immune response in a subject using a neoantigen therapeutic described herein. In some cases, the disclosure provides methods for increasing an immune response in a subject using a neoantigen peptide described herein. In some cases, the disclosure provides methods for enhancing an immune response using a neoantigen peptide. In some cases, the activating, promoting, increasing, and/or enhancing of an immune response comprises increasing cell-mediated immunity. In some cases, the activating, promoting, increasing, and/or enhancing of an immune response comprises increasing T cell activity or humoral immunity. In some cases, the activating, promoting, increasing, and/or enhancing of an immune response comprises increasing CTL or HTL activity. In some cases, the activating, promoting, increasing, and/or enhancing of an immune response comprises increasing NK cell activity. In some cases, the activating, promoting, increasing, and/or enhancing of an immune response comprises increasing T cell activity and increasing NK cell activity. In some cases, the activating, promoting, increasing, and/or enhancing of an immune response comprises increasing CTL activity and increasing NK cell activity. In some cases, the activating, promoting, increasing, and/or enhancing of an immune response comprises inhibiting or decreasing the suppressive activity of Tregs. In some cases, the immune response is a result of antigenic stimulation. In some cases, the antigenic stimulation is a tumor cell. In some cases, the antigenic stimulation is cancer.

In some cases, the disclosure provides methods of activating, promoting, increasing, and/or enhancing of an immune response using a neoantigen therapeutic described herein. In some cases, a method comprises administering to a subject in need thereof a therapeutically effective amount of a neoantigen therapeutic that delivers a neoantigen polypeptide or polynucleotide to a tumor cell. In some cases, a method comprises administering to a subject in need thereof a therapeutically effective amount of a neoantigen therapeutic that binds the tumor associated antigen and is internalized by the tumor cell. In some cases, a method comprises administering to a subject in need thereof a therapeutically effective amount of a neoantigen polypeptide that is internalized by a tumor cell, and the neoantigen peptide is processed by the cell. In some cases, a method comprises administering to a subject in need thereof a therapeutically effective amount of a neoantigen polypeptide that is internalized by a tumor cell, and an antigenic peptide is presented on the surface of the tumor cell. In some cases, a method comprises administering to a subject in need thereof a therapeutically effective amount of a neoantigen polypeptide that is internalized by the tumor cell, is processed by the cell, and an antigenic peptide is presented on the surface of the tumor cell.

In some cases, a method comprises administering to a subject in need thereof a therapeutically effective amount of a neoantigen polypeptide or polynucleotide described herein that delivers an exogenous polypeptide comprising at least one antigenic peptide to a tumor cell, wherein the antigenic peptide is presented on the surface of the tumor cell. In some cases, the antigenic peptide is presented on the surface of the tumor cell in complex with a MHC class I molecule. In some cases, the antigenic peptide is presented on the surface of the tumor cell in complex with a MHC class II molecule.

In some cases, a method comprises contacting a tumor cell with a neoantigen polypeptide or polynucleotide described herein that delivers an exogenous polypeptide comprising at least one antigenic peptide to the tumor cell, wherein the antigenic peptide is presented on the surface of the tumor cell. In some cases, the antigenic peptide is presented on the surface of the tumor cell in complex with a MHC class I molecule. In some cases, the antigenic peptide is presented on the surface of the tumor cell in complex with a MHC class II molecule.

In some cases, a method comprises administering to a subject in need thereof a therapeutically effective amount of a neoantigen polypeptide or polynucleotide described herein that delivers an exogenous polypeptide comprising at least one antigenic peptide to a tumor cell, wherein the antigenic peptide is presented on the surface of the tumor cell, and an immune response against the tumor cell is induced. In some cases, the immune response against the tumor cell is increased. In some cases, the neoantigen polypeptide or polynucleotide delivers an exogenous polypeptide comprising at least one antigenic peptide to a tumor cell, wherein the antigenic peptide is presented on the surface of the tumor cell, and tumor growth is inhibited.

In some cases, a method comprises administering to a subject in need thereof a therapeutically effective amount of a neoantigen polypeptide or polynucleotide described herein that delivers an exogenous polypeptide comprising at least one antigenic peptide to a tumor cell, wherein the antigenic peptide is presented on the surface of the tumor cell, and T cell killing directed against the tumor cell is induced. In some cases, T cell killing directed against the tumor cell is enhanced. In some cases, T cell killing directed against the tumor cell is increased.

In some cases, a method of increasing an immune response in a subject comprises administering to the subject a therapeutically effective amount of a neoantigen therapeutic described herein, wherein the agent is an antibody that specifically binds the neoantigen described herein. In some cases, a method of increasing an immune response in a subject comprises administering to the subject a therapeutically effective amount of the antibody.

The present disclosure provides methods of redirecting an existing immune response to a tumor. In some cases, a method of redirecting an existing immune response to a tumor comprises administering to a subject a therapeutically effective amount of a neoantigen therapeutic described herein. In some cases, the existing immune response is against a virus. In some cases, the virus is selected from the group consisting of: measles virus, varicella-zoster virus (VZV; chickenpox virus), influenza virus, mumps virus, poliovirus, rubella virus, rotavirus, hepatitis A virus (HAV), hepatitis B virus (HBV), Epstein Barr virus (EBV), and cytomegalovirus (CMV). In some cases, the virus is varicella-zoster virus. In some cases, the virus is cytomegalovirus. In some cases, the virus is measles virus. In some cases, the existing immune response has been acquired after a natural viral infection. In some cases, the existing immune response has been acquired after vaccination against a virus. In some cases, the existing immune response is a cell-mediated response. In some cases, the existing immune response comprises cytotoxic T cells (CTLs) or HTLs.

In some cases, a method of redirecting an existing immune response to a tumor in a subject comprises administering a fusion protein comprising (i) an antibody that specifically binds a neoantigen and (ii) at least one neoantigenic peptide described herein, wherein (a) the fusion protein is internalized by a tumor cell after binding to the tumor-associated antigen; (b) the neoantigenic peptide is processed and presented on the surface of the tumor cell associated with a MHC class I molecule; and (c) the neoantigenic peptide/MHC Class I complex is recognized by cytotoxic T cells. In some cases, the cytotoxic T cells are memory T cells. In some cases, the memory T cells are the result of a vaccination with the neoantigenic peptide.

The present disclosure provides methods of increasing the immunogenicity of a tumor. In some cases, a method of increasing the immunogenicity of a tumor comprises contacting a tumor or tumor cells with an effective amount of a neoantigen therapeutic described herein. In some cases, a method of increasing the immunogenicity of a tumor comprises administering to a subject a therapeutically effective amount of a neoantigen therapeutic described herein.

The present disclosure also provides methods for inhibiting growth of a tumor using a neoantigen therapeutic described herein. In certain cases, a method of inhibiting growth of a tumor comprises contacting a cell mixture with a neoantigen therapeutic in vitro. For example, an immortalized cell line or a cancer cell line mixed with immune cells (e.g., T cells) is cultured in medium to which a neoantigenic peptide is added. In some cases, tumor cells are isolated from a patient sample, for example, a tissue biopsy, pleural effusion, or blood sample, mixed with immune cells (e.g., T cells), and cultured in medium to which a neoantigen therapeutic is added. In some cases, a neoantigen therapeutic increases, promotes, and/or enhances the activity of the immune cells. In some cases, a neoantigen therapeutic inhibits tumor cell growth. In some cases, a neoantigen therapeutic activates killing of the tumor cells.

In certain cases, the subject is a human. In certain cases, the subject has a tumor or the subject had a tumor which was at least partially removed.

In some cases, a method of inhibiting growth of a tumor comprises redirecting an existing immune response to a new target, comprising administering to a subject a therapeutically effective amount of a neoantigen therapeutic, wherein the existing immune response is against an antigenic peptide delivered to the tumor cell by the neoantigenic peptide.

In certain cases, the tumor comprises cancer stem cells. In certain cases, the frequency of cancer stem cells in the tumor is reduced by administration of the neoantigen therapeutic. In some cases, a method of reducing the frequency of cancer stem cells in a tumor in a subject, comprising administering to the subject a therapeutically effective amount of a neoantigen therapeutic is provided.

In addition, in some aspects the disclosure provides a method of reducing the tumorigenicity of a tumor in a subject, comprising administering to the subject a therapeutically effective amount of a neoantigen therapeutic described herein. In certain cases, the tumor comprises cancer stem cells. In some cases, the tumorigenicity of a tumor is reduced by reducing the frequency of cancer stem cells in the tumor. In some cases, the methods comprise using the neoantigen therapeutic described herein. In certain cases, the frequency of cancer stem cells in the tumor is reduced by administration of a neoantigen therapeutic described herein.

In some cases, the tumor is a solid tumor. In certain cases, the tumor is a tumor selected from the group consisting of: colorectal tumor, pancreatic tumor, lung tumor, ovarian tumor, liver tumor, breast tumor, kidney tumor, prostate tumor, neuroendocrine tumor, gastrointestinal tumor, melanoma, cervical tumor, bladder tumor, glioblastoma, and head and neck tumor. In certain cases, the tumor is a colorectal tumor. In certain cases, the tumor is an ovarian tumor. In some cases, the tumor is a breast tumor. In some cases, the tumor is a lung tumor. In certain cases, the tumor is a pancreatic tumor. In certain cases, the tumor is a melanoma tumor. In some cases, the tumor is a solid tumor.

The present disclosure further provides methods for treating cancer in a subject comprising administering to the subject a therapeutically effective amount of a neoantigen therapeutic described herein.

In some cases, a method of treating cancer comprises redirecting an existing immune response to a new target, the method comprising administering to a subject a therapeutically effective amount of neoantigen therapeutic, wherein the existing immune response is against an antigenic peptide delivered to the cancer cell by the neoantigenic peptide.

The present disclosure provides for methods of treating cancer comprising administering to a subject a therapeutically effective amount of a neoantigen therapeutic described herein (e.g., a subject in need of treatment). In certain cases, the subject is a human. In certain cases, the subject has a cancerous tumor. In certain cases, the subject has had a tumor at least partially removed.

In certain cases, the cancer is a cancer selected from the group consisting of colorectal cancer, pancreatic cancer, lung cancer, ovarian cancer, liver cancer, breast cancer, kidney cancer, prostate cancer, gastrointestinal cancer, melanoma, cervical cancer, neuroendocrine cancer, bladder cancer, glioblastoma, and head and neck cancer. In certain cases, the cancer is pancreatic cancer. In certain cases, the cancer is ovarian cancer. In certain cases, the cancer is colorectal cancer. In certain cases, the cancer is breast cancer. In certain cases, the cancer is prostate cancer. In certain cases, the cancer is lung cancer. In certain cases, the cancer is melanoma. In some cases, the cancer is a solid cancer. In some cases, the cancer comprises a solid tumor.

In some cases, the cancer is a hematologic cancer. In some case, the cancer is selected from the group consisting of: acute myelogenous leukemia (AML), Hodgkin lymphoma, multiple myeloma, T cell acute lymphoblastic leukemia (T-ALL), chronic lymphocytic leukemia (CLL), hairy cell leukemia, chronic myelogenous leukemia (CML), non-Hodgkin lymphoma, diffuse large B-cell lymphoma (DLBCL), mantle cell lymphoma (MCL), and cutaneous T cell lymphoma (CTCL).

In some embodiements, the neoantigen therapeutic is administered as a combination therapy. Combination therapy with two or more therapeutic agents uses agents that work by different mechanisms of action, although this is not required. Combination therapy using agents with different mechanisms of action can result in additive or synergetic effects. Combination therapy can allow for a lower dose of each agent than is used in monotherapy, thereby reducing toxic side effects and/or increasing the therapeutic index of the agent(s). Combination therapy can decrease the likelihood that resistant cancer cells will develop. In some cases, combination therapy comprises a therapeutic agent that affects the immune response (e.g., enhances or activates the response) and a therapeutic agent that affects (e.g., inhibits or kills) the tumor/cancer cells.

Cancers include, but are not limited to, B cell cancer, e.g., multiple myeloma, Waldenstrom's macroglobulinemia, the heavy chain diseases, such as, for example, alpha chain disease, gamma chain disease, and mu chain disease, benign monoclonal gammopathy, and immunocytic amyloidosis, melanomas, breast cancer, lung cancer, bronchus cancer, colorectal cancer, prostate cancer (e.g., metastatic, hormone refractory prostate cancer), pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, and the like. Other non-limiting examples of types of cancers applicable to the methods encompassed by the present disclosure include human sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colorectal cancer, pancreatic cancer, breast cancer, ovarian cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, liver cancer, choriocarcinoma, seminoma, embryonal carcinoma, Wilms' tumor, cervical cancer, bone cancer, brain tumor, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some cases, the cancer whose phenotype is determined by the method of the disclosure is an epithelial cancer such as, but not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In other cases, the cancer is breast cancer, prostate cancer, lung cancer, or colon cancer. In still other cases, the epithelial cancer is non-small-cell lung cancer, nonpapillary renal cell carcinoma, cervical carcinoma, ovarian carcinoma (e.g., serous ovarian carcinoma), or breast carcinoma. The epithelial cancers may be characterized in various other ways including, but not limited to, serous, endometrioid, mucinous, clear cell, brenner, or undifferentiated. In some cases, the present disclosure is used in the treatment, diagnosis, and/or prognosis of lymphoma or its subtypes, including, but not limited to, mantle cell lymphoma. Lymphoproliferative disorders are also considered to be proliferative diseases.

In some cases, the combination of an agent described herein and at least one additional therapeutic agent results in additive or synergistic results. In some cases, the combination therapy results in an increase in the therapeutic index of the agent. In some cases, the combination therapy results in an increase in the therapeutic index of the additional therapeutic agent(s). In some cases, the combination therapy results in a decrease in the toxicity and/or side effects of the agent. In some cases, the combination therapy results in a decrease in the toxicity and/or side effects of the additional therapeutic agent(s).

In certain cases, in addition to administering a neoantigen therapeutic described herein, the method or treatment further comprises administering at least one additional therapeutic agent. An additional therapeutic agent can be administered prior to, concurrently with, and/or subsequently to, administration of the agent. In some cases, the at least one additional therapeutic agent comprises 1, 2, 3, or more additional therapeutic agents.

Therapeutic agents that can be administered in combination with the neoantigen therapeutic described herein include chemotherapeutic agents. Thus, in some cases, the method or treatment involves the administration of an agent described herein in combination with a chemotherapeutic agent or in combination with a cocktail of chemotherapeutic agents. Treatment with an agent can occur prior to, concurrently with, or subsequent to administration of chemotherapies. Combined administration can include co-administration, either in a single pharmaceutical formulation or using separate formulations, or consecutive administration in either order but generally within a time period such that all active agents can exert their biological activities simultaneously. Preparation and dosing schedules for such chemotherapeutic agents can be used according to manufacturers' instructions or as determined empirically by the skilled practitioner. Preparation and dosing schedules for such chemotherapy are also described in The Chemotherapy Source Book, 4th Edition, 2008, M. C. Perry, Editor, Lippincott, Williams & Wilkins, Philadelphia, PA.

Useful classes of chemotherapeutic agents include, for example, anti-tubulin agents, auristatins, DNA minor groove binders, DNA replication inhibitors, alkylating agents (e.g., platinum complexes such as cisplatin, mono(platinum), bis(platinum) and tri-nuclear platinum complexes and carboplatin), anthracyclines, antibiotics, anti-folates, antimetabolites, chemotherapy sensitizers, duocarmycins, etoposides, fluorinated pyrimidines, ionophores, lexitropsins, nitrosoureas, platinols, purine antimetabolites, puromycins, radiation sensitizers, steroids, taxanes, topoisomerase inhibitors, vinca alkaloids, or the like. In certain cases, the second therapeutic agent is an alkylating agent, an antimetabolite, an antimitotic, a topoisomerase inhibitor, or an angiogenesis inhibitor.

Chemotherapeutic agents useful in the instant disclosure include, but are not limited to, alkylating agents such as thiotepa and cyclosphosphamide (CYTOXAN); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamime; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, caminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytosine arabinoside, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenishers such as folinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK; razoxane; sizofuran; spirogermanium; tenuazonic acid; triaziquone; 2,2',2''-trichlorotriethylamine; urethan; vindesine; dacarbazine; mannomustine; mitobronitol; mitolactol; pipobroman; gacytosine; arabinoside (Ara-C); taxoids, e.g. paclitaxel (TAXOL) and docetaxel (TAXOTERE); chlorambucil; gemcitabine; 6-thioguanine; mercaptopurine; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; ibandronate; CPT11; topoisomerase inhibitor RFS 2000; difluoromethylomithine (DMFO); retinoic acid; esperamicins; capecitabine (XELODA); and pharmaceutically acceptable salts, acids or derivatives of any of the above. Chemotherapeutic agents also include anti-hormonal agents that act to regulate or inhibit hormone action on tumors such as anti-estrogens including for example tamoxifen, raloxifene, aromatase inhibiting 4(5)-imidazoles, 4 hydroxytamoxifen, trioxifene, keoxifene, LY117018, onapristone, and toremifene (FARESTON); and anti-androgens such as flutamide, nilutamide, bicalutamide, leuprolide, and goserelin; and pharmaceutically acceptable salts, acids or derivatives of any of the above. In certain cases, the additional therapeutic agent is cisplatin. In certain cases, the additional therapeutic agent is carboplatin.

In certain cases, the chemotherapeutic agent is a topoisomerase inhibitor. Topoisomerase inhibitors are chemotherapy agents that interfere with the action of a topoisomerase enzyme (e.g., topoisomerase I or II). Topoisomerase inhibitors include, but are not limited to, doxorubicin HCl, daunorubicin citrate, mitoxantrone HCl, actinomycin D, etoposide, topotecan HCl, teniposide (VM-26), and irinotecan, as well as pharmaceutically acceptable salts, acids, or derivatives of any of these. In some cases, the additional therapeutic agent is irinotecan.

In certain cases, the chemotherapeutic agent is an anti-metabolite. An anti-metabolite is a chemical with a structure that is similar to a metabolite required for normal biochemical reactions, yet different enough to interfere with one or more normal functions of cells, such as cell division. Anti-metabolites include, but are not limited to, gemcitabine, fluorouracil, capecitabine, methotrexate sodium, ralitrexed, pemetrexed, tegafur, cytosine arabinoside, thioguanine, 5-azacytidine, 6 mercaptopurine, azathioprine, 6-thioguanine, pentostatin, fludarabine phosphate, and cladribine, as well as pharmaceutically acceptable salts, acids, or derivatives of any of these. In certain cases, the additional therapeutic agent is gemcitabine.

In certain cases, the chemotherapeutic agent is an antimitotic agent, including, but not limited to, agents that bind tubulin. In some cases, the agent is a taxane. In certain cases, the agent is paclitaxel or docetaxel, or a pharmaceutically acceptable salt, acid, or derivative of paclitaxel or docetaxel. In certain cases, the agent is paclitaxel (TAXOL), docetaxel (TAXOTERE), albumin-bound paclitaxel (ABRAXANE), DHA-paclitaxel, or PG-paclitaxel. In certain alternative cases, the antimitotic agent comprises a vinca alkaloid, such as vincristine, vinblastine, vinorelbine, or vindesine, or pharmaceutically acceptable salts, acids, or derivatives thereof. In some cases, the antimitotic agent is an inhibitor of kinesin Eg5 or an inhibitor of a mitotic kinase such as Aurora A or Plk1. In certain cases, the additional therapeutic agent is paclitaxel. In some cases, the additional therapeutic agent is albumin-bound paclitaxel.

In some cases, an additional therapeutic agent comprises an agent such as a small molecule. For example, treatment can involve the combined administration of an agent of the present disclosure with a small molecule that acts as an inhibitor against tumor-associated antigens including, but not limited to, EGFR, HER2 (ErbB2), and/or VEGF. In some cases, an agent of the present disclosure is administered in combination with a protein kinase inhibitor selected from the group consisting of: gefitinib (IRESSA), erlotinib (TARCEVA), sunitinib (SUTENT), lapatanib, vandetanib (ZACTIMA), AEE788, CI-1033, cediranib (RECENTIN), sorafenib (NEXAVAR), and pazopanib (GW786034B). In some cases, an additional therapeutic agent comprises an mTOR inhibitor. In another case, the additional therapeutic agent is chemotherapy or other inhibitors that reduce the number of Treg cells. In certain cases, the therapeutic agent is cyclophosphamide or an anti-CTLA4 antibody. In another case, the additional therapeutic reduces the presence of myeloid-derived suppressor cells. In a further case, the additional therapeutic is carbotaxol. In another case, the additional therapeutic agent shifts cells to a T helper 1 response. In a further case, the additional therapeutic agent is ibrutinib.

In some cases, an additional therapeutic agent comprises a biological molecule, such as an antibody. For example, treatment can involve the combined administration of an agent of the present disclosure with antibodies against tumor-associated antigens including, but not limited to, antibodies that bind EGFR, HER2/ErbB2, and/or VEGF. In certain cases, the additional therapeutic agent is an antibody specific for a cancer stem cell marker. In certain cases, the additional therapeutic agent is an antibody that is an angiogenesis inhibitor (e.g., an anti-VEGF or VEGF receptor antibody). In certain cases, the additional therapeutic agent is bevacizumab (AVASTIN), ramucirumab, trastuzumab (HERCEPTIN), pertuzumab (OMNITARG), panitumumab (VECTIBIX), nimotuzumab, zalutumumab, or cetuximab (ERBITUX).

The agents and compositions provided herein may be used alone or in combination with conventional therapeutic regimens such as surgery, irradiation, chemotherapy and/or bone marrow transplantation (autologous, syngeneic, allogeneic or unrelated). A set of tumor antigens can be useful, *e.g.,* in a large fraction of cancer patients.

In some cases, at least one or more chemotherapeutic agents may be administered in addition to the composition comprising an immunogenic vaccine. In some cases, the one or more chemotherapeutic agents may belong to different classes of chemotherapeutic agents.

Examples of chemotherapy agents include, but are not limited to, alkylating agents such as nitrogen mustards (e.g. mechlorethamine (nitrogen mustard), chlorambucil, cyclophosphamide (Cytoxan®), ifosfamide, and melphalan); nitrosoureas (e.g. N-Nitroso-N-methylurea, streptozocin, carmustine (BCNU), lomustine, and semustine); alkyl sulfonates (e.g. busulfan); tetrazines (e.g. dacarbazine (DTIC), mitozolomide and temozolomide (Temodar®)); aziridines (e.g. thiotepa, mytomycin and diaziquone); and platinum drugs (e.g. cisplatin, carboplatin, and oxaliplatin); non-classical alkylating agents such as procarbazine and altretamine (hexamethylmelamine); anti-metabolite agents such as 5-fluorouracil (5-FU), 6-mercaptopurine (6-MP), capecitabine (Xeloda®), cladribine, clofarabine, cytarabine (Ara-C®), decitabine, floxuridine, fludarabine, nelarabine, gemcitabine (Gemzar®), hydroxyurea, methotrexate, pemetrexed (Alimta®), pentostatin, thioguanine, Vidaza; anti-microtubule agents such as vinca alkaloids (e.g. vincristine, vinblastine, vinorelbine, vindesine and vinflunine); taxanes (e.g. paclitaxel (Taxol®), docetaxel (Taxotere®)); podophyllotoxin (e.g. etoposide and teniposide); epothilones (e.g. ixabepilone (Ixempra®)); estramustine (Emcyt®); anti-tumor antibiotics such as anthracyclines (e.g. daunorubicin, doxorubicin (Adriamycin®, epirubicin, idarubicin); actinomycin-D; and bleomycin; topoisomerase I inhibitors such as topotecan and irinotecan (CPT-11); topoisomerase II inhibitors such as etoposide (VP-16), teniposide, mitoxantrone, novobiocin, merbarone and aclarubicin; corticosteroids such as prednisone, methylprednisolone (Solumedrol®), and dexamethasone (Decadron®); L-asparaginase; bortezomib (Velcade®); immunotherapeutic agents such as rituximab (Rituxan®), alemtuzumab (Campath®), thalidomide, lenalidomide (Revlimid®), BCG, interleukin-2, interferon-alfa and cancer vaccines such as Provenge®; hormone therapeutic agents such as fulvestrant (Faslodex®), tamoxifen, toremifene (Fareston®), anastrozole (Arimidex®), exemestan (Aromasin®), letrozole (Femara®), megestrol acetate (Megace®), estrogens, bicalutamide (Casodex®), flutamide (Eulexin®), nilutamide (Nilandron®), leuprolide (Lupron®) and goserelin (Zoladex®); differentiating agents such as retinoids, tretinoin (ATRA or Atralin®), bexarotene (Targretin®) and arsenic trioxide (Arsenox®); and targeted therapeutic agents such as imatinib (Gleevec®), gefitinib (Iressa®) and sunitinib (Sutent®). In some cases, the chemotherapy is a cocktail therapy. Examples of a cocktail therapy includes, but is not limited to, CHOP/R-CHOP (rituxan, cyclophosphamide, hydroxydoxorubicin, vincristine, and prednisone), EPOCH (etoposide, prednisone, vincristine, cyclophosphamide, hydroxydoxorubicin), Hyper-CVAD (cyclophosphamide, vincristine, hydroxydoxorubicin, dexamethasone), FOLFOX (fluorouracil (5-FU), leucovorin, oxaliplatin), ICE (ifosfamide, carboplatin, etoposide), DHAP (high-dose cytarabine [ara-C], dexamethasone, cisplatin), ESHAP (etoposide, methylprednisolone, cytarabine [ara-C], cisplatin) and CMF (cyclophosphamide, methotrexate, fluouracil).

In certain cases, an additional therapeutic agent comprises a second immunotherapeutic agent. In some cases, the additional immunotherapeutic agent includes, but is not limited to, a colony stimulating factor, an interleukin, an antibody that blocks immunosuppressive functions (e.g., an anti-CTLA-4 antibody, anti-CD28 antibody, anti-CD3 antibody, anti-PD-1 antibody, anti-PD-L1 antibody, anti-TIGIT antibody), an antibody that enhances immune cell functions (e.g., an anti-GITR antibody, an anti-OX-40 antibody, an anti-CD40 antibody, or an anti-4-1BB antibody), a toll-like receptor (e.g., TLR4, TLR7, TLR9), a soluble ligand (e.g., GITRL, GITRL-Fc, OX-40L, OX-40L-Fc, CD40L, CD40L-Fc, 4-1BB ligand, or 4-1BB ligand-Fc), or a member of the B7 family (e.g., CD80, CD86). In some cases, the additional immunotherapeutic agent targets CTLA-4, CD28, CD3, PD-1, PD-L1, TIGIT, GITR, OX-40, CD-40, or 4-1BB.

In some cases, the additional therapeutic agent is an immune checkpoint inhibitor. In some cases, the immune checkpoint inhibitor is an anti-PD-1 antibody, an anti-PD-L1 antibody, an anti-CTLA-4 antibody, an anti-CD28 antibody, an anti-TIGIT antibody, an anti-LAG3 antibody, an anti-TIM3 antibody, an anti-GITR antibody, an anti-4-1BB antibody, or an anti-OX-40 antibody. In some cases, the additional therapeutic agent is an anti-TIGIT antibody. In some cases, the additional therapeutic agent is an anti-PD-1 antibody selected from the group consisting of: nivolumab (OPDIVO), pembrolizumab (KEYTRUDA), pidilzumab, MEDI0680, REGN2810, BGB-A317, and PDR001. In some cases, the additional therapeutic agent is an anti-PD-L1 antibody selected from the group consisting of: BMS935559 (MDX-1105), atexolizumab (MPDL3280A), durvalumab (MEDI4736), and avelumab (MSB0010718C). In some cases, the additional therapeutic agent is an anti-CTLA-4 antibody selected from the group consisting of: ipilimumab (YERVOY) and tremelimumab. In some cases, the additional therapeutic agent is an anti-LAG-3 antibody selected from the group consisting of: BMS-986016 and LAG525. In some cases, the additional therapeutic agent is an anti-OX-40 antibody selected from the group consisting of: MEDI6469, MEDI0562, and MOXR0916. In some cases, the additional therapeutic agent is an anti-4-1BB antibody selected from the group consisting of: PF-05082566.

In some cases, the neoantigen therapeutic can be administered in combination with a biologic molecule selected from the group consisting of: adrenomedullin (AM), angiopoietin (Ang), BMPs, BDNF, EGF, erythropoietin (EPO), FGF, GDNF, granulocyte colony stimulating factor (G-CSF), granulocyte-macrophage colony stimulating factor (GM-CSF), macrophage colony stimulating factor (M-CSF), stem cell factor (SCF), GDF9, HGF, HDGF, IGF, migration-stimulating factor, myostatin (GDF-8), NGF, neurotrophins, PDGF, thrombopoietin, TGF-α, TGF-β, TNF-α, VEGF, PlGF, gamma-IFN, IL-1, IL-2, IL-3, IL-4, IL-5, IL-6, IL-7, IL-12, IL-15, and IL-18.

In some cases, treatment with a neoantigen therapeutic described herein can be accompanied by surgical removal of tumors, removal of cancer cells, or any other surgical therapy deemed necessary by a treating physician.

In certain cases, treatment involves the administration of a neoantigen therapeutic described herein in combination with radiation therapy. Treatment with an agent can occur prior to, concurrently with, or subsequent to administration of radiation therapy. Dosing schedules for such radiation therapy can be determined by the skilled medical practitioner.

Combined administration can include co-administration, either in a single pharmaceutical formulation or using separate formulations, or consecutive administration in either order but generally within a time period such that all active agents can exert their biological activities simultaneously.

It will be appreciated that the combination of a neoantigen therapeutic described herein and at least one additional therapeutic agent can be administered in any order or concurrently. In some cases, the agent will be administered to patients that have previously undergone treatment with a second therapeutic agent. In certain other cases, the neoantigen therapeutic and a second therapeutic agent will be administered substantially simultaneously or concurrently. For example, a subject can be given an agent while undergoing a course of treatment with a second therapeutic agent (e.g., chemotherapy). In certain cases, a neoantigen therapeutic will be administered within 1 year of the treatment with a second therapeutic agent. It will further be appreciated that the two (or more) agents or treatments can be administered to the subject within a matter of hours or minutes (i.e., substantially simultaneously).

For the treatment of a disease, the appropriate dosage of a neoantigen therapeutic described herein depends on the type of disease to be treated, the severity and course of the disease, the responsiveness of the disease, whether the agent is administered for therapeutic or preventative purposes, previous therapy, the patient's clinical history, and so on, all at the discretion of the treating physician. The neoantigen therapeutic can be administered one time or over a series of treatments lasting from several days to several months, or until a cure is effected or a diminution of the disease state is achieved (e.g., reduction in tumor size). Optimal dosing schedules can be calculated from measurements of drug accumulation in the body of the patient and will vary depending on the relative potency of an individual agent. The administering physician can determine optimum dosages, dosing methodologies, and repetition rates.

In some cases, a neoantigen therapeutic can be administered at an initial higher "loading" dose, followed by one or more lower doses. In some cases, the frequency of administration can also change. In some cases, a dosing regimen can comprise administering an initial dose, followed by additional doses (or "maintenance" doses) once a week, once every two weeks, once every three weeks, or once every month. For example, a dosing regimen can comprise administering an initial loading dose, followed by a weekly maintenance dose of, for example, one-half of the initial dose. Or a dosing regimen can comprise administering an initial loading dose, followed by maintenance doses of, for example one-half of the initial dose every other week. Or a dosing regimen can comprise administering three initial doses for 3 weeks, followed by maintenance doses of, for example, the same amount every other week.

As is known to those of skill in the art, administration of any therapeutic agent can lead to side effects and/or toxicities. In some cases, the side effects and/or toxicities are so severe as to preclude administration of the particular agent at a therapeutically effective dose. In some cases, therapy must be discontinued, and other agents can be tried. However, many agents in the same therapeutic class display similar side effects and/or toxicities, meaning that the patient either has to stop therapy, or if possible, suffer from the unpleasant side effects associated with the therapeutic agent.

In some cases, the dosing schedule can be limited to a specific number of administrations or "cycles". In some cases, the agent is administered for 3, 4, 5, 6, 7, 8, or more cycles. For example, the agent is administered every 2 weeks for 6 cycles, the agent is administered every 3 weeks for 6 cycles, the agent is administered every 2 weeks for 4 cycles, the agent is administered every 3 weeks for 4 cycles, etc. Dosing schedules can be decided upon and subsequently modified by those skilled in the art.

The present disclosure provides methods of administering to a subject a neoantigen therapeutic described herein comprising using an intermittent dosing strategy for administering one or more agents, which can reduce side effects and/or toxicities associated with administration of an agent, chemotherapeutic agent, etc. In some cases, a method for treating cancer in a human subject comprises administering to the subject a therapeutically effective dose of a neoantigen therapeutic in combination with a therapeutically effective dose of a chemotherapeutic agent, wherein one or both of the agents are administered according to an intermittent dosing strategy. In some cases, a method for treating cancer in a human subject comprises administering to the subject a therapeutically effective dose of a neoantigen therapeutic in combination with a therapeutically effective dose of a second immunotherapeutic agent, wherein one or both of the agents are administered according to an intermittent dosing strategy. In some cases, the intermittent dosing strategy comprises administering an initial dose of a neoantigen therapeutic to the subject, and administering subsequent doses of the agent about once every 2 weeks. In some cases, the intermittent dosing strategy comprises administering an initial dose of a neoantigen therapeutic to the subject, and administering subsequent doses of the agent about once every 3 weeks. In some cases, the intermittent dosing strategy comprises administering an initial dose of a neoantigen therapeutic to the subject, and administering subsequent doses of the agent about once every 4 weeks. In some cases, the agent is administered using an intermittent dosing strategy and the additional therapeutic agent is administered weekly.

The present disclosure provides compositions comprising the neoantigen therapeutic described herein. The present disclosure also provides pharmaceutical compositions comprising a neoantigen therapeutic described herein and a pharmaceutically acceptable vehicle. In some cases, the pharmaceutical compositions find use in immunotherapy. In some cases, the compositions find use in inhibiting tumor growth. In some cases, the pharmaceutical compositions find use in inhibiting tumor growth in a subject (e.g., a human patient). In some cases, the compositions find use in treating cancer. In some cases, the pharmaceutical compositions find use in treating cancer in a subject (e.g., a human patient).

Formulations are prepared for storage and use by combining a neoantigen therapeutic of the present disclosure with a pharmaceutically acceptable vehicle (e.g., a carrier or excipient). Those of skill in the art generally consider pharmaceutically acceptable carriers, excipients, and/or stabilizers to be inactive ingredients of a formulation or pharmaceutical composition. Exemplary formulations are listed in WO 2015/095811.

Suitable pharmaceutically acceptable vehicles include, but are not limited to, nontoxic buffers such as phosphate, citrate, and other organic acids; salts such as sodium chloride; antioxidants including ascorbic acid and methionine; preservatives such as octadecyldimethylbenzyl ammonium chloride, hexamethonium chloride, benzalkonium chloride, benzethonium chloride, phenol, butyl or benzyl alcohol, alkyl parabens, such as methyl or propyl paraben, catechol, resorcinol, cyclohexanol, 3-pentanol, and m-cresol; low molecular weight polypeptides (e.g., less than about 10 amino acid residues); proteins such as serum albumin, gelatin, or immunoglobulins; hydrophilic polymers such as polyvinylpyrrolidone; amino acids such as glycine, glutamine, asparagine, histidine, arginine, or lysine; carbohydrates such as monosaccharides, disaccharides, glucose, mannose, or dextrins; chelating agents such as EDTA; sugars such as sucrose, mannitol, trehalose or sorbitol; salt-forming counter-ions such as sodium; metal complexes such as Zn-protein complexes; and nonionic surfactants such as TWEEN or polyethylene glycol (PEG). (Remington: The Science and Practice of Pharmacy, 22st Edition, 2012, Pharmaceutical Press, London.). In some cases, the vehicle is 5% dextrose in water.

The pharmaceutical compositions described herein can be administered in any number of ways for either local or systemic treatment. Administration can be topical by epidermal or transdermal patches, ointments, lotions, creams, gels, drops, suppositories, sprays, liquids and powders; pulmonary by inhalation or insufflation of powders or aerosols, including by nebulizer, intratracheal, and intranasal; oral; or parenteral including intravenous, intraarterial, intratumoral, subcutaneous, intraperitoneal, intramuscular (e.g., injection or infusion), or intracranial (e.g., intrathecal or intraventricular).

The therapeutic formulation can be in unit dosage form. Such formulations include tablets, pills, capsules, powders, granules, solutions or suspensions in water or non-aqueous media, or suppositories.

The neoantigenic peptides described herein can also be entrapped in microcapsules. Such microcapsules are prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions as described in Remington: The Science and Practice of Pharmacy, 22st Edition, 2012, Pharmaceutical Press, London.

**In** certain cases, pharmaceutical formulations include a neoantigen therapeutic described herein complexed with liposomes. Methods to produce liposomes are known to those of skill in the art. For example, some liposomes can be generated by reverse phase evaporation with a lipid composition comprising phosphatidylcholine, cholesterol, and PEG-derivatized phosphatidylethanolamine (PEG-PE). Liposomes can be extruded through filters of defined pore size to yield liposomes with the desired diameter.

In certain cases, sustained-release preparations comprising the neoantigenic peptides described herein can be produced. Suitable examples of sustained-release preparations include semi-permeable matrices of solid hydrophobic polymers containing an agent, where the matrices are in the form of shaped articles (e.g., films or microcapsules). Examples of sustained-release matrices include polyesters, hydrogels such as poly(2-hydroxyethyl-methacrylate) or poly(vinyl alcohol), polylactides, copolymers of L-glutamic acid and 7 ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), sucrose acetate isobutyrate, and poly-D-(-)-3-hydroxybutyric acid.

The present disclosure provides methods of treatment comprising an immunogenic vaccine. Methods of treatment for a disease (such as cancer or a viral infection) are provided. A method can comprise administering to a subject an effective amount of a composition comprising an immunogenic antigen. In some cases, the antigen comprises a viral antigen. In some cases, the antigen comprises a tumor antigen.

Non-limiting examples of vaccines that can be prepared include a peptide-based vaccine, a nucleic acid-based vaccine, an antibody based vaccine, a T cell based vaccine, and an antigen-presenting cell based vaccine.

Vaccine compositions can be formulated using one or more physiologically acceptable carriers including excipients and auxiliaries which facilitate processing of the active agents into preparations which can be used pharmaceutically. Proper formulation can be dependent upon the route of administration chosen. Any of the well-known techniques, carriers, and excipients can be used as suitable and as understood in the art.

In some cases, the vaccine composition is formulated as a peptide-based vaccine, a nucleic acid-based vaccine, an antibody based vaccine, or a cell based vaccine. For example, a vaccine composition can include naked cDNA in cationic lipid formulations; lipopeptides (e.g., Vitiello, A. et al., J. Clin. Invest. 95:341, 1995), naked cDNA or peptides, encapsulated *e.g.,* in poly(DL-lactide-co-glycolide) ("PLG") microspheres (see, *e.g.,* Eldridge, et al., Molec. Immunol. 28:287-294, 1991: Alonso et al, Vaccine 12:299-306, 1994; Jones et al, Vaccine 13:675-681, 1995); peptide composition contained in immune stimulating complexes (ISCOMS) (e.g., Takahashi et al, Nature 344:873-875, 1990; Hu et al, Clin Exp Immunol. 113:235-243, 1998); or multiple antigen peptide systems (MAPs) (see *e.g.,* Tam, J. P., Proc. Natl Acad. Sci. U.S.A. 85:5409-5413, 1988; Tarn, J.P., J. Immunol. Methods 196:17-32, 1996). Sometimes, a vaccine is formulated as a peptide-based vaccine, or nucleic acid based vaccine in which the nucleic acid encodes the polypeptides. Sometimes, a vaccine is formulated as an antibody based vaccine. Sometimes, a vaccine is formulated as a cell based vaccine.

The amino acid sequence of an identified disease-specific immunogenic neoantigen peptide can be used develop a pharmaceutically acceptable composition. The source of antigen can be, but is not limited to, natural or synthetic proteins, including glycoproteins, peptides, and superantigens; antibody/antigen complexes; lipoproteins; RNA or a translation product thereof; and DNA or a polypeptide encoded by the DNA. The source of antigen may also comprise non-transformed, transformed, transfected, or transduced cells or cell lines. Cells may be transformed, transfected, or transduced using any of a variety of expression or retroviral vectors known to those of ordinary skill in the art that may be employed to express recombinant antigens. Expression may also be achieved in any appropriate host cell that has been transformed, transfected, or transduced with an expression or retroviral vector containing a DNA molecule encoding recombinant antigen(s). Any number of transfection, transformation, and transduction protocols known to those in the art may be used. Recombinant vaccinia vectors and cells infected with the vaccina vector, may be used as a source of antigen.

A composition can comprise a synthetic disease-specific immunogenic neoantigen peptide. A composition can comprise two or more disease-specific immunogenic neoantigen peptides. A composition may comprise a precursor to a disease-specific immunogenic peptide (such as a protein, peptide, DNA and RNA). A precursor to a disease-specific immunogenic peptide can generate or be generated to the identified disease-specific immunogenic neoantigen peptide. In some cases, a therapeutic composition comprises a precursor of an immunogenic peptide. The precursor to a disease-specific immunogenic peptide can be a prodrug. In some cases, the composition comprising a disease-specific immunogenic neoantigen peptide may further comprise an adjuvant. For example, the neoantigen peptide can be utilized as a vaccine. In some cases, an immunogenic vaccine may comprise a pharmaceutically acceptable immunogenic neoantigen peptide. In some cases, an immunogenic vaccine may comprise a pharmaceutically acceptable precursor to an immunogenic neoantigen peptide (such as a protein, peptide, DNA and RNA). In some cases, a method of treatment comprises administering to a subject an effective amount of an antibody specifically recognizing an immunogenic neoantigen peptide. In some cases, a method of treatment comprises administering to a subject an effective amount of a soluble TCR or TCR analog specifically recognizing an immunogenic neoantigen peptide.

The methods described herein are particularly useful in the personalized medicine context, where immunogenic neoantigen peptides are used to develop therapeutics (such as vaccines or therapeutic antibodies) for the same individual. Thus, a method of treating a disease in a subject can comprise identifying an immunogenic neoantigen peptide in a subject according to the methods described herein; and synthesizing the peptide (or a precursor thereof); and administering the peptide or an antibody specifically recognizing the peptide to the subject. In some cases, an expression pattern of an immunogenic neoantigen can serve as the essential basis for the generation of patient specific vaccines. In some cases, an expression pattern of an immunogenic neoantigen can serve as the essential basis for the generation of a vaccine for a group of patients with a particular disease. Thus, particular diseases, e.g., particular types of tumors, can be selectively treated in a patient group.

In some cases, the peptides described herein are structurally normal ("shared") antigens that can be recognized by autologous anti-disease T cells in a large patient group. In some cases, an antigen-expression pattern of a group of diseased subjects whose disease expresses structurally normal ("shared") neoantigens is determined.

There are a variety of ways in which to produce immunogenic neoantigens. Proteins or peptides may be made by any technique known to those of skill in the art, including the expression of proteins, polypeptides or peptides through standard molecular biological techniques, the isolation of proteins or peptides from natural sources, *in vitro* translation, or the chemical synthesis of proteins or peptides. In general, such disease specific neoantigens may be produced either *in vitro* or *in vivo.* Immunogenic neoantigens may be produced *in vitro* as peptides or polypeptides, which may then be formulated into a personalized vaccine or immunogenic composition and administered to a subject. *In vitro* production of immunogenic neoantigens can comprise peptide synthesis or expression of a peptide/polypeptide from a DNA or RNA molecule in any of a variety of bacterial, eukaryotic, or viral recombinant expression systems, followed by purification of the expressed peptide/polypeptide. Alternatively, immunogenic neoantigens can be produced *in vivo* by introducing molecules (*e.g.*, DNA, RNA, and viral expression systems) that encode an immunogenic neoantigen into a subject, whereupon the encoded immunogenic neoantigens are expressed. In some cases, a polynucleotide encoding an immunogenic neoantigen peptide can be used to produce the neoantigen peptide *in vitro.*

In some cases, a polynucleotide comprises a sequence with at least 60%, 65%, 70%l, 75%, 80%, 85%, 90%, 95%, 96%, 97%, 98%, 99%, or 100% identity to a polynucleotide encoding an immunogenic neoantigen.

The polynucleotide may be, *e.g.,* DNA, cDNA, PNA, CNA, RNA, single- and/or double-stranded, native or stabilized forms of polynucleotides, or combinations thereof. A nucleic acid encoding an immunogenic neoantigen peptide may or may not contain introns so long as it codes for the peptide. In some cases *in vitro* translation is used to produce the peptide.

Expression vectors comprising sequences encoding the neoantigen, as well as host cells containing the expression vectors, are also contemplated. Expression vectors suitable for use in the present disclosure can comprise at least one expression control element operationally linked to the nucleic acid sequence. The expression control elements are inserted in the vector to control and regulate the expression of the nucleic acid sequence. Examples of expression control elements are well known in the art and include, for example, the lac system, operator and promoter regions of phage lambda, yeast promoters and promoters derived from polyoma, adenovirus, retrovirus or SV40. Additional operational elements include, but are not limited to, leader sequences, termination codons, polyadenylation signals and any other sequences necessary or preferred for the appropriate transcription and subsequent translation of the nucleic acid sequence in the host system. It will be understood by one skilled in the art the correct combination of expression control elements will depend on the host system chosen. It will further be understood that the expression vector should contain additional elements necessary for the transfer and subsequent replication of the expression vector containing the nucleic acid sequence in the host system. Examples of such elements include, but are not limited to, origins of replication and selectable markers.

The neoantigen peptides may be provided in the form of RNA or cDNA molecules encoding the desired neoantigen peptides. One or more neoantigen peptides of the disclosure may be encoded by a single expression vector. Generally, the DNA is inserted into an expression vector, such as a plasmid, in proper orientation and correct reading frame for expression, if necessary, the DNA may be linked to the appropriate transcriptional and translational regulatory control nucleotide sequences recognized by the desired host (*e.g.,* bacteria), although such controls are generally available in the expression vector. The vector is then introduced into the host bacteria for cloning using standard techniques. Useful expression vectors for eukaryotic hosts, especially mammals or humans include, for example, vectors comprising expression control sequences from SV40, bovine papilloma virus, adenovirus and cytomegalovirus. Useful expression vectors for bacterial hosts include known bacterial plasmids, such as plasmids from E. coli, including pCR 1, pBR322, pMB9 and their derivatives, wider host range plasmids, such as M13 and filamentous single-stranded DNA phages.

In cases, a DNA sequence encoding a polypeptide of interest can be constructed by chemical synthesis using an oligonucleotide synthesizer. Such oligonucleotides can be designed based on the amino acid sequence of the desired polypeptide and selecting those codons that are favored in the host cell in which the recombinant polypeptide of interest is produced. Standard methods can be applied to synthesize an isolated polynucleotide sequence encoding an isolated polypeptide of interest.

Suitable host cells for expression of a polypeptide include prokaryotes, yeast, insect or higher eukaryotic cells under the control of appropriate promoters. Prokaryotes include gram negative or gram positive organisms, for example *E. coli* or *bacilli.* Higher eukaryotic cells include established cell lines of mammalian origin. Cell-free translation systems can also be employed. Appropriate cloning and expression vectors for use with bacterial, fungal, yeast, and mammalian cellular hosts are well known in the art. Various mammalian or insect cell culture systems can be employed to express recombinant protein. Exemplary mammalian host cell lines include, but are not limited to COS-7, L cells, C127, 3T3, Chinese hamster ovary (CHO), 293, HeLa and BHK cell lines. Mammalian expression vectors can comprise nontranscribed elements such as an origin of replication, a suitable promoter and enhancer linked to the gene to be expressed, and other 5' or 3' flanking nontranscribed sequences, and 5' or 3' nontranslated sequences, such as necessary ribosome binding sites, a polyadenylation site, splice donor and acceptor sites, and transcriptional termination sequences.

The proteins produced by a transformed host can be purified according to any suitable method. Such standard methods include chromatography (*e.g.*, ion exchange, affinity and sizing column chromatography, and the like), centrifugation, differential solubility, or by any other standard technique for protein purification. Affinity tags such as hexahistidine, maltose binding domain, influenza coat sequence, glutathione-S-transferase, and the like can be attached to the protein to allow easy purification by passage over an appropriate affinity column. Isolated proteins can also be physically characterized using such techniques as proteolysis, nuclear magnetic resonance and x-ray crystallography.

A vaccine can comprise an entity that binds a polypeptide sequence described herein. The entity can be an antibody. Antibody-based vaccine can be formulated using any of the well-known techniques, carriers, and excipients as suitable and as understood in the art. In some cases, the peptides described herein can be used for making neoantigen specific therapeutics such as antibody therapeutics. For example, neoantigens can be used to raise and/or identify antibodies specifically recognizing the neoantigens. These antibodies can be used as therapeutics. The antibody can be a natural antibody, a chimeric antibody, a humanized antibody, or can be an antibody fragment. The antibody may recognize one or more of the polypeptides described herein. The antibody can recognize a polypeptide that has a sequence that is at most 40%, 50%, 60%, 70%, 80%, 90%, 95%, or less in sequence homology to a polypeptide described herein. The antibody can recognize a polypeptide that has a sequence that is at least 40%, 50%, 60%, 70%, 80%, 90%, 95%, or more in sequence homology to a polypeptide described herein. The antibody can also recognize a polypeptide with sequence length is at least 30%, 40%, 50%, 60%, 70%, 80%, 90%, 95%, or more in sequence length compared to a polypeptide described herein. The antibody can recognize a polypeptide with a sequence length at most 30%, 40%, 50%, 60%, 70%, 80%, 90%, 95%, or less in sequence length compared to a polypeptide described herein.

The present disclosure also contemplates the use of nucleic acid molecules as vehicles for delivering neoantigen peptides/polypeptides to the subject in need thereof, *in vivo,* in the form of, *e.g.,* DNA/RNA vaccines.

In some cases, the vaccine is a nucleic acid vaccine. In some cases, neoantigens can be administered to a subject by use of a plasmid. Plasmids may be introduced into animal tissues by a number of different methods, *e.g.,* injection or aerosol instillation of naked DNA on mucosal surfaces, such as the nasal and lung mucosa. In some cases, physical delivery, such as with a "gene-gun" may be used. The exact choice of expression vectors can depend upon the peptide/polypeptides to be expressed, and is well within the skill of the ordinary artisan.

In some cases, the nucleic acid encodes an immunogenic peptide or peptide precursor. In some cases, the nucleic acid vaccine comprises sequences flanking the sequence coding the immunogenic peptide or peptide precursor. In some cases, the nucleic acid vaccine comprises more than one immunogenic epitope. In some cases, the nucleic acid vaccine is a DNA-based vaccine. In some cases, the nucleic acid vaccine is a RNA-based vaccine. In some cases, the RNA-based vaccine comprises mRNA. In some cases, the RNA-based vaccine comprises naked mRNA. In some cases, the RNA-based vaccine comprises modified mRNA (*e.g.*, mRNA protected from degradation using protamine. mRNA containing modified 5' CAP structure or mRNA containing modified nucleotides). In some cases, the RNA-based vaccine comprises single-stranded mRNA.

The polynucleotide may be substantially pure, or contained in a suitable vector or delivery system. Suitable vectors and delivery systems include viral, such as systems based on adenovirus, vaccinia virus, retroviruses, herpes virus, adeno-associated virus or hybrids containing elements of more than one virus. Non-viral delivery systems include cationic lipids and cationic polymers (*e.g.*, cationic liposomes).

One or more neoantigen peptides can be encoded and expressed *in vivo* using a viral based system. Viral vectors may be used as recombinant vectors in the present disclosure, wherein a portion of the viral genome is deleted to introduce new genes without destroying infectivity of the virus. The viral vector of the present disclosure is a nonpathogenic virus. In some cases the viral vector has a tropism for a specific cell type in the mammal. In another case, the viral vector of the present disclosure is able to infect professional antigen presenting cells such as dendritic cells and macrophages. In yet another case of the present disclosure, the viral vector is able to infect any cell in the mammal. The viral vector may also infect tumor cells. Viral vectors used in the present disclosure include Poxvirus such as vaccinia virus, *avipox* virus, fowlpox virus and a highly attenuated vaccinia virus (Ankara or MVA), retrovirus, adenovirus, baculovirus and the like.

A vaccine can be delivered via a variety of routes. Delivery routes can include oral (including buccal and sub-lingual), rectal, nasal, topical, transdermal patch, pulmonary, vaginal, suppository, or parenteral (including intramuscular, intraarterial, intrathecal, intradermal, intraperitoneal, subcutaneous and intravenous) administration or in a form suitable for administration by aerosolization, inhalation or insufflation. General information on drug delivery systems can be found in Ansel et al., Pharmaceutical Dosage Forms and Drug Delivery Systems (Lippencott Williams & Wilkins, Baltimore Md. (1999). The vaccine described herein can be administered to muscle, or can be administered via intradermal or subcutaneous injections, or transdermally, such as by iontophoresis. Epidermal administration of the vaccine can be employed.

In some instances, the vaccine can also be formulated for administration via the nasal passages. Formulations suitable for nasal administration, wherein the carrier is a solid, can include a coarse powder having a particle size, for example, in the range of about 10 to about 500 microns which is administered in the manner in which snuff is taken, i.e., by rapid inhalation through the nasal passage from a container of the powder held close up to the nose. The formulation can be a nasal spray, nasal drops, or by aerosol administration by nebulizer. The formulation can include aqueous or oily solutions of the vaccine.

The vaccine can be a liquid preparation such as a suspension, syrup or elixir. The vaccine can also be a preparation for parenteral, subcutaneous, intradermal, intramuscular or intravenous administration (e.g., injectable administration), such as a sterile suspension or emulsion.

The vaccine can include material for a single immunization, or may include material for multiple immunizations (i.e. a 'multidose' kit). The inclusion of a preservative is preferred in multidose arrangements. As an alternative (or in addition) to including a preservative in multidose compositions, the compositions can be contained in a container having an aseptic adaptor for removal of material.

The vaccine can be administered in a dosage volume of about 0.5 mL, although a half dose (i.e. about 0.25 mL) can be administered to children. Sometimes the vaccine can be administered in a higher dose e.g. about 1 ml.

The vaccine can be administered as a 1, 2, 3, 4, 5, 6, 7, 8, 9, 10, or more dose-course regimen. Sometimes, the vaccine is administered as a 1, 2, 3, or 4 dose-course regimen. Sometimes the vaccine is administered as a 1 dose-course regimen. Sometimes the vaccine is administered as a 2 dose-course regimen.

The administration of the first dose and second dose can be separated by about 0 day, 1 day, 2 days, 5 days, 7 days, 14 days, 21 days, 30 days, 2 months, 4 months, 6 months, 9 months, 1 year, 1.5 years, 2 years, 3 years, 4 years, or more.

The vaccine described herein can be administered every 1, 2, 3, 4, 5, 6, 7, 8, 9, 10, or more years. Sometimes, the vaccine described herein is administered every 2, 3, 4, 5, 6, 7, or more years. Sometimes, the vaccine described herein is administered every 4, 5, 6, 7, or more years. Sometimes, the vaccine described herein is administered once.

The dosage examples are not limiting and are only used to exemplify particular dosing regiments for administering a vaccine described herein. The effective amount for use in humans can be determined from animal models. For example, a dose for humans can be formulated to achieve circulating, liver, topical and/or gastrointestinal concentrations that have been found to be effective in animals. Based on animal data, and other types of similar data, those skilled in the art can determine the effective amounts of a vaccine composition appropriate for humans.

The effective amount when referring to an agent or combination of agents will generally mean the dose ranges, modes of administration, formulations, etc., that have been recommended or approved by any of the various regulatory or advisory organizations in the medical or pharmaceutical arts (e.g., FDA, AMA) or by the manufacturer or supplier.

In some aspects, the vaccine and kit described herein can be stored at between 2°C and 8°C. In some instances, the vaccine is not stored frozen. In some instances, the vaccine is stored in temperatures of such as at -20°C or -80°C. In some instances, the vaccine is stored away from sunlight.

## VIII. Kits

The neoantigen therapeutic described herein can be provided in kit form together with instructions for administration. Typically the kit would include the desired neoantigen therapeutic in a container, in unit dosage form and instructions for administration. Additional therapeutics, for example, cytokines, lymphokines, checkpoint inhibitors, antibodies, can also be included in the kit. Other kit components that can also be desirable include, for example, a sterile syringe, booster dosages, and other desired excipients.

Kits and articles of manufacture are also provided herein for use with one or more methods described herein. The kits can contain one or more neoantigen polypeptides. The kits can also contain nucleic acids that encode one or more of the polypeptides described herein, antibodies that recognize one or more of the polypeptides described herein, or APC-based cells activated with one or more of the polypeptides described herein. The kits can further contain adjuvants, reagents, and buffers necessary for the makeup and delivery of the vaccines.

The kits can also include a carrier, package, or container that is compartmentalized to receive one or more containers such as vials, tubes, and the like, each of the container(s) comprising one of the separate elements, such as the polypeptides and adjuvants, to be used in a method described herein. Suitable containers include, for example, bottles, vials, syringes, and test tubes. The containers can be formed from a variety of materials such as glass or plastic.

The articles of manufacture provided herein contain packaging materials. Examples of pharmaceutical packaging materials include, but are not limited to, blister packs, bottles, tubes, bags, containers, bottles, and any packaging material suitable for a selected formulation and intended mode of administration and treatment. A kit typically includes labels listing contents and/or instructions for use, and package inserts with instructions for use. A set of instructions will also typically be included.

The present disclosure will be described in greater detail by way of specific examples. The following examples are offered for illustrative purposes of the present disclosure. Those of skill in the art will readily recognize a variety of non-critical parameters that can be changed or modified to yield alternative cases. All patents, patent applications, and printed publications listed herein are for the assistance of the skilled person.

## EXAMPLES

### Example 1: Identification of mutant sequences with immunogenic potential

Applicants have discovered mutations that are recurrent in cancer patients. See, Tables 1 and 2.

Neoantigenic peptides described herein can be derived from the mutations listed in Tables 1 and 2. For example, a neoantigenic peptide can be derived from a substitution mutation, e.g., the KRAS G12V mutation. The neonatigenic peptide can be a peptide comprising 5, 6, 7, 8, 9, 10, 11, 12, 13, 14, 15, 16, 17, 18, 19, 20, 25, 30, 35, 40, 45, 50, 60, 70, 80, 90, or 100 amino acid residues of the protein, e.g., KRAS including the substitution (G12V). The substitution may be positioned anywhere along the length of the neoantigenic peptide. For example, it can be located in the N terminal third of the neoantigenic peptide, the central third of the neoantigenic peptide or the C terminal third of the neoantigenic peptide. In another example, the substituted residue is located 2-5 residues away from the N terminal end or 2-5 residues away from the C terminal end. Neoantigenic peptides can similarly derived from tumor specifc insertion mutations where the neonatigenic peptide comprises one or more, or all of the inserted residues.

Neoantigenic peptides can also be derived from frame shift mutation, fusion polypeptides, in-frame deletions, and splice variants. The tumor specific polypeptides resulting from these mutations can be characterized by the existence of a transition point between a polypeptide sequence encoded by the germline and the tumor specific mutant polypeptide encoded by the tumor specific mutation. The neonatigenic peptide can be a peptide of about 5, 6, 7, 8, 9, 10, 11, 12, 13, 14, 15, 16, 17, 18, 19, 20, 25, 30, 35, 40, 45, 50, 60, 70, 80, 90, or 100 amino acid residues that encompasses the transition point between the germline encoded native sequence and the tumor specific polypeptide sequence. The transition point can be positioned anywhere along the length of the neoantigenic peptide. For example, it can be located in the N terminal third of the neoantigenic peptide, the central third of the neoantigenic peptide or the C terminal third of the neoantigenic peptide. In another example, the transition point is located 2-5 residues away from the N terminal end or 2-5 residues away from the C terminal end. A neoantigenic peptide derived from frame shift mutation and splice variants can also be a peptide consisting of tumor specific mutant residues.

For each mutation event listed in Tables 1 and 2, the full-length amino acid sequence of the mutant protein was derived. Any constituent 9mer or 10mer not found in the germline protein sequence was flagged as a neo-peptide and scored for binding potential on six common HLA alleles (HLA-A01:01, HLA-A02:01. HLA-A03:01, HLA-A11:01, HLA-A24:02, HLA-B07:02, and HLA-B08:01) using available algorithms. Any peptide scoring better than 1000 nM was nominated. The nominated peptides are listed in Tables 1 and 2 followed by the high scoring HLA alleles in parenthesis.

**Table 1**

| Gene | Exemplary Protein Change | Mutation Sequence Context | Peptides (HLA allele example(s)) | Exemplary Diseases |
|---|---|---|---|---|
| **TABLE 1A** | | POINT MUTATION[1] | | |
| ABL1 | E255K | VADGLITTLHYPAPKRNK PTVYGVSPNYDKWEMER TDITMKHKLGGGQYGKV YEGVWKKYSLTVAVKTL KEDTMEVEEFLKEAAVM KEIKHPNLVQLLGVC | GQYGKVYEG (A02.01) GQYGKVYEGV (A02.01) KLGGGQYGK (A03.01) KLGGGQYGKV (A02.01) KVYEGVWKK (A02.01, A03.01) KVYEGVWKKY (A03.01) QYGKVYEGV (A24.02) QYGKVYEGVW (A24.02) | Chronic myeloid leukemia (CML), Acute lymphocytic leukemia (ALL), Gastrointestinal stromal tumors (GIST) |
| ABL1 | E255V | VADGLITTLHYPAPKRNK PTVYGVSPNYDKWEMER TDITMKHKLGGGQYGVV YEGVWKKYSLTVAVKTL KEDTMEVEEFLKEAAVM KEIKHPNLVQLLGVC | GQYGVVYEG (A02.01) GQYGVVYEGV (A02.01) KLGGGQYGV (A02.01) KLGGGQYGVV (A02.01) QYGVVYEGV (A24.02) QYGVVYEGVW (A24.02) VVYEGVWKK (A02.01, A03.01) VVYEGVWKKY (A03.01) | Chronic myeloid leukemia (CML), Acute lymphocytic leukemia (ALL), Gastrointestinal stromal tumors (GIST) |
| ABL1 | M351T | LLGVCTREPPFYIITEFMT YGNLLDYLRECNRQEVN AVVLLYMATQISSATEYL EKKNFIHRDLAARNCLVG ENHLVKVADFGLSRLMT GDTYTAHAGAKF | ATQISSATEY (A01.01) ISSATEYLEK (A03.01) SSATEYLEK (A03.01) TQISSATEYL (A02.01) YMATQISSAT (A02.01) | Chronic myeloid leukemia (CML), Acute lymphocytic leukemia (ALL), Gastrointestinal stromal tumors (GIST) |
| ABL1 | T315I | SLTVAVKTLKEDTMEVEE FLKEAAVMKEIKHPNLVQ LLGVCTREPPFYIIEFMTY GNLLDYLRECNRQEVNA VVLLYMATQISSAMEYLE KKNFIHRDLA | FYIIEFMTY (A24.02) IIEFMTYGNL (A02.01) IIIEFMTYG (A02.01) IIIEFMTYGN (A02.01) YIIIEFMTYG (A02.01) | Chronic myeloid leukemia (CML), Acute lymphocytic leukemia (ALL), Gastrointestinal stromal tumors (GIST) |

| Gene | Exemplary Protein Change | Mutation Sequence Context | Peptides (HLA allele example(s)) | Exemplary Diseases |
|---|---|---|---|---|
| **TABLE 1A** | | **POINT MUTATION**[1] | | |
| ABL1 | Y253H | STVADGLITTLHYPAPKR NKPTVYGVSPNYDKWEM ERTDITMKHKLGGGQHG EVYEGVWKKYSLTVAVK TLKEDTMEVEEFLKEAAV MKEIKHPNLVQLLG | GQHGEVYEGV (A02.01)<br><br>KLGGGQHGEV (A02.01) | Chronic myeloid leukemia (CML), Acute lymphocytic leukemia (ALL), Gastrointestinal stromal tumors (GIST) |
| ALK | G1269A | SSLAMLDLLHVARDIACG CQYLEENHFIHRDIAARN CLLTCPGPGRVAKIADFG MARDIYRASYYRKGGCA MLPVKWMPPEAFMEGIFT SKTDTWSFGVLL | KIADFGMAR (A03.01)<br><br>RVAKIADFGM (A02.01, B07.02) | NSCLC |
| ALK | L 1196M | QVAVKTLPEVCSEQDELD FLMEALIISKFNHQNIVRC IGVSLQSLPRFILMELMAG GDLKSFLRETRPRPSQPSS LAMLDLLHVARDIACGC QYLEENHFI | FILMELMAGG (A02.01) ILMELMAGG (A02.01) ILMELMAGGD (A02.01) LMELMAGGDL (A02.01) LPRFILMEL (B07.02, B08.01) LPRFILMELM (B07.02) LQSLPRFILM (A02.01, B08.01) SLPRFILMEL (A02.01, A24.02, B07.02, B08.01) | NSCLC |
| BRAF | V600E | MIKLIDIARQTAQGMDYL HAKSIIHRDLKSNNIFLHE DLTVKIGDFGLATEKSRW SGSHQFEQLSGSILWMAP EVIRMQDKNPYSFQSDVY AFGIVLYELM | LATEKSRWS (A02.01, B08.01)<br><br>LATEKSRWSG (A02.01, B08.01) | CRC, GBM, KIRP, LUAD, SKCM, THCA |

| Gene | Exemplary Protein Change | Mutation Sequence Context | Peptides (HLA allele example(s)) | Exemplary Diseases |
|---|---|---|---|---|
| TABLE 1A | | POINT MUTATION[1] | | |
| BTK | C481S | MIKEGSMSEDEFIEEAKV MMNLSHEKLVQLYGVCT KQRPIFIITEYMANGSLLN YLREMRHRFQTQQLLEM CKDVCEAMEYLESKQFL HRDLAARNCLVND | EYMANGSLL (A24.02) MANGSLLNY (A01.01, A03.01, A11.01) MANGSLLNYL (A02.01, B07.02, B08.01) SLLNYLREM (A02.01, B07.02, B08.01) YMANGSLLN (A02.01) YMANGSLLNY (A01.01, A03.01, A11.01) | CLL |
| EEF1B2 | S43G | MGFGDLKSPAGLQVLND YLADKSYIEGYVPSQADV AVFEAVSGPPPADLCHAL RWYNHIKSYEKEKASLPG VKKALGKYGPADVEDTT GSGAT | GPPPADLCHAL (B07.02) | BLCA, KIRP, PRAD, SKCM |
| EGFR | S492R | SLNITSLGLRSLKEISDGD VIISGNKNLCYANTINWK KLFGTSGQKTKIIRNRGEN SCKATGQVCHALCSPEGC WGPEPRDCVSCRNVSRG RECVDKCNLL | IIRNRGENSCK (A03.01) | CRC |
| | | | CLTSTVQLIM (A01.01, A02.01) IMQLMPFGC (A02.01) IMQLMPFGCL (A02.01, A24.02, B08.01) LIMQLMPFG (A02.01) LIMQLMPFGC (A02.01) LTSTVQLIM (A01.01) | |

EP 3 436 048 B1

(continued)

| Gene | Exemplary Protein Change | Mutation Sequence Context | Peptides (HLA allele example(s)) | Exemplary Diseases |
|---|---|---|---|---|
| TABLE 1A | | POINT MUTATION[1] | | |
| EGFR | T790M | IPVAIKELREATSPKANKE ILDEAYVMASVDNPHVC RLLGICLTSTVQLIMQLMI- FGCLLDYVREHKDNIGSQ YLLNWCVQIAKGMNYLE DRRLVHRDLAA | MQLMPFGCL (A02.01, B07.02, B08.01)  MQLMPFGCLL (A02.01, A24.02, B08.01) QLIMQLMPF (A02.01, A24.02, B08.01) QLIMQLMPFG (A02.01) STVQLIMQL (A02.01) VQLIMQLMPF (A02.01, A24.02, B08.01) | NSCLC, PRAD |
| ERBB3 | V104M | ERCEVVMGNLEIVLTGHN ADLSFLQWIREVTGYVLV AMNEFSTLPLPNLRMVRG TQVYDGKFAIFVMLNYN TNSSHALRQLRLTQLTEIL SGGVYIEKNDK | | CRC, Stomach Cancer |
| ESR1 | D538G | HLMAKAGLTLQQQHQRL AQLLLILSHIRHMSNKGM EHLYSMKCKNVVPLYGL LLEMLDAHRLHAPTSRGG ASVEETDQSHLATAGSTS SHSLQKYYITGEA | GLLLEMLDA (A02.01) LYGLLLEML (A24.02) NVVPLYGLL (A02.01) PLYGLLLEM (A02.01) PLYGLLLEML (A02.01, A24.02) VPLYGLLLEM (B07.02) VVPLYGLLL (A02.01, A24.02) | Breast Cancer |

| Gene | Exemplary Protein Change | Mutation Sequence Context | Peptides (HLA allele example(s)) | Exemplary Diseases |
|---|---|---|---|---|
| TABLE 1A | | POINT MUTATION[1] | | |
| ESR1 | S463P | NQGKCVEGMVEIFDMLL ATSSRFRMMNLQGEEFVC LKSIILLNSGVYTFLPSTLK SLEEKDHIHRVLDKITDTL IHLMAKAGLTLQQQHQR LAQLLLILSH | FLPSTLKSL (A02.01, A24.02, B08.01) GVYTFLPST (A02.01) GVYTFLPSTL (A02.01, A24.02) TFLPSTLKSL (A24.02) VYTFLPSTL (A24.02) YTFLPSTLK (A03.01) | Breast Cancer |
| ESR1 | Y537C | IHLMAKAGLTLQQQHQR LAQLLLILSHIRHMSNKG MEHLYSMKCKNVVPLCD LLLEMLDAHRLHAPTSRG GASVEETDQSHLATAGST SSHSLQKYYITGE | NVVPLCDLL (A02.01) NVVPLCDLLL (A02.01) PLCDLLLEM (A02.01) PLCDLLLEML (A02.01) VPLCDLLLEM (B07.02) VVPLCDLLL (A02.01, A24.02) | Breast Cancer |
| ESR1 | Y537N | IHLMAKAGLTLQQQHQR LAQLLLILSHIRHMSNKG MEHLYSMKCKNVVPLND LLLEMLDAHRLHAPTSRG GASVEETDQSHLATAGST SSHSLQKYYITGE | NVVPLNDLL (A02.01) NVVPLNDLLL (A02.01) PLNDLLLEM (A02.01) PLNDLLLEML (A02.01) VPLNDLLLEM (B07.02) | Breast Cancer |
| ESR1 | Y537S | IHLMAKAGLTLQQQHQR LAQLLLILSHIRHMSNKG MEHLYSMKCKNVVPLSD LLLEMLDAHRLHAPTSRG GASVEETDQSHLATAGST SSHSLQKYYITGE | NVVPLSDLL (A02.01) NVVPLSDLLL (A02.01) PLSDLLLEM (A02.01) PLSDLLLEML (A02.01) VPLSDLLLEM (B07.02) VVPLSDLLL (A02.01, A24.02) | Breast Cancer |
| FGFR3 | S249C | HRIGGIKLRHQQWSLVME SVVPSDRGNYTCVVENKF GSIRQTYTLDVLERCPHRI ILQAGLPANQTAVLGSDV EFHCKVYSDAQPHIQWLK HVEVNGSKVG | VLERCPHRPI (A02.01, B08.01) YTLDVLERC (A02.01) | BLCA, HNSC, KIRP, LUSC |

(continued)

| Gene | Exemplary Protein Change | Mutation Sequence Context | Peptides (HLA allele example(s)) | Exemplary Diseases |
|---|---|---|---|---|
| TABLE 1A | | POINT MUTATION[1] | | |
| FRG1B | L52S | AVKLSDSRIALKSGYGKY LGINSDELVGHSDAIGPRE QWEPVFQNGKMALSASN SCFIRCNEAGDIEAKSKTA GEEEMIKIRSCAEKETKK KDDIPEEDKG | FQNGKMALS (A02.01) | GBM, KIRP, PRAD, SKCM |
| HER2 | V777L (Resistance) | GSGAFGTVYKGIWIPDGE NVKIPVAIKVLRENTSPKA NKEILDEAYVMAGLGSPY VSRLLGICLTSTVQLVTQL MPYGCLLDHVRENRGRL GSQDLLNWCM | VMAGLGSPYV (A02.01, A03.01) | BRCA |
| IDH1 | R132H | RVEEFKLKQMWKSPNGTI RNILGGTVFREAIICKNIPR LVSGWVKPIIIGHHAYGD QYRATDFVVPGPGKVEIT YTPSDGTQKVTYLVHNFE EGGGVAMGM | KPIIIGHHA (B07.02) | BLCA, GBM, PRAD |
| IDH1 | R132C | RVEEFKLKQMWKSPNGTI RNILGGTVFREAIICKNIPR LVSGWVKPIIIGCHAYGD QYRATDFVVPGPGKVEIT YTPSDGTQKVTYLVHNFE EGGGVAMGM | KPIIIGCHA (B07.02) | BLCA, GBM, PRAD |
| IDH1 | R132G | RVEEFKLKQMWKSPNGTI RNILGGTVFREAIICKNIPR LVSGWVKPIIIGGHAYGD QYRATDFVVPGPGKVEIT YTPSDGTQKVTYLVHNFE EGGGVAMGM | KPIIIGGHA (B07.02) | BLCA, BRCA, CRC, GBM, HNSC, LUAD, PAAD, PRAD, UCEC |

(continued)

| Gene | Exemplary Protein Change | Mutation Sequence Context | Peptides (HLA allele example(s)) | Exemplary Diseases |
|---|---|---|---|---|
| TABLE 1A | | POINT MUTATION[1] | | |
| IDH1 | R132S | RVEEFKLKQMWKSPNGTI RNILGGTVFREAIICKNIPR LVSGWVKPIIIGSHAYGD QYRATDFVVPGPGKVEIT YTPSDGTQKVTYLVHNFE EGGGVAMGM | KPIIIGSHA (B07.02) | BLCA, BRCA, GBM, HNSC, LIHC, LUAD, LUSC, PAAD, SKCM, UCEC |
| KIT | T670I | VAVKMLKPSAHLTEREA LMSELKVLSYLGNHMNIV NLLGACTIGGPTLVIIEYC CYGDLLNFLRRKRDSFICS KQEDHAEAALYKNLLHS KESSCSDSTNE | IIEYCCYGDL (A02.01) TIGGPTLVII (A02.01) VIIEYCCYG (A02.01) | Gastrointestinal stromal tumors (GIST) |
| KIT | V654A | VEATAYGLIKSDAAMTV AVKMLKPSAHLTEREAL MSELKVLSYLGNHMNIA NLLGACTIGGPTLVITEYC CYGDLLNFLRRKRDSFICS KQEDHAEAALYK | HMNIANLLGA (A02.01) IANLLGACTI (A02.01) MNIANLLGA (A02.01) YLGNHMNIA (A02.01, B08.01) YLGNHMNIAN (A02.01) | Gastrointestinal stromal tumors (GIST) |
| MEK | C121S | ISELGAGNGGVVFKVSHK PSGLVMARKLIHLEIKPAI RNQIIRELQVLHESNSPYI VGFYGAFYSDGEISICME HMDGGSLDQVLKKAGRI PEQILGKVSI | VLHESNSPY (A03.01) VLHESNSPYI (A02.01) | Melanoma |

86

EP 3 436 048 B1

(continued)

**TABLE 1A**

| Gene | Exemplary Protein Change | Mutation Sequence Context[1] | Peptides (HLA allele example(s)) | Exemplary Diseases |
|---|---|---|---|---|
| | | POINT MUTATION[1] | | |
| MEK | P124L | LGAGNGGVVFKVSHKPS GLVMARKLIHLEIKPAIRN QIRELQVLHECNSLYIVG FYGAFYSDGEISICMEHM DGGSLDQVLKKAGRIPEQ ILGKVSIAVI | LQVLHECNSL (A02.01, B08.01) LYIVGFYGAF (A24.02) NSLYIVGFY (A01.01) QVLHECNSL (A02.01, B08.01) SLYIVGFYG (A02.01) SLYIVGFYGA (A02.01) VLHECNSLY (A03.01) VLHECNSLYI (A02.01, A03.01) | Melanoma |
| MYC | E39D | MPLNVSFTNRNYDLDYD SVQPYFYCDEEENFYQQQ QQSDLQPPAPSEDIWKKF ELLPTPPLSPSRRSGLCSPS YVAVTPFSLRGDNDGG | FYQQQQQSDL (A24.02) QQQSDLQPPA (A02.01) QQSDLQPPA (A02.01) YQQQQQSDL (A02.01, B08.01) | Lymphoid Cancer; Burkitt Lymphoma |
| MYC | P57S | FTNRNYDLDYDSVQPYFY CDEEENFYQQQQQSELQP PAPSEDIWKKFELLSTPPL SPSRRSGLCSPSYVAVTPF SLRGDNDGGGGSFSTADQ LEMVTELLG | FELLSTPPL (A02.01, B08.01) LLSTPPLSPS (A02.01) | Lymphoid Cancer |
| MYC | T58I | TNRNYDLDYDSVQPYFY CDEEENFYQQQQQSELQP PAPSEDIWKKFELLPIPPLS PSRRSGLCSPSYVAVTPFS LRGDNDGGGGSFSTADQ LEMVTELLGG | FELLPIPPL (A02.01) IWKKFELLPI (A24.02) LLPIPPLSPS (A02.01, B07.02) LPIPPLSPS (B07.02) | Neuroblastoma |
| PDGFRa | T674I | VAVKMLKPTARSSEKQA LMSELKIMTHLGPHLNIV NLLGACTKSGPIYIIIEYCF YGDLVNYLHKNRDSFLS HHPEKPKKELDIFGLNPA DESTRSYVILS | IIEYCFYGDL (A02.01) IIIEYCFYG (A02.01) YIIIEYCF (A24.02) IYIIIEYCFY (A24.02) YIIIEYCFYG (A02.01) | Chronic Eosinophilic Leukemia |

| Gene | Exemplary Protein Change | Mutation Sequence Context | Peptides (HLA allele example(s)) | Exemplary Diseases |
|---|---|---|---|---|
| TABLE 1A | | POINT MUTATION[1] | | |
| PIK3CA | E542K | IEEHANWSVSREAGFSYS HAGLSNRLARDNELREN DKEQLKAISTRDPLSKITE QEKDFLWSHRHYCVTIPE ILPKLLLSVKWNSRDEVA QMYCLVKDWPP | KITEQEKDFL (A02.01) | BLCA, BRCA, CESC, CRC, GBM, HNSC, KIRC, KIRP, LIHC, LUAD, LUSC, PRAD, UCEC |
| PIK3CA | E545K | HANWSVSREAGFSYSHA GLSNRLARDNELRENDKE QLKAISTRDPLSEITKQEK DFLWSHRHYCVTIPEILPK LLLSVKWNSRDEVAQMY CLVKDWPPIKP | STRDPLSEITK (A03.01)<br><br>DPLSEITK (A03.01) | BLCA, BRCA, CESC, CRC, GBM, HNSC, KIRC, KIRP, LIHC, LUAD, LUSC, PRAD, SKCM, UCEC |
| PIK3CA | H1047R | LFINLFSMMLGSGMPELQ SFDDIAYIRKTLALDKTEQ EALEYFMKQMNDARHGG WTTKMDWIFHTIKQHAL N | | BRCA, CESC, CRC, GBM, HNSC, LIHC, LUAD, LUSC, PRAD, UCEC |

EP 3 436 048 B1

88

| Gene | Exemplary Protein Change | Mutation Sequence Context | Peptides (HLA allele example(s)) | Exemplary Diseases |
|---|---|---|---|---|
| **TABLE 1A** | | **POINT MUTATION[1]** | | |
| POLE | P286R | QRGGVITDEEETSKKIAD QLDNIVDMREYDVPYHIR LSIDIETTKLPLKFRDAET DQIMMISYMIDGQGYLIT NREIVSEDIEDFEFTPKPE YEGPFCVFN | LPLKFRDAET (B07.02) | Colorectal adenocarcinoma; Uterine/Endometrium Adenocarcinoma; Colorectal adenocarcinoma, MSI+; Uterine/Endometrium Adenocarcinoma, MSI+; Endometrioid carcinoma; Endometrium Serous carcinoma; Endometrium Carcinosarcoma-malignant mesodermal mixed tumour; Glioma; Astrocytoma; GBM |
| PTEN | R130Q | KFNCRVAQYPFEDHNPPQ LELIKPFCEDLDQWLSED DNHVAAIHCKAGKGQTG VMICAYLLHRGKFLKAQE ALDFYGEVRTRDKKGVTI PSQRRYVYYYSY | QTGVMICAYL (A02.01) | BRCA, CESC, CRC, GBM, KIRC, LUSC, UCEC |
| RAC1 | P29S | MQAIKCVVVGDGAVGKT CLLISYTTNAFSGEYIPTV FDNYSANVMVDGKPVNL GLWDTAGQEDYDRLRPL SYPQTVGET | AFSGEYIPTV (A02.01, A24.02) | Melanoma |

| Gene | Exemplary Protein Change | Mutation Sequence Context | Peptides (HLA allele example(s)) | Exemplary Diseases |
|---|---|---|---|---|
| **TABLE 1A** | | **POINT MUTATION**[1] | | |
| TP53 | G245S | IRVEGNLRVEYLDDRNTF RHSVVVPYEPPEVGSDCT TIHYNYMCNSSCMGSMN RRPILTIITLEDSSGNLLGR NSFEVRVCACPGRDRRTE EENLRKKGEP | SMNRRPILT (A02.01, B08.01) YMCNSSCMGS (A02.01) | BLCA, BRCA, CRC, GBM, HNSC, LUSC, PAAD, PRAD |
| TP53 | R175H | TYSPALNKMFCQLAKTCP VQLWVDSTPPPGTRVRA MAIYKQSQHMTEVVRHC PHHERCSDSDGLAPPQHLI RVEGNLRVEYLDDRNTFR HSVVVPYEPPEV | | BLCA, BRCA, CRC, GBM, HNSC, LUAD, PAAD, PRAD, UCEC |
| TP53 | R248Q | EGNLRVEYLDDRNTFRHS VVVPYEPPEVGSDCTTIH YNYMCNSSCMGGMNQR PILTIITLEDSSGNLLGRNS FEVRVCACPGRDRRTEEE NLRKKGEPHHE | GMNQRPILT (A02.01) | BLCA, BRCA, CRC, GBM, HNSC, KIRC, LIHC, LUSC, PAAD, PRAD, UCEC |
| TP53 | R248W | EGNLRVEYLDDRNTFRHS VVVPYEPPEVGSDCTTIH YNYMCNSSCMGGMNWR PILTIITLEDSSGNLLGRNS FEVRVCACPGRDRRTEEE NLRKKGEPHHE | GMNWRPILT (A02.01) | BLCA, BRCA, CRC, GBM, HNSC, LIHC, LUSC, PAAD, SKCM, UCEC |
| TP53 | R273C | PEVGSDCTTIHYNYMCNS SCMGGMNRRPILTIITLED SSGNLLGRNSFEVCVCAC PGRDRRTEEENLRKKGEP HHELPPGSTKRALPNNTS SSPQPKKKPL | LLGRNSFEVC (A02.01) | BLCA, BRCA, CRC, GBM, HNSC, LUSC, PAAD, UCEC |

(continued)

| TABLE 1B Gene | Exemplary Protein Change | MSI-ASSOCIATED FRAMESHIFTS[1] Mutation Sequence Context | Peptides (HLA allele example(s)) | Exemplary Diseases |
|---|---|---|---|---|
| ACVR2A | D96fs; +1 | GVEPCYGDKDKRRHCFA TWKNISGSIEIVKQGCWL DDINCYDRTDCVEKKRQP * | | MSI+ CRC, MSI+ Uterine/Endometrium Cancer, MSI+ Sto-mach Cancer, Lynch syndrome |
| ACVR2A | D96fs; -1 | GVEPCYGDKDKRRHCFA TWKNISGSIEIVKQGCWL DDINCYDRTDCVEKKTAL KYIFVAVRAICVMKSFLIF RRWKSHSPLQIQLHLSHPI TTSCSIPWCHLC* | ALKYIFVAV (A02.01, B08.01) ALKYIFVAVR (A03.01) AVRAICVMK (A03.01) AVRAICVMKS (A03.01) CVEKKTALK (A03.01) CVEKKTALKY (A01.01) CVMKSFLIF (A24.02, B08.01) CVMKSFLIFR (A03.01) FLIFRRWKS (A02.01, B08.01) FRRWKSHSPL (B08.01) FVAVRAICV (A02.01, B08.01) FVAVRAICVM (B08.01) IQLHLSHPI (A02.01) KSFLIFRRWK (A03.01) KTALKYIFV (A02.01) KYIFVAVRAI (A24.02) RWKSHSPLQI (A24.02) TALKYIFVAV (A02.01, B08.01) VAVRAICVMK (A03.01) VMKSFLIFR (A03.01) VMKSFLIFRR (A03.01) YIFVAVRAI (A02.01) | MSI+ CRC, MSI+ Uterine/Endometrium Cancer, MSI+ Stomach Cancer, Lynch syndrome |

| TABLE 1B Gene | Exemplary Protein Change | MSI-ASSOCIATED FRAMESHIFTS[1] Mutation Sequence Context | Peptides (HLA allele example(s)) | Exemplary Diseases |
|---|---|---|---|---|
| C15ORF40 | L132fs; +1 | TAEAVNVAIAAPPSEGEA NAELCRYLSKVLELRKSD VVLDKVGLALFFFFFETK SCSVAQAGVQWRSLGSL QPPPPGFKLFSCLSFLSSW DYRRMPPCLANFCIFNRD GVSPCWSGWS* | ALFFFFFET (A02.01)<br>ALFFFFFETK (A03.01)<br>AQAGVQWRSL (A02.01)<br>CLANFCIFNR (A03.01)<br>CLSFLSSWDY (A01.01, A03.01)<br>FFETKSCSV (B08.01)<br>FFFETKSCSV (A02.01)<br>FKLFSCLSFL (A02.01)<br>FLSSWDYRRM (A02.01)<br>GFKLFSCLSF (A24.02)<br>KLFSCLSFL (A02.01, A03.01)<br>KLFSCLSFLS (A02.01, A03.01)<br>LALFFFFFET (A02.01)<br>LFFFFFETK (A03.01)<br>LSFLSSWDY (A01.01)<br>LSFLSSWDYR (A03.01)<br>RMPPCLANF (A24.02)<br>RRMPPCLANF (A24.02)<br>SLQPPPPGFK (A03.01)<br>VQWRSLGSL (A02.01) | MSI+ CRC, MSI+ Uterine/Endometrium Cancer, MSI+ Stomach Cancer, Lynch syndrome |
| CNOT1 | L1544fs; +1 | LSVIIFFFVYIWHWALPLIL NNHHICLMSSIILDCNSVR QSIMSVCFFFFSVIFSTRCL TDSRYPNICWFK* | FFFSVIFST (A02.01)<br>MSVCFFFFSV (A02.01)<br>SVCFFFFSV (A02.01, B08.01)<br>SVCFFFFSVI (A02.01) | MSI+ CRC, MSI+ Uterine/Endometrium Cancer, MSI+ Stomach Cancer, Lynch syndrome |
| CNOT1 | L1544fs; -1 | LSVIIFFFVYIWHWALPLIL NNHHICLMSSIILDCNSVR QSIMSVCFFFFCYILNTMF DR* | FFCYILNTMF (A24.02)<br>MSVCFFFFCY (A01.01)<br>SVCFFFFCYI (A02.01) | MSI+ CRC, MSI+ Uterine/Endometrium Cancer, MSI+ Stomach Cancer, Lynch syndrome |

| Gene | Exemplary Protein Change | Mutation Sequence Context | Peptides (HLA allele example(s)) | Exemplary Diseases |
|---|---|---|---|---|
| EIF2B3 | A151fs; -1 | VLVLSCDLITDVALHEVV DLFRAYDASLAMLMRKG QDSIEPVPGQKGKKKQWS SVTSLEWTAQERGCSSWL MKQTWMKSWSLRDPSYR SILEYVSTRVLWMPTSTV * | KQWSSVTSL (A02.01)<br><br>VLWMPTSTV (A02.01) | MSI+ CRC, MSI+ Uterine/Endometrium Cancer, MSI+ Stomach Cancer, Lynch syndrome |
| EPHB2 | K1020fs; -1 | SIQVMRAQMNQIQSVEGQ PLARRPRATGRTKRCQPR DVTKKTCNSNDGKKREW EKRKQILGGGGKYKEYFL KRILIRKAMTVLAGDKKG LGRFMRCVQSETKAVSLQ LPLGR* | ILIRKAMTV (A02.01) | MSI+ CRC, MSI+ Uterine/Endometrium Cancer, MSI+ Stomach Cancer, Lynch syndrome |
| ESRP1 | N512fs; +1 | LDFLGEFATDIRTHGVHM VLNHQGRPSGDAFIQMKS ADRAFMAAQKCHKKKHE GQIC* | | MSI+ CRC, MSI+ Uterine/Endometrium Cancer, MSI+ Stomach Cancer, Lynch syndrome |
| ESRP1 | N512fs; -1 | LDFLGEFATDIRTHGVHM VLNHQGRPSGDAFIQMKS ADRAFMAAQKCHKKT* | | MSI+ CRC, MSI+ Uterine/Endometrium Cancer, MSI+ Stomach Cancer, Lynch syndrome |
| FAM111B | A273fs; -1 | GALCKDGRFRSDIGEFEW KLKEGHKKIYGKQSMVD EVSGKVLEMDISKKKHY NRKISIKKLNRMKVPLMK LITRV* | RMKVPLMK (A03.01) | MSI+ CRC, MSI+ Uterine/Endometrium Cancer, MSI+ Stomach Cancer, Lynch syndrome |

| Gene | Exemplary Protein Change | Mutation Sequence Context | Peptides (HLA allele example(s)) | Exemplary Diseases |
|---|---|---|---|---|
| GBP3 | T585fs; -1 | RERAQLLEEQEKTLTSKL QEQARVLKERCQGESTQL QNEIQKLQKTLKKKPRDI CRIS* | TLKKKPRDI (B08.01) | MSI+ CRC, MSI+ Uterine/Endometrium Cancer, MSI+ Stomach Cancer, Lynch syndrome |
| JAK1 | P861fs; +1 | VNTLKEGKRLPCPPNCPD EVYQLMRKCWEFQPSNR TSFQNLIEGFEALLKTSN* | LIEGFEALLK (A03.01) | MSI+ CRC, MSI+ Uterine/Endometrium Cancer, MSI+ Stomach Cancer, Lynch syndrome |
| JAK1 | K860fs; -1 | CRPVTPSCKELADLMTRC MNYDPNQRPFFRAIMRDI NKLEEQNPDIVSEKNQQL KWTPHILKSAS* | QQLKWTPHI (A02.01) QLKWTPHILK (A03.01) IVSEKNQQLK (A03.01) QLKWTPHILK (A03.01) QQLKWTPHI (A24.02) NQQLKWTPHIL (B08.01) NQQLKWTPHI (B08.01) QLKWTPHIL (B08.01) | MSI+ CRC, MSI+ Uterine/Endometrium Cancer, MSI+ Stomach Cancer, Lynch syndrome |
| LMAN1 | E305fs; +1 | DDHDVLSFLTFQLTEPGK EPPTPDKEISEKEKEKYQE EFEHFQQELDKKKRGIPE GPPRPPRAACGGNI* | GPPRPPRAAC (B07.02)<br><br>PPRPPRAAC (B07.02) | MSI+ CRC, MSI+ Uterine/Endometrium Cancer, MSI+ Stomach Cancer, Lynch syndrome |
| LMAN1 | E305fs; -1 | DDHDVLSFLTFQLTEPGK EPPTPDKEISEKEKEKYQE EFEHFQQELDKKKRNSRR ATPTSKGSLRRKYLRV* | SLRRKYLRV (B08.01) | MSI+ CRC, MSI+ Uterine/Endometrium Cancer, MSI+ Stomach Cancer, Lynch syndrome |
| MSH3 | N385fs; +1 | TKSTLIGEDVNPLIKLDDA VNVDEIMTDTSTSYLLCIS ENKENVRDKKKGQHFYW HCGSAACHRRGCV* | SAACHRRGCV (B08.01) | MSI+ CRC, MSI+ Uterine/Endometrium Cancer, MSI+ Stomach Cancer, Lynch syndrome |

94

EP 3 436 048 B1

| Gene | Exemplary Protein Change | Mutation Sequence Context | Peptides (HLA allele example(s)) | Exemplary Diseases |
|---|---|---|---|---|
| MSH3 | K383fs; -1 | LYTKSTLIGEDVNPLIKLD DAVNVDEIMTDTSTSYLL CISENKENVRDKKRATFL LALWECSLPQARLCLIVS RTLLLVQS* | ALWECSLPQA (A02.01)<br>CLIVSRTLL (B08.01)<br>CLIVSRTLLL (A02.01, B08.01)<br>FLLALWECS (A02.01)<br>FLLALWECSL (A02.01, B08.01)<br>IVSRTLLLV (A02.01)<br>LIVSRTLLL (A02.01, B08.01)<br>LIVSRTLLLV (A02.01)<br>LLALWECSL (A02.01, B08.01)<br>LPQARLCLI (B08.01, B07.02)<br>LPQARLCLIV (B08.01)<br>NVRDKKRATF (B08.01)<br>SLPQARLCLI (A02.01, B08.01) | MSI+CRC, MSI+ Uterine/Endometrium Cancer, MSI+ Stomach Cancer, Lynch syndrome |
| NDUFC2 | A70fs; +1 | LPPPKLTDPRLLYIGFLGY CSGLIDNLIRRRPIATAGL HRQLLYITAFFFCWILSCK T* | FFCWILSCK (A03.01)<br>FFFCWILSCK (A03.01)<br>ITAFFFCWI (A02.01)<br>LYITAFFFCW (A24.02)<br>YITAFFFCWI (A02.01) | MSI+ CRC, MSI+ Uterine/Endometrium Cancer, MSI+ Stomach Cancer, Lynch syndrome |
| NDUFC2 | F69fs; -1 | SLPPPKLTDPRLLYIGFLG YCSGLIDNLIRRRPIATAG LHRQLLYITAFFLLDIIL* | ITAFFLLDI (A02.01)<br>LLYITAFFL (A02.01, B08.01)<br>LLYITAFFLL (A02.01, A24.02)<br>LYITAFFLL (A24.02)<br>LYITAFFLLD (A24.02)<br>YITAFFLLDI (A02.01) | MSI+ CRC, MSI+ Uterine/Endometrium Cancer, MSI+ Stomach Cancer, Lynch syndrome |
| RBM27 | Q817; +1 | NQSGGAGEDCQIFSTPGH PKMIYSSSNLKTPSKLCSG SKSHDVQEVLKKKTGSNE VTTRYEEKKTGSVRKAN RMPKDVNIQVRKKQKHE TRRKSKYNEDFERAWRE DLTIKR* | GSNEVTTRY (A01.01)<br>MPKDVNIQV (B07.02)<br><br><br>TGSNEVTTRY (A01.01) | MSI+ CRC, MSI+ Uterine/Endometrium Cancer, MSI+ Stomach Cancer, Lynch syndrome |

EP 3 436 048 B1

95

(continued)

| Gene | Exemplary Protein Change | Mutation Sequence Context | Peptides (HLA allele example(s)) | Exemplary Diseases |
|---|---|---|---|---|
| RPL22 | K16fs; +1 | MAPVKKLVVKGGKKKEA SSEVHS* | | MSI+ CRC, MSI+ Uterine/Endometrium Cancer, MSI+ Stomach Cancer, Lynch syndrome |
| RPL22 | K15fs; -1 | MAPVKKLVVKGGKKKRSK F* | | MSI+ CRC, MSI+ Uterine/Endometrium Cancer, MSI+ Stomach Cancer, Lynch syndrome |
| SEC31A | I462fs; +1 | MPSHQGAEQQQQHHVF ISQVVTEKEFLSRSDQLQQ AVQSQGFINYCQKKN* | | MSI+ CRC, MSI+ Uterine/Endometrium Cancer, MSI+ Stomach Cancer, Lynch syndrome |
| SEC31A | I462fs; -1 | MPSHQGAEQQQQHHVF ISQVVTEKEFLSRSDQLQQ AVQSQGFINYCQKKLMLL RLNLRKMCGPF* | KKLMLLRLNL (A02.01) KLMLLRLNL (A02.01, A03.01, B07.02, B08.01) KLMLLRLNLR (A03.01) LLRLNLRKM (B08.01) LMLLRLNL (B08.01) LMLLRLNLRK (A03.01) LNLRKMCGPF (B08.01) MLLRLNLRK (A03.01) MLLRLNLRKM (A02.01, A03.01, B08.01) NLRKMCGPF (B08.01) NYCQKKLMLL (A24.02) YCQKKLMLL (B08.01) | MSI+ CRC, MSI+ Uterine/Endometrium Cancer, MSI+ Stomach Cancer, Lynch syndrome |

| Gene | Exemplary Protein Change | Mutation Sequence Context | Peptides (HLA allele example(s)) | Exemplary Diseases |
|---|---|---|---|---|
| SEC63 | K530fs; +1 | AEVFEKEQSICAAEEQPA EDGQGETNKNRTKGGWQ QKSKGPKKTAKSKKK<u>ETF KKKTYTCAITTVKATETK AGKWSRWE*</u> | FKKKTYTCAI (B08.01)<br>ITTVKATETK (A03.01)<br>KSKKKETFK (A03.01)<br>KSKKKETFKK (A03.01)<br>KTYTCAITTV (A02.01, A24.02)<br>TFKKKTYTC (B08.01)<br>TYTCAITTV (A24.02)<br>TYTCAITTVK (A03.01)<br>YTCAITTVK (A03.01) | MSI+ CRC, MSI+ Uterine/Endometrium Cancer, MSI+ Stomach Cancer, Lynch syndrome |
| SEC63 | K529fs; -1 | MAEVFEKEQSICAAEEQP AEDGQGETNKNRTKGGW QQKSKGPKKTAKSKK<u>RN L*</u> | TAKSKKRNL (B08.01) | MSI+ CRC, MSI+ Uterine/Endometrium Cancer, MSI+ Stomach Cancer, Lynch syndrome |
| SLC35F5 | C248fs; -1 | NIMEIRQLPSSHALEAKLS RMSYPVKEQESILKTVGK LTATQVAKISFFF<u>ALCGF WQICHIKKHFQTHKLL*</u> | FALCGFWQI (A02.01) | MSI+ CRC, MSI+ Uterine/Endometrium Cancer, MSI+ Stomach Cancer, Lynch syndrome |
| SMAP1 | K172fs; +1 | YEKKKYYDKNAIAITNISS SDAPLQPLVSSPSLQAAV DKNKLEKEKEKKK<u>GREK ERKGARKAGKTTYS*</u> | | MSI+ CRC, MSI+ Uterine/Endometrium Cancer, MSI+ Stomach Cancer, Lynch syndrome |
| SMAP1 | K171fs; -1 | KYEKKKYYDKNAIAITNI SSSDAPLQPLVSSPSLQAA VDKNKLEKEKEKK<u>RKRK REKRSQKSRQNHLQLKSC RRKISNWSLKKVPALKKL RSPLWIF*</u> | LKKLRSPL (B08.01)<br>SLKKVPAL (B08.01)<br>RKISNWSLKK (A03.01)<br><br>VPAT KKT RSPL (B07.02) | MSI+ CRC, MSI+ Uterine/Endometrium Cancer, MSI+ Stomach Cancer, Lynch syndrome |

| Gene | Exemplary Protein Change | Mutation Sequence Context | Peptides (HLA allele example(s)) | Exemplary Diseases |
|---|---|---|---|---|
| TFAM | E148fs; +1 | IYQDAYRAEWQVYKEEIS RFKEQLTPSQIMSLEKEIM DKHLKRKAMTKKKRVNT AWKTKKTSFSL* | KRVNTAWKTK (A03.01) MTKKKRVNTA (B08.01) RVNTAWKTK (A03.01) RVNTAWKTKK (A03.01) TKKKRVNTA (B08.01) WKTKKTSFSL (B08.01) | MSI+ CRC, MSI+ Uterine/Endometrium Cancer, MSI+ Stomach Cancer, Lynch syndrome |
| TFAM | E148fs; -1 | IYQDAYRAEWQVYKEEIS RFKEQLTPSQIMSLEKEIM DKHLKRKAMTKKKS* | | MSI+ CRC, MSI+ Uterine/Endometrium Cancer, MSI+ Stomach Cancer, Lynch syndrome |
| TGFBR2 | P129fs; +1 | KPQEVCVAVWRKNDENI TLETVCHDPKLPYHDFILE DAASPKCIMKEKKKAW* | | MSI+ CRC, MSI+ Uterine/Endometrium Cancer, MSI+ Stomach Cancer, Lynch syndrome |
| TGFBR2 | K128fs: -1 | EKPQEVCVAVWRKNDEN ITLETVCHDPKLPYHDFIL EDAASPKCIMKEKKSLVR LSSCVPVALMSAMTTSSS QKNITPAILTCC* | ALMSAMTTS (A02.01) AMTTSSSQK (A03.01, A11.01) AMTTSSSQKN (A03.01) CIMKEKKSL (B08.01) CIMKEKKSLV (B08.01) IMKEKKSL (B08.01) IMKEKKSLV (B08.01) KSLVRLSSCV (A02.01) LVRLSSCVPV (A02.01) RLSSCVPVA (A02.01, A03.01) RLSSCVPVAL (A02.01) SAMTTSSSQK (A03.01, A11.01) SLVRLSSCV (A02.01) VPVALMSAM (B07.02) VRLSSCVPVA (A02.01) | MSI+ CRC, MSI+ Uterine/Endometrium Cancer, MSI+ Stomach Cancer, Lynch syndrome |

EP 3 436 048 B1

| Gene | Exemplary Protein Change | Mutation Sequence Context | Peptides (HLA allele example(s)) | Exemplary Diseases |
|---|---|---|---|---|
| THAP5 | K99fs; -1 | VPSKYQFLCSDHFTPDSL DIRWGIRYLKQTAVPTIFS LPEDNQGKDPSKKNPRRK TWKMRKKYAQKPSQKN HLY* | KMRKKYAQK (A03.01) | MSI+ CRC, MSI+ Uterine/Endometrium Cancer, MSI+ Stomach Cancer, Lynch syndrome |
| TTK | R854fs; -1 | GTTEEMKYVLGQLVGLN SPNSILKAAKTLYEHYSG GESHNSSSSKTFEKKGEK NDLQLFVMSDTTYKIYW TVILLNPCGNLHLKTTSL* | FVMSDTTYK (A03.01) FVMSDTTYKI (A02.01) KTFEKKGEK (A03.01) LFVMSDTTYK (A03.01)<br>MSDTTYKIY (A01.01)<br>VMSDTTYKI (A02.01)<br>VMSDTTYKIY (A01.01) | MSI+ CRC, MSI+ Uterine/Endometrium Cancer, MSI+ Stomach Cancer, Lynch syndrome |
| XPOT | F126fs; -1 | QQLIRETLISWLQAQMLN PQPEKTFIRNKAAQVFAL LFVTEYLTKWPKFFLTFS Q* | YLTKWPKFFL (A02.01) | MSI+ CRC, MSI+ Uterine/Endometrium Cancer, MSI+ Stomach Cancer, Lynch syndrome |
| TABLE 1C | | FRAMESHIFT[1] | | |
| APC | V1352fs F1354fs Q1378fs S1398fs | AKFQQCHSTLEPNPADCR VLVYLQNQPGTKLLNFLQ ERNLPPKVVLRHPKVHLN TMFRRPHSCLADVLLSVH LIVLRVVRLPAPFRVNHA VEW* | FLQERNLPP (A02.01) FRRPHSCLA (B08.01) LIVLRVVRL (B08.01)<br>LLSVHLIVL (A02.01, B08.01) | CRC, LUAD, UCEC, STAD |

99

EP 3 436 048 B1

(continued)

TABLE 1C

FRAMESHIFT[1]

| Gene | Exemplary Protein Change | Mutation Sequence Context | Peptides (HLA allele example(s)) | Exemplary Diseases |
|---|---|---|---|---|
| APC | S1421fs<br>R1435fs<br>T1438fs<br>P1442fs<br>P1443fs<br>V1452fs<br>P1453fs<br>K1462fs<br>E1464fs | APVIFQIALDKPCHQAEV<br>KHLHHLLKQLKPSEKYLK<br>IKHLLLKRERVDLSKLQ* | EVKHLHHLL (B08.01)<br>HLHHLLKQLK (A03.01)<br>HLLLKRERV (B08.01)<br>KIKHLLLKR (A03.01)<br>KPSEKYLKI (B07.02)<br>KYLKIKHLL (A24.02)<br>KYLKTKHLLL (A24.02)<br>LLKQLKPSEK (A03.01)<br>LLKRERVDL (B08.01)<br>LLLKRERVDL (B08.01)<br>QLKPSEKYLK (A03.01)<br>YLKIKHLLL (A02.01, B08.01)<br>YLKTKHLLLK (A03.01) | CRC, LUAD, UCEC, STAD |
| APC | T1487fs<br>H1490fs<br>L1488fs | MLQFRGSRFFQMLILYYIL<br>PRKVLQMDFLVHPA* | ILPRKVLQM (B08.01)<br>KVLQMDFLV (A02.01, A24.02)<br>LPRKVLQMDF (B07.02, B08.01)<br>LQMDFLVHPA (A02.01)<br>QMDFLVHPA (A02.01)<br>YILPRKVLQM (A02.01, B08.01) | CRC, LUAD, UCEC, STAD |
| ARID1A | Q1306fs<br>S1316fs<br>Y1324fs<br>T1348fs<br>G1351fs<br>G1378fs<br>P1467fs | ALGPHSRISCLPTQTRGCI<br>LLAATPRSSSSSSNDMIP<br>MAISSPPKAPLLAAPSPAS<br>RLQCINSNSRITSGQWMA<br>HMALLPSGTKGRCTACH<br>TALGRGSLSSSSCPQPSPS<br>LPASNKLPSLPLSKMYTTS<br>MAMPILPLPQLLLSADQQ<br>AAPRTNFHSSLAETVSLH<br>PLAPMPSKTCHHK* | APSPASRLQC (B07.02)<br>HPLAPMPSKT (B07.02)<br>ILPLPQLLL (A02.01)<br>LLLSADQQA (A02.01)<br>LPTQTRGCI (B07.02)<br>LPTQTRGCIL (B07.02)<br>RISCLPTQTR (A03.01)<br>SLAETVSLH (A03.01)<br>TPRSSSSSS (B07.02)<br>TPRSSSSSSS (B07.02) | STAD, UCEC, BLCA, BRCA, LUSC, CESC, KIRC, UCS |
| Gene | Exemplary Protein Change | Mutation Sequence Context | Peptides (HLA allele example(s)) | Exemplary Diseases |
| | | | ALPPVLLSL (A02.01) | |

| Gene | Exemplary Protein Change | Mutation Sequence Context | Peptides (HLA allele example(s)) | Exemplary Diseases |
|---|---|---|---|---|
| ARID1A | S674fs<br><br>P725fs<br><br><br>R727fs<br>I736fs | AHQGFPAAKESRVIQLSL LSLLIPPLTCLASEALPRPL LALPPVLLSLAQDHSRLL QCQATRCHLGHPVASRT ASCILP* | ALPPVLLSLA (A02.01)<br>ALPRPLLAL (A02.01)<br>ASRTASCIL (B07.02)<br>EALPRPLLAL (B08.01)<br>HLGHPVASR (A03.01)<br>HPVASRTAS (B07.02)<br>HPVASRTASC (B07.02)<br>IIQLSLLSLL (A02.01)<br>IQLSLLSLL (A02.01)<br>IQLSLLSLLI (A02.01, A24.02)<br>LLALPPVLL (A02.01)<br><br>LLIPPLTCL (A02.01)<br><br>LLIPPLTCLA (A02.01)<br>LLSLLIPPL (A02.01)<br>LLSLLIPPLT (A02.01)<br>LPRPLLALPP (B07.02)<br>QLSLLSLLI (A02.01)<br>RLLQCQATR (A03.01)<br>RPLLALPPV (B07.02)<br>RPLLALPPVL (B07.02)<br>SLAQDHSRL (A02.01)<br>SLAQDHSRLL (A02.01)<br>SLLIPPLTCL (A02.01)<br>SLLSLLIPP (A02.01)<br>SLLSLLIPPL (A02.01, B08.01) | STAD, UCEC, BLCA, BRCA, LUSC, CESC, KIRC, UCS |
| | | | AAATSAASTL (B07.02)<br>AAIPASTSAV (B07.02)<br>AIPASTSAV (A02.01) | |

| Gene | Exemplary Protein Change | Mutation Sequence Context | Peptides (HLA allele example(s)) | Exemplary Diseases |
|---|---|---|---|---|
| ARID1A | G414fs<br>Q473fs<br>H477fs<br>S499fs<br>P504fs<br>Q548fs<br>P549fs | PILAATGTSVRTAARTWV PRAAIRVPDPAAVPDDHA GPGAECHGRPLLYTADSS<br><br>LWTTRPQRVWSTGPDSIL<br><br>QPAKSSPSAAAATLLPAT<br><br>TVPDPSCPTFVSAAATVST<br><br>TTAPVLSASILPAAIPASTS<br><br>AVPGSIPLPAVDDTAAPPE<br><br>PAPLLTATGSVSLPAAATS | ALPAGCVSSA (A02.01)<br>APLLTATGSV (B07.02)<br>APVLSASIL (B07.02)<br>ATLLPATTV (A02.01)<br>ATVSTTTAPV (A02.01)<br>AVPANCLFPA (A02.01)<br>CLFPAALPST (A02.01)<br>CPTFVSAAA (B07.02)<br>FPAALPSTA (B07.02)<br>FPAALPSTAG (B07.02)<br>GAECHGRPL (B07.02)<br>GAISRFIWV (A02.01)<br>ILPAAIPAST (A02.01)<br>IWVSGILSPL (A24.02)<br>LLTATGSVSL (A02.01)<br>LLYTADSSL (A02.01)<br>LPAAATSAA (B07.02)<br>LPAAATSAAS (B07.02)<br>LPAAIPAST (B07.02)<br>LPAGCVSSA (B07.02)<br>LPAGCVSSAP (B07.02)<br><br>LYTADSSLW (A24.02)<br><br>QPAKSSPSA (B07.02)<br><br>QPAKSSPSAA (B07.02)<br>RFIWVSGIL (A24.02)<br>RPQRVWSTG (B07.02)<br>RVWSTGPDSI (A02.01)<br>SAVPGSIPL (B07.02)<br>SILPAAIPA (A02.01) | STAD, UCEC, BLCA, BRCA, LUSC, CESC, KIRC, UCS |

(continued)

| Gene | Exemplary Protein Change | Mutation Sequence Context | Peptides (HLA allele example(s)) | Exemplary Diseases |
|---|---|---|---|---|
|  |  |  | SLPAAATSA (A02.01)<br>SLPAAATSAA (A02.01)<br>SLWTTRPQR (A03.01)<br>SLWTTRPQRV (A02.01)<br>SPSAAAATL (B07.02)<br>SPSAAAATLL (B07.02)<br>TLDALPAGCV (A02.01)<br>TVSTTTAPV (A02.01)<br>VLSASILPA (A02.01)<br>VLSASILPAA (A02.01)<br>VPANCLFPA (B07.02)<br>VPANCLFPAA (B07.02)<br>VPDPSCPTF (B07.02)<br>VPGSIPLPA (B07.02)<br>VPGSIPLPAV (B07.02)<br>WVSGILSPL (A02.01)<br>YTADSSLWTT (A02.01) |  |
|  |  |  | APAGMVNRA (B07.02)<br>ASLHRRSYL (B08.01)<br>ASLHRRSYLK (A03.01)<br>FLLMDNKAPA (A02.01)<br>HPRRSPSRL (B07.02, B08.01)<br>HPSLHISSP (B07.02)<br>HRRSYLKIHL (B08.01)<br>HSRFLLMDNK (A03.01)<br>KLPIPSSASL (A02.01)<br>KVLTLSSSSH (A03.01)<br>LIHSRFLLM (B08.01)<br>LLMDNKAPA (A02.01)<br>LMDNKAPAGM (A02.01)<br>LPIPSSASL (B07.02)<br>MPNLRISSS (B07.02, B08.01) |  |

| Gene | Exemplary Protein Change | Mutation Sequence Context | Peptides (HLA allele example(s)) | Exemplary Diseases |
|---|---|---|---|---|
| ARID1A | T433fs<br>A441fs<br>Y447fs<br>P483fs<br>P484fs<br><br>P504fs<br><br>S519fs<br>H544fs<br>P549fs<br>P554fs<br>Q563fs | PCRAGRRVPWAASLIHSR<br>FLLMDNKAPAGMVNRAR<br>LHITTSKVLTLSSSSHPTPS<br>NHRPRPLMPNLRISSSHSL<br>NHHSSSPLSLHTPSSHPSL<br>HISSPRLHTPPSSRRHSSTP<br>RASPPTHSHRLSLLTSSSN<br>LSSOHPRRSPSRLRILSPSL<br><br>——————————————<br>SSPSKLPIPSSASLHRRSYL<br>KIHLGLRHPQPPQ* | MPNLRISSSH (B07.02)<br>NLRISSSHSL (B07.02, B08.01)<br>PPTHSHRLSL (B07.02)<br>RAGRRVPWAA (B08.01)<br>RARLHITTSK (A03.01)<br>RISSSHSLNH (A03.01)<br>RLHTPPSSR (A03.01)<br>RLHTPPSSRR (A03.01)<br><br>RLRILSPSL (A02.01, B07.02, B08.01)<br><br>RPLMPNLRI (B07.02)<br>RPRPLMPNL (B07.02)<br><br>SASLHRRSYL (B07.02, B08.01)<br><br>SLHISSPRL (A02.01)<br>SLHRRSYLK (A03.01)<br>SLHRRSYLKI (B08.01)<br>SLIHSRFLL (A02.01)<br>SLIHSRFLLM (A02.01, B08.01)<br>SLLTSSSNL (A02.01)<br>SLNHHSSSPL (A02.01, B07.02, B08.01)<br>SLSSPSKLPI (A02.01)<br>SPLSLHTPS (B07.02)<br>SPLSLHTPSS (B07.02)<br>SPPTHSHRL (B07.02)<br>SPRLHTPPS (B07.02)<br>SPRLHTPPSS (B07.02)<br>SPSLSSPSKL (B07.02)<br>SYLKIHLGL (A24.02)<br>TPSNHRPRPL (B07.02, B08.01)<br>TPSSHPSLHI (B07.02) | STAD, UCEC, BLCA, BRCA, LUSC, CESC, KIRC, UCS |

(continued)

| Gene | Exemplary Protein Change | Mutation Sequence Context | Peptides (HLA allele example(s)) | Exemplary Diseases |
|---|---|---|---|---|
| ARID1A | A2137fs | | CVPFWTGRIL (B07.02)<br>HCVPFWTGRIL (B07.02)<br>ILLPSAASV (A02.01)<br>ILLPSAASVC (A02.01)<br>LLPSAASVCPI (A02.01)<br>LPSAASVCPI (B07.02)<br>MRPHCVPF (B08.01)<br>RILLPSAASV (A02.01) | STAD, UCEC, BLCA, BRCA, LUSC, CESC, KIRC, UCS |
| | P2139fs | RTNPTVRMRPHCVPFWT<br>GRILLPSAASVCPIPFEAC<br>HLCQAMTLRCPNTQGCC<br>SSWAS* | RMRPHCVPF (A24.02, B07.02, 808.01) | |
| | L1970fs<br>V1994fs | | RMRPHCVPFW (A24.02)<br>RTNPTVRMR (A03.01)<br>SVCPIPFEA (A02.01)<br>TVRMRPHCV (B08.01)<br>TVRMRPHCVPF (B08.01)<br>VPFWTGRIL (B07.02)<br>VPFWTGRILL (B07.02)<br>VRMRPHCVPF (B08.01) | |

| Gene | Exemplary Protein Change | Mutation Sequence Context | Peptides (HLA allele example(s)) | Exemplary Diseases |
|---|---|---|---|---|
| ARID1A | N756fs S764fs T783fs Q799fs A817fs | TNQALPKIEVICRGTPRCP STVPPSPAQPYLRVSLPED RYTQAWAPTSRTPWGAM VPRGVSMAHKVATPGSQ TIMPCPMPTTPVQAWLEA * | AMVPRGVSM (B07.02, B08.01) AMVPRGVSMA (A02.01) AWAPTSRTPW (A24.02) CPMPTTPVQA (B07.02) CPSTVPPSPA (B07.02) GAMVPRGVSM (B07.02, B08.01) MPCPMPTTPV (B07.02) MPTTPVQAW (B07.02) MPTTPVQAWL (B07.02) SLPEDRYTQA (A02.01) SPAQPYLRV (B07.02) SPAQPYLRVS (B07.02) TIMPCPMPT (A02.01) TPVQAWLEA (B07.02) TSRTPWGAM (B07.02) VPPSPAQPYL (B07.02) VPRGVSMAH (B07.02) | STAD, UCEC, BLCA, BRCA, LUSC, CESC, KIRC, UCS |
| β2M | N62fs E67fs L74fs F82fs T91fs E94fs | RMERELKKWSIQTCLSAR TGLSISCTTLNSPPLKKMS MPAV* | CLSARTGLSI (B08.01) CTTLNSPPLK (A03.01) GLSISCTTL (A02.01) SPPLKKMSM (B07.02, B08.01) TLNSPPLKK (A03.01) TTLNSPPLK (A03.01) TTLNSPPLKK (A03.01) | CRC, STAD, SKCM, HNSC |
| β2M | L13fs S14fs | LCSRYSLFLAWRLSSVLQ RFRFTHVIQQRMESQIS* | LQRFRFTHV (B08.01) LQRFRFTHVI (B08.01) RLSSVLQRF (A24.02) RLSSVLQRFR (A03.01) VLQRFRFTHV (A02.01, B08.01) | CRC, STAD, SKCM, HNSC |

EP 3 436 048 B1

| Gene | Exemplary Protein Change | Mutation Sequence Context | Peptides (HLA allele example(s)) | Exemplary Diseases |
|---|---|---|---|---|
| CDH1 | A691fs<br>P708fs<br>L711fs | RSACVTVKGPLASVGRHS<br>LSKQDCKFLPFWGFLEEF<br>LLC* | ASVGRHSLSK (A03.01)<br>KFLPFWGFL (A24.02)<br>LASVGRHSL (B07.02)<br>LPFWGFLEEF (B07.02)<br>PFWGFLEEF (A24.02)<br>SVGRHSLSK (A03.01) | ILC LumA Breast Cancer |
| CDH1 | H121fs<br>P126fs<br>H128fs<br>N144fs<br>V157fs<br><br>P159fs<br><br>N166fs<br>N181fs<br>F189fs<br>P201fs<br>F205fs | IQWGTTTAPRPIRPPFLES<br>KQNCSHFPTPLLASEDRR<br>ETGLFLPSAAQKMKKAHF<br>LKTWFRSNPTKTKKARFS<br>TASLAKELTHPLLVSLLL<br>KEKQDG* | APRPIRPPF (B07.02)<br>APRPIRPPFL (B07.02)<br>AQKMKKAHFL (B08.01)<br>FLPSAAQKM (A02.01)<br>GLFLPSAAQK (A03.01)<br>HPLLVSLLL (B07.02)<br>KAHFLKTWFR (A03.01)<br>KARFSTASL (B07.02)<br>KMKKAHFLK (A03.01)<br><br>KTWFRSNPTK (A03.01)<br><br>LAKELTHPL (B07.02, B08.01)<br>LAKELTHPLL (B08.01)<br>NPTKTKKARF (B07.02)<br>QKMKKAHFL (B08.01)<br>RFSTASLAK (A03.01)<br>RPIRPPFLES (B07.02)<br>RSNPTKTKK (A03.01)<br>SLAKELTHPL (A02.01, B08.01)<br>TKKARFSTA (B08.01) | ILC LumA Breast Cancer |

EP 3 436 048 B1

107

| Gene | Exemplary Protein Change | Mutation Sequence Context | Peptides (HLA allele example(s)) | Exemplary Diseases |
|---|---|---|---|---|
| CDH1 | V114fs P127fs V132fs P160fs | PTDPFLGLRLGLHLQKVF HQSHAEYSGAPPPPPAPS GLRFWNPSRIAHISQLLS WPQKTEERLGYSSHQLPR K* | GLRFWNPSR (A03.01) ISQLLSWPQK (A03.01) RIAHISQLL (A02.01) RLGYSSHQL (A02.01) SQLLSWPQK (A03.01) SRIAHISQL (B08.01) WPQKTEERL (B07.02) YSSHQLPRK (A03.01) | ILC LumA Breast Cancer |
| CDH1 | L731fs R749fs E757fs G759fs | FCCSCCFFGGERWSKSPY CPQRMTPGTTFITMMKKE AEKRTRTLT* | CPQRMTPGTT (B07.02) EAEKRTRTL (B08.01) GTTFITMMK (A03.01) 3TTFITMMKK (A03.01) ITMMKKEAEK (A03.01) RMTPGTTFI (A02.01) SPYCPQRMT (B07.02) TMMKKEAEK (A03.01) TPGTTFITM (B07.02) TPGTTFITMM (B07.02) TTFITMMKK (A03.01) | ILC LumA Breast Cancer |
| CDH1 | S19fs E24fs S36fs | WRRNCKAPVSLRKSVQT PARSSPARPDRTRRLPSLG VPGQPWALGAAASRRCC CCCRSPLGSARSRSPATLA LTPRATRSRCPGATWREA ASWAE* | CPGATWREA (B07.02) CPGATWREAA (B07.02) RSRCPGATWR (A03.01) TPRATRSRC (B07.02) | ILC LumA Breast Cancer |
| | P394fs P387fs S398fs H400fs M401fs S408fs P409fs | | | |

| Gene | Exemplary Protein Change | Mutation Sequence Context | Peptides (HLA allele example(s)) | Exemplary Diseases |
|---|---|---|---|---|
| GATA3 | S408fs<br><br>P409fs<br><br><br><br>T419fs<br><br><br>H424fs<br>P425fs<br>S427fs<br>F431fs<br>S430fs<br>H434fs<br>H435fs<br>S438fs<br>M443fs<br>G444fs<br>*445fs | PGRPLQTHVLPEPHLALQ<br>PLQPHADHAHADAPAIQP<br>VLWTTPPLQHGHRHGLEP<br>―――――――――――――<br>CSMLTGPPARVPAVPFDL<br>HFCRSSIMKPKRDGYMFL<br>KAESKIMFATLQRSSLWC<br>LCSNH* | HVLPEPHLAL (B07.02)<br><br><br>RPLQTHVLPE (B07.02)<br>VLWTTPPLQH (A03.01) | Breast Cancer |
| GATA3 | P426fs<br>H434fs<br>P433fs<br>T441fs | PRPRRCTRHPACPLDHTT<br>PPAWSPPWVRALLDAHR<br>APSESPCSPFRLAFLQEQY<br>HEA* | APSESPCSPF (B07.02)<br>CPLDHTTPPA (B07.02)<br>FLQEQYHEA (A02.01, B08.01)<br>RLAFLQEQYH (A03.01)<br>SPCSPFRLAF (B07.02)<br>SPPWVRALL (B07.02)<br>YPACPLDHTT (B07.02) | Breast Cancer |

EP 3 436 048 B1

109

(continued)

| Gene | Exemplary Protein Change | Mutation Sequence Context | Peptides (HLA allele example(s)) | Exemplary Diseases |
|---|---|---|---|---|
| MLL2 | P519fs<br>E524fs<br>P647fs<br>S654fs<br>L656fs<br>R755fs<br>L761fs<br>Q773fs | TRRCHCCPHLRSHPCPHH<br>LRNHPRPHHLRHHACHH<br>HLRNCPHPHFLRHCTCPG<br>RWRNRPSLRRLRSLLCLP<br>HLNHHLFLHWRSRPCLHR<br>KSHPHLLHLRRLYPHHLK<br>HRPCPHHLKNLLCPRHLR<br>NCPLPRHLKHLACLHHLR<br>SHPCPLHLKSHPCLHHRR<br>HLVCSHHLKSLLCPLHLR<br>SLPFPHHLRHHACPHHLR<br>TRLCPHHLKNHLCPPHLR<br>YRAYPPCLWCHACLHRL<br>RNLPCPHRLRSLPRPLHLR<br>LHASPHHLRTPPHPHHLR<br>THLLPHHRRTRSCPCRWR<br>SHPCCHYLRSRNSAPGPR<br>GRTCHPGLRSRTCPPGLR<br>SHTYLRRLRSHTCPPSLRS<br>HAYALCLRSHTCPPRLRD<br>HICPLSLRNCTCPPRLRSR<br>TCLLCLRSHACPPNLRNH<br>TCPPSLRSHACPPGLRNRI<br>CPLSLRSHPCPLGLKSPLR<br>SQANALHLRSCPCSLPLG<br>NHPYLPCLESQPCLSLGN<br>HLCPLCPRSCRCPHLGSHF<br>CRLS* | ALHLRSCPC (B08.01)<br>CLHHRRHLV (B08.01)<br>CLHHRRHLVC (B08.01)<br>CLHRKSHPHL (B08.01)<br>CLRSHACPP (B08.01)<br>CLRSHTCPP (B08.01)<br>CLWCHACLH (A03.01)<br>CPHHT KNHL (B07.02)<br>CPHHT KNLL (B07.02)<br>CPHHLRTRL (B07.02, B08.01)<br>CPLHLRSLPF (B07.02, B08.01)<br>CPLPRHT KHT (B07.02, B08.01)<br>CPLSLRSHPC (B07.02)<br>CPRHLRNCPL (B07.02, B08.01)<br>FPHHLRHHA (B07.02, B08.01)<br>FPHHLRHHAC (B07.02, B08.01)<br>GLRSRTCPP (B08.01)<br>HACLHRLRNL (B08.01)<br>HLACLHHLR (A03.01)<br>HLCPPHLRY (A03.01)<br>HLCPPHLRYR (A03.01)<br>HLKHTACLH (A03.01)<br>HLKHRPCPH (B08.01)<br>HLKNHLCPP (B08.01)<br>HLKSHPCLH (A03.01)<br>HLKSLLCPL (A02.01, B08.01)<br>HLLHLRRLY (A03.01)<br>HLRNCPLPR (A03.01)<br>HLRNCPLPRH (A03.01)<br>HLRRLYPHHL (B08.01) | STAD, BLCA, CRC, HNSC, BRCA |
| | | | HLRSHPCPL (B07.02, B08.01) | |

(continued)

| Gene | Exemplary Protein Change | Mutation Sequence Context | Peptides (HLA allele example(s)) | Exemplary Diseases |
|---|---|---|---|---|
| | | | HLRSHPCPLH (A03.01) | |
| | | | HLRSLPFPH (A03.01) | |
| | | | HLRTRLCPH (A03.01, B08.01) | |
| | | | HLVCSHHLK (A03.01) | |
| | | | HPCLHHRRHL (B07.02, B08.01) | |
| | | | HPGLRSRTC (B07.02) | |
| | | | HPHLLHLRRL (B07.02, B08.01) | |
| | | | HRKSHPHLL (B08.01) | |
| | | | HRRTRSCPC (B08.01) | |
| | | | KSHPHLLHLR (A03.01) | |
| | | | KSLLCPLHLR (A03.01) | |
| | | | LLCPLHLRSL (A02.01, B08.01) | |
| | | | LLHLRRLYPH (B08.01) | |
| | | | LPRHLKHLA (B07.02) | |
| | | | LPRHLKHLAC (B07.02, B08.01) | |
| | | | LRRLRSHTC (B08.01) | |
| | | | LRRLYPHHL (B08.01) | |
| | | | LVCSHHLKSL (B08.01) | |
| | | | NLRNHTCPPS (B08.01) | |
| | | | PLHLRSLPF (B08.01) | |
| | | | RLCPHHLKNH (A03.01) | |
| | | | RLYPHHLKH (A03.01) | |
| | | | RLYPHHLKHR (A03.01) | |
| | | | RPCPHHLKNL (B07.02) | |
| | | | RSHPCPLHLK (A03.01) | |
| | | | RSLPFPHHLR (A03.01) | |
| | | | RTRLCPHHL (B07.02) | |
| | | | RTRLCPHHLK (A03.01) | |
| | | | SLLCPLHLR (A03.01) | |
| | | | SLRSHACPP (B08.01) | |
| | | | SPLRSQANA (B07.02) | |
| | | | YLRRLRSHT (B08.01) | |
| | | | YPHHLKHRPC (B07.02, B08.01) | |

(continued)

| Gene | Exemplary Protein Change | Mutation Sequence Context | Peptides (HLA allele example(s)) | Exemplary Diseases |
|---|---|---|---|---|
| PTEN | I122fs<br>I135fs<br>A148fs<br>L152fs<br>D162fs<br>I168fs | SWKGTNWCNDMCIFITSG QIFKGTRGPRFLWGSKDQ RQKGSNYSQSEALCVLL* | FITSGQIFK (A03.01)<br>IFITSGQIF (A24.02)<br>SQSEALCVL (A02.01)<br>SQSEALCVLL (A02.01) | UCEC, PRAD, SKCM, STAD, BRCA, LUSC, KIRC, LIHC, KIRP, GBM |
| PTEN | L265fs<br>K266fs | KRTKCFTFG* |  | UCEC, PRAD, SKCM, STAD, BRCA, LUSC, KIRC, LIHC, KIRP, GBM |
| PTEN | A39fs<br>E40fs<br>V45fs<br>R47fs<br>N48fs | PIFIQTLLLWDFLQKDLKA YTGTILMM* | AYTGTILMM (A24.02)<br>DLKAYTGTIL (B08.01) | UCEC, PRAD, SKCM, STAD, BRCA, LUSC, KIRC, LIHC, KIRP, GBM |
| PTEN | T319fs<br>T321fs<br>K327fs<br>A328fs<br>A333fs | QKMILTKQIKTKPTDTFL QILR* | ILTKQIKTK (A03.01)<br>KMILTKQIK (A03.01)<br>KPTDTFLQI (B07.02)<br>KPTDTFLQIL (B07.02)<br>MILTKQIKTK (A03.01) | UCEC, PRAD, SKCM, STAD, BRCA, LUSC, KIRC, LIHC, KIRP, GBM |
| PTEN | N63fs<br>E73fs<br>A86fs<br>N94fs | GFWIQSIKTITRYTIFVLK DIMTPPNLIAELHNILLKTI THHS* | ITRYTIFVLK (A03.01)<br>LIAELHNIL (A02.01)<br>LIAELHNILL (A02.01)<br>MTPPNLIAEL (A02.01)<br>NLIAELHNI (A02.01)<br>NLIAELHNIL (A02.01)<br>RYTIFVLKDI (A24.02)<br>TITRYTIFVL (A02.01)<br>TPPNLIAEL (B07.02) | UCEC, PRAD, SKCM, STAD, BRCA, LUSC, KIRC, LIHC, KIRP, GBM |

(continued)

| Gene | Exemplary Protein Change | Mutation Sequence Context | Peptides (HLA allele example(s)) | Exemplary Diseases |
|---|---|---|---|---|
| PTEN | T202fs<br>G209fs<br>C211fs<br>I224fs<br>G230fs<br>P231fs<br>R233fs<br>D236fs | NYSNVQWRNLQSSVCGL<br>PAKGEDIFLQFRTHTTGR<br>QVHVL* | FLQFRTHTT (A02.01, B08.01)<br>LPAKGEDIFL (B07.02)<br>LQFRTHTTGR (A03.01)<br>NLQSSVCGL (A02.01)<br>SSVCGLPAK (A03.01)<br>VQWRNLQSSV (A02.01) | UCEC, PRAD, SKCM, STAD, BRCA, LUSC, KIRC, LIHC, KIRP, GBM |
| PTEN | G251fs<br>E256fs<br>K260fs<br>Q261fs<br><br>L265fs<br><br>M270fs<br>H272fs<br>T286fs<br>E288fs | YQSRVLPQTEQDAKKGQ<br>NVSLLGKYILHTRTRGNL<br>RKSRKWKSM* | GQNVSLLGK (A03.01)<br>HTRTRGNLRK (A03.01)<br>ILHTRTRGNL (B08.01)<br>KGQNVSLLGK (A03.01)<br><br>LLGKYILHT (A02.01)<br><br>LRKSRKWKSM (B08.01)<br>SLLGKYILH (A03.01)<br>SLLGKYILHT (A02.01) | UCEC, PRAD, SKCM, STAD, BRCA, LUSC, KIRC, LIHC, KIRP, GBM |
| | A70fs<br>P72fs<br>A76fs<br>A79fs<br>P89fs<br>W91fs<br>S96fs<br>V97fs | | CTSPLLAPV (A02.01)<br>FPENLPGQL (B07.02)<br>GLLAFWDSQV (A02.01)<br>IFCPFPENL (A24.02)<br>LLAFWDSQV (A02.01)<br>LLAPVIFCP (A02.01)<br>LLAPVIFCPF (A02.01, A24.02)<br>LPCPQQDVL (B07.02) | |

EP 3 436 048 B1

| Gene | Exemplary Protein Change | Mutation Sequence Context | Peptides (HLA allele example(s)) | Exemplary Diseases |
|---|---|---|---|---|
| TP53 | V97fs | SSQNARGCSPRGPCTSSSY TGGPCTSPLLAPVIFCPFPE NLPGQLRFPSGLLAFWDS QVCDLHVLPCPQQDVLPT GQDLPCAAVG* | RFPSGLLAF (A24.02) | BRCA, CRC, LUAD, PRAD, HNSC, LUSC, PAAD, STAD, BLCA, OV, LIHC, SKCM, UCEC, LAML, UCS, KICH, GBM, ACC |
| | G108fs | | RFPSGLLAFW (A24.02) | |
| | G117fs S121fs V122fs C124fs K139fs V143fs | | SPLLAPVIF (B07.02) SPRGPCTSS (B07.02) SPRGPCTSSS (B07.02) SQVCDLHVL (A02.01) VIFCPFPENL (A02.01) | |
| | V173fs H178fs D186fs H193fs L194fs E198fs | | AMVWPLLSI (A02.01) AMVWPLLSIL (A02.01) AQIAMVWPL (A02.01, A24.02) AQIAMVWPLL (A02.01) CPMSRLRLA (B07.02, B08.01) CPMSRLRLAL (B07.02, B08.01) IAMVWPLLSI (A02.01, A24.02, B08.01) ILSEWKEICV (A02.01) IVWWCPMSR (A03.01) IVWWCPMSRL (A02.01) | |

(continued)

| Gene | Exemplary Protein Change | Mutation Sequence Context | Peptides (HLA allele example(s)) | Exemplary Diseases |
|---|---|---|---|---|
| TP53 | V203fs | GAAPTMSAAQIAMVWPLLSILSEWKEICVWWCPMSRLRLALTVPPSTTTTCVTVPAWAA* | WMTETLFDI (A24.02) | BRCA, CRC, LUAD, PRAD, HNSC, LUSC, PAAD, STAD, BLCA, OV, LIHC, SKCM, UCEC, LAML, UCS, KICH, GBM, ACC |
| | E204fs | | LLSILSEWK (A03.01) | |
| | L206fs | | MSAAQIAMV (A02.01) | |
| | D207fs | | MSRLRLALT (B08.01) | |
| | N210fs | | MSRLRLALTV (B08.01) | |
| | T211fs | | MVWPLLSIL (A02.01) | |
| | F212fs | | RLALTVPPST (A02.01) | |
| | V225fs | | TLFDIVWWC (A02.01) | |
| | S241fs | | TLFDIVWWCP (A02.01) | |
| | | | TMSAAQIAMV (A02.01) | |
| | | | VWSIWMTETL (A24.02) | |
| | | | WMTETLFDI (A02.01, A24.02) | |
| | | | WMTETLFDIV (A01.01, A02.01) | |
| | R248fs | | ALRCVFVPV (A02.01, B08.01) | |
| | P250fs | | ALRCVFVPVL (A02.01, B08.01) | |
| | S260fs | | ALSEHCPTT (A02.01) | |
| | N263fs | | AQRKRISARK (A03.01) | |
| | G266fs | | GAQRKRISA (B08.01) | |
| | N268fs | | | |
| | V272fs | | | |
| | V274fs | | | |
| | P278fs | | | |
| | D281fs | | | |
| | R282fs | | | |

| Gene | Exemplary Protein Change | Mutation Sequence Context | Peptides (HLA allele example(s)) | Exemplary Diseases |
|---|---|---|---|---|
| TP53 | T284fs<br>E285fs<br>L289fs | | HWMENISPF (A24.02)<br>LPSQRRNHW (B07.02)<br>LPSQRRNHWM (B07.02, B08.01) | BRCA, CRC, LUAD, PRAD, HNSC, LUSC, PAAD, STAD, BLCA, OV, LIHC, SKCM, UCEC, LAML, UCS, KICH, GBM, ACC |
| | K292fs | TGGPSSPSSHWKTPVVIY WDGTALRCVFVPVLGET GAQRKRISARKGSLTTSC PQGALSEHCPTTPAPLPSQ RRNHWMENISPFRSVGVS ASRCSES* | NISPFRSVGV (A02.01) | |
| | P301fs | | RISARKGSL (B07.02, B08.01) | |
| | S303fs<br>T312fs<br>S314fs<br>K319fs<br>K320fs<br>P322fs<br>Y327fs<br>F328fs<br>L330fs<br>R333fs<br>R335fs<br>R337fs<br>E339fs | | SPFRSVGVSA (B07.02)<br>SPSSHWKTPV (B07.02, B08.01)<br>TALRCVFVPV (A02.01)<br>VIYWDGTAL (A02.01)<br>VIYWDGTALR (A03.01)<br>VLGETGAQRK (A03.01) | |

| Gene | Exemplary Protein Change | Mutation Sequence Context | Peptides (HLA allele example(s)) | Exemplary Diseases |
|---|---|---|---|---|
| TP53 | S149fs<br>P151fs<br>P152fs<br>V157fs<br>Q165fs<br>S166fs<br>H168fs<br>V173fs | FHTPARHPRPRHGHLQAV<br>TAHDGGCEALPPP* | HPRPRHGHL (B07.02, B08.01)<br>HPRPRHGHLQ (B07.02)<br>RPRHGHLQA (B07.02)<br>RPRHGHLQAV (B07.02, B08.01) | BRCA, CRC, LUAD, PRAD, HNSC, LUSC, PAAD, STAD, BLCA, OV, LIHC, SKCM, UCEC, LAML, UCS, KICH, GBM, ACC |
| TP53 | P47fs<br>D48fs<br>D49fs<br>Q52fs<br>F54fs<br>E56fs<br>P58fs<br>P60fs<br>E62fs<br>M66fs<br>P72fs<br>V73fs<br><br><br>P75fs<br><br><br>A78fs<br>P82fs<br>P85fs<br>S96fs<br>P98fs<br>T102fs<br>Y103fs | CCPRTILNNGSLKTQVQM<br>KLPECQRLLPPWPLHQQL<br>LHRRPLHQPPPGPCHLLSL<br>PRKPTRAATVSVWASCIL<br>GQPSL* | GSLKTQVQMK (A03.01)<br>PPGPCHLLSL (B07.02)<br>RTILNNGSLK (A03.01)<br>SLKTQVQMK (A03.01)<br><br><br>SLKTQVQMKL (B08.01)<br><br><br>TILNNGSLK (A03.01) | BRCA, CRC, LUAD, PRAD, HNSC, LUSC, PAAD, STAD, BLCA, OV, LIHC, SKCM, UCEC, LAML, UCS, KICH, GBM, ACC |

| Gene | Exemplary Protein Change | Mutation Sequence Context | Peptides (HLA allele example(s)) | Exemplary Diseases |
|---|---|---|---|---|
| | G108fs<br>F109fs<br>R110fs<br>G117fs | | | |
| TP53 | L26fs<br>P27fs<br>P34fs<br>P36fs<br>A39fs<br>Q38fs | VRKHFQTYGNYFLKTTFC<br>PPCRPKQWMI* | CPPCRPKQWM (B07.02)<br>TTFCPPCRPK (A03.01) | BRCA, CRC, LUAD, PRAD, HNSC, LUSC, PAAD, STAD, BLCA, OV, LIHC, SKCM, UCEC, LAML, UCS, KICH, GBM, ACC |
| TP53 | C124fs<br>L130fs<br>N131fs<br>C135fs<br>K139fs<br>A138fs<br>T140fs<br>V143fs<br><br><br>Q144fs<br><br><br>V147fs<br>T150fs<br>P151fs<br>P152fs<br>G154fs<br>R156fs<br>R158fs<br>A161fs | LARTPLPSTRCFANWPRP<br>ALCSCGLIPHPRPAPASAP<br>WPSTSSHST* | CFANWPRPAL (A24.02)<br>FANWPRPAL (B07.02, B08.01)<br>GLIPHPRPA (A02.01)<br>HPRPAPASA (B07.02, B08.01)<br>HPRPAPASAP (B07.02)<br>IPHPRPAPA (B07.02, B08.01)<br><br><br>IPHPRPAPAS (B07.02)<br><br><br>RPALCSCGL (B07.02)<br>RPALCSCGLI (B07.02)<br>TPLPSTRCF (B07.02)<br>WPRPALCSC (B07.02)<br>WPRPALCSCG (B07.02) | BRCA, CRC, LUAD, PRAD, HNSC, LUSC, PAAD, STAD, BLCA, OV, LIHC, SKCM, UCEC, LAML, UCS, KICH, GBM, ACC |

118

EP 3 436 048 B1

(continued)

| Gene | Exemplary Protein Change | Mutation Sequence Context | Peptides (HLA allele example(s)) | Exemplary Diseases |
|---|---|---|---|---|
| VHL | L178fs<br>D179fs<br>L 184fs<br>T202fs<br>R205fs<br>D213fs<br>G212fs | ELQETGHRQVALRRSGRP<br>PKCAERPGAADTGAHCTS<br>TDGRLKISVETYTVSSQLL<br>MVLMSLDLDTGLVPSLVS<br>KCLILRVK* | ALRRSGRPPK (A03.01)<br>GLVPSLVSK (A03.01)<br>KISVETYTV (A02.01)<br>LLMVLMSLDL (A02.01, B08.01)<br>LMSLDLDTGL (A02.01)<br>LMVLMSLDL (A02.01)<br>LVSKCLILRV (A02.01)<br>QLLMVLMSL (A02.01, B08.01)<br>RPGAADTGA (B07.02)<br>RPGAADTGAH (B07.02)<br>SLDLDTGLV (A02.01)<br>SLVSKCLIL (A02.01, B08.01)<br>SQLLMVLMSL (A02.01)<br>TVSSQLLMV (A02.01)<br>TYTVSSQLL (A24.02)<br>TYTVSSQLLM (A24.02)<br>VLMSLDLDT (A02.01)<br>VPSLVSKCL (B07.02)<br>VSKCLILRVK (A03.01)<br>YTVSSQLLM (A01.01)<br>YTVSSQLLMV (A02.01) | KIRC, KIRP |
| VHL | L158fs<br>K159fs<br>R161fs<br>Q164fs | KSDASRLSGA* | | KIRC, KIRP |

(continued)

| Gene | Exemplary Protein Change | Mutation Sequence Context | Peptides (HLA allele example(s)) | Exemplary Diseases |
|---|---|---|---|---|
| VHL | P146fs, I147fs, F148fs, L158fs | RTAYFCQYHTASVYSERAMPPGCPEPSQA* | FCQYHTASV (B08.01) | KIRC, KIRP |
| VHL | S68fs, S72fs, I75fs, S80fs, P86fs, P97fs, I109fs, H115fs, L116fs, G123fs, T124fs, N131fs, L135fs, V137fs, G144fs, D143fs, I147fs | TRASPPRSSSAIAVRASCCPYGSTSTASRSPTQRCRLARAAASTATEVTFGSSEMQGHTMGFWLTKLNYLCHLSMLTDSLFLPISHCQCIL* | CPYGSTSTA (B07.02), CPYGSTSTAS (B07.02), LARAAASTAT (B07.02), MLTDSLFLP (A02.01), PPRSSSAIAV (B07.02), RAAASTATEV (B07.02), SPPRSSSAI (B07.02), SPPRSSSAIA (B07.02), SPTQRCRLA (B07.02), TQRCRLARA (B08.01), TQRCRLARAA (B08.01) | KIRC, KIRP |
| VHL | K171fs, P172fs, N174fs, L178fs, D179fs, L188fs | SSLRITGDWTSSGRSTKIWKTTQMCRKTWSG* | KIWKTTQMCR (A03.01), WTSSGRSTK (A03.01) | KIRC, KIRP |

| Gene | Exemplary Protein Change | Mutation Sequence Context | Peptides (HLA allele example(s)) | Exemplary Diseases |
|---|---|---|---|---|
| VHL | V62fs<br>V66fs<br>Q73fs<br>V84fs<br>F91fs<br><br>T100fs<br><br>P103fs<br>S111fs<br>L116fs<br>H115fs<br>D126fs | <u>RRRRGGVGRRGVRPGRV</u><br><u>RPGGTGRRGGDGGRAAA</u><br><u>ARAALGELARALPGHLLQ</u><br><u>SQSARRAARMAQLRRRA</u><br><u>AALPNAAAWHGPPHPQL</u><br><u>PRSPLALQRCRDTRWASG</u><br><u>*</u> | ALGELARAL (A02.01)<br>AQLRRRAAA (B08.01)<br>AQLRRRAAAL (B08.01)<br>ARRAARMAQL (B08.01)<br>HPQLPRSPL (B07.02, B08.01)<br>HPQLPRSPLA (B07.02)<br>LARALPGHL (B07.02)<br><br>LARALPGHLL (B07.02)<br><br>MAQLRRRAA (B07.02, B08.01)<br>MAQLRRRAAA (B07.02, B08.01)<br>QLRRRAAAL (B07.02, B08.01)<br>RAAALPNAAA (B07.02)<br>RMAQLRRRAA (B07.02, B08.01)<br>SQSARRAARM (B08.01) | KIRC, KIRP |

| **TABLE 1D** | | **CRYPTIC EXON** [1] | | |
|---|---|---|---|---|
| AR-v7 | cryptic final exon | <u>SCKVFFKRAAEGKQKYL</u><br><u>CASRNDCTIDKFRRKNCP</u><br><u>SCRLRKCYEAGMTLGEKF</u><br><u>RVGNCKHLKMTRP*</u> | GMTLGEKFRV (A02:01)<br><br>RVGNCKHLK (A03.01) | Prostate Cancer, Castration-resistant Prostate Cancer |

| **TABLE 1E** | | **OUT OF FRAME FUSIONS** [1,3] | | |
|---|---|---|---|---|
| AC011997.1 :LRRC69 | AC011997.1:LRRC69<br>*out-of-frame | MAGAPPPASLPPCSLISDC<br>CASNQRDSVGVGPSEP:<u>G</u>:<br>**NNIKICNESASRK***  | GPSEPGNNI (B07.02)<br><br>KICNESASRK (A03.01) | LUSC, Breast Cancer, Head and Neck Cancer, LUAD |

(continued)

| TABLE 1E | | OUT OF FRAME FUSIONS [1,3] | | |
|---|---|---|---|---|
| EEF1DP3 | EEF1DP3:FRY *out-of-frame | HGWRPFLPVRARSRWNR RLDVTVANGR::S:**WKYG WSLLRVPQVNGIQVLNV SLKSSSNVISYE***  | GIQVLNVSLK (A03.01), IQVLNVSLK (A03.01), KSSSNVISY (A01.01, A03.01), KYGWSLLRV (A24.02), RSWKYGWSL (A02.01), SLKSSSNVI (B08.01), SWKYGWSLL (A24.02), TVANGRSWK (A03.01), VPQVNGIQV (B07.02), VPQVNGIQVL (B07.02), VTVANGRSWK (A03.01), WSLLRVPQV (B08.01) | Breast Cancer |
| MAD1L1:M AFK | MAD1L1:MAFK | RLKEVFQTKIQEFRKACY TLTGYQIDITTENQYRLTS LYAEHPGDCLIFK::**LRVP GSSVLVTVPGL*** | HPGDCLIFKL (B07.02), KLRVPGSSV (B07.02), KLRVPGSSVL (B07.02), RVPGSSVLV (A02.01), SVLVTVPGL (A02.01), VPGSSVLVTV (B07.02) | CLL |
| PPP1R1B:S TARD3 | PPP1R1B:STAR D3 | AEVLKVIRQSAGQKTTCG QGLEGPWERPPPLDESER DGGSEDQVEDPALS:A:**LL LRPRPPREVGAHQDEQ AAQGADPRLGAQPACR GLPGLLTVPQPEPLLAP PSAA*** | ALLLRPRPPR (A03.01), ALSALLLRPR (A03.01) | Breast Cancer |

| Table 1F | | IN FRAME DELETIONS and FUSIONS [1,2] | | |
|---|---|---|---|---|
| **Gene** | **Exemplary Protein Change** | **Mutation Sequence Context** | **Peptides (HLA allele example(s))** | **Exemplary Diseases** |
| BCR:ABL | BCR:ABL | ERAEWRENIREQQKKCFR SFSLTSVELQMLTNSCVK LQTVHSIPLTINKE::**EALQ RPVASDFEPQGLSEAAR WNSKENLLAGPSENDPN LFVALYDFVASG** | LTINKEEAL (A02.01, B08.01) | CML, AML |
| BCR:ABL | BCR:ABL | ELQMLTNSCVKLQTVHSI PLTINKEDDESPGLYGFLN VIVHSATGFKQSS:<u>K</u>:**ALQ RPVASDFEPQGLSEAAR WNSKENLLAGPSENDPN LFVALYDFVASGD** | IVHSATGFK (A03.01)<br><br>ATGFKQSSK (A03.01) | CML, AML |
| C11orf95:RE LA | C11orf95:RELA | ISNSWDAHLGLGACGEAE GLGVQGAEEEEEEEEEEE EEGAGVPACPPKGP:<u>E</u>:**LF PLIFPAEPAQASGPYVEII EQPKQRGMRFRYKCEG RSAGSIPGERSTD** | ELFPLIFPA (A02.01, B08.01)<br><br>KGPELFPLI (A02.01, A24.02)<br><br>KGPELFPLIF (A24.02) | Supretentorial epen-dyomas |
| CBFB:MYH 11 | (variant "type a") | LQRLDGMGCLEFDEERA QQEDALAQQAFEEARRR TREFEDRDRSHREEME::**V HELEKSKRALETQMEE MKTQLEELEDELQATE DAKLRLEVNMQALKGQ F** | | AML |

| Table 1F | | IN FRAME DELETIONS and FUSIONS [1,2] | | |
|---|---|---|---|---|
| **Gene** | **Exemplary Protein Change** | **Mutation Sequence Context** | **Peptides (HLA allele example(s))** | **Exemplary Diseases** |
| CD74:ROS1 | (exon6:exon32) | KGSFPENLRHLKNTMETI DWKVFESWMHHWLLFE MSRHSLEQKPTDAPPK::**A GVPNKPGIPKLLEGSKN SIQWEKAEDNGCRITYY ILEIRKSTSNNLQNQ** | KPTDAPPKAGV (B07.02) | NSCLC, Crizotinib resistance |
| EGFR | EGFRvIII (internal deletion) | MRPSGTAGAALLALLAA LCPASRALEEKK:<u>G</u>:NYVV TDHGSCVRACGADSYEM EEDGVRKCKKCEGPCRK VCNGIGIGEFKD | ALEEKKGNYV (A02.01) | GBM |
| EGFR:SEPI 14 | EGFR:SEPT14 | LPQPPICTIDVYMIMVKC WMIDADSRPKFRELIIEFS KMARDPQRYLVIQ::**LQD KFEHLKMIQQEEIRKLE EEKKQLEGEIIDFYKMK AASEALQTQLSTD** | IQLQDKFEHL (A02.01, B08.01) QLQDKFEHL (A02.01, B08.01) QLQDKFEHLK (A03.01) YLVIQLQDKF (A02.01, A24.02) | GBM, Glioma, Head and Neck Cancer |
| EML4:ALK | EML4:ALK | SWENSDDSRNKLSKIPSTP KLIPKVTKTADKHKDVIIN QAKMSTREKNSQ:<u>V</u>:**YRR KHQELQAMQMELQSPE YKLSKLRTSTIMTDYNP NYCFAGKTSSISDL** | QVYRRKHQEL (B08.01) STREKNSQV (B08.01) VYRRKHQEL (A24.02, B08.01) | NSCLC |

| Table 1F | | IN FRAME DELETIONS and FUSIONS [1,2] | | |
|---|---|---|---|---|
| Gene | Exemplary Protein Change | Mutation Sequence Context | Peptides (HLA allele example(s)) | Exemplary Diseases |
| FGFR3:TAC C3 | FGFR3:TACC3 | EGHRMDKPANCTHDLYM IMRECWHAAPSQRPTFKQ LVEDLDRVLTVTSTD::**VK ATQEENRELRSRCEELH GKNLELGKIMDRFEEVV YQAMEEVQKQKELS** | VLTVTSTDV (A02.01)<br><br>VLTVTSTDVK (A03.01) | Bladder Cancer, LUSC |
| NAB:STAT6 | NAB:STAT6 ("variant 1" of Chmielecki et al.) | RDNTLLLRRVELFSLSRQ VARESTYLSSLKGSRLHP EELGGPPLKKLKQE::<u>ATS</u> <u>KSQ</u>**IMSLWGLVSKMPPE KVQRLYVDFPQHLRHL LGDWLESQPWEFLVGS DAFCC** | IMSLWGLVS (A02.01)<br>IMSLWGLVSK (A03.01)<br>KLKQEATSK (A03.01)<br>QIMSLWGLV (A02.01)<br>SQIMSLWGL (A02.01, A24.02, B08.01)<br>SQIMSLWGLV (A02.01)<br>TSKSQIMSL (B08.01) | Solitary fibrous tumors |
| NDRG1:ER G | NDRG1:ERG | MSREMQDVDLAEVKPLV EKGETITGLLQEFDVQ::**E ALSVVSEDQSLFECAYG TPHLAKTEMTASSSSDY GQTSKMSPRVPQQDW** | LLQEFDVQEA (A02.01)<br><br>LQEFDVQEAL (A02.01) | Prostate Cancer |
| PML:RARA | PML:RARA (exon3:exon3) | VLDMHGFLRQALCRLRQ EEPQSLQAAVRTDGFDEF KVRLQDLSSCITQGK:<u>A</u>:**IE TQSSSSEEIVPSPPSPPPL PRIYKPCFVCQDKSSGY HYGVSACEGCKG** | | Acute promyelocytic leukemia |
| PML:RARA | PML:RARA (exon6:exon3) | RSSPEQPRPSTSKAVSPPH LDGPPSPRSPVIGSEVFLP NSNHVASGAGEA:**A**:**IETQ SSSSEEIVPSPPSPPPLPRI YKPCFVCQDKSSGYHYG VSACEGCKG** | | Acute promyelocytic leukemia |

| Table 1F | | IN FRAME DELETIONS and FUSIONS [1,2] | | |
|---|---|---|---|---|
| Gene | Exemplary Protein Change | Mutation Sequence Context | Peptides (HLA allele example(s)) | Exemplary Diseases |
| RUNX1 | RUN-X1(ex5)-RUNX1-T1(ex2) | VARFNDLRFVGRSGRGKS FTLTITVFTNPPQVATYHR AIKITVDGPREPR:N:**RTEK** **HSTMPDSPVDVKTQSRL** **TPPTMPPPPTTQGAPRT** **SSFTPTTLTNGT** | GPREPRNRT (B07.02)<br><br>RNRTEKHSTM (B08.01) | AML |
| TMPRSS2:E RG | TMPRSS2:ERG | MALNS::EALSVVSEDQSL FECAYGTPHLAKTEMTAS SSSDYGQTSKMSPRVPQQ DW | ALNSEALSV (A02.01)<br>ALNSEALSVV (A02.01)<br><br>MALNSEALSV (A02.01, B08.01) | Prostate Cancer |

[1]Underlined AAs represent non-native AAs

[2]Bolded AAs represent native AAs of the amino acid sequence encoded by the second of the two fused genes

[3]Bolded and underlined AAs represent non-native AAs of the amino acid sequence encoded by the second of the two fused genes due to a framshift.

**Table 2**

| Gene | Exemplary Protein Change | Mutation Sequence Context | Peptides (HLA allele example(s)) | Exemplary Diseases |
|---|---|---|---|---|
| Table 2A | | POINT MUTATIONS [1] | | |
| AKT1 | E17K | MSDVAIVKEGWLHKRG KYIKTWRPRYFLLKNDG TFIGYKERPQDVDQREAP LNNFSVAQCQLMKTER | KYIKTWRPRY (A24.02) WLHKRGKYI (A02.01, B07.02, B08.01) WLHKRGKYIK (A03.01) | BRCA, CESC, HNSC, LUSC, PRAD, SKCM, THCA |
| ANAPC1 | T537A | TMLVLEGSGNLVLYTGV VRVGKVFIPGLPAPSLTM SNTMPRPSTPLDGVSAPK PLSKLLGSLDEVVLLSPV PELRDSSKLHDSLYNEDC TFQQLGTYIHSI | APKPLSKLL (B07.02) GVSAPKPLSK (A03.01) VSAPKPLSK (A03.01) | GBM, LUSC, PAAD, PRAD, SKCM |
| FGFR3 | S249C | HRIGGIKLRHQQWSLVM ESVVPSDRGNYTCVVEN KFGSIRQTYTLDVLERCP HRPILQAGLPANQTAVL GSDVEFHCKVYSDAQPH IQWLKHVEVNGSKVG | CPHRPILQA (B07.02) | BLCA, HNSC, KIRP, LUSC |
| FRG1B | I10T | MREPIYMHSTMVFLPWE LHTKKGPSPPEQFMAVK LSDSRTALKSGYGKYLGI NSDELVGHSDAIGPREQ WEPVFQNGKMALLASNS CFIR | KLSDSRTAL (A02.01, B07.02, B08.01) KLSDSRTALK (A03.01) LSDSRTALK (A01.01, A03.01) RTALKSGYGK (A03.01) TALKSGYGK (A03.01) | KIRP, PRAD, SKCM |
| FRG1B | L52S | AVKLSDSRIALKSGYGK YLGINSDELVGHSDAIGP REQWEPVFQNGKMALS ASNSCFIRCNEAGDIEAK SKTAGEEEMIKIRSCAEK ETKKKDDIPEEDKG | ALSASNSCF (A02.01, A24.02, B07.02) ALSASNSCFI (A02.01) FQNGKMALSA (A02.01, B08.01) | GBM, KIRP, PRAD, SKCM |
| HER2 | L755S (Resistance) | AMPNQAQMRILKETELR KVKVLGSGAFGTVYKGI WIPDGENVKIPVAIKVSR ENTSPKANKEILDEAYV MAGVGSPYVSRLLGICLT STVQLVTQLMPYGC | KVSRENTSPK (A03.01) | BRCA |

(continued)

| Gene | Exemplary Protein Change | Mutation Sequence Context | Peptides (HLA allele example(s)) | Exemplary Diseases |
|---|---|---|---|---|
| **Table 2A** | | **POINT MUTATIONS [1]** | | |
| IDH1 | R132G | RVEEFKLKQMWKSPNGT IRNILGGTVFREAIICKNIP RLVSGWVKPIIIG<u>G</u>HAYG DQYRATDFVVPGPGKVE ITYTPSDGTQKVTYLVHN FEEGGGVAMGM | KPIIIGGHAY (B07.02) | BLCA, BRCA, CRC, GBM, HNSC, LUAD, PAAD, PRAD, UCEC |
| KRAS | G12C | MTEYKLVVVGA<u>C</u>GVGK SALTIQLIQNHFVDEYDP TIEDSYRKQVVIDGETCL LDILDTAGQE | KLVVVGACGV (A02.01) LVVVGACGV (A02.01) VVGACGVGK (A03.01, A11.01) VVVGACGVGK (A03.01) | BRCA, CESC, CRC, HNSC, LUAD, PAAD, UCEC |
| KRAS | G12D | MTEYKLVVVGA<u>D</u>GVGK SALTIQLIQNHFVDEYDP TIEDSYRKQVVIDGETCL LDILDTAGQE | VVGADGVGK (A11.01) VVVGADGVGK (A11.01) KLVVVGADGV (A02.01) LVVVGADGV (A02.01) | BLCA, BRCA, CESC, CRC, GBM, HNSC, KIRP, LIHC, LUAD, PAAD, SKCM, UCEC |
| KRAS | G12V | MTEYKLVVVGA<u>V</u>GVGK SALTIQLIQNHFVDEYDP TIEDSYRKQVVIDGETCL LDILDTAGQE | KLVVVGAVGV (A02.01) LVVVGAVGV (A02.01) VVGAVGVGK (A03.01, A11.01) VVVGAVGVGK (A03.01, A11.01) | BRCA, CESC, CRC, LUAD, PAAD, THCA, UCEC |
| KRAS | Q61H | AGGVGKSALTIQLIQNHF VDEYDPTIEDSYRKQVVI DGETCLLDILDTAG<u>H</u>EEY SAMRDQYMRTGEGFLC VFAINNTKSFEDIHHYRE QIKRVKDSEDVPM | ILDTAGHEEY (A01.01) | CRC, LUSC, PAAD, SKCM, UCEC |
| KRAS | Q61L | AGGVGKSALTIQLIQNHF VDEYDPTIEDSYRKQVVI DGETCLLDILDTAG<u>L</u>EEY SAMRDQYMRTGEGFLC VFAINNTKSFEDIHHYRE QIKRVKDSEDVPM | ILDTAGLEEY (A01.01) LLDILDTAGL (A02.01) | CRC, GBM, HNSC, LUAD, SKCM, UCEC |

(continued)

| Gene | Exemplary Protein Change | Mutation Sequence Context | Peptides (HLA allele example(s)) | Exemplary Diseases |
|---|---|---|---|---|
| Table 2A | | POINT MUTATIONS [1] | | |
| NRAS | Q61K | AGGVGKSALTIQLIQNHF VDEYDPTIEDSYRKQVVI DGETCLLDILDTAGKEEY SAMRDQYMRTGEGFLC VFAINNSKSFADINLYRE QIKRVKDSDDVPM | ILDTAGKEEY (A01.01) | BLCA, CRC, LIHC, LUAD, LUSC, SKCM, THCA, UCEC |
| NRAS | Q61R | AGGVGKSALTIQLIQNHF VDEYDPTIEDSYRKQVVI DGETCLLDILDTAGREEY SAMRDQYMRTGEGFLC VFAINNSKSFADINLYRE QIKRVKDSDDVPM | ILDTAGREEY (A01.01) | BLCA, CRC, LUSC, PAAD, PRAD, SKCM, THCA, UCEC |
| PIK3CA | E542K | IEEHANWSVSREAGFSYS HAGLSNRLARDNELREN DKEQLKAISTRDPLSKITE QEKDFLWSHRHYCVTIP EILPKLLLSVKWNSRDEV AQMYCLVKDWPP | AISTRDPLSK (A03.01) | BLCA, BRCA, CESC, CRC, GBM, HNSC, KIRC, KIRP, LIHC, LUAD, LUSC, PRAD, UCEC |
| PTEN | R130Q | KFNCRVAQYPFEDHNPP QLELIKPFCEDLDQWLSE DDNHVAAIHCKAGKGQT GVMICAYLLHRGKFLKA QEALDFYGEVRTRDKKG VTIPSQRRYVYYYSY | QTGVMICAY (A01.01) | BRCA, CESC, CRC, GBM, KIRC, LUSC, UCEC |
| RAC1 | P29S | MQAIKCVVVGDGAVGK TCLLISYTTNAFSGEYIPT VFDNYSANVMVDGKPV NLGLWDTAGQEDYDRL RPLSYPQTVGET | FSGEYIPTV (A02.01) TTNAFSGEY (A01.01) YTTNAFSGEY (A01.01) | Melanoma |
| SF3B 1 | K700E | AVCKSKKSWQARHTGIK IVQQIAILMGCAILPHLRS LVEIIEHGLVDEQQEVRTI SALAIAALAEAATPYGIE SFDSVLKPLWKGIRQHR GKGLAAFLKAI | GLVDEQQEV (A02.01) | AML associated with MDS; Chronic lymphocytic leukaemia-small lympho-cytic lymphoma; Myelo-dysplastic syndrome; AML; Luminal NS carcinoma of breast; Chronic myeloid leukaemia; Ductal carcinoma of pancreas; Chronic myelomonocytic leukaemia; Chronic lymphocytic leukaemia-small lymphocytic lymphoma; Myelofibrosis; Myelodys-plastic syndrome; PRAD; Essential thrombocythae-mia; Medullomyoblastoma |

(continued)

| Gene | Exemplary Protein Change | Mutation Sequence Context | Peptides (HLA allele example(s)) | Exemplary Diseases |
|---|---|---|---|---|
| Table 2A | | POINT MUTATIONS [1] | | |
| SPOP | F133L | YLSLYLLLVSCPKSEVRA KFKFSILNAKGEETKAME SQRAYRFVQGKDWG_L_K KFIRRDFLLDEANGLLPD DKLTLFCEVSVVQDSVNI SGQNTMNMVKVPE | FVQGKDWGL (A02.01, B08.01) | PRAD |
| SPOP | F133V | YLSLYLLLVSCPKSEVRA KFKFSILNAKGEETKAME SQRAYRFVQGKDWG_V_K KFIRRDFLLDEANGLLPD DKLTLFCEVSVVQDSVNI SGQNTMNMVKVPE | FVQGKDWGV (A02.01) | PRAD |
| TP53 | G245S | IRVEGNLRVEYLDDRNTF RHSVVVPYEPPEVGSDCT TIHYNYMCNSSCMG_S_MN RRPILTIITLEDSSGNLLG RNSFEVRVCACPGRDRR TEEENLRKKGEP | CMGSMNRRPI (A02.01, B08.01) GSMNRRPIL (B08.01) MGSMNRRPI (B08.01) MGSMNRRPIL (B08.01) SMNRRPILTI (A02.01, A24.02, B08.01) | BLCA, BRCA, CRC, GBM, HNSC, LUSC, PAAD, PRAD |
| TP53 | R248Q | EGNLRVEYLDDRNTFRH SVVVPYEPPEVGSDCTTI HYNYMCNSSCMGG_MNQ_ RPILTIITLEDSSGNLLGR NSFEVRVCACPGRDRRT EEENLRKKGEPHHE | CMGGMNQRPI (A02.01, B08.01) GMNQRPILTI (A02.01, B08.01) NQRPILTII (A02.01, B08.01) | BLCA, BRCA, CRC, GBM, HNSC, KIRC, LIHC, LUSC, PAAD, PRAD, UCEC |
| TP53 | R248W | EGNLRVEYLDDRNTFRH SVVVPYEPPEVGSDCTTI HYNYMCNSSCMGG_MN_ _W_RPILTIITLEDSSGNLLG RNSFEVRVCACPGRDRR TEEENLRKKGEPHHE | CMGGMNWRPI (A02.01, A24.02, B08.01) GMNWRPILTI (A02.01, B08.01) MNWRPILTI (A02.01, A24.02, B08.01) MNWRPILTII (A02.01, A24.02) | BLCA, BRCA, CRC, GBM, HNSC, LIHC, LUSC, PAAD, SKCM, UCEC |
| TP53 | R273C | PEVGSDCTTIHYNYMCN SSCMGGMNRRPILTIITLE DSSGNLLGRNSFEV_C_VC ACPGRDRRTEEENLRKK GEPHHELPPGSTKRALPN NTSSSPQPKKKPL | NSFEVCVCA (A02.01) | BLCA, BRCA, CRC, GBM, HNSC, LUSC, PAAD, UCEC |

(continued)

| Gene | Exemplary Protein Change | Mutation Sequence Context | Peptides (HLA allele example(s)) | Exemplary Diseases |
|---|---|---|---|---|
| Table 2A | | POINT MUTATIONS [1] | | |
| TP53 | R273H | PEVGSDCTTIHYNYMCN SSCMGGMNRRPILTIITLE DSSGNLLGRNSFEV<u>H</u>VC ACPGRDRRTEEENLRKK GEPHHELPPGSTKRALPN NTSSSPQPKKKPL | NSFEVHVCA (A02.01) | BRCA, CRC, GBM, HNSC, LIHC, LUSC, PAAD, UCEC |
| TP53 | Y220C | TEVVRRCPHHERCSDSD GLAPPQHLIRVEGNLRVE YLDDRNTFRHSVVVP<u>C</u>E PPEVGSDCTTIHYNYMC NSSCMGGMNRRPILTIIT LEDSSGNLLGRNSF | VVPCEPPEV (A02.01) VVVPCEPPEV (A02.01) | BLCA, BRCA, GBM, HNSC, LIHC, LUAD, LUSC, PAAD, SKCM, UCEC |
| Table 2B | | MSI-ASSOCIATED FRAMESHIFTS [1] | | |
| MSH6 | F1088fs; +1 | YNFDKNYKDWQSAVECI AVLDVLLCLANYSRGGD GPMCRPVILLPEDTPP<u>LL</u> RA | ILLPEDTPPL (A02.01) LLPEDTPPL (A02.01) | MSI+ CRC, MSI+ Uterine/Endometrium Cancer, MSI+ Stomach Cancer, Lynch syndrome |
| Table 2C Gene | Exemplary Protein Change | FRAMESHIFT [1] Mutation Sequence Context | Peptides (HLA allele example(s)) | Exemplary Diseases |
| | | <u>AKFQQCHSTLEPNPADC RVLVYLQNQPGTKLLNF LQERNLPPKVVLRHPKV HLNTMFRRPHSCLADVL LSVHLIVLRVVRLPAPFR VNHAVEW</u>* | APFRVNHAV (B07.02)<br><br>CLADVLLSV (A02.01)<br>FLQERNLPPK (A03.01)<br>HLIVLRVVRL (A02.01, B08.01)<br>HPKVHLNTM (B07.02, B08.01)<br>HPKVHLNTMF (B07.02, B08.01)<br>KVHLNTMFR (A03.01)<br>KVHLNTMFRR (A03.01)<br>LPAPFRVNHA (B07.02)<br>MFRRPHSCL (B07.02, B08.01)<br>MFRRPHSCLA (B08.01) | CRC, LUAD, UCEC, STAD |

(continued)

| Table 2C Gene | Exemplary Protein Change | FRAMESHIFT [1] Mutation Sequence Context | Peptides (HLA allele example(s)) | Exemplary Diseases |
|---|---|---|---|---|
| APC | F1354fs | | NTMFRRPHSC (B08.01) RPHSCLADV (B07.02) RPHSCLADVL (B07.02) RVVRLPAPFR (A03.01) SVHLIVLRV (A02.01) TMFRRPHSC (B08.01) TMFRRPHSCL (A02.01, B08.01) VLLSVHLIV (A02.01) VLLSVHLIVL (A02.01) VLRVVRLPA (B08.01) VVRLPAPFR (A03.01) | |

| Gene | Exemplary Protein Change | Mutation Sequence Context | Peptides (HLA allele example(s)) | Exemplary Diseases |
|---|---|---|---|---|
| ARID1A | Y1324fs | ALGPHSRISCLPTQTRGCILLAATPRSSSSSSSSNDMIPMAISSPPKAPLLAAPSPASRLQCINSNSRITSGQWMAHMALLPSGTKGRCTACHTALGRGSLSSSSCPQPSPSLPASNKLPSLPLSKMYTTSMAMPILPLPQLLLLSADQQAAPRTNFHSSLAETVSLHPLAPMPSKTCHHK* | AMPILPLPQL (A02.01) APLLAAPSPA (B07.02) APRTNFHSS (B07.02) APRTNFHSSL (B07.02, B08.01) CPQPSPSLPA (B07.02) GQWMAHMAL (A02.01) GQWMAHMALL (A02.01) HMALLPSGTK (A03.01) HTALGRGSL (B07.02) IPMAISSPP (B07.02) IPMAISSPPK (B07.02) KLPSLPLSK (A03.01) | STAD, UCEC, BLCA, BRCA, LUSC, CESC, KIRC, UCS |

(continued)

| Gene | Exemplary Protein Change | Mutation Sequence Context | Peptides (HLA allele example(s)) | Exemplary Diseases |
|---|---|---|---|---|
| | | | KLPSLPLSKM (A02.01) | |
| | | | KMYTTSMAM (A02.01, A03.01) | |
| | | | LLAAPSPASR (A03.01) | |
| | | | LLLSADQQAA (A02.01) | |
| | | | LLSADQQAA (A02.01) | |
| | | | LPASNKLPS (B07.02) | |
| | | | LPASNKLPSL (B07.02, B08.01) | |
| | | | LPLPQLLLSA (B07.02) | |
| | | | LPSLPLSKM (B07.02) | |
| | | | LSKMYTTSM (B08.01) | |
| | | | MALLPSGTK (A03.01) | |
| | | | MPILPLPQL (B07.02) | |
| | | | MPILPLPQLL (B07.02) | |
| | | | MYTTSMAMPI (A24.02) | |
| | | | PMAISSPPK (A03.01) | |
| | | | QWMAHMALL (A24.02) | |
| | | | SKMYTTSMAM (B07.02) | |
| | | | SMAMPILPL (A02.01, B07.02, B08.01) | |
| | | | SNKLPSLPL (B08.01) | |
| | | | SPASRLQCI (B07.02, B08.01) | |
| | | | SPPKAPLLAA (B07.02) | |
| | | | SPSLPASNKL (B07.02) | |
| | | | YTTSMAMPI (A02.01) | |
| | | | YTTSMAMPIL (A02.01) | |

133

(continued)

| Gene | Exemplary Protein Change | Mutation Sequence Context | Peptides (HLA allele example(s)) | Exemplary Diseases |
|---|---|---|---|---|
| ARID1A | G1848fs | RSYRRMIHLWWTAQISL GVCRSLTVACCTGGLVG GTPLSISRPTSRARQSCCL PGLTHPAHQPLGSM* | CLPGLTHPA (A02.01) GLTHPAHQPL (A02.01) HPAHQPLGSM (B07.02) LTHPAHQPL (B07.02) RPTSRARQSC (B07.02) RQSCCLPGL (A02.01) TSRARQSCCL (B08.01) | STAD, UCEC, BLCA, BRCA, LUSC, CESC, KIRC, UCS |
| β2M | L13fs | QHSGRDVSLRGLSCARA TLSFWPGGYPAYSKDSG LLTSSSREWKVKFPELLC VWVSSIRH* | ELLCVWVSSI (A02.01) EWKVKFPEL (B08.01) KFPELLCVW (A24.02) LLCVWVSSI (A02.01) LLTSSSREWK (A03.01) LTSSSREWK (A03.01) YPAYSKDSGL (B07.02) | CRC, STAD, SKCM, HNSC |
| GATA3 | L328fs N334fs | AQAKAVCSQESRDVLCE LSDHHNHTLEEECQWGP CLQCLWALLQASQY* | CLQCLWALL (A02.01) CQWGPCLQCL (A02.01) QWGPCLQCL (A24.02) QWGPCLQCLW (A24.02) | Breast Cancer |
| GATA3 | | PGRPLQTHVLPEPHLALQ PLQPHADHAHADAPAIQ PVLWTTPPLQHGHRHGL EPCSMLTGPPARVPAVPF DLHFCRSSIMKPKRDGY MFLKAESKIMFATLQRSS LWCLCSNH* | AIQPVLWTT (A02.01) ALQPLQPHA (A02.01) DLHFCRSSIM (B08.01) EPHLALQPL (B07.02, B08.01) ESKIMFATL (B08.01) FATLQRSSL (B07.02, B08.01) | Breast Cancer |

(continued)

| Gene | Exemplary Protein Change | Mutation Sequence Context | Peptides (HLA allele example(s)) | Exemplary Diseases |
|---|---|---|---|---|
| | | | FLKAESKIM (B08.01) | |
| | | | FLKAESKIMF (B08.01) | |
| | | | GPPARVPAV (B07.02) | |
| | | | IMKPKRDGYM (B08.01) | |
| | H400fs | | KIMFATLQR (A03.01) | |
| | S408fs | | KPKRDGYMF (B07.02) | |
| | S408fs | | KPKRDGYMFL (B07.02) | |
| | S430fs | | LHFCRSSIM (B08.01) | |
| | H434fs | | LQHGHRHGL (B08.01) | |
| | | | MFATLQRSSL (B07.02, B08.01) | |
| | | | MFLKAESKI (A24.02) | |
| | | | MLTGPPARV (A02.01) | |
| | | | QPVLWTTPPL (B07.02) | |
| | | | SMLTGPPARV (A02.01) | |
| | H435fs | | TLQRSSLWCL (A02.01) | |
| | | | VLPEPHLAL (A02.01) | |
| | | | VPAVPFDLHF (B07.02) | |
| | | | YMFLKAESK (A03.01) | |
| | | | YMFLKAESKI (A02.01, A03.01, A24.02, B08.01) | |

(continued)

| Gene | Exemplary Protein Change | Mutation Sequence Context | Peptides (HLA allele example(s)) | Exemplary Diseases |
|---|---|---|---|---|
| MLL2 | | TRRCHCCPHLRSHPCPHH LRNHPRPHHLRHHACHH HLRNCPHPHFLRHCTCPG RWRNRPSLRRLRSLLCLP HLNHHLFLHWRSRPCLH RKSHPHLLHLRRLYPHH LKHRPCPHHLKNLLCPR HLRNCPLPRHLKHLACL HHLRSHPCPLHLKSHPCL HHRRHLVCSHHLKSLLC PLHLRSLPFPHHLRHHAC PHHLRTRLCPHHLKNHL CPPHLRYRAYPPCLWCH ACLHRLRNLPCPHRLRSL —————————— PRPLHLRLHASPHHLRTP PHPHHLRTHLLPHHRRTR SCPCRWRSHPCCHYLRS RNSAPGPRGRTCHPGLRS —————————— RTCPPGLRSHTYLRRLRS HTCPPSLRSHAYALCLRS HTCPPRLRDHICPLSLRN CTCPPRLRSRTCLLCLRS HACPPNLRNHTCPPSLRS HACPPGLRNRICPLSLRS HPCPLGLKSPLRSQANAL —————————— HLRSCPCSLPLGNHPYLP CLESQPCLSLGNHLCPLC PRSCRCPHLGSHPCRLS* | APGPRGRTC (B07.02) CLRSHTCPPR (A03.01) CLWCHACLHR (A03.01) CPHLGSHPC (B07.02) CPLGLKSPL (B07.02) CPRSCRCPH (B07.02) CPRSCRCPHL (B07.02, B08.01) CSLPLGNHPY (A01.01) GLRNRICPL (A02.01, B07.02, B08.01) GLRSHTYLR (A03.01) GLRSHTYLRR (A03.01) GPRGRTCHPG (B07.02) HT GSHPCRL (B08.01) HLRLHASPH (A03.01) | STAD, BLCA, CRC, HNSC, BRCA |

(continued)

| Gene | Exemplary Protein Change | Mutation Sequence Context | Peptides (HLA allele example(s)) | Exemplary Diseases |
|---|---|---|---|---|
| | P647fs | | HLRSCPCSL (B07.02, B08.01) HLRTHLLPH (A03.01) HT RTHLLPHH (A03.01) HLRYRAYFP (B08.01) HLRYRAYPPC (B08.01) HPHHLRTHL (B07.02) HPHHLRTHLL (B07.02, B08.01) HTYLRRLRSH (A03.01) LPCPHRLRSL (B07.02, B08.01) T PHHRRTRSC (B07.02, B08.01) LPLGNHPYL (B07.02) LPRPLHLRL (B07.02, B08.01) NLRNHTCPP (B08.01) PPRLRSRTCL (B07.02, B08.01) RLHASPHHL (A02.01) RLHASPHHLR (A03.01) RLRDHICPL (A02.01, B07.02, | |
| | L656fs | | | |
| | | | B08.01) RLRNLPCPH (A03.01) RLRNLPCPHR (A03.01) RLRSHTCPP (B08.01) RLRSLPRPL (B07.02, B08.01) RLRSLPRPLH (A03.01) RLRSRTCLL (B07.02, B08.01) RNRICPLSL (B07.02, B08.01) | |

(continued)

| Gene | Exemplary Protein Change | Mutation Sequence Context | Peptides (HLA allele example(s)) | Exemplary Diseases |
|---|---|---|---|---|
| | | | RPLHLRLHA (B07.02) | |
| | | | RPLHLRLHAS (B07.02) | |
| | | | RSHACPPGLR (A03.01) | |
| | | | RSHACPPNLR (A03.01) | |
| | | | RSHAYALCLR (A03.01) | |
| | | | RSHPCCHYLR (A03.01) | |
| | | | RSHPCPLGLK (A03.01) | |
| | | | RSHTCPPSLR (A03.01) | |
| | | | RSLPRPLHLR (A03.01) | |
| | | | RSRTCLLCL (B07.02) | |
| | | | RSRTCLLCLR (A03.01) | |
| | | | RSRTCPPGL (B07.02) | |
| | | | RSRTCPPGLR (A03.01) | |
| | | | RTHLLPHHRR (A03.01) | |
| | | | RTRSCPCRWR (A03.01) | |
| | | | RYRAYPPCL (A24.02) | |
| | | | RYRAYPPCLW (A24.02) | |
| | | | SLGNHLCPL (A02.01, B07.02, B08.01) | |
| | | | SLPLGNHPYL (A02.01) | |
| | | | SLPRPLHLRL (A02.01) | |
| | | | SLRNCTCPPR (A03.01) | |
| | | | SLRSHAYAL (A02.01, B07.02, B08.01) | |
| | | | SLRSHPCPL (A02.01, B07.02, B08.01) | |
| | | | SPHHLRTPP (B07.02) | |

138

(continued)

| Gene | Exemplary Protein Change | Mutation Sequence Context | Peptides (HLA allele example(s)) | Exemplary Diseases |
|---|---|---|---|---|
| | | | SPHHLRTPPH (B07.02) SPLRSQANAL (B07.02, B08.01) YLRRLRSHTC (B08.01) YLRSRNSAP (B08.01) YLRSRNSAPG (B08.01) | |
| MLL2 | P2354fs | GPRSHPLPRLWHLLLQV TQTSFALAPTLTHMLSPH * | ALAPTLTHM (A02.01) ALAPTLTHML (A02.01) LLQVTQTSFA (A02.01) LQVTQTSFAL (A02.01) RLWHLLLQV (A02.01) RLWHLLLQVT (A02.01) | STAD, BLCA, CRC, HNSC, BRCA |
| RNF43 | G659fs | PLGLVPWTRWCPQGKPR FPAMSTTTATGTTTTKSG SSGMAGSLAQKPESPSPG LLFLGHSPSQSHLLLISKS PDPTQQPLRGGSLTHSAP GPSLSQPLAQLTPPASAP VPAVCSTCKNPASLPDTH RGKGGGVPPSPPLALGPR MQLCTQLARFFPITPPVW HILGPQRHTP* | CTQLARFFPI (A24.02) FFPITPPVW (A24.02) FPITPPVWHI (B07.02) GPRMQLCTQL (B07.02, B08.01) ITPPVWHIL (A24.02) LALGPRMQL (B07.02) MQLCTQLARF (A24.02) RFFPITPPV (A02.01, A24.02) RFFPITPPVW (A24.02) RMQLCTQLA (A02.01) RMQLCTQLAR (A03.01) SPPLALGPRM (B07.02) TQLARFFPI (A02.01, A24.02, B08.01) | STAD |

(continued)

| Gene | Exemplary Protein Change | Mutation Sequence Context | Peptides (HLA allele example(s)) | Exemplary Diseases |
|---|---|---|---|---|
| SMAP1 | E169fs | KYEKKKYYDKNAIAITNI SSSDAPLQPLVSSPSLQA AVDKNKLEKEKEKKRKR KREKRSQKSRQNHLQLK SCRRKISNWSLKKVPAL KKLRSPLWIF | KSRQNHLQL (B07.02) ALKKLRSPL (B08.01, B07.02) HLQLKSCRRK (A03.01) KISNWSLKK (A03.01, A11.01) KISNWSLKKV (A03.01) KLRSPLWIF (A24.02) KSRQNHLQLK (A03.01) NWSLKKVPAL (B08.01) SLKKVPALK (A03.01, A11.01) SLKKVPALKK (A03.01) SQKSRQNHL (B08.01) WSLKKVPAL (B08.01) WSLKKVPALK (A03.01) | MSI+ CRC, MSI+ Uterine/Endometrium Cancer, MSI+ Stomach Cancer |
| TP53 | P58fs P72fs G108fs R110fs | CCPRTILNNGSLKTQVQ MKLPECQRLLPPWPLHQ QLLHRRPLHQPPPGPCHL LSLPRKPTRAATVSVWA SCILGQPSL* | KLPECQRLL (A02.01) KPTRAATVSV (B07.02) LPPWPLHQQL (B07.02) LPRKPTRAA (B07.02, B08.01) LPRKPTRAAT (B07.02) QQLLHRRPL (B08.01) RLLPPWPLH (A03.01) | BRCA, CRC, LUAD, PRAD, HNSC, LUSC, PAAD, STAD, BLCA, OV, LIHC, SKCM, UCEC, LAML, UCS, KICH, GBM, ACC |
| TP53 | P152fs | LARTPLPSTRCFANWPRP ALCSCGLIPHPRPAPASA PWPSTSSHST* | APASAPWPST (B07.02) APWPSTSSH (B07.02) RPAPASAPW (B07.02) WPSTSSHST (B07.02) | BRCA, CRC, LUAD, PRAD, HNSC, LUSC, PAAD, STAD, BLCA, OV, LIHC, SKCM, UCEC, LAML, UCS, KICH, GBM, ACC |

(continued)

| Gene | Exemplary Protein Change | Mutation Sequence Context | Peptides (HLA allele example(s)) | Exemplary Diseases |
|---|---|---|---|---|
| UBR5 | K2120fs | SQGLYSSSASSGKCLME VTVDRNCLEVLPTKMSY AANLKNVMNMQNRQKK KGKNSPCCQKKLRVQNQ GHLLMILLHN* | RVQNQGHLL (B07.02) | |
| VHL | L116fs<br><br>G123fs | TRASPPRSSSAIAVRASC CPYGSTSTASRSPTQRCR LARAAASTATEVTFGSSE MQGHTMGFWLTKLNYL CHLSMLTDSLFLPISHCQ CIL* | FLPISHCQCI (A02.01)<br>FWLTKLNYL (A24.02, B08.01)<br>HLSMLTDSL (A02.01)<br>HTMGFWLTK (A03.01)<br>HTMGFWLTKL (A02.01)<br>KLNYLCHLSM (A02.01)<br>LPISHCQCI (B07.02, B08.01)<br>LPISHCQCIL (B07.02, B08.01)<br>LTDSLFLPI (A01.01, A02.01)<br>LTKLNYLCHL (B08.01)<br>MLTDSLFLPI (A01.01, A02.01, B08.01)<br>MQGHTMGFWL (A02.01)<br>NYLCHLSML (A24.02)<br>SMLTDSLFL (A02.01)<br>TMGFWLTKL (A02.01)<br>YLCHLSMLT (A02.01) | KIRC, KIRP |
| TABLE2D | | INSERT [1] | | |
| HER2 | G776insYVMA | LGSGAFGTVYKGIWIPDG ENVKIPVAIKVLRENTSP KANKEILDEAYVMAYV MAGVGSPYVSRLLGICLT STVQLVTQLMPYGCLLD HVRENRGRLGSQDLLNW | ILDEAYVMAY (A01.01)<br>VMAYVMAGV (A02.01)<br>YVMAYVMAG (A02.01, B07.02, B08.01) | Lung Cancer |

(continued)

| Gene | Exemplary Protein Change | Mutation Sequence Context | Peptides (HLA allele example(s)) | Exemplary Diseases |
|---|---|---|---|---|
| | | | YVMAYVMAGV (A02.01, B07.02, B08.01) | |

[1] Underlined AAs represent non-native AAs
[2] Bolded AAs represent native AAs of the amino acid sequence encoded by the second of the two fused genes
[3] Bolded and underlined AAs represent non-native AAs of the amino acid sequence encoded by the second of the two fused genes due to a framshift.

In the Tables above, for one or more of the exemplary fusions, a sequence that comes before the first ":" belongs to an exon sequence of a polypeptide encoded by a first gene, a sequence that comes after the second ":" belongs to an exon sequence of a polypeptide encoded by a second gene, and an amino acid that appears between ":" symbols is encoded by a codon that is split between the exon sequence of a polypeptide encoded by a first gene and the exon sequence of a polypeptide encoded by a second gene.

However, in some cases, for example, NAB:STAT6, the NAB exon is linked to the 5' UTR of STAT6 and the first amino acid that appears after the junction is the normal start codon of STAT6 (there is no frame present at this site (as it is not normally translated)).

AR-V7 in the tables above can also be considered, in some cases, a splice variant of the AR gene that encodes a protein that lacks the ligand binding domain found in full length AR.

## Example 2: HLA Class I and Class II Binding Assays

The following example of peptide binding to HLA molecules demonstrates quantification of binding affinities of HLA class I and class II peptides. Binding assays can be performed with peptides that are either motif-bearing or not motif-bearing.

Epstein-Barr virus (EBV)-transformed homozygous cell lines, fibroblasts, CIR, or 721.22 transfectants are used as sources of HLA class I molecules. Cell lysates are prepared and HLA molecules purified in accordance with disclosed protocols (Sidney et al., Current Protocols in Immunology 18.3.1 (1998); Sidney, et al., J. Immunol. 154:247 (1995); Sette, et al., Mol. Immunol. 31:813 (1994)). HLA molecules are purified from lysates by affinity chromatography. The lysates are passed over a column of Sepharose CL-4B beads coupled to an appropriate antibody. The anti-HLA column is then washed with 10 mM Tris-HCL, pH 8.0, in 1% NP-40, PBS, and PBS containing 0.4% n-octylglucoside and HLA molecules are eluted with 50 mM diethylamine in 0.15 M NaCl containing 0.4% n-octylglucoside, pH 11.5. A 1/25 volume of 2.0 M Tris, pH 6.8, is added to the eluate to reduce the pH to ~8.0. Eluates are then concentrated by centrifugation in Centriprep 30 concentrators (Amicon, Beverly, MA). Protein content is evaluated by a BCA protein assay (Pierce Chemical Co., Rockford, IL) and confirmed by SDS-PAGE.

A detailed description of the protocol utilized to measure the binding affinity of peptides to Class I and Class II MHC has been published (Sette et al., Mol. Immunol. 31:813, 1994; Sidney et al., in Current Protocols in Immunology, Margulies, Ed., John Wiley & Sons, New York, Section 18.3, 1998). Briefly, purified MHC molecules (5 to 500 nM) are incubated with various unlabeled peptide inhibitors and 1-10 nM $^{125}$I-radiolabeled probe peptides for 48 h in PBS containing 0.05% Nonidet P-40 (NP40) (or 20% w/v digitonin for H-2 IA assays) in the presence of a protease inhibitor cocktail. All assays are at pH 7.0 with the exception of DRB1*0301, which was performed at pH 4.5, and DRB1*1601 (DR2w21β1) and DRB4*0101 (DRw53), which were performed at pH 5.0.

Following incubation, MHC-peptide complexes are separated from free peptide by gel filtration on 7.8 mm x 15 cm TSK200 columns (TosoHaas 16215, Montgomeryville, PA). Because the large size of the radiolabeled peptide used for the DRB1*1501 (DR2w2β1) assay makes separation of bound from unbound peaks more difficult under these conditions, all DRB1*1501 (DR2w2β1) assays were performed using a 7.8 mm x 30 cm TSK2000 column eluted at 0.6 mLs/min. The eluate from the TSK columns is passed through a Beckman 170 radioisotope detector, and radioactivity is plotted and integrated using a Hewlett-Packard 3396A integrator, and the fraction of peptide bound is determined.

Radiolabeled peptides are iodinated using the chloramine-T method. Typically, in preliminary experiments, each MHC preparation is titered in the presence of fixed amounts of radiolabeled peptides to determine the concentration of HLA molecules necessary to bind 10-20% of the total radioactivity. All subsequent inhibition and direct binding assays are performed using these HLA concentrations.

Since under these conditions [label] < [HLA] and $IC_{50} \geq$ [HLA], the measured $IC_{50}$ values are reasonable

approximations of the true $K_D$ values. Peptide inhibitors are typically tested at concentrations ranging from 120 μg/ml to 1.2 ng/ml, and are tested in two to four completely independent experiments. To allow comparison of the data obtained in different experiments, a relative binding figure is calculated for each peptide by dividing the $IC_{50}$ of a positive control for inhibition by the $IC_{50}$ for each tested peptide (typically unlabeled versions of the radiolabeled probe peptide). For database purposes, and inter-experiment comparisons, relative binding values are compiled. These values can subsequently be converted back into $IC_{50}$ nM values by dividing the $IC_{50}$ nM of the positive controls for inhibition by the relative binding of the peptide of interest. This method of data compilation has proven to be the most accurate and consistent for comparing peptides that have been tested on different days, or with different lots of purified MHC.

Because the antibody used for HLA-DR purification (LB3.1) is α-chain specific, β1 molecules are not separated from β3 (and/or β4 and β5) molecules. The β1 specificity of the binding assay is obvious in the cases of DRB1*0101 (DR1), DRB1*0802 (DR8w2), and DRB1*0803 (DR8w3), where no β3 is expressed. It has also been demonstrated for DRB1*0301 (DR3) and DRB3*0101 (DR52a), DRB1*0401 (DR4w4), DRB1*0404 (DR4w14), DRB1*0405 (DR4w15), DRB1*1101 (DRS), DRB1*1201 (DR5w12), DRB1*1302 (DR6w19) and DRB1*0701 (DR7). The problem of β chain specificity for DRB1* 1501 (DR2w2β1), DRB5*0101 (DR2w2β2), DRB1*1601 (DR2w21β1), DRB5*0201 (DR51Dw21), and DRB4*0101 (DRw53) assays is circumvented by the use of fibroblasts. Development and validation of assays with regard to DRβ molecule specificity have been described previously (see, e.g., Southwood et al., J. Immunol. 160:3363-3373, 1998).

A detailed description of the protocol utilized to measure the binding stability of peptides to Class I MHC has been published (Harndahl et al. J Immunol Methods. 374:5-12, 2011). Briefly, synthetic genes encoding biotinylated MHC-I heavy and light chains are expressed in E. coli and purified from inclusion bodies using standard methods. The light chain (β2m) is radio-labeled with iodine ($^{125}$I), and combined with the purified MHC-I heavy chain and peptide of interest at 18°C to initiate pMHC-I complex formation. These reactions are carried out in streptavidin coated microplates to bind th biotinylated MHC-I heavy chains to the surface and allow measurement of radiolabeled light chain to monitor complex formation. Dissociation is initiated by addition of higher concentrations of unlabled light-chain and incubation at 37°C. Stability is defined as the length of time in hours it takes for half of the complexes to dissociate, as measured by scintillation counts

Live cell/flow cytometry-based assays can also be used, e.g., an assay utilizing a TAP-deficient hybridoma cell line T2 (American Type Culture Collection (ATCC Accession No. CRL-1992), Manassas, Va.). TAP deficiency in this cell line leads to inefficient loading of MHCI in the ER and an excess of empty MHCIs. Salter and Cresswell, EMBO J. 5:943-49 (1986); Salter, Immunogenetics 21:235-46 (1985). Empty MHCIs are highly unstable and short-lived. When T2 cells are cultured at reduced temperatures, empty MHCIs appear transiently on the cell surface, where they can be stabilized by exogenous addition of MHCI-binding peptides. To perform this binding assay, peptide-receptive MHCIs were induced by culturing aliquots of $10^7$ T2 cells overnight at 26 °C in serum free AIM-V medium alone, or in medium containing escalating concentrations (0.1 to 100 μM) of peptide. Cells were then washed twice with PBS, and subsequently incubated with a fluorescent tagged HLA-A0201-specific monoclonal antibody, BB7.2, to quantify cell surface expression. Samples were acquired on a FACS Calibur instrument (Becton Dickinson) and the mean fluorescence intensity (MFI) determined using the accompanying Cellquest software.

## Example 3: HLA Class I Binding Affinity

HLA binding assays were used to evaluate either the affinity of interaction of a given epitope with a selected HLA molecule or the stability. Each of these measurements was, e.g., indicative of the ability of an epitope to elicit a CD8+ T cell response.

A subset of peptides from Table 1 (n=562) were synthesized and their affinity for their given HLA class I molecule was measured as described. The values are shown in Table 3. The measurements are plotted against the predicted affinity in Figure 1. These data show a strong correlation between prediction and measurement (dotted line represents best fit, $R^2$ = 0.45), demonstrating the value of the predictions. However, the outliers demonstrate the importance of these measurements. Thick vertical and horizontal lines are shown at 500 nM for the predicted affinity and observed affinity, respectively. 500nM is commonly accepted in the field as the maximum affinity for an epitope that is a "weak binder" to HLA class I. Therefore, the points in the lower right quadrant (prediction greater than 500 nM, measurement less than 500 nM) are epitopes that were considered very weak binders but were observed to bind within an acceptable range. Epitopes in this quadrant (n=75) represent 30.5% of epitopes not considered to be binders by prediction (combination of bottom right and top right quadrants, n=246).

### Table 3: HLA Class I Binding Affinity and Stability

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| ABL1, M351T | A02.01 | TQISSATEYL | 2921.0 | 2644.0 | 0 |
| ABL1, T315I | A02.01 | YIIIEFMTYG | 3502.0 | 186.0 | 0 |
| ABL1, T315I | A02.01 | IIIEFMTYG | 1991.0 | 779.0 | 0 |
| ABL1, T315I | A02.01 | IIIEFMTYGN | 16793.0 | 1551.0 | 0 |
| ABL1, T315I | A02.01 | IIEFMTYGNL | 2134.0 | 9702.0 | 0 |
| ABL1, Y253H | A02.01 | KLGGGQHGEV | 1705.0 | 387.0 | 0.4 |
| AKT1, E17K | B08.01 | WLHKRGKYI | 47.0 | 417.0 | 1.3 |
| AKT1, E17K | A02.01 | WLHKRGKYI | 4972.0 | 1250.0 | 1.2 |
| AKT1, E17K | B07.02 | WLHKRGKYI | 7185.0 | 2648.0 | 0 |
| ALK, G1269A | A02.01 | RVAKIADFGM | 5258.0 | 125.0 | 0.5 |
| ALK, G1269A | B07.02 | RVAKIADFGM | 7260.0 | 9723.0 | 0.2 |
| ALK, L1196M | A02.01 | SLPRFILMEL | 94.0 | 26.0 | 0.5 |
| ALK, L1196M | A02.01 | ILMELMAGG | 192.0 | 223.0 | 0.5 |
| ALK, L1196M | A02.01 | LMELMAGGDL | 5617.0 | 311.0 | 8.9 |
| ALK, L1196M | A02.01 | LQSLPRFILM | 2519.0 | 413.0 | 0 |
| ALK, L1196M | B07.02 | SLPRFILMEL | 17.0 | 583.0 | 0.4 |
| ALK, L1196M | B08.01 | LQSLPRFILM | 1288.0 | 1547.0 | 0 |
| ALK, L1196M | A02.01 | FILMELMAGG | 189.0 | 1580.0 | 0 |
| ALK, L1196M | B08.01 | SLPRFILMEL | 686.0 | 1762.0 | 0 |
| ALK, L1196M | A24.02 | SLPRFILMEL | 5143.0 | 2774.0 | 0.2 |
| ALK, L1196M | A02.01 | ILMELMAGGD | 5761.0 | 3451.0 | 0 |
| APC, AVEW | A02.01 | VLLSVHLIV | 36.0 | 72.0 | 11 |
| APC, AVEW | A02.01 | CLADVLLSV | 5.0 | 219.0 | 24 |
| APC, VHPA | A02.01 | KVLQMDFLV | 25.0 | 11.0 | 6.4 |
| APC, VHPA | A02.01 | LQMDFLVHPA | 26.0 | 68.0 | 1.5 |
| β2M, ...MPAV | A03.01 | TTLNSPPLKK | 62.5 | 14.3 | not measured |
| β2M, ...MPAV | A03.01 | TTLNSPPLK | 165.4 | 9.8 | not measured |
| β2M, ...MPAV | A03.01 | TLNSPPLKK | 27.5 | 5.4 | not measured |
| β2M, ...MPAV | A03.01 | CTTLNSPPLK | 225.3 | 63.6 | not measured |
| β2M, ...MPAV | B08.01 | CLSARTGLSI | 1106.6 | 149.6 | not measured |
| β2M, ...MPAV | A02.01 | GLSISCTTL | 669.0 | 114.5 | not measured |
| β2M, ...SIRH | A03.01 | LTSSSREWK | 413.9 | 117.8 | not measured |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| β2M, ...SIRH | A03.01 | LLTSSSREWK | 206.1 | 1769.8 | not measured |
| β2M, ...SIRH | B07.02 | YPAYSKDSGL | 41.1 | 79.5 | not measured |
| β2M, ...SIRH | B08.01 | EWKVKFPEL | 488.7 | 538.4 | not measured |
| β2M, ...SIRH | A24.02 | KFPELLCVW | 83.7 | 13.7 | not measured |
| β2M, ...SQIS | B08.01 | LQRFRFTHV | 55.5 | 37.3 | not measured |
| β2M, ...SQIS | A24.02 | RLSSVLQRF | 288.9 | 28.2 | not measured |
| β2M, ...SQIS | A02.01 | VLQRFRFTHV | 163.4 | 106.7 | not measured |
| β2M, ...SQIS | B08.01 | VLQRFRFTHV | 264.1 | 480.1 | not measured |
| β2M, ...SQIS | A03.01 | RLSSVLQRFR | 168.1 | 12.5 | not measured |
| BCR:ABL (e13a2, aka b2a2) | B08.01 | LTINKEEAL | 4972.0 | 895.0 | 0 |
| BCR:ABL (e13a2, aka b2a2) | A02.01 | LTINKEEAL | 12671.0 | 4413.0 | 0 |
| BRAF, V600E | A02.01 | LATEKSRWSG | 39130.0 | 23337.0 | 0 |
| BRAF, V600E | B08.01 | LATEKSRWS | 24674.0 | 36995.0 | 0 |
| BRAF, V600E | B08.01 | LATEKSRWSG | 13368.0 | 46582.0 | 0 |
| BRAF, V600E | A02.01 | LATEKSRWS | 39109.0 | 60997.0 | 0 |
| BTK, C481S | A02.01 | SLLNYLREM | 48.0 | 87.0 | 3 |
| BTK, C481S | A02.01 | MANGSLLNYL | 2979.0 | 1082.0 | 0 |
| BTK, C481S | B07.02 | SLLNYLREM | 6544.0 | 1110.0 | 0 |
| BTK, C481S | B08.01 | SLLNYLREM | 1091.0 | 1230.0 | 0 |
| BTK, C481S | A02.01 | YMANGSLLN | 7856.0 | 4444.0 | 0 |
| BTK, C481S | B07.02 | MANGSLLNYL | 8921.0 | 17715.0 | 0 |
| BTK, C481S | B08.01 | MANGSLLNYL | 7639.0 | 19853.0 | 0 |
| BTK, C481S | A03.01 | MANGSLLNY | 1030.3 | 35.6 | not measured |
| BTK, C481S | A01.01 | MANGSLLNY | 285.7 | 439.0 | not measured |
| BTK, C481S | A24.02 | EYMANGSLL | 213.2 | 5.0 | not measured |
| BTK, C481S | A01.01 | YMANGSLLNY | 95.7 | 13.2 | not measured |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| BTK, C481S | A03.01 | YMANGSLLNY | 109.4 | 95.9 | not measured |
| C11orf95:RELA | A02.01 | ELFPLIFPA | 13.0 | 13.0 | 5.1 |
| C11orf95:RELA | A24.02 | KGPELFPLI | 909.0 | 14.0 | 1.7 |
| C11orf95:RELA | A02.01 | KGPELFPLI | 6840.0 | 101.0 | 0.3 |
| C11orf95:RELA | B08.01 | ELFPLIFPA | 7316.0 | 449.0 | 0 |
| C15ORF40(+1) | A02.01 | KLFSCLSFL | 6.0 | 6.0 | 14.3 |
| C15ORF40(+1) | A03.01 | KLFSCLSFL | 1488.0 | 308.0 | 0.8 |
| C15ORF40(+1) | A03.01 | SLQPPPPGFK | 26.3 | 19.0 | not measured |
| C15ORF40(+1) | A03.01 | LFFFFFETK | 658.4 | 413.3 | not measured |
| C15ORF40(+1) | A02.01 | ALFFFFFET | 28.9 | 470.7 | not measured |
| C15ORF40(+1) | A03.01 | ALFFFFFETK | 31.5 | 216.4 | not measured |
| C15ORF40(+1) | A02.01 | FFFETKSCSV | 754.5 | 61.2 | not measured |
| C15ORF40(+1) | B08.01 | FFETKSCSV | 807.6 | 7.6 | not measured |
| C15ORF40(+1) | A01.01 | LSFLSSWDY | 211.1 | 52.9 | not measured |
| C15ORF40(+1) | A02.01 | FLSSWDYRRM | 62.2 | 323.5 | not measured |
| C15ORF40(+1) | A03.01 | LSFLSSWDYR | 508.7 | 100.9 | not measured |
| C15ORF40(+1) | A02.01 | FKLFSCLSFL | 9.9 | 662.9 | not measured |
| C15ORF40(+1) | A02.01 | VQWRSLGSL | 986.0 | 4733.2 | not measured |
| C15ORF40(+1) | A02.01 | KLFSCLSFLS | 65.1 | 0.6 | not measured |
| C15ORF40(+1) | A03.01 | KLFSCLSFLS | 805.1 | 104.0 | not measured |
| C15ORF40(+1) | A02.01 | AQAGVQWRSL | 630.2 | 670.0 | not measured |
| C15ORF40(+1) | A24.02 | RRMPPCLANF | 253.0 | 141.1 | not measured |
| C15ORF40(+1) | A03.01 | CLSFLSSWDY | 890.5 | 2705.8 | not measured |
| C15ORF40(+1) | A24.02 | GFKLFSCLSF | 387.4 | 643.0 | not measured |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| C15ORF40(+1) | A24.02 | RMPPCLANF | 34.4 | 8.7 | not measured |
| C15ORF40(+1) | A03.01 | CLANFCIFNR | 575.4 | 221.8 | not measured |
| C15ORF40(+1) | A01.01 | CLSFLSSWDY | 538.7 | 987.3 | not measured |
| CNOT1(+1) | A02.01 | SVCFFFFSV | 27.0 | 175.0 | 9.4 |
| CNOT1(+1) | B08.01 | SVCFFFFSV | 4940.0 | 10599.0 | 0 |
| CNOT1(+1) | A02.01 | MSVCFFFFSV | 131.0 | 1706.4 | not measured |
| CNOT1(+1) | A02.01 | FFFSVIFST | 608.9 | 4556.0 | not measured |
| CNOT1(-1) | A01.01 | MSVCFFFFCY | 310.4 | 4369.3 | not measured |
| CNOT1(-1) | A02.01 | SVCFFFFCYI | 237.2 | 519.8 | not measured |
| CNOT1(-1) | A24.02 | FFCYILNTMF | 583.4 | 73.4 | not measured |
| EGFR, T790M | A02.01 | MQLMPFGCLL | 21.0 | 20.0 | 0.4 |
| EGFR, T790M | A02.01 | MQLMPFGCL | 842.0 | 166.0 | 0.4 |
| EGFR, T790M | A02.01 | LIMQLMPFGC | 1984.0 | 177.0 | 0.4 |
| EGFR, T790M | A02.01 | QLIMQLMPF | 2511.0 | 227.0 | 0.3 |
| EGFR, T790M | B08.01 | QLIMQLMPF | 891.0 | 388.0 | 0 |
| EGFR, T790M | B08.01 | IMQLMPFGCL | 1302.0 | 548.0 | 0 |
| EGFR, T790M | A02.01 | CLTSTVQLIM | 3465.0 | 716.0 | 0 |
| EGFR, T790M | A02.01 | IMQLMPFGCL | 143.0 | 837.0 | 0.5 |
| EGFR, T790M | A02.01 | IMQLMPFGC | 1123.0 | 1607.0 | 0.4 |
| EGFR, T790M | B07.02 | MQLMPFGCL | 10169.0 | 2270.0 | 0 |
| EGFR, T790M | A24.02 | QLIMQLMPF | 3209.0 | 2389.0 | 0.4 |
| EGFR, T790M | A02.01 | LIMQLMPFG | 4961.0 | 3513.0 | 0 |
| EGFR, T790M | A24.02 | VQLIMQLMPF | 1455.0 | 4559.0 | 0 |
| EGFR, T790M | A02.01 | VQLIMQLMPF | 4464.0 | 5492.0 | 0 |
| EGFR, T790M | A02.01 | QLIMQLMPFG | 5751.0 | 5926.0 | 0 |
| EGFR, T790M | B08.01 | MQLMPFGCL | 1105.0 | 7045.0 | 0 |
| EGFR, T790M | A02.01 | STVQLIMQL | 2151.0 | 8537.0 | 0 |
| EGFR, T790M | A01.01 | CLTSTVQLIM | 2998.0 | 11036.0 | 0 |
| EGFR, T790M | B08.01 | MQLMPFGCLL | 970.0 | 14056.0 | 0 |
| EGFR, T790M | B08.01 | VQLIMQLMPF | 3370.0 | 17898.0 | 0 |
| EGFR, T790M | A24.02 | IMQLMPFGCL | 4394.0 | 18102.0 | 0 |
| EGFR, T790M | A24.02 | MQLMPFGCLL | 4168.0 | 23572.0 | 0 |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| EGFR, T790M | A01.01 | LTSTVQLIM | 1000.7 | 2891.1 | not measured |
| EGFR:SEPT14 | B08.01 | QLQDKFEHL | 917.0 | 989.0 | 0 |
| EGFR:SEPT14 | A02.01 | QLQDKFEHL | 422.0 | 1155.0 | 0.6 |
| EGFR:SEPT14 | A02.01 | YLVIQLQDKF | 9963.0 | 2057.0 | 0 |
| EGFR:SEPT14 | A24.02 | YLVIQLQDKF | 9508.0 | 2152.0 | 2.6 |
| EGFR:SEPT14 | A02.01 | IQLQDKFEHL | 820.0 | 4265.0 | 0.2 |
| EGFR:SEPT14 | B08.01 | IQLQDKFEHL | 4278.0 | 10247.0 | 0 |
| EGFRvIII (internal deletion) | A02.01 | ALEEKKGNYV | 2445.0 | 141.0 | 0 |
| EIF2B3(-1) | A02.01 | KQWSSVTSL | 54.4 | 26.5 | not measured |
| EML4:ALK | B08.01 | QVYRRKHQEL | 194.0 | 160.0 | 0 |
| EPHB2(-1) | A02.01 | ILIRKAMTV | 38.2 | 19.5 | not measured |
| ESR1, D538G | A24.02 | PLYGLLLEML | 1519.0 | 444.0 | 6.3 |
| ESR1, D538G | A02.01 | GLLLEMLDA | 705.0 | 558.0 | 0.4 |
| ESR1, D538G | A02.01 | PLYGLLLEM | 349.0 | 640.0 | 0.7 |
| ESR1, D538G | A24.02 | VVPLYGLLL | 2965.0 | 658.0 | 0.8 |
| ESR1, D538G | A02.01 | PLYGLLLEML | 542.0 | 797.0 | 0 |
| ESR1, D538G | A02.01 | VVPLYGLLL | 4432.0 | 1039.0 | 0.6 |
| ESR1, D538G | A02.01 | NVVPLYGLL | 4835.0 | 10471.0 | 0 |
| ESR1, D538G | B07.02 | VPLYGLLLEM | 145.1 | 27.9 | not measured |
| ESR1, D538G | A24.02 | LYGLLLEML | 218.3 | 0.8 | not measured |
| ESR1, S463P | A02.01 | FLPSTLKSL | 71.0 | 21.0 | 2.1 |
| ESR1, S463P | A02.01 | GVYTFLPST | 307.0 | 779.0 | 1.3 |
| ESR1, S463P | A24.02 | FLPSTLKSL | 10723.0 | 995.0 | 1 |
| ESR1, S463P | A02.01 | GVYTFLPSTL | 248.0 | 1197.0 | 0.4 |
| ESR1, S463P | B08.01 | FLPSTLKSL | 2314.0 | 1968.0 | 0 |
| ESR1, S463P | A24.02 | GVYTFLPSTL | 954.0 | 7696.0 | 0 |
| ESR1, Y537C | A02.01 | PLCDLLLEM | 1067.0 | 602.0 | 0.8 |
| ESR1, Y537C | A02.01 | VVPLCDLLL | 5533.0 | 1200.0 | 0 |
| ESR1, Y537C | A02.01 | NVVPLCDLLL | 1964.0 | 1373.0 | 0 |
| ESR1, Y537C | A02.01 | PLCDLLLEML | 1320.0 | 2008.0 | 0.9 |
| ESR1, Y537C | A02.01 | NVVPLCDLL | 3473.0 | 3027.0 | 0 |
| ESR1, Y537C | A24.02 | VVPLCDLLL | 7992.0 | 3888.0 | 0.4 |
| ESR1, Y537N | A02.01 | PLNDLLLEM | 1062.0 | 151.0 | 4.2 |
| ESR1, Y537N | A02.01 | NVVPLNDLL | 4725.0 | 2900.0 | 0 |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| ESR1, Y537N | A02.01 | NVVPLNDLLL | 2606.0 | 4190.0 | 0 |
| ESR1, Y537N | A02.01 | PLNDLLLEML | 1741.0 | 11957.0 | 0 |
| ESR1, Y537S | A02.01 | PLSDLLLEM | 713.0 | 404.0 | 2.7 |
| ESR1, Y537S | A02.01 | NVVPLSDLLL | 2510.0 | 741.0 | 0 |
| ESR1, Y537S | A02.01 | NVVPLSDLL | 4259.0 | 916.0 | 0 |
| ESR1, Y537S | A02.01 | VVPLSDLLL | 8320.0 | 2551.0 | 0 |
| ESR1, Y537S | A02.01 | PLSDLLLEML | 1138.0 | 6469.0 | 0 |
| ESR1, Y537S | A24.02 | VVPLSDLLL | 8463.0 | 8252.0 | 0.5 |
| FAM111B(-1) | A03.01 | RMKVPLMK | 58.9 | 33.0 | not measured |
| FGFR3, S249C | A02.01 | YTLDVLERC | 3309.0 | 1764.0 | 6.6 |
| FGFR3, S249C | B08.01 | VLERCPHRPI | 3629.0 | 7223.0 | 0 |
| FGFR3, S249C | A02.01 | VLERCPHRPI | 4505.0 | 15321.0 | 0 |
| FGFR3:TACC3 | A02.01 | VLTVTSTDV | 1255.0 | 295.0 | 1.1 |
| FRG1B, I10T | B07.02 | KLSDSRTAL | 225.0 | 9.0 | 6.8 |
| FRG1B, I10T | A02.01 | KLSDSRTAL | 275.0 | 111.0 | 2.8 |
| FRG1B, I10T | B08.01 | KLSDSRTAL | 3276.0 | 122.0 | 0 |
| FRG1B, L52S | A02.01 | ALSASNSCFI | 327.0 | 226.0 | 0.8 |
| FRG1B, L52S | B08.01 | FQNGKMALSA | 7796.0 | 425.0 | 0 |
| FRG1B, L52S | B07.02 | ALSASNSCF | 13989.0 | 684.0 | 0 |
| FRG1B, L52S | A02.01 | ALSASNSCF | 7913.0 | 728.0 | 0.3 |
| FRG1B, L52S | A02.01 | FQNGKMALS | 2305.0 | 3276.0 | 0 |
| FRG1B, L52S | A02.01 | FQNGKMALSA | 1205.0 | 6158.0 | 0 |
| FRG1B, L52S | A24.02 | ALSASNSCF | 9672.0 | 16338.0 | 0.2 |
| GATA3 ...CSNH | B08.01 | FLKAESKIM | 263.4 | 21.9 | not measured |
| GATA3 ...CSNH | B08.01 | LQHGHRHGL | 693.0 | 550.4 | not measured |
| GATA3 ...CSNH | B07.02 | EPHLALQPL | 106.6 | 17.0 | not measured |
| GATA3 ...CSNH | B07.02 | RPLQTHVLPE | 968.0 | 2534.4 | not measured |
| GATA3 ...CSNH | B08.01 | FATLQRSSL | 138.0 | 26.6 | not measured |
| GATA3 ...CSNH | B07.02 | MFATLQRSSL | 1285.0 | 266.9 | not measured |
| GATA3 ...CSNH | A24.02 | MFLKAESKI | 1065.7 | 332.1 | not measured |
| GATA3 ...CSNH | B07.02 | FATLQRSSL | 261.9 | 14.0 | not measured |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| GATA3 ...CSNH | A02.01 | MLTGPPARV | 145.4 | 10.6 | not measured |
| GATA3 ...CSNH | B08.01 | EPHLALQPL | 1128.3 | 12.4 | not measured |
| GATA3 ...CSNH | B07.02 | GPPARVPAV | 297.6 | 221.2 | not measured |
| GATA3 ...CSNH | B08.01 | MFATLQRSSL | 220.5 | 53.4 | not measured |
| GATA3 ...CSNH | A02.01 | ALQPLQPHA | 644.4 | 603.9 | not measured |
| GATA3 ...CSNH | A03.01 | VLWTTPPLQH | 962.3 | 16.0 | not measured |
| GATA3 ...CSNH | A02.01 | VLPEPHLAL | 140.7 | 16.0 | not measured |
| GATA3 ...CSNH | B07.02 | HVLPEPHLAL | 1057.2 | 1332.6 | not measured |
| GATA3 ...CSNH | A03.01 | YMFLKAESK | 53.1 | 79.8 | not measured |
| GATA3 ...CSNH | B07.02 | VPAVPFDLHF | 1996.2 | 2114.2 | not measured |
| GATA3 ...CSNH | A02.01 | AIQPVLWTT | 229.3 | 8.1 | not measured |
| GATA3 ...CSNH | A02.01 | TLQRSSLWCL | 319.2 | 117.7 | not measured |
| GATA3 ...CSNH | A03.01 | KIMFATLQR | 62.5 | 2.5 | not measured |
| GATA3 ...CSNH | B07.02 | QPVLWTTPPL | 54.4 | 109.3 | not measured |
| GATA3 ...CSNH | B08.01 | ESKIMFATL | 253.7 | 17.7 | not measured |
| GATA3 ...CSNH | B08.01 | IMKPKRDGYM | 342.1 | 33.2 | not measured |
| GATA3 ...CSNH | B07.02 | KPKRDGYMF | 109.7 | 28.2 | not measured |
| GATA3 ...CSNH | B08.01 | FLKAESKIMF | 1539.9 | 82.3 | not measured |
| GATA3 ...CSNH | B07.02 | KPKRDGYMFL | 32.5 | 98.1 | not measured |
| GATA3 ...CSNH | B08.01 | LHFCRSSIM | 2141.2 | 118.7 | not measured |
| GATA3 CSNH | A02.01 | SMLTGPPARV | 57.0 | 15.0 | 21.7 |
| GATA3 CSNH | B08.01 | YMFLKAESKI | 606.0 | 32.0 | 0.4 |
| GATA3 CSNH | A02.01 | YMFLKAESKI | 163.0 | 166.0 | 0.6 |
| GATA3 CSNH | A03.01 | YMFLKAESKI | 1338.0 | 21111.0 | 0 |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| GATA3 YHEA | A02.01 | FLQEQYHEA | 7.0 | 11.0 | 9.5 |
| GATA3 YHEA | B08.01 | FLQEQYHEA | 1222.0 | 2285.0 | 0 |
| GBP3(-1) | B08.01 | TLKKKPRDI | 286.3 | 3.3 | not measured |
| HER2, G776insYVMA | A02.01 | VMAYVMAGV | 6.0 | 2.0 | 16.5 |
| HER2, G776insYVMA | A02.01 | YVMAYVMAGV | 5.0 | 57.0 | 20.9 |
| HER2, G776insYVMA | B07.02 | YVMAYVMAG | 6910.0 | 170.0 | 0 |
| HER2, G776insYVMA | B08.01 | YVMAYVMAGV | 721.0 | 353.0 | 0 |
| HER2, G776insYVMA | A02.01 | YVMAYVMAG | 841.0 | 11535.0 | 2.5 |
| HER2, G776insYVMA | B08.01 | YVMAYVMAG | 836.0 | 19413.0 | 1.2 |
| HER2, G776insYVMA | B07.02 | YVMAYVMAGV | 11445.0 | 52630.0 | 0 |
| HER2, L755S | A03.01 | KVSRENTSPK | 66.0 | 7.0 | 13.5 |
| HER2, V777L | A02.01 | VMAGLGSPYV | 20.0 | 102.0 | 2.9 |
| HER2, V777L | A03.01 | VMAGLGSPYV | 3951.0 | 11222.0 | 0 |
| JAK1(-1) | A02.01 | SLMPAHWSI | 4.0 | 48.0 | 5 |
| JAK1(-1) | A02.01 | LSLMPAHWSI | 21.0 | 164.0 | 0.4 |
| JAK1(-1) | B08.01 | SLMPAHWSI | 282.0 | 177.0 | 0 |
| JAK1(-1) | A02.01 | FQMQPLSLM | 33.0 | 553.0 | 0.3 |
| JAK1(-1) | A24.02 | SLMPAHWSI | 194.0 | 633.0 | 0.4 |
| JAK1(-1) | B08.01 | LSLMPAHWSI | 1914.0 | 860.0 | 0 |
| JAK1(-1) | B07.02 | SLMPAHWSI | 3907.0 | 1040.0 | 0 |
| JAK1(-1) | B08.01 | FQMQPLSLM | 2261.0 | 6714.0 | 0 |
| JAK1(-1) | B07.02 | FQMQPLSLM | 3458.0 | 10207.0 | 0 |
| JAK1(-1) | A24.02 | LSLMPAHWSI | 2125.0 | 12398.0 | 0 |
| JAK1(-1) | A24.02 | FQMQPLSLM | 4021.0 | 14612.0 | 0 |
| KIT, T670I | A02.01 | VIIEYCCYG | 4225.0 | 191.0 | 0.5 |
| KIT, T670I | A02.01 | IIEYCCYGDL | 3918.0 | 7310.0 | 0 |
| KIT, T670I | A02.01 | TIGGPTLVII | 5425.0 | 10685.0 | 0 |
| KIT, V654A | A02.01 | YLGNHMNIA | 92.0 | 117.0 | 0.6 |
| KIT, V654A | A02.01 | MNIANLLGA | 4522.0 | 128.0 | 0.3 |
| KIT, V654A | A02.01 | HMNIANLLGA | 294.0 | 430.0 | 0 |
| KIT, V654A | B08.01 | YLGNHMNIA | 2480.0 | 872.0 | 0 |
| KIT, V654A | A02.01 | YLGNHMNIAN | 7103.0 | 1342.0 | 0 |
| KIT, V654A | A02.01 | IANLLGACTI | 11214.0 | 6417.0 | 0 |
| KRAS, G12C | A02.01 | KLVVVGACGV | 204.0 | 150.0 | 1 |
| KRAS, G12C | A02.01 | LVVVGACGV | 658.0 | 1213.0 | 0.6 |
| KRAS, G12C | A03.01 | VVVGACGVGK | 300.7 | 1.6 | not measured |

(continued)

| Mutation | Allele | Peptide | Predicted affinity ($IC_{50}$; (nM)) | Observed Affinity ($IC_{50}$; (nM)) | Stability ($T_{1/2}$ (h)) |
|---|---|---|---|---|---|
| KRAS, G12C | A03.01 | VVGACGVGK | 182.0 | 4.1 | not measured |
| KRAS, G12D | A02.01 | KLVVVGADGV | 361.0 | 184.0 | 0.9 |
| KRAS, G12D | A02.01 | LVVVGADGV | 2120.0 | 1192.0 | 0 |
| KRAS, G12V | A02.01 | KLVVVGAVGV | 163.0 | 96.0 | 0.9 |
| KRAS, G12V | A02.01 | LVVVGAVGV | 453.0 | 975.0 | 0.6 |
| KRAS, G12V | A03.01 | VVGAVGVGK | 168.9 | 1.9 | not measured |
| KRAS, Q61H | A01.01 | ILDTAGHEEY | 131.8 | 64.2 | not measured |
| KRAS, Q61L | A01.01 | ILDTAGLEEY | 65.9 | 8.6 | not measured |
| KRAS, Q61L | A02.01 | LLDILDTAGL | 113.4 | 715.7 | not measured |
| LMAN1(+1) | B07.02 | GPPRPPRAAC | 69.3 | 48.6 | not measured |
| LMAN1(+1) | B07.02 | PPRPPRAAC | 263.7 | 32.8 | not measured |
| LMAN1(-1) | B08.01 | SLRRKYLRV | 28.0 | 0.4 | not measured |
| MEK, C121S | A02.01 | VLHESNSPYI | 189.0 | 131.0 | 1.9 |
| MEK, P124L | A02.01 | VLHECNSLYI | 67.0 | 10.0 | 5.1 |
| MEK, P124L | A02.01 | SLYIVGFYGA | 104.0 | 390.0 | 0.4 |
| MEK, P124L | A02.01 | SLYIVGFYG | 2987.0 | 1063.0 | 0 |
| MEK, P124L | A02.01 | LQVLHECNSL | 5803.0 | 4723.0 | 0 |
| MEK, P124L | A02.01 | QVLHECNSL | 8695.0 | 7774.0 | 0.5 |
| MEK, P124L | A03.01 | VLHECNSLYI | 4733.0 | 10500.0 | 0 |
| MEK, P124L | B08.01 | QVLHECNSL | 6854.0 | 14532.0 | 0 |
| MEK, P124L | B08.01 | LQVLHECNSL | 2782.0 | 19316.0 | 0 |
| MLL2, ...LSPH | A02.01 | LLQVTQTSFA | 1935.0 | 676.9 | not measured |
| MLL2, ...LSPH | A02.01 | RLWHLLLQV | 8.3 | 1.3 | not measured |
| MLL2, ...LSPH | A02.01 | LQVTQTSFAL | 1147.4 | 718.3 | not measured |
| MLL2, ...LSPH | A02.01 | RLWHLLLQVT | 140.8 | 50.9 | not measured |
| MLL2, ...LSPH | A02.01 | ALAPTLTHM | 98.4 | 59.0 | not measured |
| MLL2, ...LSPH | A02.01 | ALAPTLTHML | 66.4 | 39.0 | not measured |
| MLL2, CRLS | B08.01 | SLGNHLCPL | 136.0 | 6.0 | 0.5 |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| MLL2, CRLS | A02.01 | SLGNHLCPL | 28.0 | 18.0 | 3.5 |
| MLL2, CRLS | B07.02 | SLGNHLCPL | 3967.0 | 2590.0 | 0 |
| MSH3(-1) | A02.01 | LLALWECSL | 46.0 | 15.0 | 4 |
| MSH3(-1) | A02.01 | FLLALWECSL | 17.0 | 114.0 | 10.8 |
| MSH3(-1) | B08.01 | LLALWECSL | 1454.0 | 154.0 | 0 |
| MSH3(-1) | B08.01 | FLLALWECSL | 671.0 | 13100.0 | 0 |
| MSH3(-1) | A02.01 | LIV SRTLLL | 755.0 | 173.5 | not measured |
| MSH3(-1) | A02.01 | LIVSRTLLLV | 146.6 | 10920.6 | not measured |
| MSH3(-1) | B08.01 | LIVSRTLLL | 270.7 | 881.7 | not measured |
| MSH3(-1) | A02.01 | IVSRTLLLV | 166.2 | 12.7 | not measured |
| MSH3(-1) | B08.01 | SLPQARLCLI | 632.3 | 4313.9 | not measured |
| MSH3(-1) | B08.01 | CLIVSRTLLL | 835.7 | 1100.4 | not measured |
| MSH3(-1) | B08.01 | LPQARLCLI | 136.5 | 15.0 | not measured |
| MSH3(-1) | A02.01 | SLPQARLCLI | 782.4 | 112.9 | not measured |
| MSH3(-1) | A02.01 | CLIVSRTLLL | 560.5 | 2005.1 | not measured |
| MSH3(-1) | A02.01 | FLLALWECS | 686.6 | 93.2 | not measured |
| MSH3(-1) | A02.01 | FLLALWECSL | 16.6 | 0.9 | not measured |
| MSH3(-1) | A02.01 | LLALWECSL | 46.1 | 12.5 | not measured |
| MSH3(-1) | B07.02 | LPQARLCLI | 134.6 | 72.6 | not measured |
| MSH3(-1) | B08.01 | CLIVSRTLL | 915.0 | 126.7 | not measured |
| MSH3(-1) | A02.01 | ALWECSLPQA | 24.6 | 9.0 | not measured |
| MSH3(-1) | B08.01 | LPQARLCLIV | 591.4 | 152.1 | not measured |
| MSH6(+1) | A02.01 | LLPEDTPPL | 8.9 | 2.8 | not measured |
| MSH6(+1) | A02.01 | ILLPEDTPPL | 16.3 | 6.7 | not measured |
| MYC, E39D | A02.01 | QQSDLQPPA | 4930.0 | 70.0 | 0 |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| MYC, E39D | A02.01 | QQQSDLQPPA | 11835.0 | 646.0 | 0 |
| MYC, E39D | A02.01 | YQQQQQSDL | 8842.0 | 799.0 | 0.4 |
| MYC, E39D | B08.01 | YQQQQQSDL | 5259.0 | 18868.0 | 0 |
| MYC, P57S | A02.01 | FELLSTPPL | 2509.0 | 225.0 | 0 |
| MYC, P57S | A02.01 | LLSTPPLSPS | 5226.0 | 1770.0 | 0 |
| MYC, P57S | B08.01 | FELLSTPPL | 4208.0 | 3179.0 | 0 |
| MYC, T58I | A02.01 | LLPIPPLSPS | 2071.0 | 449.0 | 0 |
| MYC, T58I | A02.01 | FELLPIPPL | 2472.0 | 553.0 | 0 |
| NAB:STAT6 ("variant 1" of Chmielecki et al.) | A02.01 | SQIMSLWGL | 14.0 | 62.0 | 1 |
| NAB:STAT6 ("variant 1" of Chmielecki et al.) | A02.01 | IMSLWGLVS | 3630.0 | 7321.0 | 0 |
| NAB:STAT6 ("variant 1" of Chmielecki et al.) | A24.02 | SQIMSLWGL | 1604.0 | 8516.0 | 0 |
| NAB:STAT6 ("variant 1" of Chmielecki et al.) | B08.01 | SQIMSLWGL | 4587.0 | 15997.0 | 0 |
| NDRG1:ERG | A02.01 | LLQEFDVQEA | 200.0 | 45.0 | 2.5 |
| NDRG1:ERG | A02.01 | LQEFDVQEAL | 2229.0 | 50280.0 | 0 |
| NDUFC2(-KCDT14)(+1) | A02.01 | ITAFFFCWI | 437.7 | 7490.4 | not measured |
| NDUFC2(-KCDT14)(+1) | A24.02 | LYITAFFFCW | 46.6 | 45.3 | not measured |
| NDUFC2(-KCDTI14)(+1) | A03.01 | FFFCWILSCK | 325.9 | 597.1 | not measured |
| NDUFC2(-KCDT14)(+1) | A03.01 | FFCWILSCK | 985.7 | 184.9 | not measured |
| NDUFC2(-KCDT14)(-1) | A02.01 | LLYITAFFL | 24.0 | 713.0 | 17 |
| NDUFC2(-KCDT14)(-1) | B08.01 | LLYITAFFL | 3588.0 | 9592.0 | 0 |
| NDUFC2(-KCDT14)(-1) | A02.01 | ITAFFLLDI | 699.0 | 78.7 | not measured |
| NDUFC2(-KCDT14)(-1) | A02.01 | YITAFFLLDI | 157.0 | 64.5 | not measured |
| NDUFC2(-KCDT14)(-1) | A24.02 | LYITAFFLL | 15.6 | 0.1 | not measured |
| NDUFC2(-KCDT14)(-1) | A02.01 | LLYITAFFLL | 43.7 | 323.2 | not measured |
| NDUFC2(-KCDT14)(-1) | A24.02 | LLYITAFFLL | 59.7 | 60.1 | not measured |
| NDUFC2(-KCDT14)(-1) | A24.02 | LYITAFFLLD | 414.3 | 0.4 | not measured |
| NRAS, Q61K | A01.01 | ILDTAGKEEY | 272.6 | 14.3 | not measured |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| NRAS, Q61R | A01.01 | ILDTAGREEY | 255.8 | 7.0 | not measured |
| PDGFRa, T6741 | A02.01 | IIIEYCFYG | 693.0 | 16.0 | 1.2 |
| PDGFRa, T674I | A02.01 | YIIIEYCFYG | 1529.0 | 113.0 | 0 |
| PDGFRa, T674I | A02.01 | IIEYCFYGDL | 3049.0 | 1090.0 | 0 |
| PIK3CA, E542K | A02.01 | KITEQEKDFL | 12548.0 | 1397.0 | 0 |
| PIK3CA, E542K | A03.01 | AISTRDPLSK | 41.3 | 57.5 | not measured |
| PTEN, R130Q | A02.01 | QTGVMICAYL | 3786.0 | 9760.0 | 0 |
| RAC1, P29S | A02.01 | FSGEYIPTV | 21.0 | 3.0 | 6.8 |
| RAC1, P29S | A02.01 | AFSGEYIPTV | 1008.0 | 781.0 | 0 |
| RAC1, P29S | A01.01 | TTNAFSGEY | 23.0 | 4.4 | not measured |
| RAC1, P29S | A01.01 | YTTNAFSGEY | 20.0 | 10.5 | not measured |
| RBM27(+1) | B07.02 | MPKDVNIQV | 291.6 | 12.5 | not measured |
| RBM27(+1) | A01.01 | TGSNEVTTRY | 343.9 | 15545.2 | not measured |
| RBM27(+1) | A01.01 | GSNEVTTRY | 151.6 | 605.5 | not measured |
| RNF43, RHTP | A02.01 | TQLARFFPI | 17.0 | 19.0 | 0 |
| RNF43, RHTP | A24.02 | TQLARFFPI | 268.0 | 52.0 | 0 |
| RNF43, RHTP | B08.01 | TQLARFFPI | 41.0 | 9150.0 | 0 |
| SEC31A(-1) | A02.01 | KLMLLRLNL | 58.0 | 17.0 | 16.9 |
| SEC31A(-1) | B08.01 | KLMLLRLNL | 421.0 | 29.0 | 0 |
| SEC31A(-1) | B07.02 | KLMLLRLNL | 2969.0 | 133.0 | 1.5 |
| SEC31A(-1) | A03.01 | KLMLLRLNL | 4664.0 | 210.0 | 0 |
| SEC31A(-1) | B08.01 | LLRLNLRKM | 185.1 | 68.2 | not measured |
| SEC31A(-1) | A03.01 | MLLRLNLRKM | 171.4 | 116.9 | not measured |
| SEC31A(-1) | A03.01 | KLMLLRLNLR | 95.4 | 48.4 | not measured |
| SEC31A(-1) | A03.01 | MLLRLNLRK | 14.6 | 1.3 | not measured |
| SEC31A(-1) | A03.01 | LMLLRLNLRK | 23.9 | 6.0 | not measured |
| SEC31A(-1) | A02.01 | MLLRLNLRKM | 508.5 | 2507.4 | not measured |
| SEC31A(-1) | B08.01 | MLLRLNLRKM | 565.9 | 95.3 | not measured |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| SEC31A(-1) | A02.01 | KLMLLRLNL | 57.6 | 2.6 | not measured |
| SEC31A(-1) | B08.01 | LMLLRLNL | 116.0 | 9.3 | not measured |
| SEC31A(-1) | B08.01 | KLMLLRLNL | 420.6 | 58.4 | not measured |
| SEC31A(-1) | A02.01 | KKLMLLRLNL | 275.4 | 288.9 | not measured |
| SEC31A(-1) | B08.01 | NLRKMCGPF | 163.5 | 35.9 | not measured |
| SEC31A(-1) | B08.01 | YCQKKLMLL | 203.1 | 222.1 | not measured |
| SEC31A(-1) | B08.01 | LNLRKMCGPF | 782.2 | 438.7 | not measured |
| SEC63(+1) | A03.01 | YTCAITTVK | 279.0 | 122.4 | not measured |
| SEC63(+1) | A03.01 | TYTCAITTVK | 556.4 | 2362.2 | not measured |
| SEC63(+1) | A03.01 | ITTVKATETK | 795.8 | 1245.3 | not measured |
| SEC63(+1) | A03.01 | KSKKKETFKK | 744.0 | 39.6 | not measured |
| SEC63(+1) | B08.01 | TFKKKTYTC | 648.2 | 77.9 | not measured |
| SEC63(+1) | A03.01 | KSKKKETFK | 411.0 | 74.3 | not measured |
| SEC63(+1) | B08.01 | FKKKTYTCAI | 562.8 | 384.9 | not measured |
| SEC63(-1) | B08.01 | TAKSKKRNL | 213.8 | 30.6 | not measured |
| SF3B1, K700E | A02.01 | GLVDEQQEV | 50.0 | 44.0 | 7.4 |
| SLC35F5(-1) | A02.01 | FALCGFWQI | 10.5 | 0.4 | not measured |
| SMAP1(-1) | A03.01 | KSRQNHLQLK | 88.1 | 4.7 | not measured |
| SMAP1(-1) | B07.02 | KSRQNHLQL | 329.5 | 78.0 | not measured |
| SMAP1(-1) | A24.02 | KLRSPLWIF | 504.5 | 828.2 | not measured |
| SMAP1(-1) | A03.01 | KISNWSLKK | 11.5 | 8.8 | not measured |
| SMAP1(-1) | A11.01 | KISNWSLKK | 15.3 | 9.8 | not measured |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| SMAP1(-1) | A11.01 | SLKKVPALK | 117.6 | 129.1 | not measured |
| SMAP1(-1) | B08.01 | SLKKVPAL | 66.8 | 7.9 | not measured |
| SMAP1(-1) | A03.01 | WSLKKVPALK | 148.9 | 94.9 | not measured |
| SMAP1(-1) | A03.01 | KISNWSLKKV | 168.3 | 114.6 | not measured |
| SMAP1(-1) | A03.01 | RKISNWSLKK | 20.8 | 130.6 | not measured |
| SMAP1(-1) | A03.01 | SLKKVPALK | 29.6 | 4.4 | not measured |
| SMAP1(-1) | B08.01 | SQKSRQNHL | 305.0 | 44.6 | not measured |
| SMAP1(-1) | B07.02 | ALKKLRSPL | 355.5 | 223.2 | not measured |
| SMAP1(-1) | B08.01 | ALKKLRSPL | 58.9 | 0.5 | not measured |
| SMAP1(-1) | B08.01 | WSLKKVPAL | 110.7 | 12.5 | not measured |
| SMAP1(-1) | A03.01 | HLQLKSCRRK | 216.7 | 96.9 | not measured |
| SMAP1(-1) | B08.01 | LKKLRSPL | 139.6 | 0.6 | not measured |
| SMAP1(-1) | A03.01 | SLKKVPALKK | 43.1 | 9.6 | not measured |
| SPOP, F133L | A02.01 | FVQGKDWGL | 121.0 | 34.0 | 2.1 |
| SPOP, F133L | B08.01 | FVQGKDWGL | 1401.0 | 207.0 | 0 |
| TFAM(+1) | A03.01 | RVNTAWKTK | 136.4 | 8.6 | not measured |
| TFAM(+1) | A03.01 | RVNTAWKTKK | 70.6 | 2.3 | not measured |
| TFAM(+1) | B08.01 | TKKKRVNTA | 312.4 | 159.4 | not measured |
| TFAM(+1) | A03.01 | KRVNTAWKTK | 304.1 | 331.6 | not measured |
| TFAM(+1) | B08.01 | WKTKKTSFSL | 930.6 | 112.2 | not measured |
| TFAM(+1) | B08.01 | MIKKKRVNTA | 534.2 | 186.9 | not measured |
| TGFBR2(-1) | A02.01 | RLSSCVPVA | 83.0 | 4.0 | 18.7 |
| TGFBR2(-1) | A03.01 | RLSSCVPVA | 4264.0 | 439.0 | 0 |
| TGFBR2(-1) | A03.01 | AMTTSSSQK | 48.5 | 8.3 | not measured |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| TGFBR2(-1) | A03.01 | AMTTSSSQKN | 887.2 | 2336.5 | not measured |
| TGFBR2(-1) | B08.01 | IMKEKKSL | 69.8 | 14.1 | not measured |
| TGFBR2(-1) | A02.01 | KSLVRLSSCV | 903.1 | 279.8 | not measured |
| TGFBR2(-1) | A02.01 | SLVRLSSCV | 177.3 | 29.9 | not measured |
| TGFBR2(-1) | A11.01 | SAMTTSSSQK | 36.4 | 15.8 | not measured |
| TGFBR2(-1) | B08.01 | IMKEKKSLV | 80.8 | 16.8 | not measured |
| TGFBR2(-1) | A11.01 | AMTTSSSQK | 89.9 | 161.6 | not measured |
| TGFBR2(-1) | A03.01 | SAMTTSSSQK | 96.7 | 15.7 | not measured |
| TGFBR2(-1) | A02.01 | RLSSCVPVAL | 84.5 | 54.2 | not measured |
| TGFBR2(-1) | A02.01 | VRLSSCVPVA | 640.6 | 1206.8 | not measured |
| TGFBR2(-1) | A02.01 | RLSSCVPVA | 82.7 | 49.5 | not measured |
| TGFBR2(-1) | B08.01 | CIMKEKKSL | 218.5 | 7.5 | not measured |
| TGFBR2(-1) | A02.01 | ALMSAMTTS | 320.4 | 139.1 | not measured |
| TGFBR2(-1) | A02.01 | LVRLSSCPV | 132.7 | 1237.6 | not measured |
| THAP5(-1) | A03.01 | KMRKKYAQK | 23.7 | 5.7 | not measured |
| TMPRSS2:ERG | A02.01 | ALNSEALSV | 66.0 | 14.0 | 9.1 |
| TMPRSS2:ERG | A02.01 | ALNSEALSVV | 84.0 | 15.0 | 2.9 |
| TMPRSS2:ERG | A02.01 | MALNSEALSV | 198.0 | 129.0 | 0.7 |
| TMPRSS2:ERG | B08.01 | MALNSEALSV | 8512.0 | 13457.0 | 0 |
| TP53, AAVG | A02.01 | GLLAFWDSQV | 57.0 | 10.0 | 14.2 |
| TP53, AAVG | A02.01 | LLAFWDSQV | 13.0 | 68.0 | 12.8 |
| TP53, AWAA | A02.01 | WMTETLFDI | 7.0 | 14.0 | 4 |
| TP53, AWAA | A02.01 | WMTETLFDIV | 15.0 | 40.0 | 0.4 |
| TP53, AWAA | A24.02 | WMTETLFDI | 4936.0 | 713.0 | 0 |
| TP53, AWAA | A01.01 | WMTETLFDIV | 4046.0 | 14394.0 | 0 |
| TP53, CSES | B07.02 | LPSQRRNHWM | 89.0 | 10.0 | 6.5 |
| TP53, CSES | B08.01 | LPSQRRNHWM | 325.0 | 47.0 | 0.7 |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| TP53, CSES | A02.01 | ALSEHCPTT | 208.0 | 79.0 | 27.3 |
| TP53, G245S | B08.01 | CMGSMNRRPI | 1204.0 | 80.0 | 0 |
| TP53, G245S | A02.01 | YMCNSSCMGS | 2485.0 | 81.0 | 0.8 |
| TP53, G245S | B08.01 | SMNRRPILTI | 260.0 | 337.0 | 0 |
| TP53, G245S | A02.01 | SMNRRPILTI | 1644.0 | 1198.0 | 0.3 |
| TP53, G245S | B08.01 | SMNRRPILT | 2536.0 | 1282.0 | 0 |
| TP53, G245S | A02.01 | CMGSMNRRPI | 7822.0 | 1989.0 | 0 |
| TP53, G245S | A02.01 | SMNRRPILT | 7251.0 | 3839.0 | 0 |
| TP53, G245S | A24.02 | SMNRRPILTI | 10308.0 | 16292.0 | 0 |
| TP53, G245S | B08.01 | GSMNRRPIL | 636.7 | 15.5 | not measured |
| TP53, G245S | B08.01 | MGSMNRRPIL | 89.1 | 6.3 | not measured |
| TP53, G245S | B08.01 | MGSMNRRPI | 324.2 | 29.1 | not measured |
| TP53, QPSL | B07.02 | LPRKPTRAAT | 47.0 | 3.0 | 3.7 |
| TP53, QPSL | B07.02 | LPRKPTRAA | 8.0 | 8.0 | 5.5 |
| TP53, QPSL | B07.02 | KPTRAATVSV | 12.0 | 8.0 | 3 |
| TP53, QPSL | B08.01 | LPRKPTRAA | 873.0 | 1158.0 | 0 |
| TP53, R248Q | B08.01 | NQRPILTII | 3433.0 | 20.0 | 0 |
| TP53, R248Q | A02.01 | GMNQRPILTI | 1787.0 | 709.0 | 0.4 |
| TP53, R248Q | A02.01 | GMNQRPILT | 8115.0 | 3029.0 | 0 |
| TP53, R248Q | A02.01 | CMGGMNQRPI | 3025.0 | 3673.0 | 0 |
| TP53, R248Q | A02.01 | NQRPILTII | 10855.0 | 9606.0 | 0 |
| TP53, R248Q | B08.01 | CMGGMNQRPI | 6364.0 | 18766.0 | 0 |
| TP53, R248Q | B08.01 | GMNQRPILTI | 3266.0 | 29251.0 | 0 |
| TP53, R248W | B08.01 | MNWRPILTI | 6447.0 | 1.0 | 0 |
| TP53, R248W | A02.01 | GMNWRPILTI | 189.0 | 282.0 | 0.5 |
| TP53, R248W | A02.01 | CMGGMNWRPI | 354.0 | 346.0 | 0.4 |
| TP53, R248W | A02.01 | MNWRPILTI | 5834.0 | 516.0 | 3.8 |
| TP53, R248W | A02.01 | MNWRPILTII | 8158.0 | 1026.0 | 0.4 |
| TP53, R248W | B08.01 | GMNWRPILTI | 3990.0 | 1045.0 | 0 |
| TP53, R248W | A02.01 | GMNWRPILT | 3416.0 | 1130.0 | 0 |
| TP53, R248W | B08.01 | CMGGMNWRPI | 3218.0 | 2248.0 | 0 |
| TP53, R248W | A24.02 | CMGGMNWRPI | 9521.0 | 4453.0 | 0 |
| TP53, R248W | A24.02 | MNWRPILTI | 3634.0 | 6977.0 | 0.2 |
| TP53, R248W | A24.02 | MNWRPILTII | 1517.0 | 44901.0 | 0 |
| TP53, R273C | A02.01 | LLGRNSFEVC | 1272.0 | 2081.0 | 0 |
| TP53, R273C | A02.01 | NSFEVCVCA | 4239.0 | 2200.0 | 0 |

(continued)

| Mutation | Allele | Peptide | Predicted affinity (IC$_{50}$; (nM)) | Observed Affinity (IC$_{50}$; (nM)) | Stability (T$_{1/2}$ (h)) |
|---|---|---|---|---|---|
| TP53, R273H | A02.01 | NSFEVHVCA | 6768.0 | 503.0 | 0 |
| TP53, SHST | B07.02 | HPRPAPASA | 13.0 | 11.0 | 4.9 |
| TP53, SHST | B08.01 | HPRPAPASA | 1718.0 | 25.0 | 0 |
| TP53, Y220C | A02.01 | VVPCEPPEV | 1268.0 | 187.0 | 0.9 |
| TTK(-1) | A02.01 | VMSDTTYKI | 15.8 | 19.6 | not measured |
| TTK(-1) | A03.01 | LFVMSDTTYK | 57.9 | 749.4 | not measured |
| TTK(-1) | A02.01 | FVMSDTTYKI | 16.0 | 62.4 | not measured |
| TTK(-1) | A03.01 | FVMSDTTYK | 63.1 | 66.9 | not measured |
| TTK(-1) | A03.01 | KTFEKKGEK | 81.3 | 32.2 | not measured |
| TTK(-1) | A01.01 | VMSDTTYKIY | 245.1 | 375.8 | not measured |
| TTK(-1) | A01.01 | MSDTTYKIY | 18.9 | 10.2 | not measured |
| UBR5(-1) | B07.02 | RVQNQGHLL | 429.1 | 826.5 | not measured |
| VHL, QCIL | A02.01 | MLTDSLFLPI | 8.0 | 16.0 | 1 |
| VHL, QCIL | A02.01 | SMLTDSLFL | 14.0 | 31.0 | 9.8 |
| VHL, QCIL | B08.01 | MLTDSLFLPI | 2581.0 | 110.0 | 0 |
| VHL, QCIL | A01.01 | MLTDSLFLPI | 429.0 | 7673.0 | 0 |
| XPOT(-1) | A02.01 | YLTKWPKFFL | 10.7 | 42.9 | not measured |

## Example 3: HLA Class I Binding Stability

A subset of the peptides used for affinity measurements were also used for stability measurements using the assay described (n=275). These data are shown in Table 3. The data are plotted according to 4 bins in Figure 2, with the median and interquartile intervals overlaid. Less than 50 nM was considered by the field as a strong binder, 50-150 nM was considered an intermediate binder, 150-500 nM was considered a weak binder, and greater than 500 nM was considered a very weak binder. The connection between the observed stability and observed affinity was evident by the decreasing median stability across these binned stability intervals. However, there is considerable overlap between the bins, and importantly there are epitopes in all bins with observed stability in the multiple hour range, including the very weak binders.

## Example 4: Immunogencity Assays

Immunogenicity assays are used to test the ability of each test peptide to expand T cells. Mature professional APCs are prepared for these assays in the following way. Monocytes are enriched from healthy human donor PBMCs using a bead-based kit (Miltenyi). Enriched cells are plated in GM-CSF and IL-4 to induce immature DCs. After 5 days, immature DCs are incubated at 37°C with each peptide for 1 hour before addition of a cytokine maturation cocktail (GM-CSF, IL-1β, IL-4, IL-6, TNFα, PGE1β). Cells are incubated at 37°C to mature DCs.

After maturation of DCs, PBMCs (either bulk or enriched for T cells) are added to mature dendritic cells with proliferation cytokines. Cultures are monitored for peptide-specific T cells using a combination of functional assays and/or tetramer staining. Parallel immunogenicity assays with the modified and parent peptides allowed for comparisons of the relative efficiency with which the peptides expanded peptide-specific T cells.

*Tetramer Staining.* MHC tetramers are purchased or manufactured on-site, and are used to measure peptide-specific T cell expansion in the immunogenicity assays. For the assessment, tetramer is added to $1 \times 10^5$ cells in PBS containing 1% FCS and 0.1% sodium azide (FACS buffer) according to manufacturer's instructions. Cells are incubated in the dark for 20 minutes at room temperature. Antibodies specific for T cell markers, such as CD8, are then added to a final concentration suggested by the manufacturer, and the cells are incubated in the dark at 4 °C for 20 minutes. Cells are washed with cold FACS buffer and resuspended in buffer containing 1% formaldehyde. Cells are acquired on a FACS Calibur (Becton Dickinson) instrument, and are analyzed by use of Cellquest software (Becton Dickinson). For analysis of tetramer positive cells, the lymphocyte gate is taken from the forward and side-scatter plots. Data are reported as the percentage of cells that were CD8$^+$/Tetramer$^+$.

*Intracellular cytokine staining.* In the absence of well-established tetramer staining to identify antigen-specific T cell populations, antigen-specificity can be estimated using assessment of cytokine production using well-established flow cytometry assays. Briefly, T cells are stimulated with the peptide of interest and compared to a control. After stimulation, production of cytokines by CD4$^+$ T cells (e.g., IFNy and TNF$\alpha$) are assessed by intracellular staining. These cytokines, especially IFN$\gamma$, used to identify stimulated cells.

*ELISPOT.* Peptide-specific T cells are functionally enumerated using the ELISPOT assay (BD Biosciences), which measures the release of IFNgamma from T cells on a single cell basis. Target cells (T2 or HLA-A0201 transfected C1Rs) were pulsed with 10 uM peptide for 1 hour at 37 °C, and washed three times. $1 \times 10^5$ peptide-pulsed targets are co-cultured in the ELISPOT plate wells with varying concentrations of T cells ($5 \times 10^2$ to $2 \times 10^3$) taken from the immunogenicity culture. Plates are developed according to the manufacturer's protocol, and analyzed on an ELISPOT reader (Cellular Technology Ltd.) with accompanying software. Spots corresponding to the number of IFNgamma-producing T cells are reported as the absolute number of spots per number of T cells plated. T cells expanded on modified peptides are tested not only for their ability to recognize targets pulsed with the modified peptide, but also for their ability to recognize targets pulsed with the parent peptide.

*CD107 staining.* CD107a and b are expressed on the cell surface of CD8$^+$ T cells following activation with cognate peptide. The lytic granules of T cells have a lipid bilayer that contains lysosomal-associated membrane glycoproteins ("LAMPs"), which include the molecules CD107a and b. When cytotoxic T cells are activated through the T cell receptor, the membranes of these lytic granules mobilize and fuse with the plasma membrane of the T cell. The granule contents are released, and this leads to the death of the target cell. As the granule membrane fuses with the plasma membrane, C107a and b are exposed on the cell surface, and therefore are markers of degranulation. Because degranulation as measured by CD107 a and b staining is reported on a single cell basis, the assay is used to functionally enumerate peptide-specific T cells. To perform the assay, peptide is added to HLA-A0201-transfected cells C1R to a final concentration of 20 $\mu$M, the cells were incubated for 1 hour at 37 °C, and washed three times. $1 \times 10^5$ of the peptide-pulsed C1R cells were aliquoted into tubes, and antibodies specific for CD107 a and b are added to a final concentration suggested by the manufacturer (Becton Dickinson). Antibodies are added prior to the addition of T cells in order to "capture" the CD107 molecules as they transiently appear on the surface during the course of the assay. $1 \times 10^5$ T cells from the immunogenicity culture are added next, and the samples were incubated for 4 hours at 37 °C. The T cells are further stained for additional cell surface molecules such as CD8 and acquired on a FACS Calibur instrument (Becton Dickinson). Data is analyzed using the accompanying Cellquest software, and results were reported as the percentage of CD8$^+$ CD107 a and b$^+$ cells.

*Cytotoxicity assays.* Cytotoxic activity is measured using a chromium release assay. Target T2 cells are labeled for 1 hour at 37 °C with Na$^{51}$Cr and washed $5 \times 10^3$ target T2 cells were then added to varying numbers of T cells from the immunogenicity culture. Chromium release is measured in supernatant harvested after 4 hours of incubation at 37°C. The percentage of specific lysis is calculated as:

$$\text{Experimental release-spontaneous release/Total release-spontaneous release x 100}$$

Immunogenicity assays were carried out to assess whether each peptide can elicit a T cell response by antigen-specific expansion. Though current methods are imperfect, and therefore negative results do not imply a peptide is incapable of inducing a response, a positive result demonstrates that a peptide can induce a T cell response. Several peptides from Table 3 were tested for their capacity to elicit CD8$^+$ T cell responses with multimer readouts as described. Each positive result was measured with a second multimer preparation to avoid any preparation biases. Several examples of positive multimer staining are shown in Figures 3-6, with multimer negative cells (left) and antigen-specific (multimer-double-positive) cells (right) shown.

HLA-A02:01$^+$ T cells were co-cultured with monocyte-derived dendritic cells loaded with TMPRSS2::ERG fusion neoepitope (ALNSEALSV; HLA-A02:01) for 10 days. CD8$^+$ T cells were analyzed for antigen-specificity for TMPRSS2::ERG fusion neoepitope using multimers (initial: BV421 and PE; validation: APC and BUV396) (Figure 3).

HLA-A02:01[+] T cells were co-cultured with monocyte-derived dendritic cells loaded with GATA3 frameshift neoepitope (SMLTGPPARV; HLA-A02:01) for 10 days. CD8[+] T cells were analyzed for antigen-specificity for GATA3 frameshift neoepitope using multimers (initial: APC and BUV396; validation: PE and BV421) (Figure 4).

HLA-A02:01[+] T cells were co-cultured with monocyte-derived dendritic cells loaded with β2M frameshift neoepitope (LLCVWVSSI; HLA-A02:01) for 10 days. CD8[+] T cells were analyzed for antigen-specificity for β2M frameshift neoepitope using multimers (initial: PE and APC; validation: PE and BV421) (Figure 5).

HLA-A02:01[+] T cells were co-cultured with monocyte-derived dendritic cells loaded with KRAS G12C neoepitope (KLVVVGACGV; HLA-A02:01) for 10 days. CD8[+] T cells were analyzed for antigen-specificity for KRAS G12C frameshift neoepitope using multimers (initial: BUV396 and BV421; validation: APC and BUV396) (Figure 6).

While antigen-specific CD8[+] T cell responses are readily assessed using well-established HLA Class I multimer technology, CD4[+] T cell responses require a separate assay to evaluate because HLA Class II multimer technology is not well-established. In order to assess CD4[+] T cell responses, T cells were re-stimulated with the peptide of interest and compared to a control. In the case of a completely novel sequence (e.g., arising from a frame-shift or fusion), the control was no peptide. In the case of a point-mutation, the control was the WT peptide. After stimulation, production of cytokines by CD4[+] T cells (e.g., IFNy and TNFα) were assessed by intracellular staining. These cytokines, especially IFNy, used to identify stimulated cells. Antigen-specific CD4[+] T cell responses showed increased cytokine production relative to control. Examples of antigen-specific CD4[+] T cell responses generated against neopeptides are shown in Figures 7-9.

T cells were co-cultured with monocyte-derived dendritic cells loaded β2M frameshift neopeptides for 20 days (restimulation with fresh monocyte-derived dendritic cells on day 20). CD4[+] T cells were analyzed for antigen-specificity by intracellular cytokine staining after restimulation with monocyte-derived dendritic cells loaded with β2M frameshift peptide for 24 hours, compared to controls without peptide (Figure 7).

Naive T cells were co-cultured with monocyte-derived dendritic cells loaded BTK C481S neopeptide for 20 days (restimulation with fresh monocyte-derived dendritic cells on day 20). CD4[+] T cells were analyzed for antigen-specificity by intracellular cytokine staining after restimulation with monocyte-derived dendritic cells loaded with BTK C481S neopeptide for 24 hours, compared to controls wild-type BTK peptide (Figure 8).

Naive T cells were co-cultured with monocyte-derived dendritic cells loaded GATA3 frameshift neopeptides for 20 days (restimulation with fresh monocyte-derived dendritic cells on day 20). CD4[+] T cells were analyzed for antigen-specificity by intracellular cytokine staining after restimulation with monocyte-derived dendritic cells loaded with GATA3 frameshift peptide for 24 hours, compared to controls without peptide (Figure 9).

## Example 5: Selection of CTL and HTL epitopes for inclusion in an tumor-specific vaccine.

This example illustrates the procedure for the selection of peptide epitopes for vaccine compositions of the disclosure. The peptides in the composition can be in the form of a nucleic acid sequence, either single or one or more sequences (i.e., minigene) that encodes peptide(s), or may be single and/or polyepitopic peptides.

Epitopes are selected which, upon administration, mimic immune responses that have been observed to be correlated with tumor clearance. For example, vaccine can include 1-2 epitopes that come from at least one tumor antigen region. Epitopes from one region can be used in combination with epitopes from one or more additional tumor antigen regions.

Epitopes can be selected, for example, that have a binding affinity of an $IC_{50}$ of 500 nM or less for an HLA class I molecule, or for class II, an $IC_{50}$ of 1000 nM or less.

When creating a polyepitopic compositions, e.g. a minigene, it is typically desirable to generate the smallest peptide possible that encompasses the epitopes of interest. The principles employed are similar, if not the same, as those employed when selecting a peptide comprising nested epitopes. Additionally, however, upon determination of the nucleic acid sequence to be provided as a minigene, the peptide sequence encoded thereby is analyzed to determine whether any "junctional epitopes" have been created. A junctional epitope is a potential HLA binding epitope, e.g., as predicted by motif analysis. Junctional epitopes are generally to be avoided because the recipient may bind to an HLA molecule and generate an immune response to that epitope, which is not present in a native protein sequence.

Peptide epitopes for inclusion in vaccine compositions are, for example, selected from those listed in the Tables. A vaccine composition comprised of selected peptides, when administered, is safe, efficacious, and elicits an immune response similar in magnitude of an immune response that inhibits tumor growth.

## Example 6: Peptide Composition for Prophylactic or Therapeutic Uses

Immunogenic or vaccine compositions of the invention are used to inhibit tumor growth. For example, a polyepitopic composition (or a nucleic acid comprising the same) containing multiple CTL and HTL epitopes is administered to individuals having tumors. The dose of peptide for the immunization is from about 1 to about 50,000 μg, generally 100-5,000 μg, for a 70 kg patient. The initial administration may be followed by booster dosages at 4 weeks followed by evaluation of the magnitude of the immune response in the patient, by techniques that determine the presence of epitope-specific CTL populations in a PBMC sample. Additional booster doses are administered as required. The composition is found to be both safe and efficacious to inhibit tumor growth.

Alternatively, the polyepitopic composition can be administered as a nucleic acid, for example as RNA, in accordance with methodologies known in the art and disclosed herein.

Neoantigen binding agents, such as TCR or CARs can be can be administered in accordance with methodologies known in the art and disclosed herein. The binding agents can be administered as polynucleotides, for example DNA or RNA, encoding the binding agents as part of cellular therapy. Alternatively, the binding agents can be prepared as antibodies or fragments thereof capable of recognizing the specific peptide:MHC complex coupled to cytotoxic agents or T cell binding agents capable of re-directing patient T cells to tumor cells expressing the epitopes listed in the Tables.

Neoantigen peptides, polynucleotides, binding agents, or cells expressing these molecules can be delivered to the same patient via multiple methodologies known in the art, and can further be combined with other cancer therapies (e.g., chemotherapy, surgery, radiation, checkpoint inhibitors, etc.).

**Example 7: Administration of Compositions Using Dendritic Cells**

Vaccines comprising epitopes of the disclosure may be administered using dendritic cells. In this example, peptide-pulsed or nucleic acid-pulsed dendritic cells can be administered to a patient to stimulate a CTL response *in vivo.* In this method dendritic cells are isolated, expanded, and pulsed with a vaccine comprising peptide or encoding DNA or RNA CTL and HTL epitopes of the disclosure. The dendritic cells are infused back into the patient to elicit CTL and HTL responses *in vivo.* The induced CTL and HTL then destroy (CTL) or facilitate destruction (HTL) of the specific target tumor cells that bear the proteins from which the epitopes in the vaccine are derived.

Alternatively, *ex vivo* CTL or HTL responses to a particular tumor-associated antigen can be induced by incubating in tissue culture the patient's, or genetically compatible, CTL or HTL precursor cells together with a source of antigen-presenting cells, such as dendritic cells, and the appropriate immunogenic peptides or nucleic acids. After an appropriate incubation time (typically about 7-28 days), in which the precursor cells are activated and expanded into effector cells, the cells are infused back into the patient, where they will destroy (CTL) or facilitate destruction (HTL) of their specific target cells, i.e., tumor cells.

**Example 8: Identification of Neoepitopes**

Cancer cells containing distinct genetic changes that alter amino acid sequence could generate potential novel T cell epitopes, such as those shown in the Tables. Identifying which patient's tumors contain tumor-specific neoepitopes as identified in the Tables can comprise identification of DNA mutations. As one approach, whole genome or whole exome sequencing using well-established techniques of tumor versus matched germline samples from patients can be carried out. As an additional approach, RNA sequencing of tumor and appropriately matched normal samples may also be conducted. As an additional approach, specific assays based on targeted sequencing, either next generation sequencing techniques or well-established Sanger sequencing can also be conducted. Additionally, highly specific Polymerase-chain reaction based assays may be developed.

**Example 9: Specificity of neoantigen peptide recognition by CD8⁺ or CD4⁺ cells**

In various cases, the present teachings include disclosure of discrimination between mutated and wild-type sequences by vaccine-induced CD8⁺ cells. To determine whether vaccine-induced T cells could recognize naturally processed antigen, a tumor cell line can be transduced with a multi-mini-gene construct encoding mutated (MUT) or wild-type (WT) sequences of peptides incorporated into a vaccine. Each minigene can consists of 21 aa encoding either the MUT or WT sequences. Vaccine-induced cells, specific for a protein containing a particular mutation can be incubated with MUT or WT expressing cancer cells, supernatants can be collected after 24 h of incubation, and IFN-y produced by cells can be measured in supernatants by ELISA.

Neoantigen-specific cells recognition of mutated and wild type peptides can also be determined in a standard 4 h $^{51}$Cr-release assay.

**Claims**

**1.** A pharmaceutical composition comprising:

a recombinant or synthetic neoantigenic peptide or a polynucleotide encoding the recombinant neoantigenic peptide for use in treatment of cancer in a subject in need thereof;
wherein the neoantigenic peptide comprises a tumor-specific neoepitope,
wherein the tumor-specific neoepitope comprises at least 8 contiguous amino acids of an amino acid sequence represented by a formula:

$$A_xB_yC_z,$$

wherein:
each A is an amino acid corresponding to a native polypeptide encoded by a GATA3 gene;
$B_y$ is absent;
each C is an amino acid encoded by a frameshift of a sequence encoding the native polypeptide;
the at least 8 contiguous amino acids comprises at least one C;
x + y + z is at least 8; and
wherein $C_z$ is

(i) PGRPLQTHVLPEPHLALQPLQPHADHAHADAPAIQPVLWTTPPL QHGHRHGLEPCSMLTGPPARVPAVPFDLHFCRSSIMKPKRDGY MFLKAESKIMFATLQRSSLWCLCSNH; and

(a) the tumor-specific neoepitope is FLKAESKIMF (SEQ ID NO: 536) and the subject express an MHC molecule encoded by a B08:01 HLA allele,
(b) the tumor-specific neoepitope is IMKPKRDGYM (SEQ ID NO: 550) and the subject express an MHC molecule encoded by a B08:01 HLA allele,
(c) the tumor-specific neoepitope is LHFCRSSIM (SEQ ID NO: 556) and the subject express an MHC molecule encoded by a B08:01 HLA allele,
(d) the tumor-specific neoepitope is MFATLQRSSL (SEQ ID NO: 567) and the subject express an MHC molecule encoded by a B07:02 HLA allele or a B08:01 HLA allele,
(e) the tumor-specific neoepitope is MFLKAESKI (SEQ ID NO: 568) and the subject express an MHC molecule encoded by a A24:02 HLA allele,
(f) the tumor-specific neoepitope is YMFLKAESKI (SEQ ID NO: 598) and the subject express an MHC molecule encoded by a A24:02 HLA allele or a B08:01 HLA allele,
(g) the tumor-specific neoepitope is HVLPEPHLAL (SEQ ID NO: 1428),
(h) the tumor-specific neoepitope is RPLQTHVLPE (SEQ ID NO: 1429),
(i) the tumor-specific neoepitope is VLWTTPPLQH (SEQ ID NO: 1430) and optionally the subject expresses an MHC molecule encoded by a A03:01 HLA allele,
(j) the tumor-specific neoepitope is EPHLALQPL (SEQ ID NO: 529) and the subject express an MHC molecule encoded by a B07:02 or B08:01 HLA allele, or
(k) the tumor-specific neoepitope is VLPEPHLAL (SEQ ID NO: 594) and the subject express an MHC molecule encoded by a A02.01 HLA allele; or

wherein $C_z$ is
(ii) PRPRRCTRHPACPLDHTTPPAWSPPWVRALLDAHRAPSESPCSP FRLAFLQEQYHEA and

(a) the tumor-specific neoepitope sequence is APSESPCSPF (SEQ ID NO: 1431),
(b) the tumor-specific neoepitope sequence is CPLDHTTPPA (SEQ ID NO: 1432),
(c) the tumor-specific neoepitope sequence is FLQEQYHEA (SEQ ID NO: 1433),
(d) the tumor-specific neoepitope sequence is RLAFLQEQYH (SEQ ID NO: 1434),
(e) the tumor-specific neoepitope sequence is SPCSPFRLAF (SEQ ID NO: 1435), or
(f) the tumor-specific neoepitope sequence is SPPWVRALL (SEQ ID NO: 1436);
wherein the cancer is breast cancer.

2. The pharmaceutical composition for use according to claim 1, wherein $C_z$ is PRPRRCTRHPACPLDHTTPPAWSP PWVRALLDAHRAPSESPCSPFRLAFL QEQYHEA and

(a) the tumor-specific neoepitope is APSESPCSPF (SEQ ID NO: 1431),
(b) the tumor-specific neoepitope is CPLDHTTPPA (SEQ ID NO: 1432),
(c) the tumor-specific neoepitope is FLQEQYHEA (SEQ ID NO: 1433),
(d) the tumor-specific neoepitope is RLAFLQEQYH (SEQ ID NO: 1434),
(e) the tumor-specific neoepitope is SPCSPFRLAF (SEQ ID NO: 1435), or

(f) the tumor-specific neoepitope is SPPWVRALL (SEQ ID NO: 1436).

3. The pharmaceutical composition for use according to claim 1, wherein Cz is: PGRPLQTHVLPEPHLALQPLQPHADHAHADAPAIQPVLWTTPPLQHGHR HGLEPCSMLTGPPARVPAVPFDLHFCRSSIMKPKRDGYMFLKAESKIMFA TLQRSSLWCLCSNH and

(a) the tumor-specific neoepitope is FLKAESKIMF (SEQ ID NO: 536) and the subject express an MHC molecule encoded by a B08:01 HLA allele,

(b) the tumor-specific neoepitope is IMKPKRDGYM (SEQ ID NO: 550) and the subject express an MHC molecule encoded by a B08:01 HLA allele,

(c) the tumor-specific neoepitope is LHFCRSSIM (SEQ ID NO: 556) and the subject express an MHC molecule encoded by a B08:01 HLA allele,

(d) the tumor-specific neoepitope is MFATLQRSSL (SEQ ID NO: 567) and the subject express an MHC molecule encoded by a B07:02 HLA allele or a B08:01 HLA allele,

(e) the tumor-specific neoepitope is MFLKAESKI (SEQ ID NO: 568) and the subject express an MHC molecule encoded by a A24:02 HLA allele,

(f) the tumor-specific neoepitope is YMFLKAESKI (SEQ ID NO: 598) and the subject express an MHC molecule encoded by a A24:02 HLA allele or a B08:01 HLA allele,

(g) the tumor-specific neoepitope is HVLPEPHLAL (SEQ ID NO: 1428),

(h) the tumor-specific neoepitope is RPLQTHVLPE (SEQ ID NO: 1429),

(i) the tumor-specific neoepitope is VLWTTPPLQH (SEQ ID NO: 1430) and optionally the subject expresses an MHC molecule encoded by a A03:01 HLA allele,

(j) the tumor-specific neoepitope is EPHLALQPL (SEQ ID NO: 529) and the subject express an MHC molecule encoded by a B07:02 or B08:01 HLA allele, or

(k) the tumor-specific neoepitope is VLPEPHLAL (SEQ ID NO: 594) and the subject express an MHC molecule encoded by a A02.01 HLA allele.

4. The pharmaceutical composition for use according to any one of claims 1-3,
wherein the neoantigenic peptide is at least 9 and at most 500 amino acids in length.

5. The pharmaceutical composition for use according to any one of claims 1-4,
wherein the tumor-specific neoepitope binds to a MHC class I molecule with a binding affinity of 500 nM or less.

6. The pharmaceutical composition for use according to any one of claims 1-5,
wherein the pharmaceutical composition comprises at least two different neoantigenic peptides, wherein the gene encoding the tumor-specific neoepitope of each of the at least two different neoantigenic peptides is GATA3.

7. The pharmaceutical composition for use according to any one of claims 1 and 3-6, wherein the tumor-specific neoepitope is FLKAESKIMF (SEQ ID NO: 536) and the subject express an MHC molecule encoded by a B08:01 HLA allele.

8. The pharmaceutical composition for use according to any one of claims 1 and 3-6, wherein the tumor-specific neoepitope is IMKPKRDGYM (SEQ ID NO: 550) and the subject express an MHC molecule encoded by a B08:01 HLA allele.

9. The pharmaceutical composition for use according to any one of claims 1 and 3-6, wherein the tumor-specific neoepitope is LHFCRSSIM (SEQ ID NO: 556) and the subject express an MHC molecule encoded by a B08:01 HLA allele.

10. The pharmaceutical composition for use according to one of claims 1, and 3-6, wherein the tumor-specific neoepitope is MFATLQRSSL (SEQ ID NO: 567) and the subject express an MHC molecule encoded by a B07:02 HLA allele or a B08:01 HLA allele.

11. The pharmaceutical composition for use according to any one of claims 1 and 3-6, wherein the tumor-specific neoepitope is MFLKAESKI (SEQ ID NO: 568) and the subject express an MHC molecule encoded by a A24:02 HLA allele.

12. The pharmaceutical composition for use according to any one of claims 1 and 3-6, wherein the tumor-specific neoepitope is YMFLKAESKI (SEQ ID NO: 598) and the subject express an MHC molecule encoded by a A24:02 HLA allele or a B08:01 HLA allele.

13. The pharmaceutical composition for use according to any one of claims 1 and 3-6, wherein the tumor-specific neoepitope is VLWTTPPLQH (SEQ ID NO: 1430) and optionally the subject expresses an MHC molecule encoded by a A03:01 HLA allele.

**Patentansprüche**

1. Eine pharmazeutische Zusammensetzung, umfassend:

Ein rekombinantes oder synthetisches neoantigenes Peptid oder ein Polynukleotid,
das das rekombinante neoantigene Peptid für die Verwendung bei der Behandlung von Krebs bei einem Patienten, der dies benötigt, codiert;
wobei das neoantigene Peptid ein tumorspezifisches Neoepitop umfasst, wobei das tumorspezifische Neoepitop mindestens 8 benachbarte Aminosäuren einer Aminosäure-Sequenz umfasst, die von folgender Formel repräsentiert ist:

$$A_xB_yC_z,$$

wobei:

Jedes A eine Aminosäure ist, die einem nativen Polypeptid entspricht, das durch ein GATA3-Gen codiert ist;
$B_y$ nicht vorhanden ist;
jedes C eine Aminosäure ist, die durch einen Frameshift einer Sequenz codiert ist, die das native Polypeptid codiert;
die mindestens 8 benachbarten Aminosäuren mindestens ein C umfassen;
x + y + z mindestens 8 ist; und
wobei $C_z$

(i) PGRPLQTHVLPEPHLALQPLQPHADHAHADAPAIQPVLWTTPPLQHGHRHGL EPCSMLTGPPAR VPAVPFDLHFCRSSIMKPKRDGYMFLKAESKIMFATLQRSSL WCLCSNH ist; und

(a) das tumorspezifische Neoepitop FLKAESKIMF (SEQ ID NO: 536) ist und der Patient ein MHC-Molekül exprimiert, das durch ein B08:01 HLA-Allel codiert ist,
(b) das tumorspezifische Neoepitop IMKPKRDGYM (SEQ ID NO: 550) ist und der Patient ein MHC-Molekül exprimiert, das durch ein B08:01 HLA-Allel codiert ist,
(c) das tumorspezifische Neoepitop LHFCRSSIM (SEQ ID NO: 556) ist und der Patient ein MHC-Molekül exprimiert, das durch ein B08:01 HLA-Allel codiert ist,
(d) das tumorspezifische Neoepitop MFATLQRSSL (SEQ ID NO: 567) ist und der Patient ein MHC-Molekül exprimiert, das durch ein B07:02 HLA-Allel oder ein B08:01 HLA-Allel codiert ist,
(e) das tumorspezifische Neoepitop MFLKAESKI (SEQ ID NO: 568) ist und der Patient ein MHC-Molekül exprimiert, das durch ein A24:02 HLA-Allel codiert ist,
(f) das tumorspezifische Neoepitop YMFLKAESKI (SEQ ID NO: 598) ist und der Patient ein MHC-Molekül exprimiert, das durch ein A24:02 HLA-Allel oder ein B08:01 HLA-Allel codiert ist,
(g) das tumorspezifische Neoepitop HVLPEPHLAL (SEQ ID NO: 1428) ist,
(h) das tumorspezifische Neoepitop RPLQTHVLPE (SEQ ID NO: 1429) ist,
(i) das tumorspezifische Neoepitop VLWTTPPLQH (SEQ ID NO: 1430) ist und optional der Patient ein MHC-Molekül exprimiert, das durch ein A03:01 HLA-Allel codiert ist,
(j) das tumorspezifische Neoepitop EPHLALQPL (SEQ ID NO: 529) ist und der Patient ein MHC-Molekül exprimiert, das durch ein B07:02 oder B08:01 HLA-Allel codiert ist, oder
(k) das tumorspezifische Neoepitop VLPEPHLAL (SEQ ID NO: 594) ist und der Patient ein MHC-Molekül exprimiert, das durch ein A02.01 HLA-Allel codiert ist, oder

wobei $C_z$
(ii) PRPRRCTRHPACPLDHTTPPAWSPPWVRALLDAHRAPSESPCSPFRLAFLQE QYHEA ist und

(a) die tumorspezifische Neoepitop-Sequenz APSESPCSPF (SEQ ID NO: 1431) ist,

(b) die tumorspezifische Neoepitop-Sequenz CPLDHTTPPA (SEQ ID NO: 1432) ist,

(c) die tumorspezifische Neoepitop-Sequenz FLQEQYHEA (SEQ ID NO: 1433) ist,

(d) die tumorspezifische Neoepitop-Sequenz RLAFLQEQYH (SEQ ID NO: 1434) ist,

(e) die tumorspezifische Neoepitop-Sequenz SPCSPFRLAF (SEQ ID NO: 1435) ist, oder

(f) die tumorspezifische Neoepitop-Sequenz SPPWVRALL (SEQ ID NO: 1436) ist;

wobei der Krebs ein Brustkrebs ist.

**2.** Pharmazeutische Zusammensetzung für die Verwendung nach Anspruch 1, wobei $C_z$ PRPRRCTRHPACPLDHT TPPAVVSPPWVRALLDAHRAPSESPCSPFRLAFLQEQYHE A ist und

(a) das tumorspezifische Neoepitop APSESPCSPF (SEQ ID NO: 1431) ist,

(b) das tumorspezifische Neoepitop CPLDHTTPPA (SEQ ID NO: 1432) ist,

(c) das tumorspezifische Neoepitop FLQEQYHEA (SEQ ID NO: 1433) ist,

(d) das tumorspezifische Neoepitop RLAFLQEQYH (SEQ ID NO: 1434) ist,

(e) das tumorspezifische Neoepitop SPCSPFRLAF (SEQ ID NO: 1435) ist, oder

(f) das tumorspezifische Neoepitop SPPWVRALL (SEQ ID NO: 1436) ist.

**3.** Pharmazeutische Zusammensetzung für die Verwendung nach Anspruch 1, wobei $C_z$: PGRPLQTHVLPEPHLAL QPLQPHADHAHADAPAIQPVLWTTPPLQHGHRHGLEPCS MLTGPPARVPAVPFDLHFCRSSIMKPKRDGYMFLK AESKIMFATLQRSSLWCLCSNH ist; und

(a) das tumorspezifische Neoepitop FLKAESKIMF (SEQ ID NO: 536) ist und der Patient ein MHC-Molekül exprimiert, das durch ein B08:01 HLA-Allel codiert ist,

(b) das tumorspezifische Neoepitop IMKPKRDGYM (SEQ ID NO: 550) ist und der Patient ein MHC-Molekül exprimiert, das durch ein B08:01 HLA-Allel codiert ist,

(c) das tumorspezifische Neoepitop LHFCRSSIM (SEQ ID NO: 556) ist und der Patient ein MHC-Molekül exprimiert, das durch ein B08:01 HLA-Allel codiert ist,

(d) das tumorspezifische Neoepitop MFATLQRSSL (SEQ ID NO: 567) ist und der Patient ein MHC-Molekül exprimiert, das durch ein B07:02 HLA-Allel oder ein B08:01 HLA-Allel codiert ist,

(e) das tumorspezifische Neoepitop MFLKAESKI (SEQ ID NO: 568) ist und der Patient ein MHC-Molekül exprimiert, das durch ein A24:02 HLA-Allel codiert ist,

(f) das tumorspezifische Neoepitop YMFLKAESKI (SEQ ID NO: 598) ist und der Patient ein MHC-Molekül exprimiert, das durch ein A24:02 HLA-Allel oder ein B08:01 HLA-Allel codiert ist,

(g) das tumorspezifische Neoepitop HVLPEPHLAL (SEQ ID NO: 1428) ist,

(h) das tumorspezifische Neoepitop RPLQTHVLPE (SEQ ID NO: 1429) ist,

(i) das tumorspezifische Neoepitop VLWTTPPLQH (SEQ ID NO: 1430) ist und optional der Patient ein MHC-Molekül exprimiert, das durch ein A03:01 HLA-Allel codiert ist,

(j) das tumorspezifische Neoepitop EPHLALQPL (SEQ ID NO: 529) ist und der Patient ein MHC-Molekül exprimiert, das durch ein B07:02 oder B08:01 HLA-Allel codiert ist, oder

(k) das tumorspezifische Neoepitop VLPEPHLAL (SEQ ID NO: 594) ist und der Patient ein MHC-Molekül exprimiert, das durch ein A02.01 HLA-Allel codiert ist.

**4.** Pharmazeutische Zusammensetzung für die Verwendung nach einem der Ansprüche 1 bis 3, wobei das neoantigene Peptid mindestens 9 und höchstens 500 Aminosäuren lang ist.

**5.** Pharmazeutische Zusammensetzung für die Verwendung nach einem der Ansprüche 1 bis 4, wobei sich das tumorspezifische Neoepitop an ein MHC-Klasse-I-Moleküle mit einer Bindungsaffinität von 500 nM oder weniger bindet.

**6.** Pharmazeutische Zusammensetzung für die Verwendung nach einem der Ansprüche 1 bis 5, wobei die pharmazeutische Zusammensetzung mindestens zwei verschiedene neoantigene Peptide umfasst, wobei das Gen, das das tumorspezifische Neoepitop jedes der mindestens zwei verschiedenen neoantigenen Peptide codiert, GATA3 ist.

**7.** Pharmazeutische Zusammensetzung für die Verwendung nach einem der Ansprüche 1 und 3 bis 6, wobei das tumorspezifische Neoepitop FLKAESKIMF (SEQ ID NO: 536) ist und der Patient ein MHC-Molekül exprimiert, das durch ein B08:01 HLA-Allel codiert ist.

8. Pharmazeutische Zusammensetzung für die Verwendung nach einem der Ansprüche 1 und 3 bis 6, wobei das tumorspezifische Neoepitop IMKPKRDGYM (SEQ ID NO: 550) ist und der Patient ein MHC-Molekül exprimiert, das durch ein B08:01 HLA-Allel codiert ist.

9. Pharmazeutische Zusammensetzung für die Verwendung nach einem der Ansprüche 1 und 3 bis 6, wobei das tumorspezifische Neoepitop LHFCRSSIM (SEQ ID NO: 556) ist und der Patient ein MHC-Molekül exprimiert, das durch ein B08:01 HLA-Allel codiert ist.

10. Pharmazeutische Zusammensetzung für die Verwendung nach einem der Ansprüche 1 und 3 bis 6, wobei das tumorspezifische Neoepitop MFATLQRSSL (SEQ ID NO: 567) ist und der Patient ein MHC-Molekül exprimiert, das durch ein B07:02 HLA-Allel oder ein B08:01 HLA-Allel codiert ist.

11. Pharmazeutische Zusammensetzung für die Verwendung nach einem der Ansprüche 1 und 3 bis 6, wobei das tumorspezifische Neoepitop MFLKAESKI (SEQ ID NO: 568) ist und der Patient ein MHC-Molekül exprimiert, das durch ein A24:02 HLA-Allel codiert ist.

12. Pharmazeutische Zusammensetzung für die Verwendung nach einem der Ansprüche 1 und 3 bis 6, wobei das tumorspezifische Neoepitop YMFLKAESKI (SEQ ID NO: 598) ist und der Patient ein MHC-Molekül exprimiert, das durch ein A24:02 HLA-Allel oder ein B08:01 HLA-Allel codiert ist.

13. Pharmazeutische Zusammensetzung für die Verwendung nach einem der Ansprüche 1 und 3 bis 6, wobei das tumorspezifische Neoepitop VLWTTPPLQH (SEQ ID NO: 1430) ist und optional der Patient ein MHC-Molekül exprimiert, das durch ein A03:01 HLA-Allel codiert ist.

**Revendications**

1. Composition pharmaceutique comprenant :

   un peptide néoantigénique recombinant ou synthétique ou un polynucléotide codant pour le peptide néoantigénique recombinant destiné à être utilisé dans le traitement du cancer chez un sujet qui en a besoin ;
   dans laquelle le peptide néoantigénique comprend un néoépitope spécifique à la tumeur,
   dans lequel le néoépitope spécifique à la tumeur comprend au moins 8 acides aminés contigus d'une séquence d'acides aminés représentée par une formule :

   $$A_xB_yC_z,$$

   dans laquelle :

   chaque A est un acide aminé correspondant à un polypeptide natif codé par un gène GATA3 ;
   $B_y$ est absent ;
   chaque C est un acide aminé codé par un décalage de trame d'une séquence codant pour le polypeptide natif ;
   lesdits au moins 8 acides aminés contigus comprennent au moins un C ;
   $x + y + z$ vaut au moins 8 ; et
   dans laquelle $C_z$ est

   (i) PGRPLQTHVLPEPHLALQPLQPHADHAHADAPAIQPVLWTTPPLQHGHRHGLE PCSMLTGPPA RVPAVPFDLHFCRSSIMKPKRDGYMFLKAESKIMFATLQRSSLWCL CSNH ; et

   (a) le néoépitope spécifique à la tumeur est FLKAESKIMF (SEQ ID n° : 536) et le sujet exprime une molécule du CMH codée par un allèle B08:01 HLA,
   (b) le néoépitope spécifique à la tumeur est IMKPKRDGYM (SEQ ID n° : 550) et le sujet exprime une molécule du CMH codée par un allèle B08:01 HLA,
   (c) le néoépitope spécifique à la tumeur est LxHFCRSSIM (SEQ ID n° : 556) et le sujet exprime une molécule du CMH codée par un allèle B08:01 HLA,
   (d) le néoépitope spécifique à la tumeur est MFATLQRSSL (SEQ ID n° : 567) et le sujet exprime une molécule du CMH codée par un allèle B07:02 HLA ou un allèle B08:01 HLA,

(e) le néoépitope spécifique à la tumeur est MFLKAESKI (SEQ ID n° : 568) et le sujet exprime une molécule du CMH codée par un allèle A24:02 HLA,

(f) le néoépitope spécifique à la tumeur est YMFLKAESKI (SEQ ID n° : 598) et le sujet exprime une molécule du CMH codée par un allèle A24:02 HLA ou un allèle B08:01 HLA,

(g) le néoépitope spécifique à la tumeur est HVLPEPHLAL (SEQ ID n° : 1428),

(h) le néoépitope spécifique à la tumeur est RPLQTHVLPE (SEQ ID n° : 1429),

(i) le néoépitope spécifique à la tumeur est LHFCRSSIM (SEQ ID n° : 1430) et éventuellement, le sujet exprime une molécule du CMH codée par un allèle A03:01 HLA,

(j) le néoépitope spécifique à la tumeur est EPHLALQPL (SEQ ID n° : 529) et le sujet exprime une molécule du CMH codée par un allèle B07:02 ou B08:01 HLA, ou

(k) le néoépitope spécifique à la tumeur est VLPEPHLAL (SEQ ID n° : 594) et le sujet exprime une molécule du CMH codée par un allèle A02.01 HLA ; ou

dans laquelle $C_z$ est

(ii) PRPRRCTRHPACPLDHTTPPAWSPPWVRALLDAHRAPSESPCSPFRLAFLQEQ YHEA et

(a) la séquence néoépitope spécifique à la tumeur est APSESPCSPF (SEQ ID n° : 1431),

(b) la séquence néoépitope spécifique à la tumeur est CPLDHTTPPA (SEQ ID n° : 1432),

(c) la séquence néoépitope spécifique à la tumeur est FLQEQYHEA (SEQ ID n° : 1433),

(d) la séquence néoépitope spécifique à la tumeur est RLAFLQEQYH (SEQ ID n° : 1434),

(e) la séquence néoépitope spécifique à la tumeur est SPCSPFRLAF (SEQ ID n° : 1435), ou

(f) la séquence néoépitope spécifique à la tumeur est SPPWVRALL (SEQ ID n° : 1436) ;

dans laquelle le cancer est le cancer du sein.

2. Composition pharmaceutique destinée à être utilisée selon la revendication 1, dans laquelle $C_z$ est PRPRRCTR HPACPLDHTTPPAWSPPWVRALLDAHRAPSESPCSPFRLAFLQEQYHEA et

(a) le néoépitope spécifique à la tumeur est APSESPCSPF (SEQ ID n° : 1431),

(b) le néoépitope spécifique à la tumeur est CPLDHTTPPA (SEQ ID n° : 1432),

(c) le néoépitope spécifique à la tumeur est FLQEQYHEA (SEQ ID n° : 1433),

(d) le néoépitope spécifique à la tumeur est RLAFLQEQYH (SEQ ID n° : 1434),

(e) le néoépitope spécifique à la tumeur est SPCSPFRLAF (SEQ ID n° : 1435), ou

(f) le néoépitope spécifique à la tumeur est SPPWVRALL (SEQ ID n° : 1436).

3. Composition pharmaceutique destinée à être utilisée selon la revendication 1, dans laquelle $C_z$ est : PGRPLQTH VLPEPHLALQPLQPHADHAHADAPAIQPVLWTTPPLQHGHRHGLEPCSML TGPPARVPAVPFDLHFCRSSIMKP KRDGYMFLKAESKIMFATLQRSSLWCLCSNH et

(a) le néoépitope spécifique à la tumeur est FLKAESKIMF (SEQ ID n° : 536) et le sujet exprime une molécule du CMH codée par un allèle B08:01 HLA,

(b) le néoépitope spécifique à la tumeur est IMKPKRDGYM (SEQ ID n° : 550) et le sujet exprime une molécule du CMH codée par un allèle B08:01 HLA,

(c) le néoépitope spécifique à la tumeur est LxHFCRSSIM (SEQ ID n° : 556) et le sujet exprime une molécule du CMH codée par un allèle B08:01 HLA,

(d) le néoépitope spécifique à la tumeur est MFATLQRSSL (SEQ ID n° : 567) et le sujet exprime une molécule du CMH codée par un allèle B07:02 HLA ou un allèle B08:01 HLA,

(e) le néoépitope spécifique à la tumeur est MFLKAESKI (SEQ ID n° : 568) et le sujet exprime une molécule du CMH codée par un allèle A24:02 HLA,

(f) le néoépitope spécifique à la tumeur est YMFLKAESKI (SEQ ID n° : 598) et le sujet exprime une molécule du CMH codée par un allèle A24:02 HLA ou un allèle B08:01 HLA,

(g) le néoépitope spécifique à la tumeur est HVLPEPHLAL (SEQ ID n° : 1428),

(h) le néoépitope spécifique à la tumeur est RPLQTHVLPE (SEQ ID n° : 1429),

(i) le néoépitope spécifique à la tumeur est LHFCRSSIM (SEQ ID n° : 1430) et éventuellement, le sujet exprime une molécule du CMH codée par un allèle A03:01 HLA,

(j) le néoépitope spécifique à la tumeur est EPHLALQPL (SEQ ID n° : 529) et le sujet exprime une molécule du CMH codée par un allèle B07:02 ou B08:01 HLA, ou

(k) le néoépitope spécifique à la tumeur est VLPEPHLAL (SEQ ID n° : 594) et le sujet exprime une molécule du CMH codée par un allèle A02.01 HLA.

4. Composition pharmaceutique destinée à être utilisée selon l'une quelconque parmi les revendications 1 à 3, dans laquelle le peptide néoantigénique a une longueur d'au moins 9 et d'au plus 500 acides aminés.

5. Composition pharmaceutique destinée à être utilisée selon l'une quelconque parmi les revendications 1 à 4, dans laquelle le néoépitope spécifique à la tumeur se lie à une molécule du CMH de classe 1 avec une affinité de liaison de 500 nm ou moins.

6. Composition pharmaceutique destinée à être utilisée selon l'une quelconque parmi les revendications 1 à 5, dans laquelle la composition pharmaceutique comprend au moins deux peptides néoantigéniques différents, dans laquelle le gène codant pour le néoépitope spécifique à la tumeur de chacun desdits au moins deux peptides néoantigéniques différents est GATA3.

7. Composition pharmaceutique destinée à être utilisée selon l'une quelconque parmi les revendications 1 et 3 à 6, dans laquelle le néoépitope spécifique à la tumeur est FLKAESKIMF (SEQ ID n° : 536) et le sujet exprime une molécule du CMH codée par un allèle B08:01 HLA.

8. Composition pharmaceutique destinée à être utilisée selon l'une quelconque parmi les revendications 1 et 3 à 6, dans laquelle le néoépitope spécifique à la tumeur est IMKPKRDGYM (SEQ ID n° : 550) et le sujet exprime une molécule du CMH codée par un allèle B08:01 HLA.

9. Composition pharmaceutique destinée à être utilisée selon l'une quelconque parmi les revendications 1 et 3 à 6, dans laquelle le néoépitope spécifique à la tumeur est LHFCRSSIM (SEQ ID n° : 556) et le sujet exprime une molécule du CMH codée par un allèle B08:01 HLA.

10. Composition pharmaceutique destinée à être utilisée selon l'une des revendications 1 et 3 à 6, dans laquelle le néoépitope spécifique à la tumeur est MFATLQRSSL (SEQ ID n° : 567) et le sujet exprime une molécule du CMH codée par un allèle B07:02 HLA ou un allèle B08:01 HLA.

11. Composition pharmaceutique destinée à être utilisée selon l'une quelconque parmi les revendications 1 et 3 à 6, dans laquelle le néoépitope spécifique à la tumeur est MFLKAESKI (SEQ ID n° : 568) et le sujet exprime une molécule du CMH codée par un allèle A24:02 HLA.

12. Composition pharmaceutique destinée à être utilisée selon l'une quelconque parmi les revendications 1 et 3 à 6, dans laquelle le néoépitope spécifique à la tumeur est YMFLKAESKI (SEQ ID n° : 598) et le sujet exprime une molécule du CMH codée par un allèle A24:02 HLA ou un allèle B08:01 HLA.

13. Composition pharmaceutique destinée à être utilisée selon l'une quelconque parmi les revendications 1 et 3 à 6, dans laquelle le néoépitope spécifique à la tumeur est VLWTTPPLQH (SEQ ID n° : 1430) et éventuellement, le sujet exprime une molécule du CMH codée par un allèle A03:01 HLA.

FIG. 1

**FIG. 2**

Initial Stain

Validation

FIG. 3A

FIG. 3B

EP 3 436 048 B1

FIG. 4B

Validation

BV421

PE

FIG. 4A

Initial Stain

BUV396

APC

FIG. 5A

FIG. 5B

Initial Stain

Validation

FIG. 6A

FIG. 6B

EP 3 436 048 B1

FIG. 7

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62316530
- US 62316533
- US 62316547
- US 62316552
- US 62316567
- US 62316571
- WO 2016187508 A
- WO 2016164833 A
- WO 2016172722 A
- WO 9420127 A
- WO 9403205 A
- WO 2012159643 A
- EP 2006009448 W
- US 4722848 A
- US 5580859 A
- US 5589466 A
- WO 2011051489 A
- US 20070134197 A
- US 20060258607 A
- US 4588585 A
- WO 2012159754 A
- US 6955808 B
- US 6943019 B
- US 20080254008 A
- US 9405700 W
- US 7029848 B
- US 6974695 B
- US 6913922 B
- US 6869794 B
- US 6780407 B
- US 6537594 B
- US 6309647 B
- US 6265189 B
- US 6156567 A
- US 6090393 A
- US 5942235 A
- US 5833975 A
- US 6277558 B
- US 5658785 A
- US 7115391 B
- US 7172893 B
- US 6953690 B
- US 6936466 B
- US 6924128 B
- US 6893865 B
- US 6793926 B
- US 6537540 B
- US 6475769 B
- US 6258595 B
- US 6991797 B
- US 7255862 B
- US 8309098 B
- US 5185146 A
- US 4235871 A
- US 4501728 A
- US 4837028 A
- US 5019369 A
- US 5204253 A
- WO 9618372 A
- WO 9324640 A
- US 5279833 A
- WO 9106309 A
- WO 2015095811 A
- US 5849589 A
- US 6406705 B1
- US 20130123169
- WO 9507707 A

**Non-patent literature cited in the description**

- **FENG DU et al.** *Medicinal Research Reviews*, 2015, vol. 35 (6), 1300-1315
- **FRITSCH et al.** *Cancer Immunology Research*, 2014, vol. 2 (6), 522-529
- **SIDNEY et al.** *Current Protocols in Immunology*, 1998, 18.3.1
- **SIDNEY et al.** *J. Immunol.*, 1995, vol. 154, 247
- **SETTE et al.** *Mol. Immunol.*, 1994, vol. 31, 813
- **CEPPELLINI et al.** *Nature*, 1989, vol. 339, 392
- **CHRISTNICK et al.** *Nature*, 1991, vol. 352, 67
- **BUSCH et al.** *Int. Immunol.*, 1990, vol. 2, 443
- **HILL et al.** *J. Immunol.*, 1991, vol. 147, 189
- **DEL GUERCIO et al.** *J. Immunol.*, 1995, vol. 154, 685
- **CERUNDOLO et al.** *J. Immunol.*, 1991, vol. 21, 2069
- **HILL et al.** *J. Immunol.*, 1994, vol. 152, 2890

- **MARSHALL et al.** *J. Immunol.*, 1994, vol. 152, 4946
- **REAY et al.** *EMBO J.*, 1992, vol. 11, 2829
- **KHILKO et al.** *J. Biol. Chem.*, 1993, vol. 268, 15425
- **HAMMER et al.** *J. Exp. Med.*, 1994, vol. 180, 2353
- **LJUNGGREN et al.** *Nature*, 1990, vol. 346, 476
- **SCHUMACHER et al.** *Cell*, 1990, vol. 62, 563
- **TOWNSEND et al.** *Cell*, 1990, vol. 62, 285
- **PARKER et al.** *J. Immunol.*, 1992, vol. 149, 1896
- **STITES et al.** IMMUNOLOGY. Lange Publishing, 1994
- **PAUL**. FUNDAMENTAL IMMUNOLOGY. Raven Press, 1993
- **VERHOEF et al.** *Eur. J. Drug Metab. Pharmacokinetics*, 1986, vol. 11, 291
- **STEWART ; YOUNG**. SOLID PHASE PEPTIDE SYNTHESIS. Pierce Chemical Co., 1984
- **SAMBROOK et al.** MOLECULAR CLONING, A LABORATORY MANUAL. Cold Spring Harbor Press, 1989
- **BRITO et al.** *Adv. Genet.*, 2015, vol. 89, 179-233
- **MATTEUCCI et al.** *J. Am. Chem. Soc.*, 1981, vol. 103, 3185
- **GLUZMAN**. *Cell*, 1981, vol. 23, 175
- **STOVER et al.** *Nature*, 1991, vol. 351, 456-460
- Remington's Pharmaceutical Sciences. Mack Publishing Co., 2000
- **MERRIFIELD**. *Science*, 1986, vol. 232, 341-347
- **BARANY ; MERRIFIELD**. The Peptides. Academic Press, 1979, 1-284
- **STEWART ; YOUNG**. Solid Phase Peptide Synthesis. 1984
- **MERRIFIELD RB**. Solid phase peptide synthesis. I. The synthesis of a tetrapeptide. *J. Am. Chem. Soc.*, 1963, vol. 85, 2149-54
- **SAMBROOK et al.** Molecular Cloning, A Laboratory Manual. Cold Spring Harbor Laboratory, 1989
- **ZOELLER et al.** *Proc. Nat'l. Acad. Sci. USA*, 1984, vol. 81, 5662-5066
- **POUWELS et al.** Cloning Vectors: A Laboratory Manual. Elsevier, 1985
- **LUCKOW ; SUMMERS**. *Bio/Technology*, 1988, vol. 6, 47
- **MOR et al.** *The Journal of Immunology*, 1995, vol. 155 (4), 2039-2046
- **LEITNER et al.** *The Journal of Immunology*, 1997, vol. 159 (12), 6112-6119
- **ALARCON et al.** *Adv. Parasitol. Advances in Parasitology*, 1999, vol. 42, 343-410
- **ROBINSON et al.** *Adv. Virus Res. Advances in Virus Research*, 2000, vol. 55, 1-74
- **BÖHMET**. *Journal of Immunological Methods*, 1996, vol. 193 (1), 29-40
- **LEWIS et al.** Advances in Virus Research. Academic Press, 1999, vol. 54, 129-88
- **WEINER et al.** *Scientific American*, 1999, vol. 281 (1), 34-41
- **SHAREI et al.** Ex Vivo Cytosolic Delivery of Functional Macromolecules to Immune Cells. *PLOS ONE*, 13 April 2015
- **BUCHSCHER et al.** *J. Virol.*, 1992, vol. 66, 2731-2739
- **JOHANN et al.** *J. Virol.*, 1992, vol. 66, 1635-1640
- **SOMMNERFELT et al.** *Virol.*, 1990, vol. 176, 58-59
- **WILSON et al.** *J. Virol.*, 1998, vol. 63, 2374-2378
- **MILLER et al.** *J. Virol.*, 1991, vol. 65, 2220-2224
- **CHROBOCZEK, J. ; BIEBER, F. ; JACROT, B.** The Sequence of the Genome of Adenovirus Type 5 and Its Comparison with the Genome of Adenovirus Type 2. *Virology*, 1992, vol. 186, 280-285
- **VERARDI et al.** *Hum Vaccin Immunother.*, July 2012, vol. 8 (7), 961-70
- **MOSS**. *Vaccine.*, 2013, vol. 31 (39), 4220-4222
- **ROLPH et al.** Recombinant viruses as vaccines and immunological tools. *Curr Opin Immunol*, 1997, vol. 9, 517-524
- **HORIG H ; LEE DS ; CONKRIGHT W et al.** Phase I clinical trial of a recombinant canarypoxvirus (ALVAC) vaccine expressing human carcinoembryonic antigen and the B7.1 co-stimulatory molecule. *Cancer Immunol Immunother*, 2000, vol. 49, 504-14
- **VON MEHREN M ; ARLEN P ; TSANG KY et al.** Pilot study of a dual gene recombinant avipox vaccine containing both carcinoembryonic antigen (CEA) and B7.1 transgenes in patients with recurrent CEA-expressing adenocarcinomas. *Clin Cancer Res*, 2000, vol. 6, 2219-28
- **MUSEY L ; DING Y ; ELIZAGA M et al.** HIV-1 vaccination administered intramuscularly can induce both systemic and mucosal T cell immunity in HIV-1-uninfected individuals. *J Immunol*, 2003, vol. 171, 1094-101
- **PAOLETTI E.** Applications of pox virus vectors to vaccination: an update. *Proc Natl Acad Sci U S A*, 1996, vol. 93, 11349-53
- **MARSHALL JL ; HAWKINS MJ ; TSANG KY et al.** Phase I study in cancer patients of a replication-defective avipox recombinant vaccine that expresses human carcinoembryonic antigen. *J Clin Oncol*, 1999, vol. 17, 332-7
- **MAYR, A. et al.** *Infection*, 1975, vol. 3, 6-14

- **MEYER, H. et al.** *J. Gen. Virol.*, 1991, vol. 72, 1031-1038
- **MANDL et al.** *Cancer Immunol Immunother.*, January 2012, vol. 61 (1), 19-29
- **SAMBROOK et al.** Molecular Cloning: A Laboratory Manual. Cold Spring Harbor Laboratory Press, 2001
- **SIOUD et al.** *FASEB J*, 2013, vol. 27, 3272-3283
- **MURPHY et al.** *The Prostate*, 1996, vol. 29, 371-380
- **TJUA et al.** *The Prostate*, 1997, vol. 32, 272-278
- **SZOKA et al.** *Ann. Rev. Biophys. Bioeng.*, 1980, vol. 9, 467
- **WOLFF et al.** *Science*, 1990, vol. 247, 1465-1468
- **GEALL et al.** *Proc Natl Acad Sci U S A.*, 2012, vol. 109, 14604-14609
- **MANNINO ; GOULD-FOGERITE.** *BioTechniques*, 1988, vol. 6 (7), 682-691
- **FELGNER et al.** *Proc. Natl. Acad. Sci. USA*, 1987, vol. 84, 7413-7414
- **DUPUIS M et al.** *Cell Immunol.*, 1998, vol. 186 (1), 18-27
- **ALLISON A C**. *Dev Biol Stand.*, 1998, vol. 92, 3-11
- **MOSCA et al.** *Frontiers in Bioscience*, 2007, vol. 12, 4050-4060
- **GAMVRELLIS et al.** *Immunol & Cell Biol.*, 2004, vol. 82, 506-516
- **GABRILOVICH D I et al.** *J Immunother Emphasis Tumor Immunol.*, 1996 (6), 414-418
- **ARTHUR M. KRIEG**. Nature Reviews. *Drug Discovery*, 05 June 2006, 471-484
- **KOIDE et al.** *Methods Enzymol.*, 2012, vol. 503, 135-56
- Remington's Pharmaceutical Sciences. 1980
- Remington' pharmaceutical Sciences. Mack Publishing Co., 1990
- Dermatological Formulations: Percutaneous absorption. Marcel Dekker Incl, 1983
- **DERES et al.** *Nature*, 1989, vol. 342, 561
- **AN, L.** ; **WHITTON, J. L.** *J. Virol.*, 1997, vol. 71, 2292
- **THOMSON, S. A. et al.** *J. Immunol.*, 1996, vol. 157, 822
- **WHITTON, J. L. et al.** *J. Virol.*, 1993, vol. 67, 348

- **HANKE, R. et al.** *Vaccine*, 1998, vol. 16, 426
- **MANNINO ; GOULD-FOGERITE.** *BioTechniques*, 1988, vol. 6 (7), 682
- **FELGNER et al.** *Proc. Nat'l Acad. Sci. USA*, 1987, vol. 84, 7413
- **SADELAIN, M. et al.** *Nat Rev Cancer*, 2003, vol. 3, 35-45
- **MORGAN, R. A. et al.** *Science*, 2006, vol. 314, 126-129
- **PANELLI, M. C. et al.** *J Immunol*, 2000, vol. 164, 495-504
- **PANELLI, M. C. et al.** *J Immunol*, 2000, vol. 164, 4382-4392
- **DUPONT, J. et al.** *Cancer Res*, 2005, vol. 65, 5417-5427
- **PAPANICOLAOU, G. A. et al.** *Blood*, 2003, vol. 102, 2498-2505
- The Chemotherapy Source Book. Lippincott, Williams & Wilkins, 2008
- Remington: The Science and Practice of Pharmacy. Pharmaceutical Press, 2012
- **VITIELLO, A. et al.** *J. Clin. Invest.*, 1995, vol. 95, 341
- **ELDRIDGE et al.** *Molec. Immunol.*, 1991, vol. 28, 287-294
- **ALONSO et al.** *Vaccine*, 1994, vol. 12, 299-306
- **JONES et al.** *Vaccine*, 1995, vol. 13, 675-681
- **TAKAHASHI et al.** *Nature*, 1990, vol. 344, 873-875
- **HU et al.** *Clin Exp Immunol.*, 1998, vol. 113, 235-243
- **TAM, J. P.** *Proc Natl Acad. Sci. U.S.A.*, 1988, vol. 85, 5409-5413
- **TARN, J.P.** *J. Immunol. Methods*, 1996, vol. 196, 17-32
- **ANSEL et al.** Pharmaceutical Dosage Forms and Drug Delivery Systems. Lippencott Williams & Wilkins, 1999
- **SIDNEY et al.** Current Protocols in Immunology. John Wiley & Sons, 1998
- **SOUTHWOOD et al.** *J. Immunol.*, 1998, vol. 160, 3363-3373
- **HARNDAHL et al.** *J Immunol Methods*, 2011, vol. 374, 5-12
- **SALTER** ; **CRESSWELL**. *EMBO J.*, 1986, vol. 5, 943-49
- **SALTER**. *Immunogenetics*, 1985, vol. 21, 235-46